# EUROPEAN PATENT APPLICATION

(11) **EP 4 471 140 A1**
(43) Date of publication of application: **04.12.2024**
(21) Application number: 23746448.2
(22) Date of filing: 29.01.2023
(51) Int. Cl.: C12N 15/11, A61K 47/54, A61K 31/7088, A61P 1/16

(54) **TARGETING LIGAND CONTAINING N-ACETYLGALACTOSAMINE**

(30) Priority: 30.01.2022 CN 202210114045; 01.04.2022 CN 202210347977; 11.01.2023 CN 202310064105
(71) Applicant: Rona Bioscience, Limited, Admiralty, Hong Kong (HK)
(72) Inventor: HUANG, Jinyu, Shanghai 201315 (CN); GUO, Hongli, Shanghai 201315 (CN)
(74) Representative: Cabinet Becker et Associés
(86) International application number: PCT/CN2023/073708
(87) International publication number: WO 2023/143571

(57) **Abstract**

Provided in the present invention is a ligand containing N-acetylgalactosamine, wherein the ligand contains a conjugated group as represented by formula (X') and a nucleic acid molecule containing the targeting ligand.

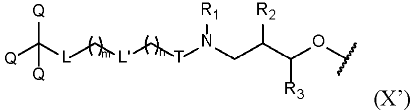

## Description

### FIELD OF TECHNOLOGY

The present invention belongs to the field of medicine, and specifically relates to a targeting ligand containing N-acetylgalactosamine and a nucleic acid molecule containing the targeting ligand.

### BACKGROUND

N-acetylgalactosamine (GalNAc) can specifically bind to an asialoglycoprotein receptor (ASGPR) on surfaces of hepatocytes. Coupling of a targeting ligand containing GalNAc to a small interfering RNA (siRNA) can achieve liver targeted delivery of the siRNA.

Targeting ligands containing GalNAc are disclosed in the prior art, but the field has needs for targeting ligands with better stability, targetability and the like.

### SUMMARY

The present invention provides a novel targeting ligand containing N-acetylgalactosamine.

In a first aspect, the present invention relates to a ligand containing N-acetylgalactosamine, where the ligand includes a conjugated group shown in Formula (X'): 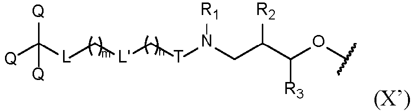 where, 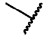
represents a position connected with a biomolecule
Q is independently H, 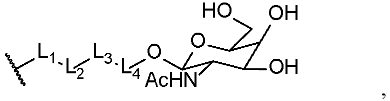 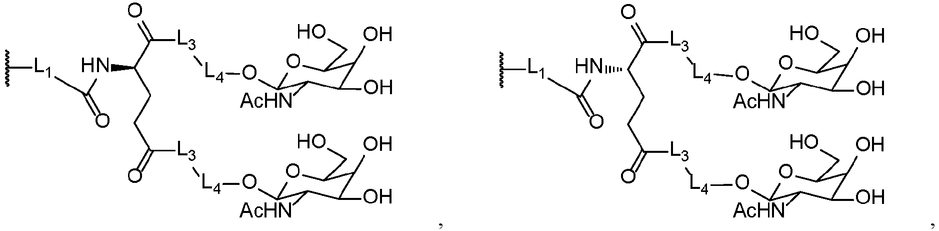 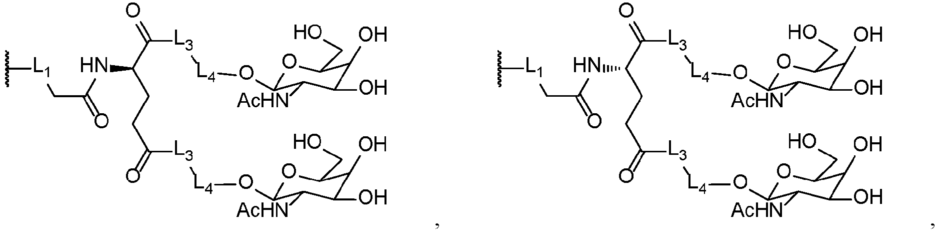 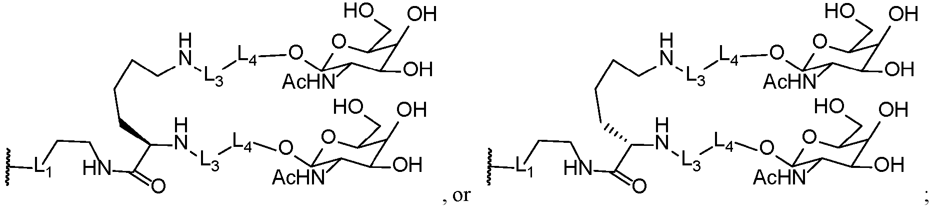
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
where e is 1, 2, 3, 4, or 5;
T is bond, -CH₂-, -C(O)-, -M-, -CH₂-M-, or -C(O)-M-;
where M is 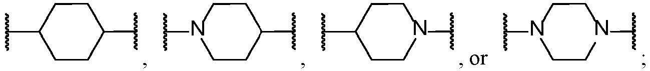
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In another aspect, the present invention relates to a ligand containing N-acetylgalactosamine, where the conjugated group is shown in Formula (I'): 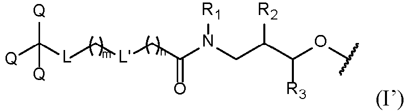 where, 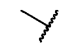
represents a position connected with a biomolecule;
Q is independently H, 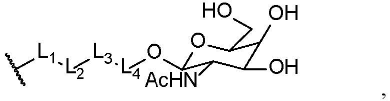 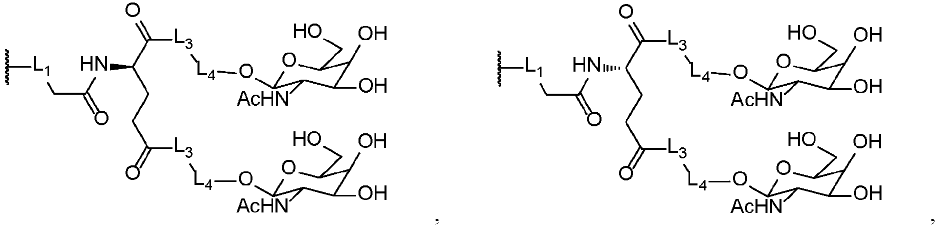 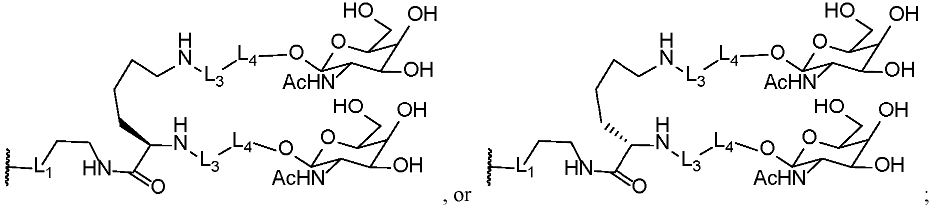
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond, -C(O)NH-, or -NHC(O)-;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
nis 0, 1,2, 3, 4, 5, 6, 7, 8,9, or 10.

In another aspect, the present invention relates to a ligand containing N-acetylgalactosamine, where the conjugated group is shown in Formula (X'):
Q is independently 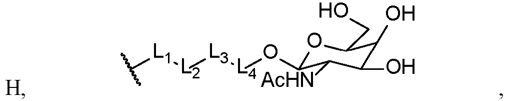 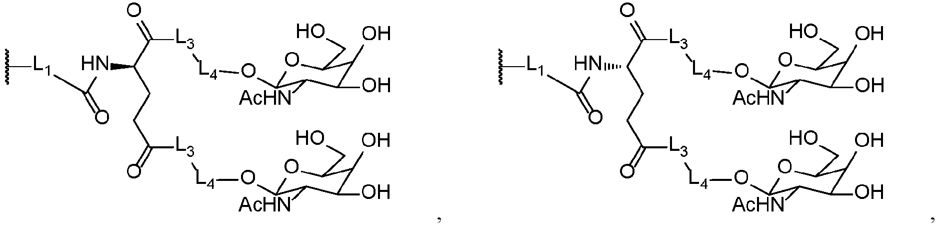 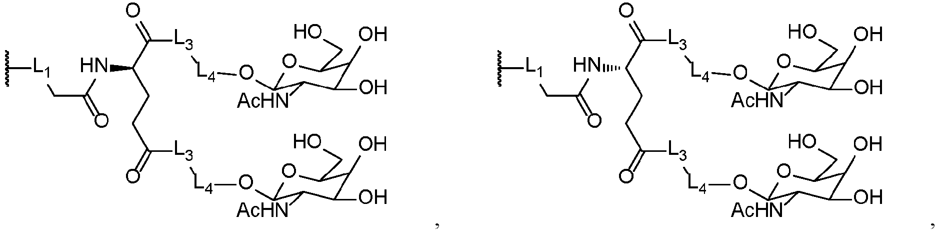 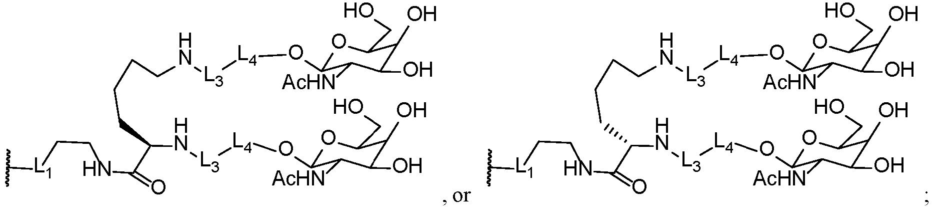
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
where the e is 1, 2, 3, 4, or 5;
T is bond, -CH₂-, -M-, -CH₂-M-, or -C(O)-M-;
where the M is 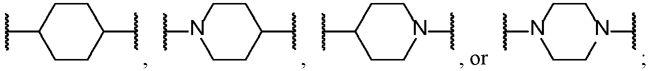
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In another aspect, the present invention relates to a ligand containing N-acetylgalactosamine, where the conjugated group is shown in Formula (X'):
Q is independently H, 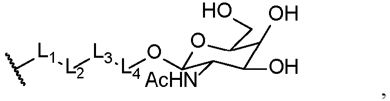 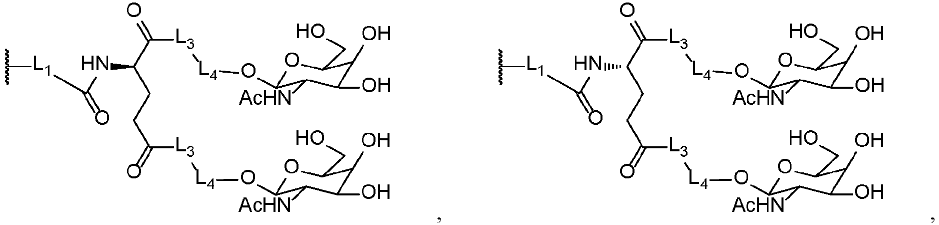 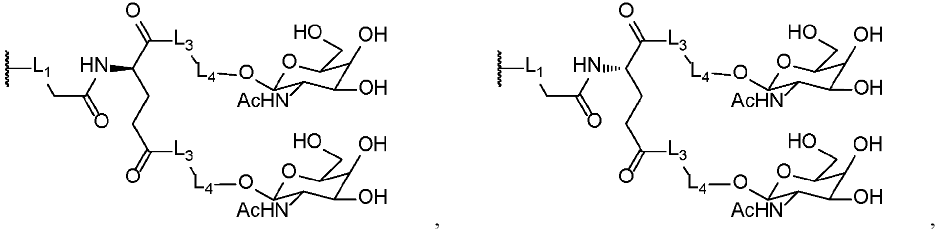 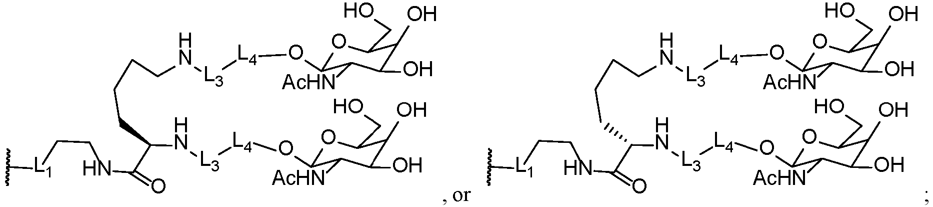
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is -O(CH₂CH₂O)ₑ-;
where the e is 1, 2, 3, 4, or 5;
T is bond, -CH₂-, -C(O)-, -M-, -CH₂-M-, or -C(O)-M-;
where the M is 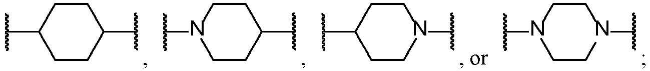
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In another aspect, the present invention relates to a compound of Formula (X), or a pharmaceutically acceptable salt, stereoisomer or isotope variant thereof: 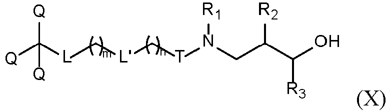 where
Q is independently H, 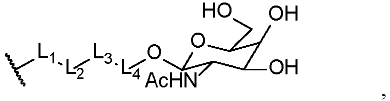 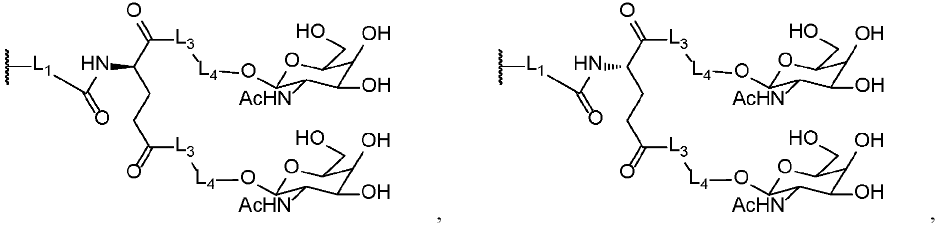 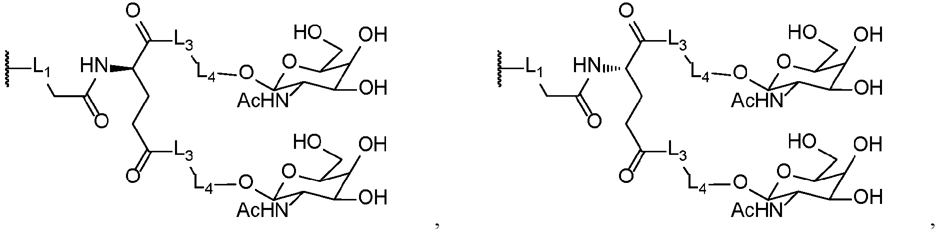 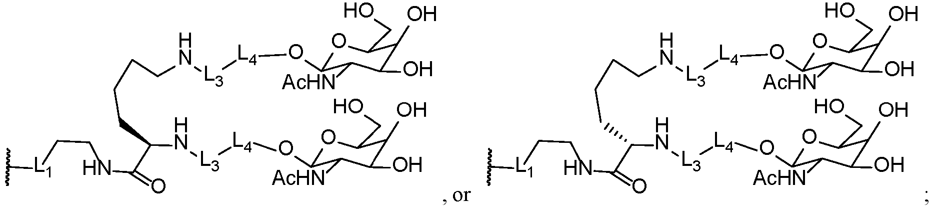
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
where e is 1, 2, 3, 4, or 5;
T is bond, -CH₂-, -C(O)-, -M-, -CH₂-M-, or -C(O)-M-;
where M is 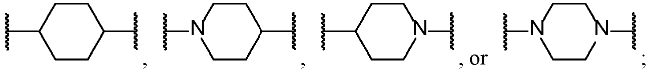
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In another aspect, the present invention relates to a compound of Formula (I), or a pharmaceutically acceptable salt, stereoisomer or isotope variant thereof: 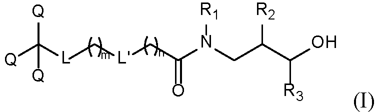 where
Q is independently H, 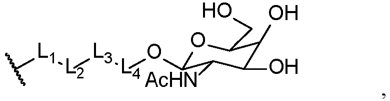 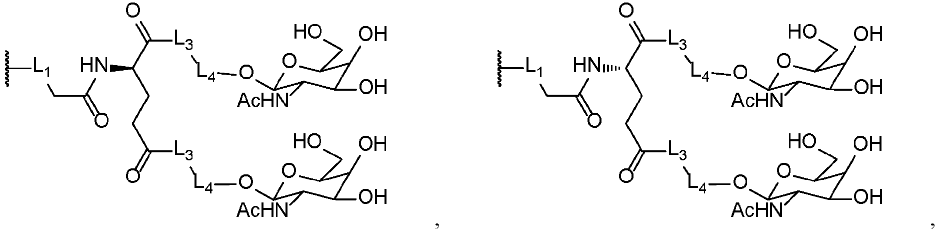 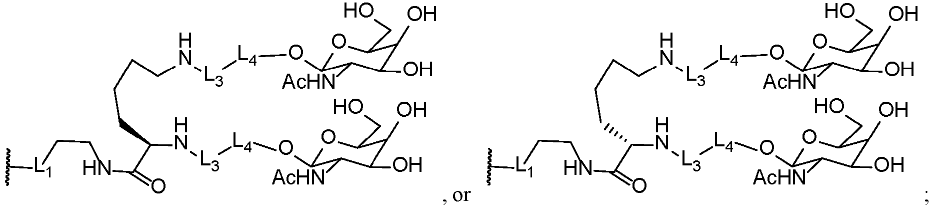
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond, -C(O)NH-, or -NHC(O)-;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In another aspect, the present invention relates to a compound of Formula (X), or a pharmaceutically acceptable salt, stereoisomer or isotope variant thereof, where:
Q is independently H, 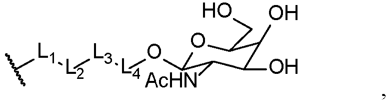 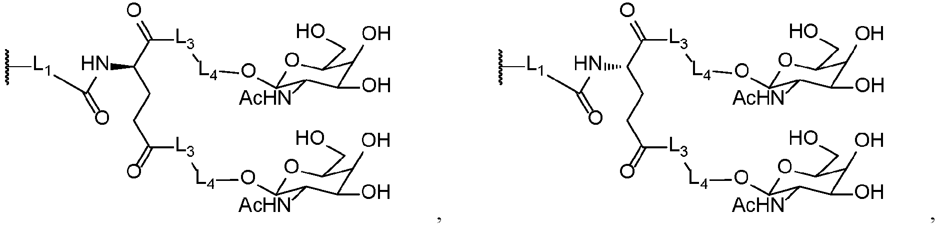 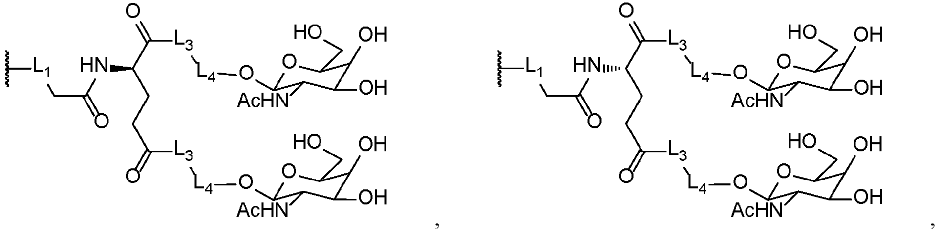 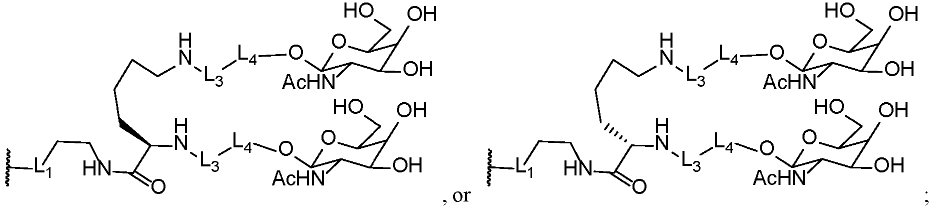
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
where the e is 1, 2, 3, 4, or 5;
T is bond, -CH₂-, -M-, -CH₂-M-, or -C(O)-M-;
where the M is 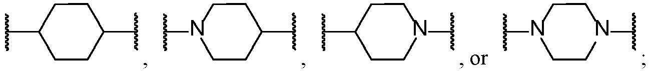
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In another aspect, the present invention relates to a compound of Formula (X), or a pharmaceutically acceptable salt, stereoisomer or isotope variant thereof, where:
Q is independently H, 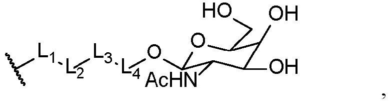 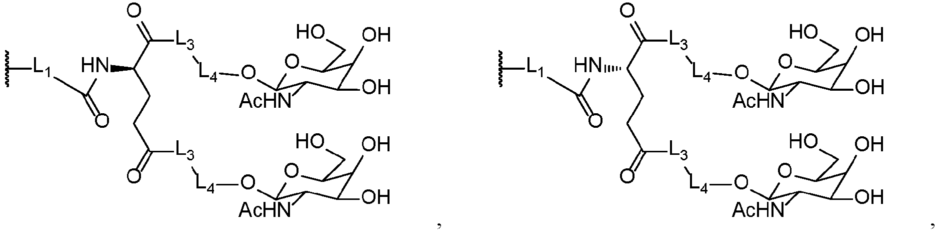 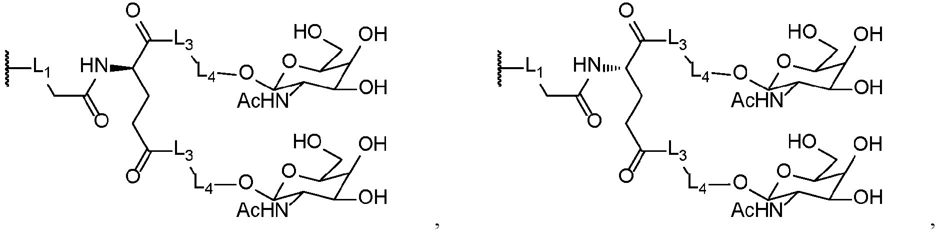 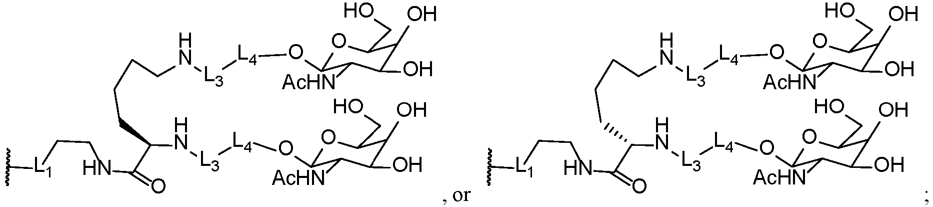
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is -O(CH₂CH₂O)ₑ-;
where the e is 1, 2, 3, 4, or 5;
T is bond, -CH₂-, -C(O)-, -M-, -CH₂-M-, or -C(O)-M-;
where the M is 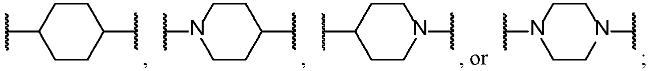
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In another aspect, the present invention relates to an intermediate compound, which is a compound of Formula (V): 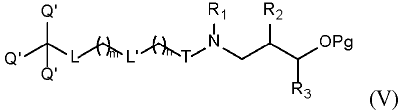 where,
Pg is a hydroxyl protective group, for example, selected from the following: 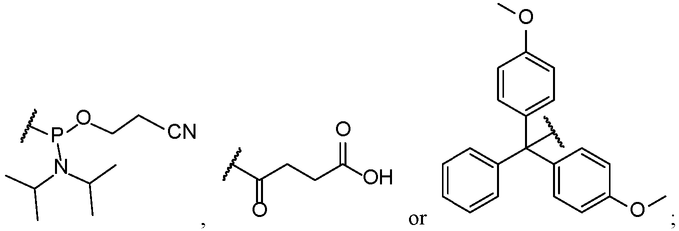
Q' is independently H, 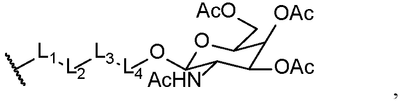 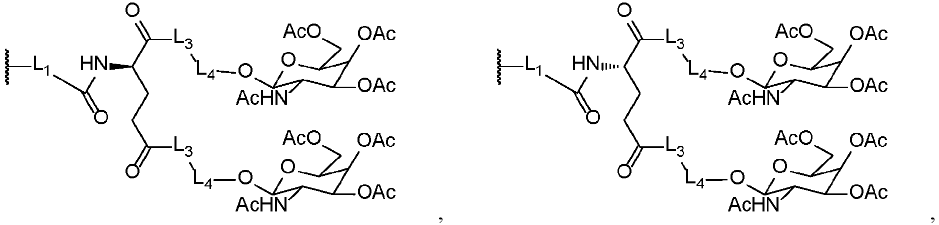 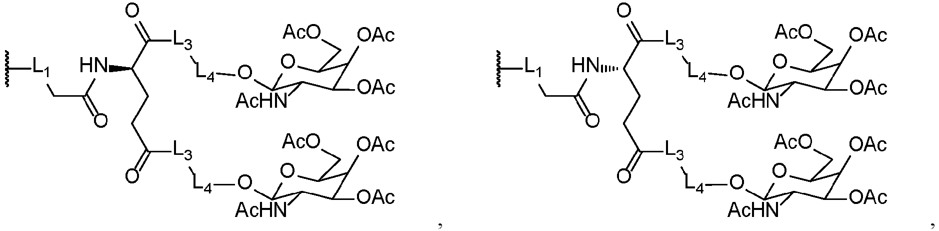 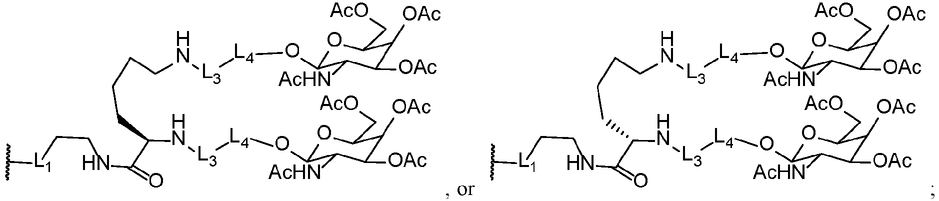 and
other groups are as defined above.

In another aspect, the present invention relates to a method for preparing the compound of Formula (I), including treating the compound of Formula (V) under the condition of removing the protective hydroxyl group: 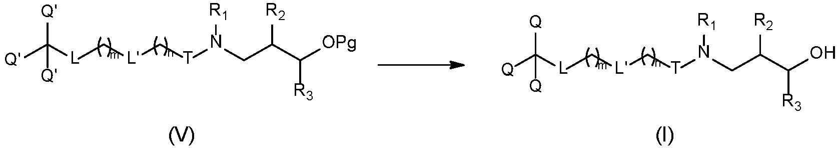 where various groups are as defined above.

In another aspect, the present invention relates to a nucleic acid molecule, which includes a nucleic acid and the ligand connected thereto as defined above.

In another aspect, the present invention relates to a pharmaceutical composition, which includes the nucleic acid molecule as described herein and a pharmaceutically acceptable carrier or excipient.

In another aspect, the present invention relates to a kit, which includes the nucleic acid molecule as described herein.

In specific embodiments of the ligand, the compounds, the intermediate compound and the nucleic acid molecule, groups are preferably defined as follows.
Q

In one embodiment, Q is H; in another embodiment, Q is 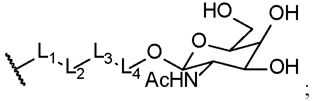 in another embodiment, Q is 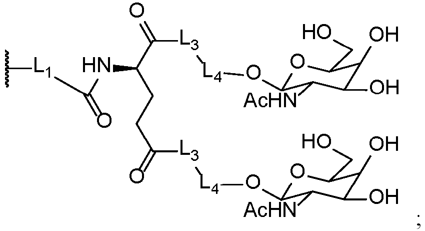 in another embodiment, Q is 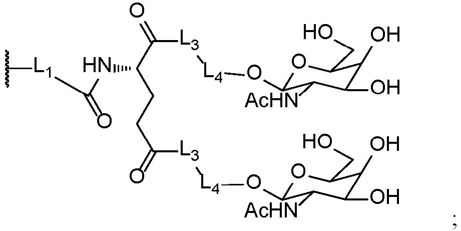 in another embodiment, Q is 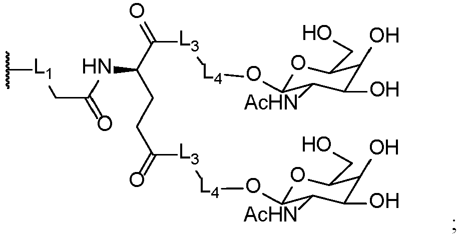 in another embodiment, Q is 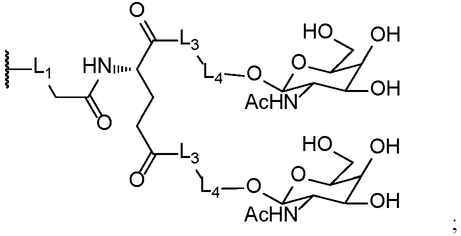 in another embodiment, Q is 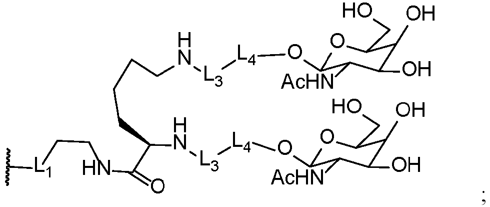 in another embodiment, Q is 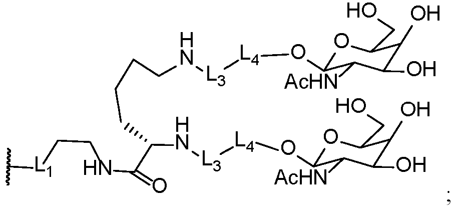 and in another embodiment, Q is any combination of the above groups.
L₁, L₂, L₃, and L₄

In one embodiment, L₁ is bond; in another embodiment, L₁ is -CH₂-; in another embodiment, L₁ is - CH₂CH₂-; in another embodiment, L₁ is -C(O)-; in another embodiment, L₁ is -CH₂O-; in another embodiment, L₁ is -CH₂O-CH₂CH₂O-; and in another embodiment, L₁ is -NHC(O)-(CH₂NHC(O))ₐ-.

In one embodiment, L₂ is H; and in another embodiment, L₂ is -CH₂CH₂C(O)-.

In one embodiment, L₃ is bond; in another embodiment, L₃ is -(NHCH₂CH₂)_{b}-; in another embodiment, L₃ is -(NHCH₂CH₂CH₂)_{b}-; and in another embodiment, L₃ is -C(O)CH₂-.

In one embodiment, L₄ is -(OCH₂CH₂)_{c}-; in another embodiment, L₄ is -(OCH₂CH₂CH₂)_{c}-; in another embodiment, L₄ is -(OCH₂CH₂CH₂CH₂)_{c}-; in another embodiment, L₄ is -(OCH₂CH₂CH₂CH₂CH₂)_{c}-; and in another embodiment, L₄ is -NHC(O)-(CH₂)_{d}-.
L, L', and T

In one embodiment, L is bond; in another embodiment, L is -CH₂O-; and in another embodiment, L is - NHC(O)-.

In one embodiment, L' is bond; in another embodiment, L' is -C(O)NH-; in another embodiment, L' is - NHC(O)-; and in another embodiment, L' is -O(CH₂CH₂O)ₑ-.

In one embodiment, T is bond; in another embodiment, T is -CH₂-; in another embodiment, T is -C(O)-; in another embodiment, T is -M-; in another embodiment, T is -CH₂-M-; and in another embodiment, T is - C(O)-M-.

In one embodiment, the M is 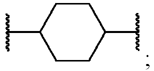 in another embodiment, the M is 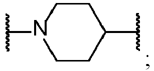 in another embodiment, the M is 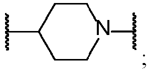 and in another embodiment, the M is 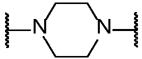 R₁, R₂, and R₃

In one embodiment, R₁ and R₂ together form -CH₂CH₂O-, and R₃ is H; in another embodiment, R₁ and R₂ together form -CH₂CH(R)-O-, and R₃ is H; and in another embodiment, R₁ and R₃ together form -C₁₋₂ alkylene-, and R₂ is H.

In one embodiment, R is -OR'; in another embodiment, R is -CH₂OR'; in another embodiment, R is - CH₂CH₂OR'; in a more specific embodiment, R' is H; in another more specific embodiment, R' is a hydroxyl protective group; in another more specific embodiment, R' is a solid support; in another more specific embodiment, R' 'is -C(O)CH₂CH₂C(O)OH; and in another more specific embodiment, R' is 4,4'-dimethoxytriphenylmethyl.

### DETAILED DESCRIPTION OF THE INVENTION

### Definition

### Chemical definition

Definitions of specific functional groups and chemical terms are described in more detail below.

When a numerical range is listed, it is agreed to include each value and subranges within Range. For example, the "C₁₋₆ alkyl" includes C₁ alkyl, C₂ alkyl, C₃ alkyl, C₄ alkyl, C₅ alkyl, C₆ alkyl, C₁₋₆ alkyl, C₁₋₅ alkyl, C₁₋₄ alkyl, C₁₋₃ alkyl, C₁₋₂ alkyl, C₂₋₆ alkyl, C₂₋₅ alkyl, C₂₋₄ alkyl, C₂₋₃ alkyl, C₃₋₆ alkyl, C₃₋₅ alkyl, C₃₋₄ alkyl, C₄₋₆ alkyl, C₄₋₅ alkyl, and C₅₋₆ alkyl.

The "C₁₋₆ alkyl" refers to a straight-chain or branched-chain saturated hydrocarbon group having 1 to 6 carbon atoms. In some embodiments, C₁₋₄ alkyl and C₁₋₂ alkyl are preferred. Examples of the C₁₋₆ alkyl include: methyl (C₁), ethyl (C₂), n-propyl (C₃), isopropyl (C₃), n-butyl (C₄), tert-butyl (C₄), sec-butyl (C₄), isobutyl (C₄), n-pentyl (C₅), 3-pentyl (C₅), pentyl (C₅), neopentyl (C₅), 3-methyl-2-butyl (C₅), tert-pentyl (C₅), and n-hexyl (C₆). The term "C₁₋₆ alkyl" further includes heteroalkyl, in which one or more (such as 1, 2, 3, or 4) carbon atoms are substituted with a heteroatom (such as oxygen, sulfur, nitrogen, boron, silicon, or phosphorus). An alkyl group can be substituted with one or more substituents, for example, substituted with 1 to 5 substituents, 1 to 3 substituents, or 1 substituent. Common alkyl abbreviations include: Me(-CH₃), Et(-CH₂CH₃), iPr(-CH(CH₃)₂), nPr(-CH₂CH₂CH₃), n-Bu(-CH₂CH₂CH₂CH₃), or i-Bu(-CH₂CH(CH₃)₂).

The "halo" or "halogen" refers to fluorine (F), chlorine (Cl), bromine (Br), and iodine (I).

Therefore, the "C₁₋₆ haloalkyl" refers to the "C₁₋₆ alkyl" that is substituted with one or more halogen groups. In some embodiments, C₁₋₄ haloalkyl is particularly preferred, and C₁₋₂ haloalkyl is more preferred. Exemplary haloalkyl includes, but is not limited to: -CF₃, -CH₂F, -CHF₂, -CHFCH₂F, -CH₂CHF₂, -CF₂CF₃, - CCl₃, -CH₂Cl, -CHCl₂, 2,2,2-trifluoro-1,1-dimethyl-ethyl, etc. A haloalkyl group can be substituted at any available connection point with, for example, 1 to 5 substituents, 1 to 3 substituents, or 1 substituent.

"C₁₋₆ alkylene" refers to a divalent group formed by removing another hydrogen of C₁₋₆ alkyl, and can be substituted or unsubstituted. In some embodiments, C₁₋₄ alkylene, C₂₋₄ alkylene, and C₁₋₂ alkylene are preferred. Unsubstituted alkylene includes, but is not limited to: methylene (-CH₂-), ethylidene (-CH₂CH₂-), propylidene (-CH₂CH₂CH₂-), butylidene (-CH₂CH₂CH₂CH₂-), pentylidene (-CH₂CH₂CH₂CH₂CH₂-), hexylidene (-CH₂CH₂CH₂CH₂CH₂CH₂-), etc. Exemplary substituted alkylene, such as, alkylene substituted with one or more alkyls (methyl), includes, but is not limited to: substituted methylene (-CH(CH₃)-, -C(CH₃)₂-), substituted ethylidene (-CH(CH₃)CH₂-, -CH₂CH(CH₃)-, -C(CH₃)₂CH₂-, -CH₂C(CH₃)₂₋), substituted propylidene (-CH(CH₃)CH₂CH₂-, -CH₂CH(CH₃)CH₂-, -CH₂CH₂CH(CH₃)-, -C(CH₃)₂CH₂CH₂-, - CH₂C(CH₃)₂CH₂-, -CH₂CH₂C(CH₃)₂-), etc.

### Other definition

The term "siRNA" herein is a double-stranded RNA molecule that can mediate silencing of a complementary target RNA (such as mRNA, such as a transcript of a gene that encodes a protein). The siRNA is usually double-stranded and includes an antisense strand complementary to the target RNA and a sense strand complementary to the antisense strand. For convenience, such mRNA is also called an mRNA to be silenced herein. Such gene is also called a target gene. Usually, an RNA to be silenced is an endogenous gene or a pathogen gene. In addition, an RNA except for the mRNA (such as a tRNA) and a viral RNA can also be targeted.

An "shRNA" refers to a short hairpin RNA. The shRNA includes two short inverted repeat sequences. The shRNA cloned into an shRNA expression vector includes two short inverted repeat sequences, which is separated by a loop sequence in the middle to form a hairpin structure and is controlled by a promoter polIII. Then, 5-6 T(s) are linked to serve as a transcriptional terminator of an RNA polymerase III.

The term "antisense strand" refers to such a strand of the siRNA that includes a region that is completely, fully, or substantially complementary to a target sequence. The term "sense strand" refers to such a strand of the siRNA that includes a region that is completely, fully, or substantially complementary to Region of the term antisense strand defined herein.

The term "complementary region" refers to a region, on the antisense strand, that is completely, fully, or substantially complementary to a target mRNA sequence. In the case that the complementary region is not completely complementary to the target sequence, mispairing can be located in an internal or terminal region of a molecule. Usually, mispairing with highest tolerance is located in the terminal region, for example, within 5, 4, 3, 2, or 1 nucleotide at the 5' and/or 3' end. A part, most sensitive to mispairing, of the antisense strand is called a "seed region". For example, in an siRNA including a 19nt strand, some mispairings can be tolerated at position 19 (from 5' to 3').

The term "complementary" refers to the ability of a first polynucleotide to hybridize with a second polynucleotide under certain conditions such as strict conditions. For example, the strict conditions may include 400 mM NaCl, 40 mM PIPES with a pH value of 6.4, and 1 mM EDTA that are lasted at 50°C or 70°C for 12-16 hours. In order to meet the above requirements with respect to the hybridize ability, a "complementary" sequence may further include or completely form base pairs that are formed from non-Watson-Crick base pairs and/or from unnatural and modified nucleotides. Such non-Watson-Crick base pairs include, but are not limited to, G:U Wobble base pairs or Hoogstein base pairs.

A polynucleotide that is "at least partially complementary", "fully complementary", or "substantially complementary" to a messenger RNA (mRNA) refers to a polynucleotide that is substantially complementary to a continuous part of the mRNA of interest. For example, when a sequence is substantially complementary to a non-interrupted part of an mRNA that encodes PCSK9, a polynucleotide is at least partially complementary to the PCSK9 mRNA. Herein, the terms "complementary", "completely complementary", "fully complementary" and "substantially complementary" may be used relative to base pairs between the sense strand and the antisense strand of the siRNA, or between an antisense strand of an siRNA reagent and a target sequence.

The "fully complementarity" refers to an extent to which the sense strand merely needs to be complementary to the antisense strand in order to maintain overall double-stranded features of a molecule. In other words, although perfect complementarity is usually required, in some cases, especially in the antisense strand, one or more mispairings, such as 6, 5, 4, 3, 2, or 1 mispairing (relative to a target mRNA) can be included, and the sense strand and the antisense strand can still maintain the overall double-stranded features of the molecule.

A "nucleoside" is a compound composed of two substances including a purine base or a pyrimidine base, and ribose or deoxyribose. A "nucleotide" is a compound composed of three substances including a purine base or a pyrimidine base, ribose or deoxyribose, and phosphoric acid. An "oligonucleotide" refers to, for example, a nucleic acid molecule (RNA or DNA) that has a length of less than 100, 200, 300, or 400 nucleotides.

The "base" is a basic composition unit for synthesis of nucleosides, nucleotides, and nucleic acids, and due to nitrogen in composition elements, is also called "nitrogenous base". Herein, unless otherwise specified, capital letters A, U, T, G and C represent base composition of nucleotides, which are adenine, uracil, thymine, guanine, and cytosine, respectively.

"Modification" of nucleotides described herein include, but are not limited to, methoxy modification, fluorinated modification, thiophosphate group ligation, or protection with conventional protective groups, etc. For example, the nucleotides with fluorinated modification refer to nucleotides formed by substitution of hydroxyl at site 2' of ribosyl of nucleotides with fluorine, and the nucleotides with methoxy modification refer to nucleotides formed by substitution of 2'-hydroxyl of ribosyl with methoxyl.

Herein, "modified nucleotides" include, but are not limited to, 2'-O-methyl modified nucleotides, 2'-fluoro modified nucleotides, 2'-deoxy- modified nucleotides, inosine ribonucleotides, debasified nucleotides, reverse baseless deoxyribonucleotides, nucleotides containing a thiophosphate group, vinyl phosphate modified nucleotides, locked nucleotides, 2'-amino- modified nucleotides, 2'-alkyl- modified nucleotides, morpholino nucleotides, aminophosphates, unnatural bases containing nucleotides, and terminal nucleotides linked to cholesterol-based derivatives or a sebacamide dodecanoate group, deoxyribonucleotides, or nucleotides protected with conventional protective groups, etc. For example, the 2'-fluoro modified nucleotides refer to nucleotides formed by substitution of hydroxyl at site 2' of ribosyl of nucleotides with fluorine. The 2'-deoxy- modified nucleotides refer to nucleotides formed by substitution of 2'-hydroxyl of ribosyl with methoxyl.

The "hydroxyl protective group" refers to a group that can prevent hydroxyl from a chemical reaction and can also be removed under certain conditions to restore the hydroxyl. The hydroxyl protective group mainly includes a silane type protective group, an acyl type protective group, or an ether type protective group, and preferably includes:
trimethylsilyl (TMS), triethylsilyl (TES), dimethylisopropylsilyl (DMIPS), diethylisopropylsilyl (DEIPS), tert-butyldimethylsilyl (TBDMS), tert-butyldiphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), carbobenzoxy (Cbz), tert-butoxycarbonyl (Boc), phenylmethyl (Bn), p-methoxybenzyl (PMB), allyl, triphenylmethyl (Tr), di-p-methoxytriphenylmethyl (DMTr), methoxymethyl (MOM), phenoxymethyl (BOM), 2,2,2-trichloroethoxymethyl, 2-methoxyethoxymethyl (MEM), methylthiomethyl (MTM), or p-methoxybenzyloxymethyl (PMBM).

The term "pharmaceutically acceptable salt" used herein refers to carboxylates or amino acid addition salts of the compound of the present invention, which are suitable for contact with tissues of patients without producing inappropriate toxicity, irritant effects, allergic reactions and the like within a reliable medical judgment range, are effective in their expected applications relative to a reasonable benefit/risk ratio, and include (if possible) a zwitterionic form of the compound of the present invention.

The compound of the present invention may include one or more asymmetric centers, and thus may exist in a variety of stereoisomer forms, such as an enantiomer form and/or a diastereoisomer form. For example, the compound of the present invention may be a separate enantiomer, a diastereoisomer or a geometric isomer (such as a cis-isomer and a trans-isomer), or may be in the form of a mixture of stereoisomers, including a racemate mixture and a mixture containing one or more stereoisomers. Isomers can be separated from a mixture by a method known to persons skilled in the art. The method includes: chiral high-pressure liquid chromatography (HPLC) and formation and crystallization of a chiral salt; and alternatively, preferred isomers can be prepared by asymmetric synthesis.

The present invention further includes isotope labeled compounds (isotope variants) that are equivalent to the one of Formula (I), but one or more atoms are substituted with atoms with atomic masses or mass numbers different from atomic masses or mass numbers commonly found in nature. Examples of isotopes that can be introduced into the compound of the present invention include isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. All compounds of the present invention containing the isotopes and/or other isotopes of other atoms, precursor drugs thereof, and pharmaceutically acceptable salts of the compounds or the precursor drugs fall within the scope of the present invention. Certain isotope labeled compounds of the present invention, such as those into which radioisotopes (such as ³H and ¹⁴C) are introduced, can be used for determining distribution of drugs and/or substrate tissues. Tritium, namely ³H, and carbon-14, namely ¹⁴C isotope, are particularly preferred because they are easy to prepare and detect. Furthermore, substitution with heavier isotopes, such as deuterium, namely ²H, may be preferred in some cases because higher metabolic stability can provide therapeutic benefits, such as prolonging the half-life in vivo or reducing dose requirements. Isotope labeled compounds of Formula (I) of the present invention and precursor drugs thereof can generally be prepared by substituting non-isotope labeled reagents with readily available isotope labeled reagents in processes described below and/or technologies disclosed in examples and preparative examples.

The present invention specifically relates to the following technical solutions:
1. A ligand containing N-acetylgalactosamine, where the ligand includes a conjugated group shown in Formula (X'): 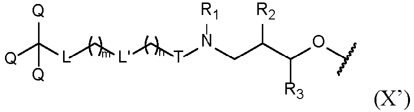 where, 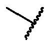
represents a position connected with a biomolecule;
Q is independently H, 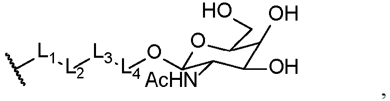 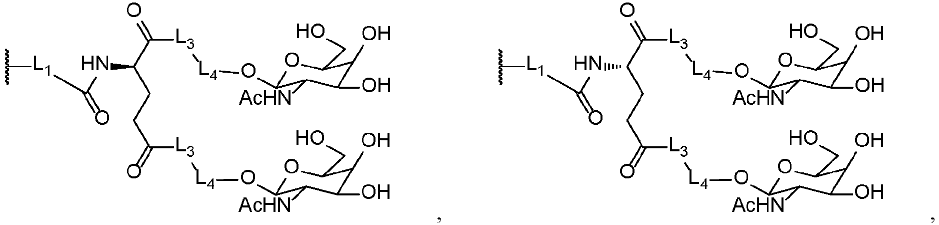 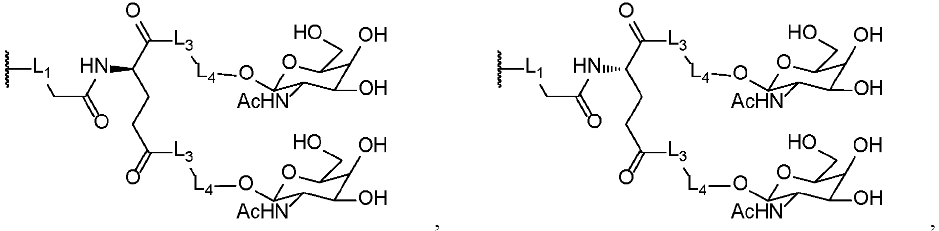 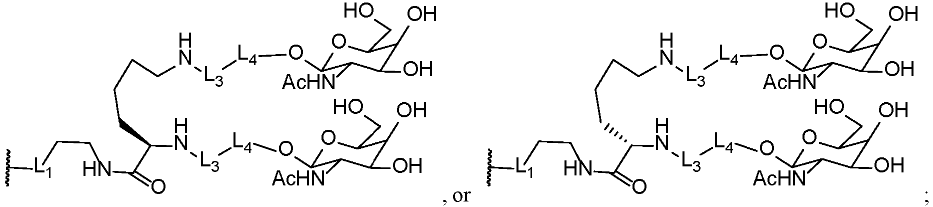
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
where e is 1, 2, 3, 4, or 5;
T is bond, -CH₂-, -C(O)-, -M-, -CH₂-M-, or -C(O)-M-;
where M is 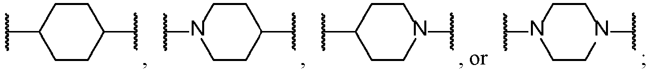
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

2. The ligand according to the technical solution 1, where the conjugated group is shown in Formula (I'): 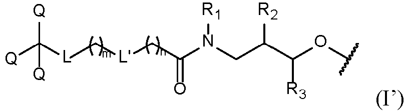 where, 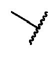
represents a position connected with a biomolecule;
Q is independently H, 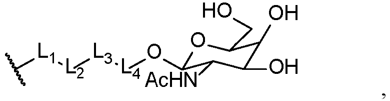 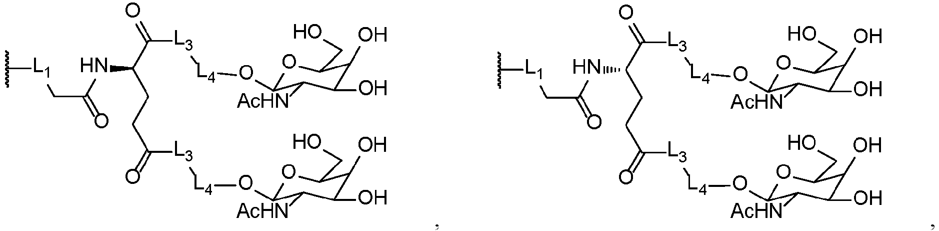 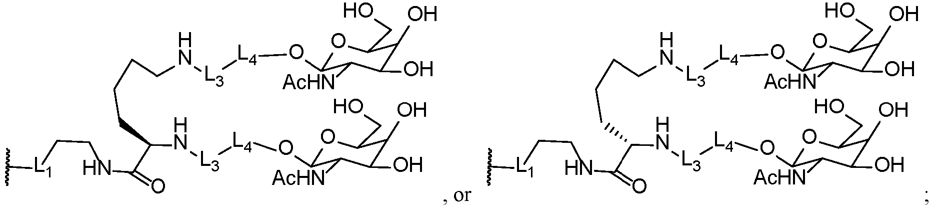
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond, -C(O)NH-, or -NHC(O)-;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
nis 0, 1,2, 3, 4, 5, 6, 7, 8,9, or 10.

3. The ligand according to the technical solution 2, where,
Q is independently H or 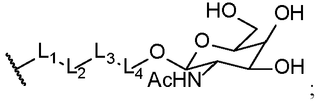
where L₁ is -CH₂O- or -NHC(O)-(CH₂NHC(O))ₐ-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O-;
L' is bond;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
nis0, 1, 2, 3, 4, 5, 6, 7, 8, 9, or 10.

4. The ligand according to the technical solution 2, where the conjugated group is shown in Formula (I'-1), Formula (I'-2), or Formula (I'-3): 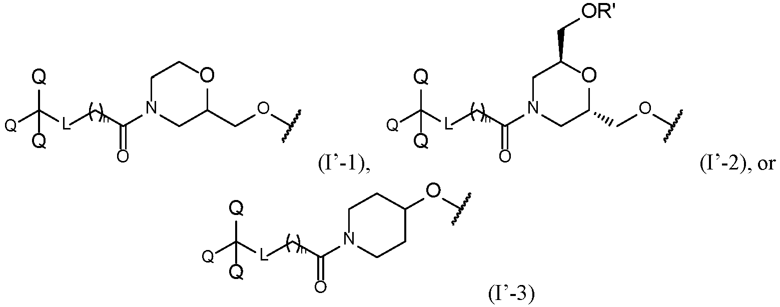 where, 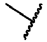
represents a position connected with a biomolecule;
Q is 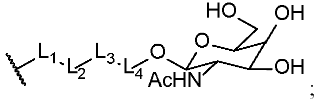
where L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1, 2, 3, 4, 5, 6, 7, or 8;
L is -CH₂O-;
R' is H, a hydroxyl protective group, or a solid support, and the hydroxyl protective group is preferably - C(O)CH₂CH₂C(O)OH or 4,4'-dimethoxytriphenylmethyl; and
n is 0, 1, 2, 3, 4, 5, 6, 7, 8, 9, or 10.

5. The ligand according to the technical solution 2, where,
Q is independently H, 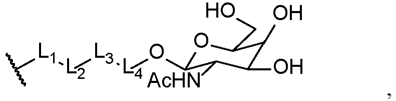 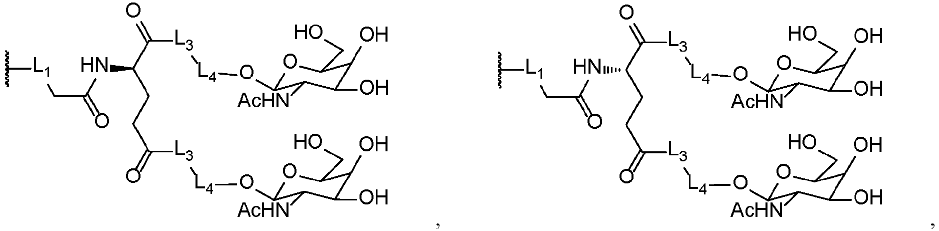 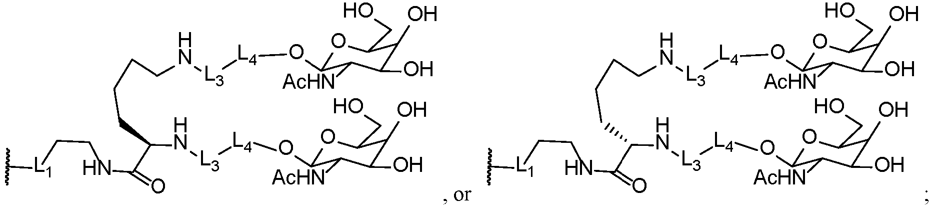
where L₁ is -CH₂O-, -CH₂O-CH₂CH₂O-, or -NHC(O)-(CH₂NHC(O))ₐ-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

6. The ligand according to the technical solution 5, where the conjugated group is shown in Formula (II'-1) or Formula (II'-2): 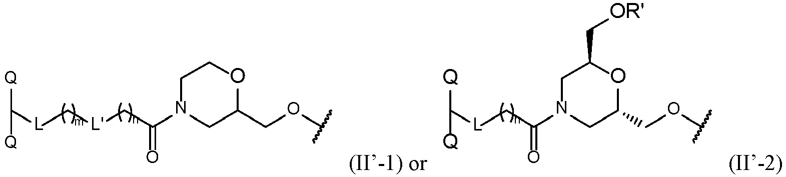 where, 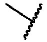
represents a position connected with a biomolecule;
Q is independently 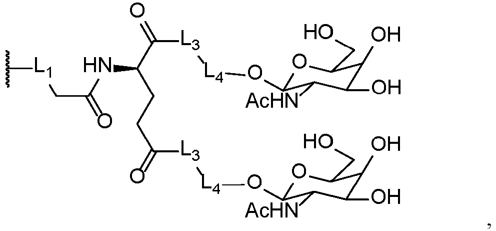 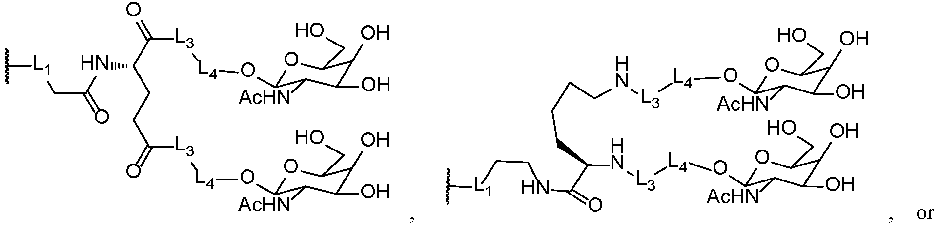 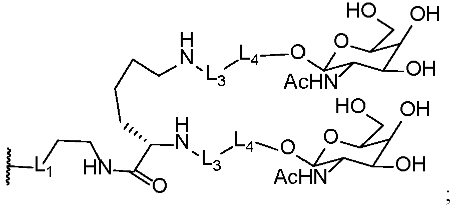
where L₁ is -CH₂O- or -CH₂O-CH₂CH₂O-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1, 2, 3, 4, 5, 6, 7, or 8;
L is -NHC(O)-;
L' is bond or -C(O)NH-;
R' is H, a hydroxyl protective group, or a solid support, and the hydroxyl protective group is preferably - C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

7. The ligand according to the technical solution 2, where,
Q is independently H, 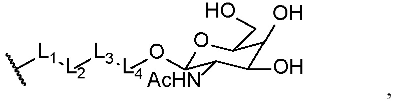 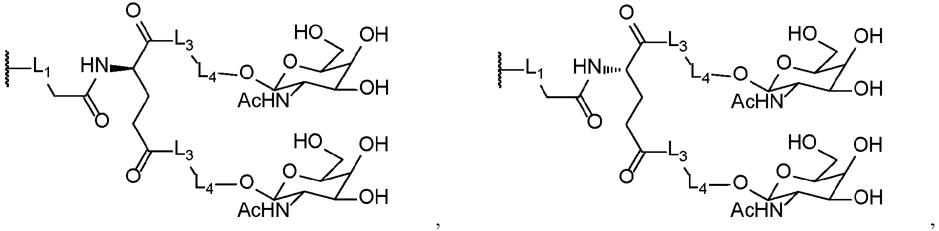 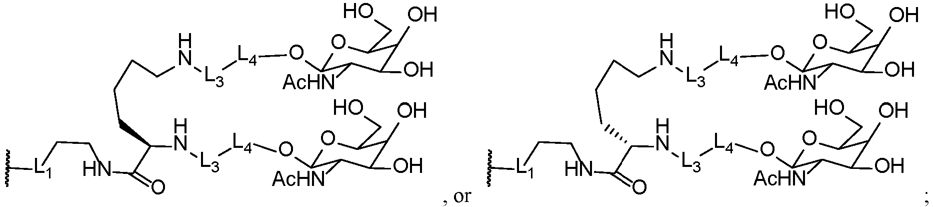
where L₁ is -CH₂-, -C(O)-, -CH₂O-, -CH₂O-CH₂CH₂O-, or -NHC(O)-(CH₂NHC(O))ₐ-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

8. The ligand according to the technical solution 7, where the conjugated group is shown in Formula (II' -2): 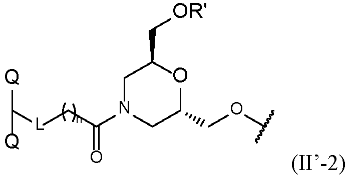 where, 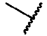
represents a position connected with a biomolecule;
Q is 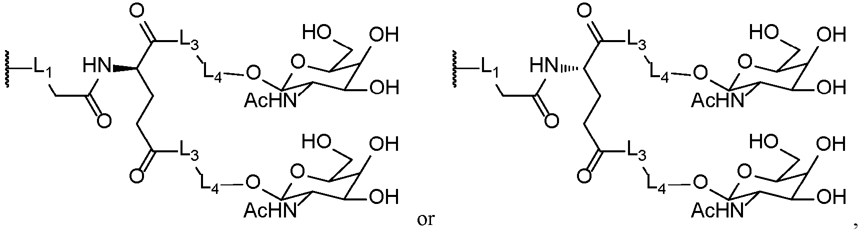 preferably 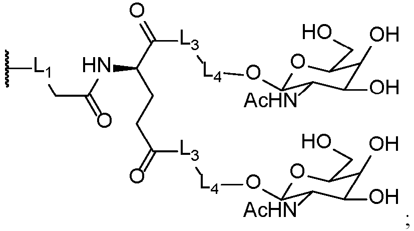
L₁ is -CH₂O-;
L₃ is -NHCH₂CH₂-;
L₄ is -(OCH₂CH₂)_{c}-,
c is 1, 2, or 3;
L is -NHC(O)-;
R' is H; and
n is 6, 7, 8, 9, or 10, preferably 7, 8, or 9, more preferably 8.

9. The ligand according to the technical solution 7, where the conjugated group is shown in Formula (II' -2): 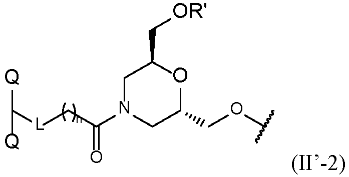 where, 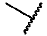
represents a position connected with a biomolecule;
Q is independently 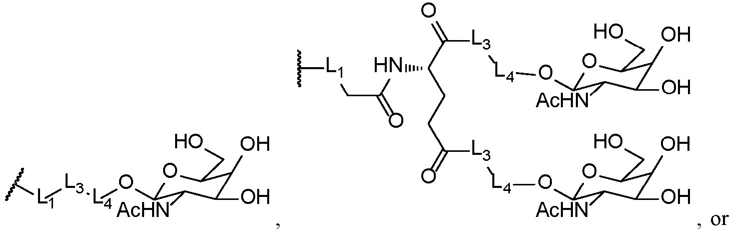 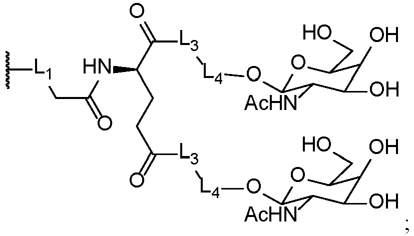
where L₁ is -CH₂- or -C(O)-;
L₃ is -(NHCH₂CH₂)_{b}-,
L₄ is -(OCH₂CH₂)_{c}-,
where b is 1, 2, 3, 4, or 5;
c is 1, 2, 3, 4, or 5;
L is -CH₂O- or -NHC(O)-;
R' is H, a hydroxyl protective group, or a solid support, and the hydroxyl protective group is preferably - C(O)CH₂CH₂C(O)OH or 4,4'-dimethoxytriphenylmethyl; and
n is 0, 1, 2, 3, 4, 5, 6, 7, 8, 9, or 10.

10. The ligand according to the technical solution 9, where the conjugated group is shown in Formula (II' -3): 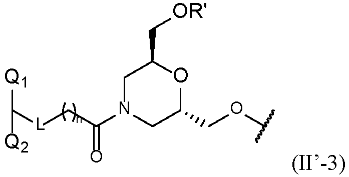 where, 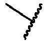
represents a position connected with a biomolecule;
Q₁ is 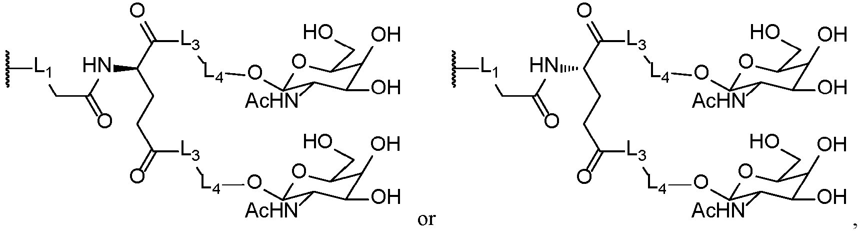 preferably 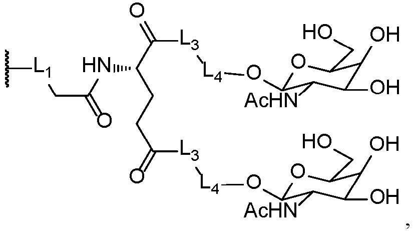 where L₁ is -CH₂-;
Q₂ is 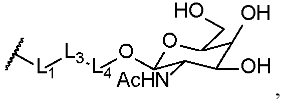 where L₁ is -C(O)-;
L₃ is -NHCH₂CH₂-;
L₄ is -(OCH₂CH₂)_{c}-,
c is 1, 2, or 3;
L is -NHC(O)-;
R' is H; and
n is 6, 7, 8, 9, or 10, preferably 7, 8, or 9, more preferably 8.

11. The ligand according to the technical solution 1, where:
Q is independently H, 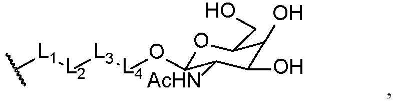 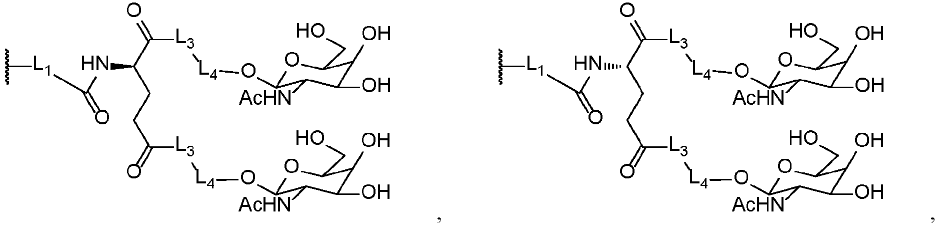 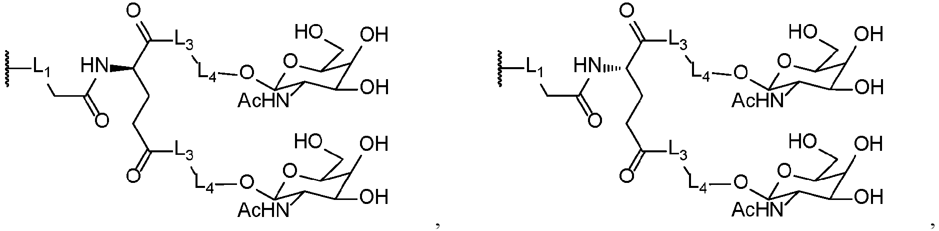 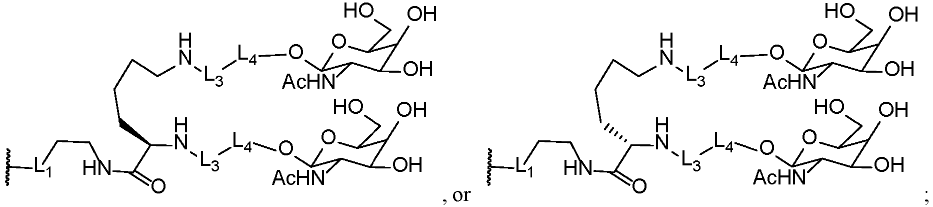
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
where the e is 1, 2, 3, 4, or 5;
T is bond, -CH₂-, -M-, -CH₂-M-, or -C(O)-M-;
where the M is 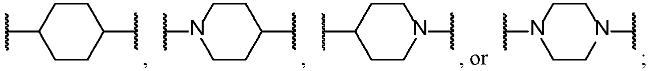
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

12. The ligand according to the technical solution 11, where,
T is -M-, -CH₂-M-, or -C(O)-M-, where the M is 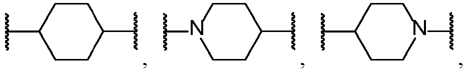 or 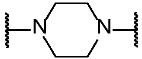
13. The ligand according to the technical solution 11 or 12, where,
Q is independently H or 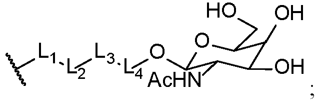
where L₁ is -CH₂O- or -NHC(O)-(CH₂NHC(O))ₐ-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
where a is 0, 1, 2, or 3;
the b is 1, 2, 3, 4, or 5;
c is 1, 2, 3, 4, or 5;
the d is 1, 2, 3, 4, 5, 6, 7, or 8;
L is bond or -CH₂O-;
L' is bond or -O(CH₂CH₂O)ₑ-;
where the e is 1, 2, 3, 4, or 5;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to the technical solution 11 or 12.

14. The ligand according to the technical solution 13, where the conjugated group is shown in Formula (III'-1), Formula (III'-2), or Formula (III'-3): 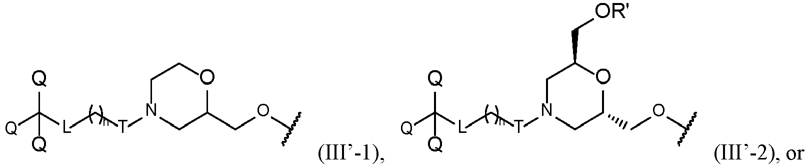 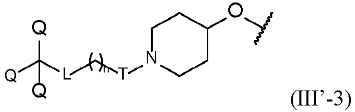 where,
Q is 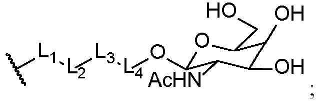
where L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1, 2, 3, 4, 5, 6, 7, or 8;
L is bond or -CH₂O-;
where R' is H, a hydroxyl protective group, or a solid support, and the hydroxyl protective group is preferably -C(O)CH₂CH₂C(O)OH or 4,4'-dimethoxytriphenylmethyl;
n is 0, 1, 2, 3, 4, 5, 6, 7, 8, 9, or 10; and
T is as defined according to the technical solution 11 or 12.

15. The ligand according to the technical solution 11 or 12, where,
Q is independently H, 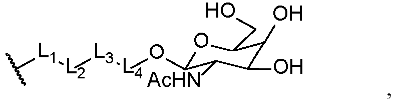 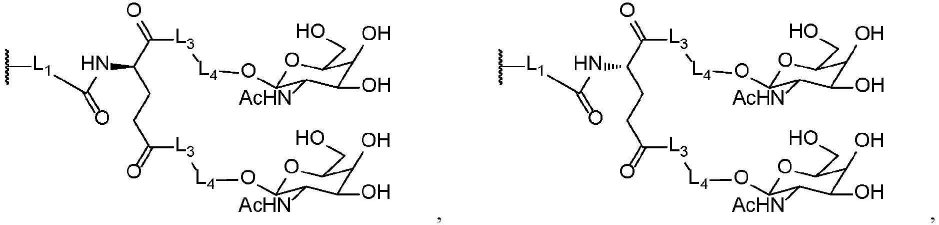 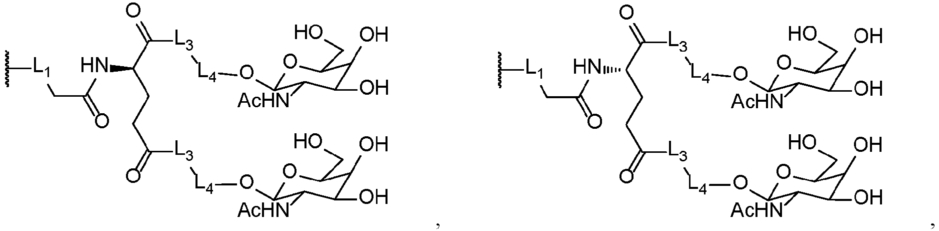 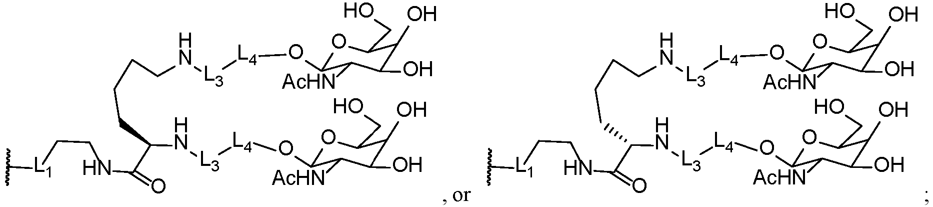
where L₁ is -CH₂-, -CH₂O-, or -C(O)-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1, 2, 3, 4, 5, 6, 7, or 8;
L is bond or -NHC(O)-;
L' is bond;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to the technical solution 11 or 12.

16. The ligand according to the technical solution 11 or 12, where the conjugated group is shown in Formula (IV'-1) or Formula (IV'-2): 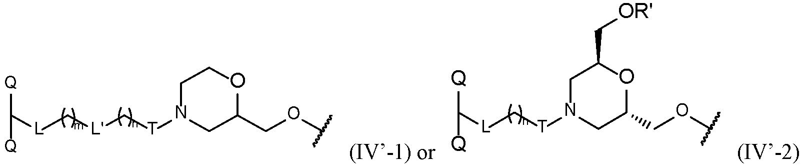 where,
Q is independently 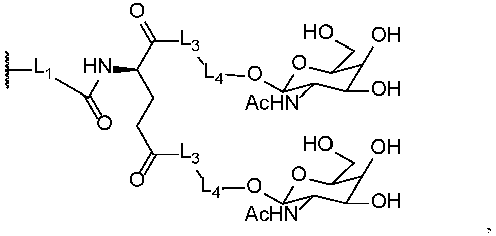 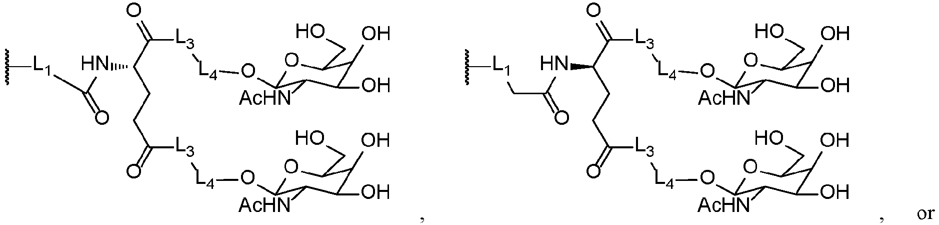 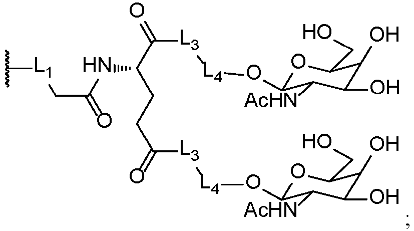
where L₁ is -CH₂-, -CH₂O-, or -C(O)-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1, 2, 3, 4, 5, 6, 7, or 8;
L is bond or -NHC(O)-;
L' is bond;
where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to the technical solution 11 or 12.

17. The ligand according to the technical solution 1, where the conjugated group is shown in Formula (II'-3): 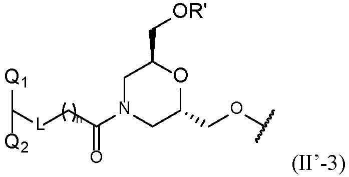 where, 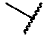
represents a position connected with a biomolecule;
Q₁ is 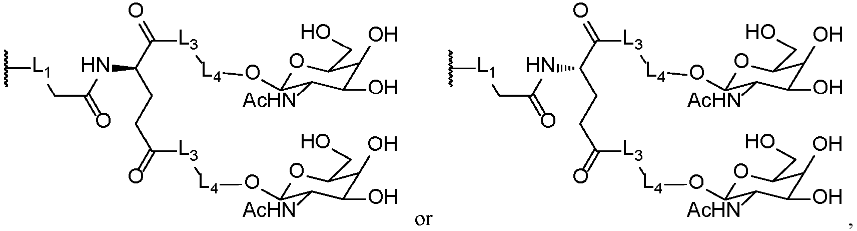
preferably 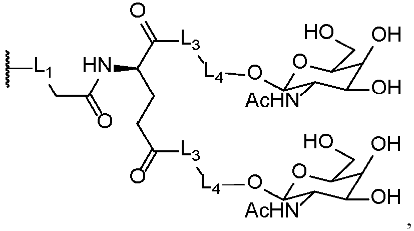 where L₁ is -CH₂-;
Q₂ is 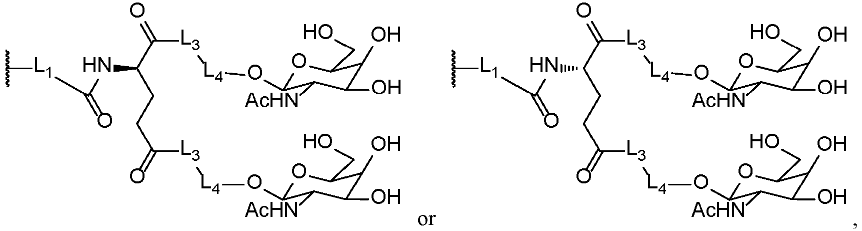
preferably 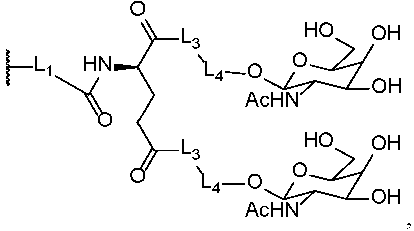 where L₁ is bond;
   L₃ is -NHCH₂CH₂-;
   L₄ is -(OCH₂CH₂)_{c}-,
   c is 1, 2, or 3;
   L is -NHC(O)-;
   R' is H; and
   n is 6, 7, 8, 9, or 10, preferably 7, 8, or 9, more preferably 8.

18. The ligand according to the technical solution 1, where:
Q is independently H, 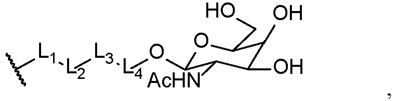 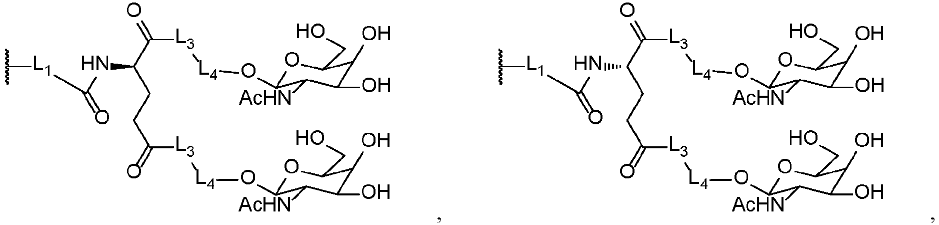 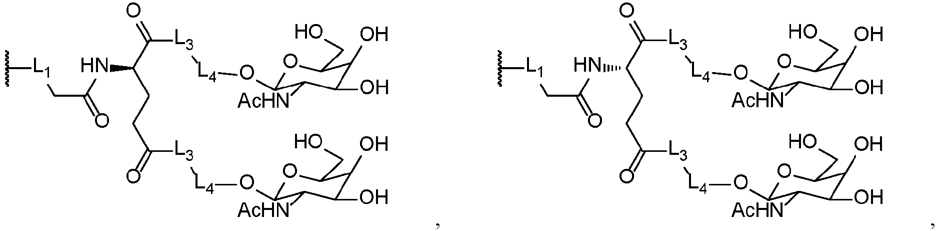 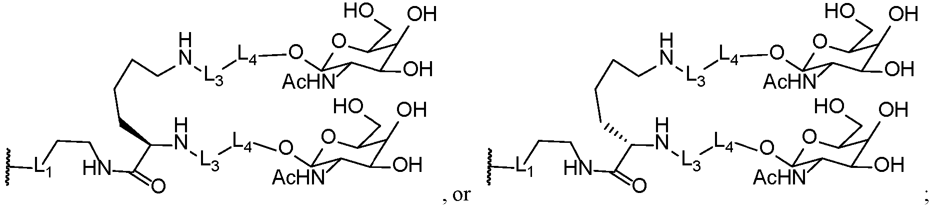
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is -O(CH₂CH₂O)ₑ-;
where the e is 1, 2, 3, 4, or 5;
T is bond, -CH₂-, -C(O)-, -M-, -CH₂-M-, or -C(O)-M-;
where the M is 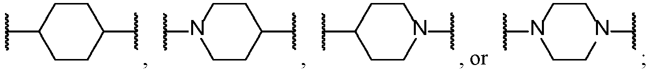
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

19. The ligand according to the technical solution 1, where the conjugated group is selected from the following:

| Number | Structure |
|---|---|
| GL1 | 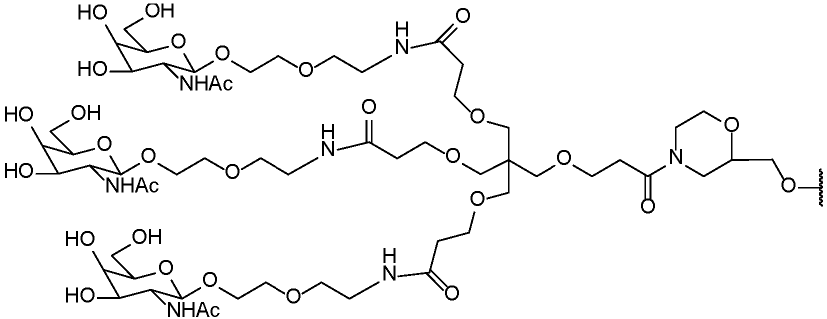 |
| GL2 | 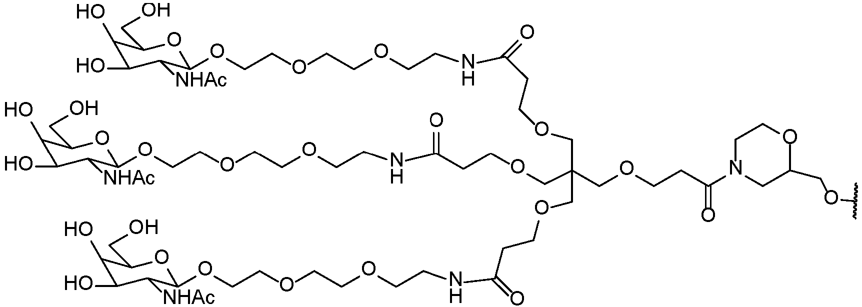 |
| GL3 | 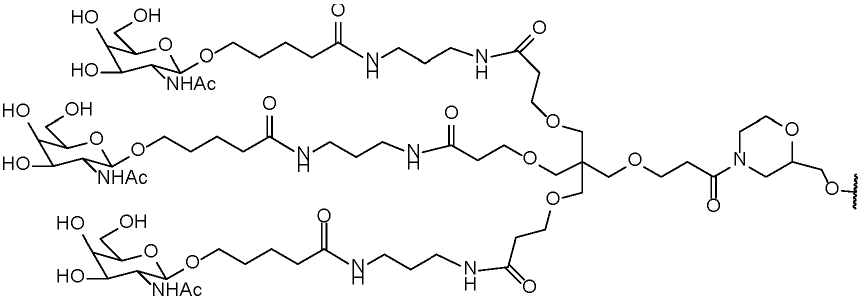 |
| GL4 | 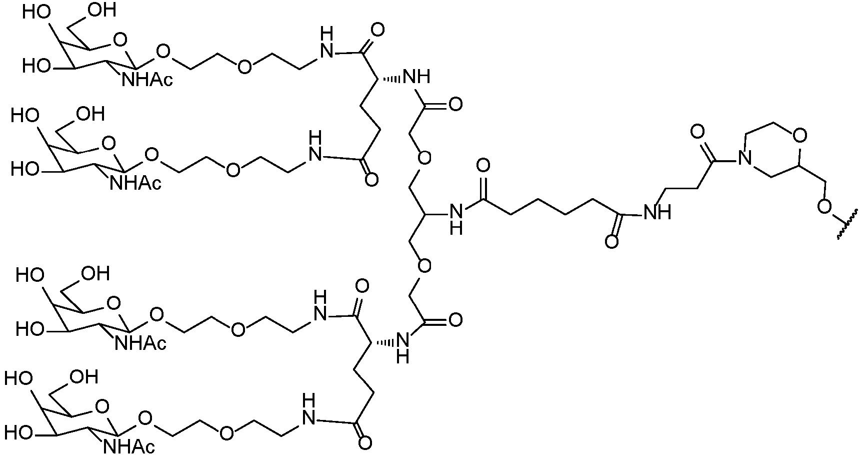 |
| GL5 | 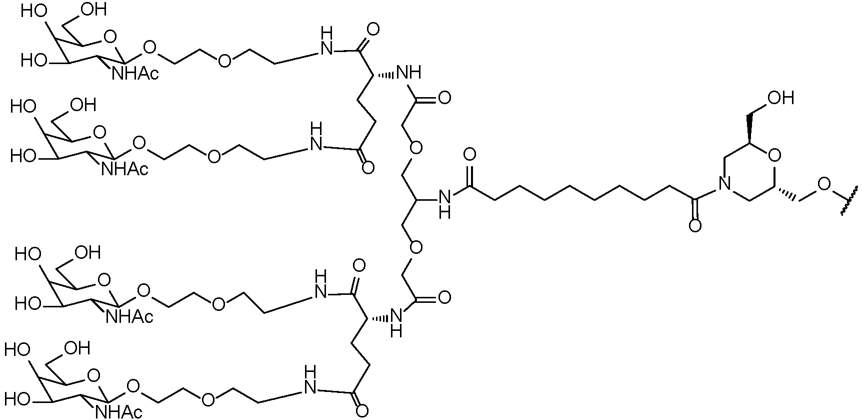 |
| GL6 | 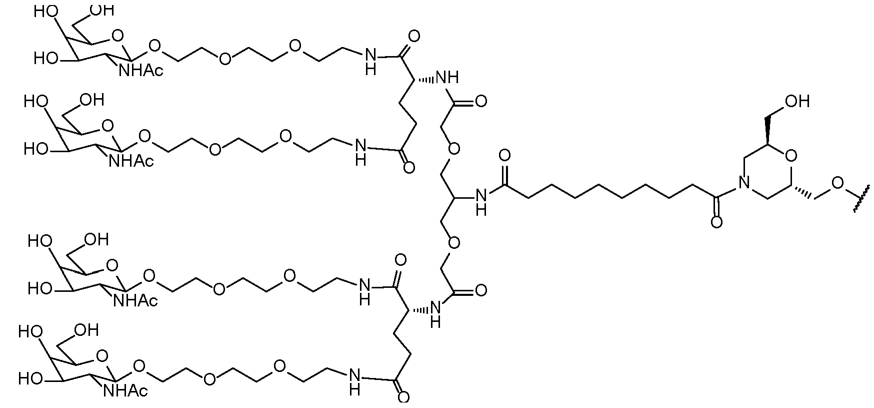 |
| GL7 | 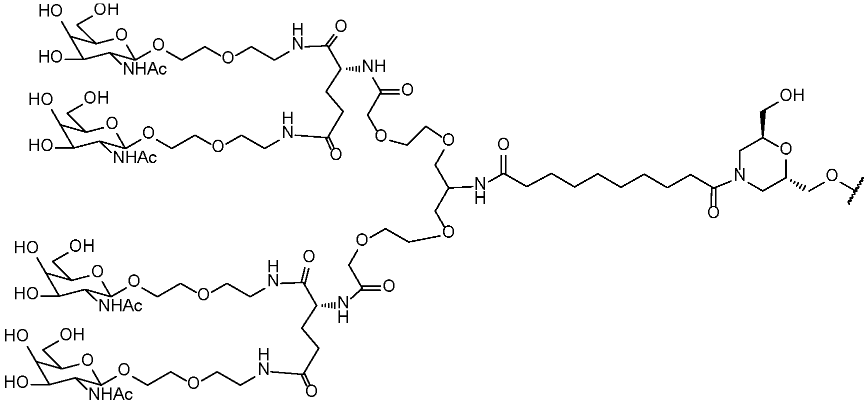 |
| GL8 | 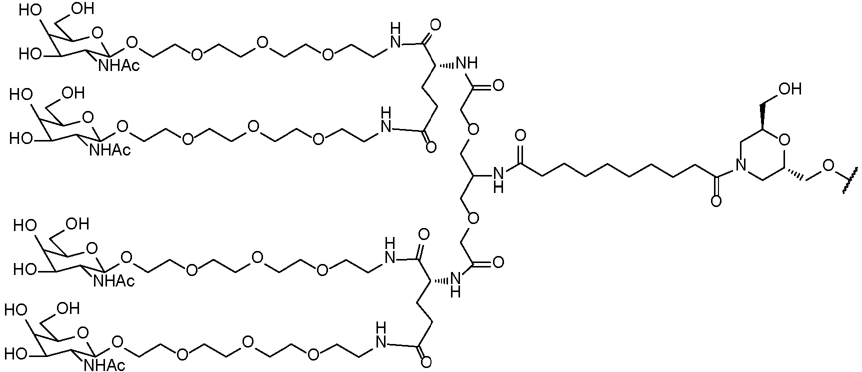 |
| GL9 | 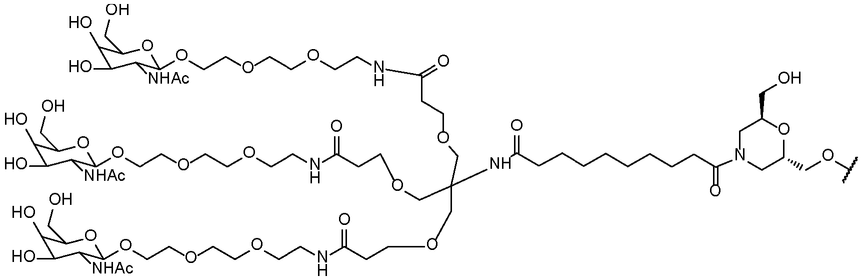 |
| GL10 | 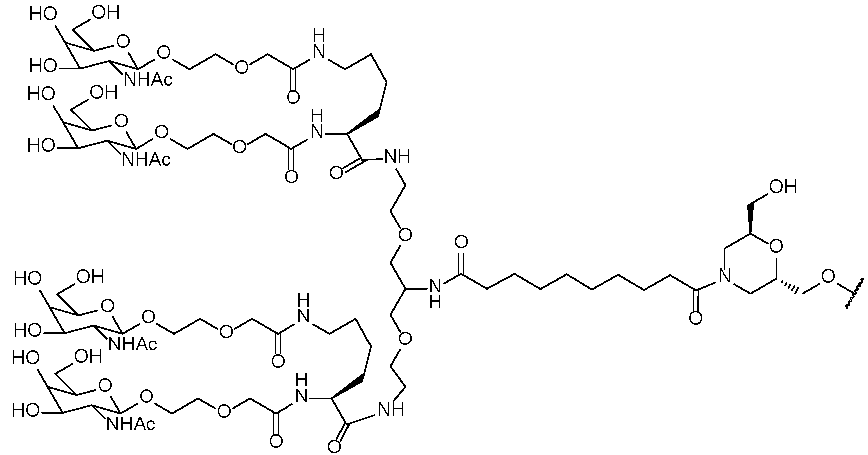 |
| GL11 | 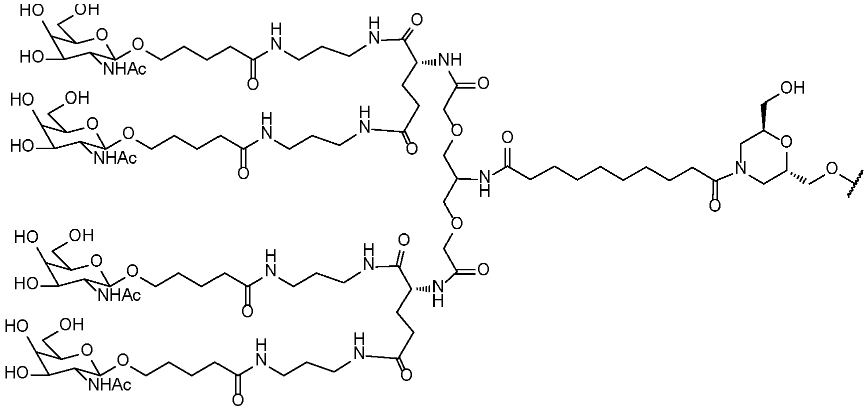 |
| GL12 | 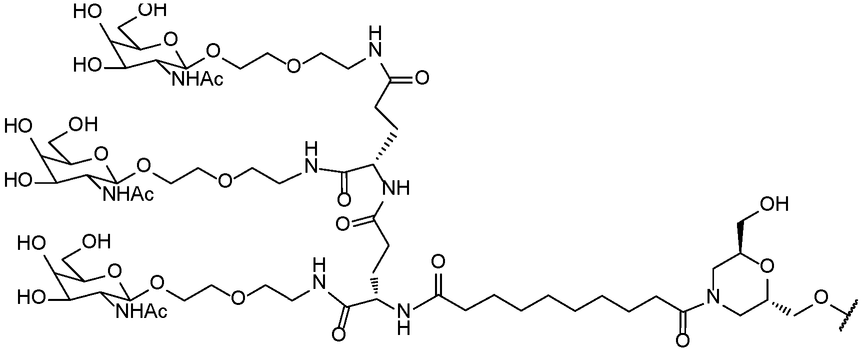 |
| GL13 | 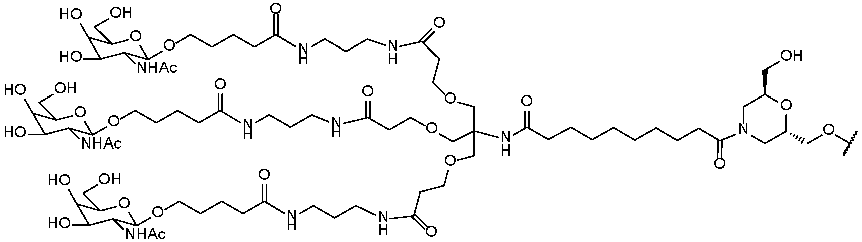 |
| GL14 | 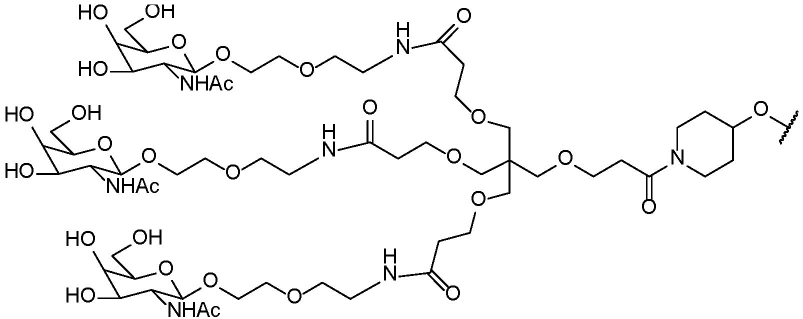 |
| GL15 | 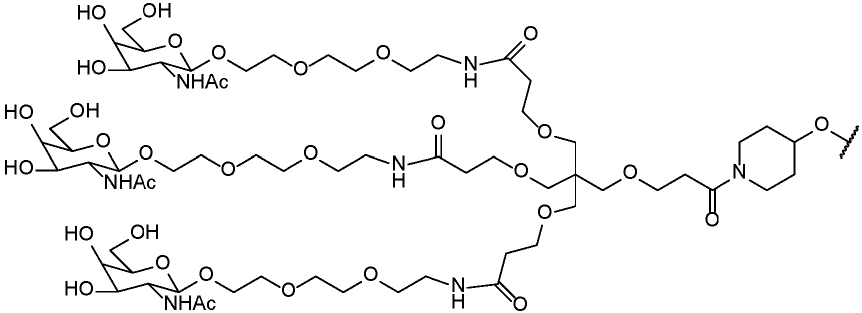 |
| GL16 | 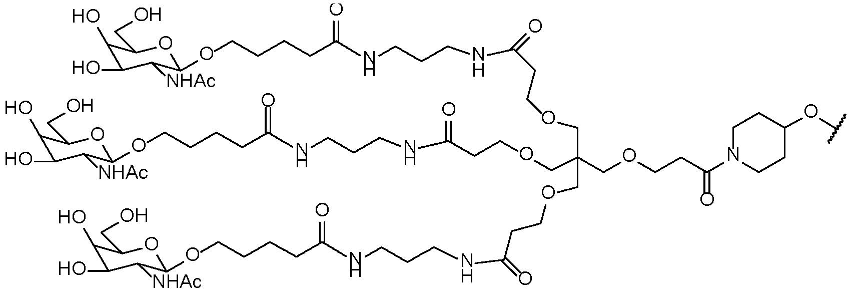 |

20. The ligand according to the technical solution 1, where the conjugated group is selected from the following:

| Numb er | Structure |
|---|---|
| GL17 | 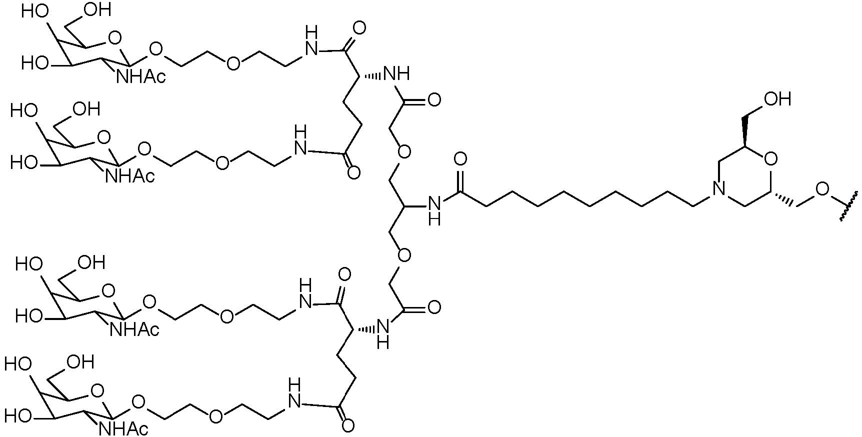 |
| GL18 | 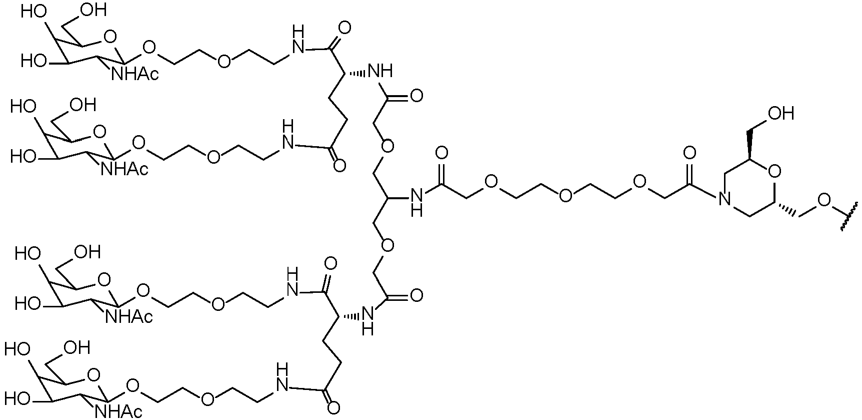 |
| GL19 | 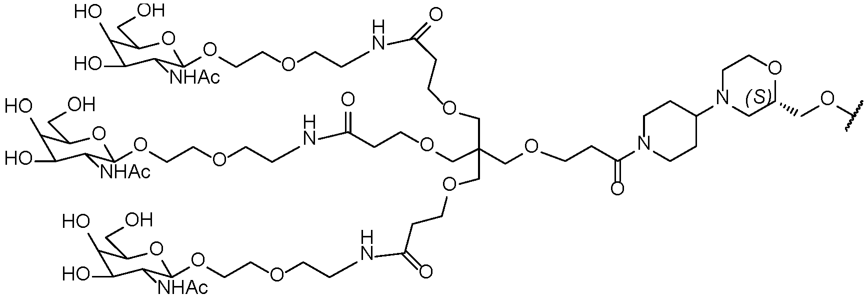 |
| GL20 | 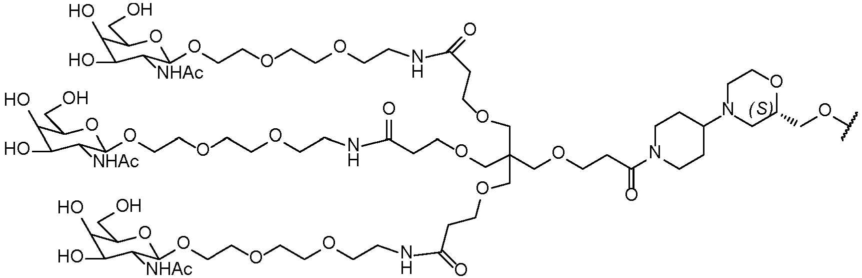 |
| GL21 | 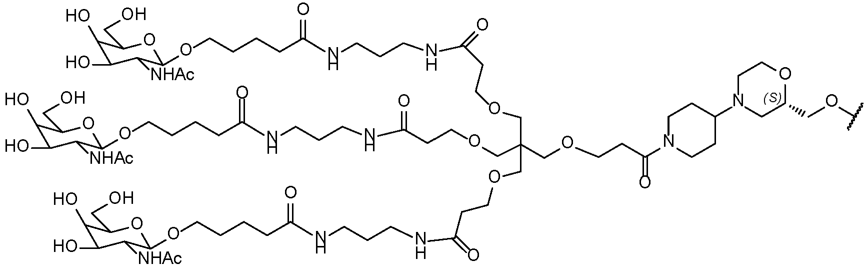 |
| GL22 | 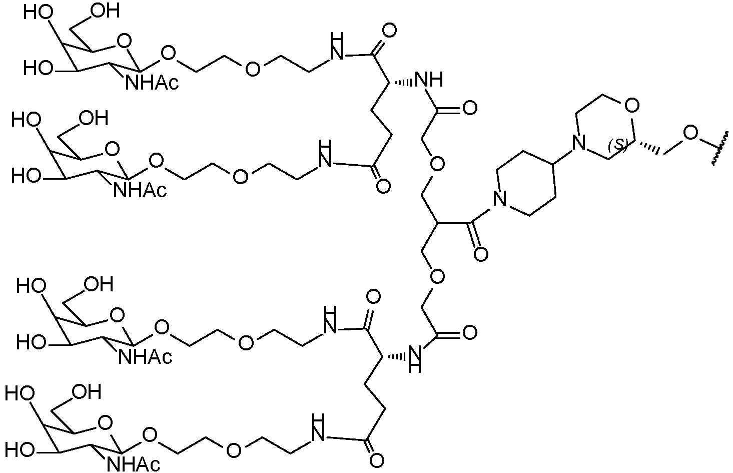 |
| GL23-1 | 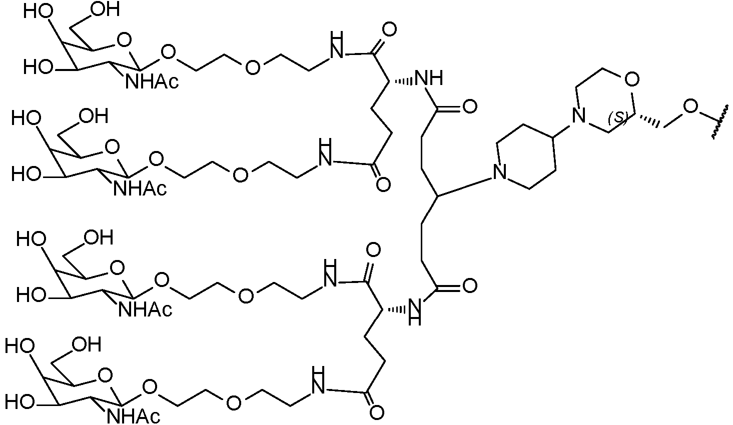 |
| GL23-2 | 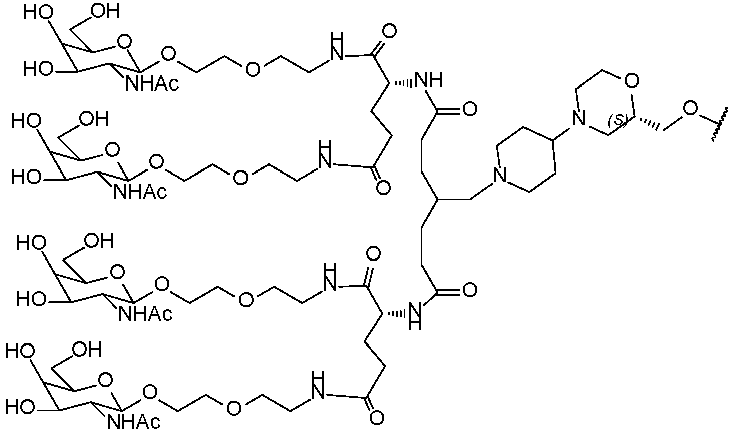 |
| GL24 | 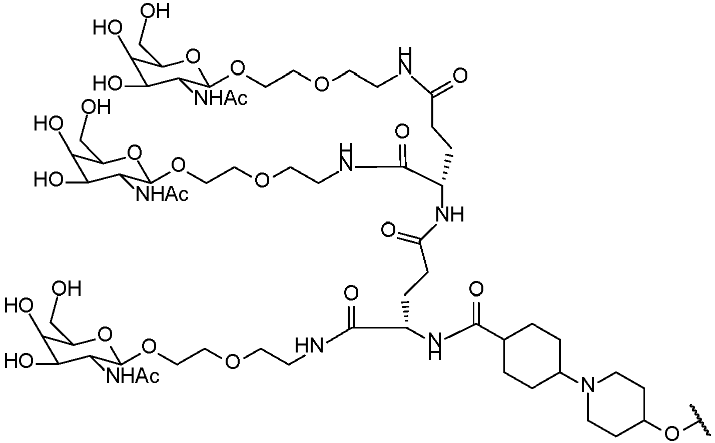 |
| GL25 | 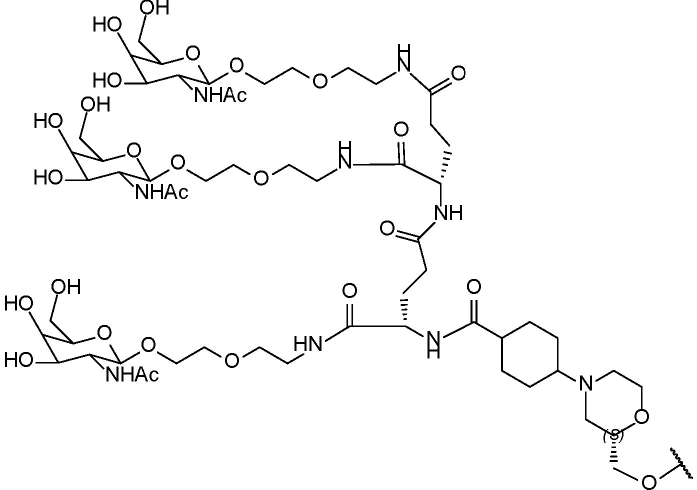 |
| GL26 | 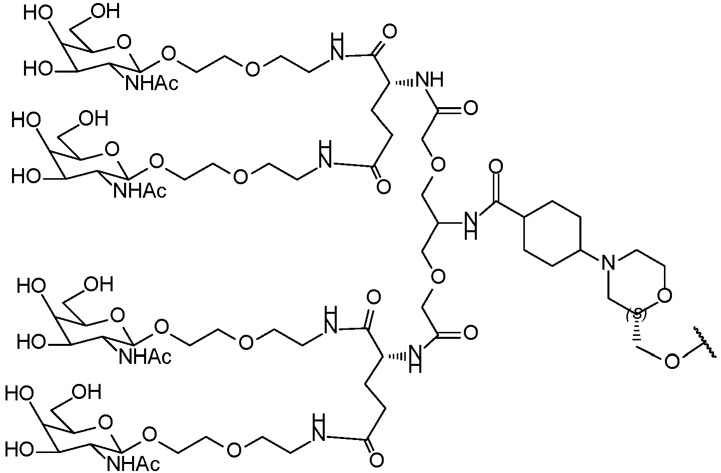 |
| GL27 | 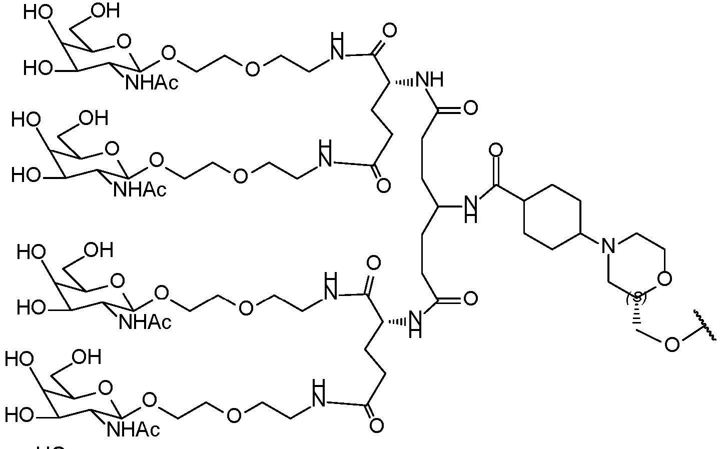 |
| GL28 | 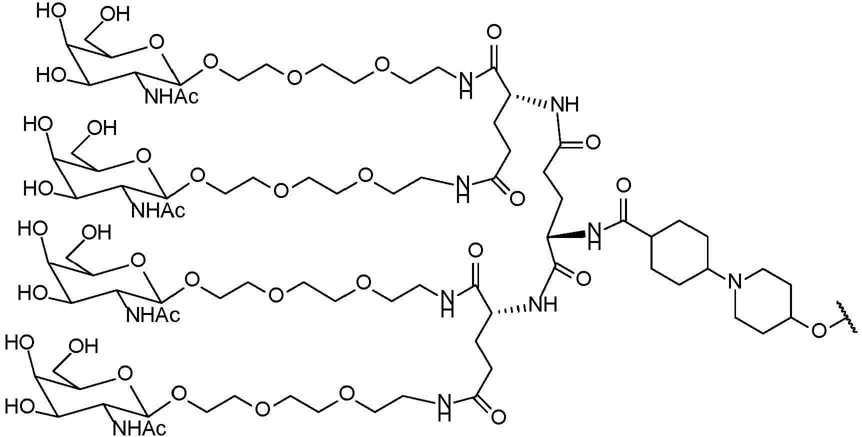 |
| GL29 | 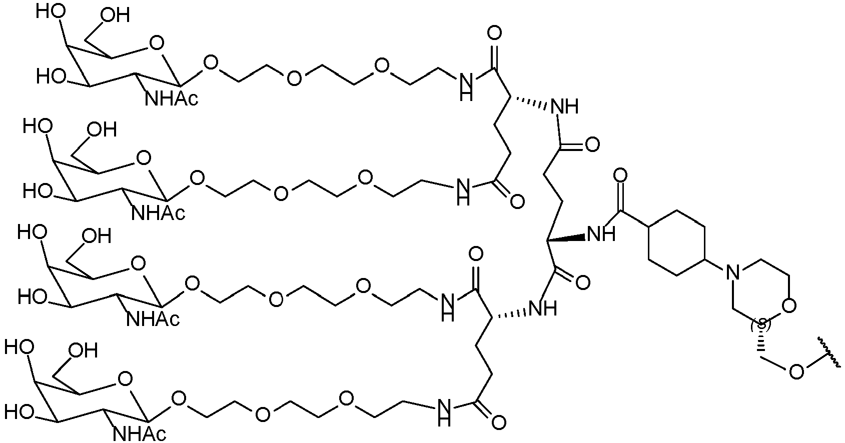 |
| GL30 | 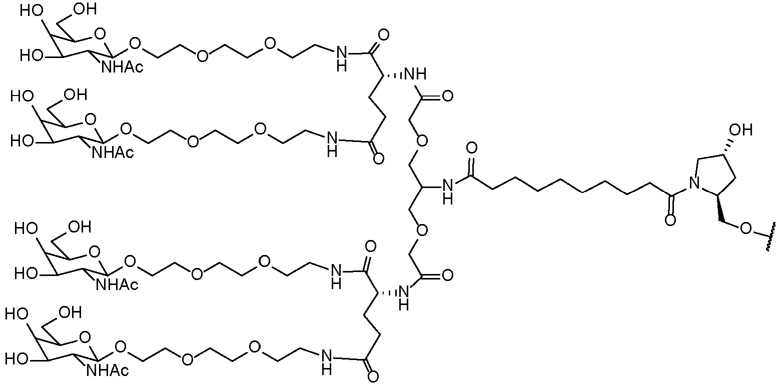 |
| GL31 | 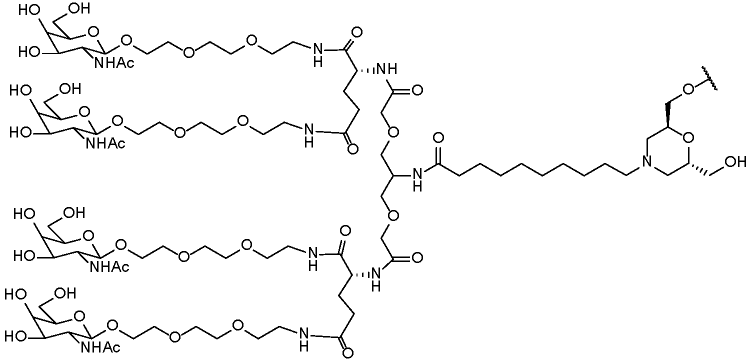 |
| GL32 | 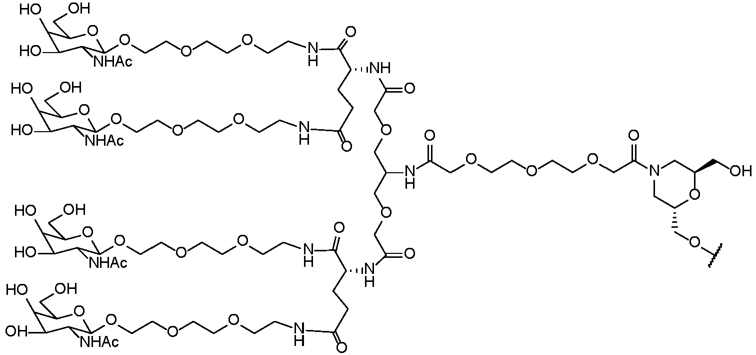 |
| GL33 | 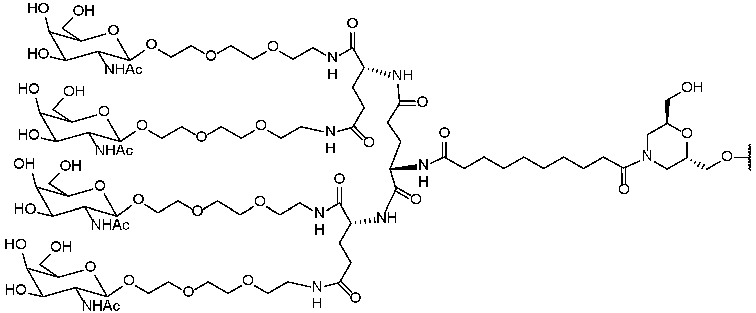 |

21. The ligand according to any one of the technical solutions 1-20, targeting ASGPR.
22. A nucleic acid molecule, including a nucleic acid and the ligand connected thereto according to any one of the technical solutions 1-21.
23. The nucleic acid molecule according to the technical solution 22, where the nucleic acid is selected from a DNA, an RNA, and a DNA/RNA hybrid.
24. The nucleic acid molecule according to the technical solution 23, where the nucleic acid molecule is single-stranded or double-stranded.
25. The nucleic acid molecule according to the technical solution 23, where the nucleic acid molecule is selected from a small interfering RNA (siRNA) and a short hairpin RNA (shRNA).
26. The nucleic acid molecule according to the technical solution 23, where the nucleic acid molecule is a double-stranded RNA molecule which includes a sense strand and an antisense strand, and a conjugated group is connected to a 3' end or 5' end of the sense strand of the double-stranded RNA molecule, preferably the 3' end.
27. The nucleic acid molecule according to the technical solution 26, where the sense strand and the antisense strand of the double-stranded RNA molecule each has 10 to 30 nucleotides, preferably 15 to 25 nucleotides, more preferably 20 to 25 nucleotides.
28. The nucleic acid molecule according to the technical solution 26 or 27, where one or more nucleotides in the sense strand and/or the antisense strand of the double-stranded RNA molecule include chemical modification.
29. The nucleic acid molecule according to the technical solution 26 or 27, where one or more nucleotides in the sense strand and/or the antisense strand of the double-stranded RNA molecule are substituted with nucleotide analogs.
30. The nucleic acid molecule according to any one of the technical solutions 22-29, specifically binding to an asialoglycoprotein receptor (ASGPR) on surfaces of hepatocytes.
31. A pharmaceutical composition, including the nucleic acid molecule according to any one of the technical solutions 22-30 and a pharmaceutically acceptable carrier or excipient.
32. A kit, including the nucleic acid molecule according to any one of the technical solutions 22-30.
33. A compound of Formula (X), or a pharmaceutically acceptable salt, stereoisomer or isotope variant thereof: 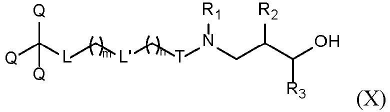 where,
Q is independently H, 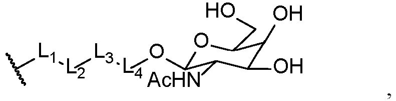 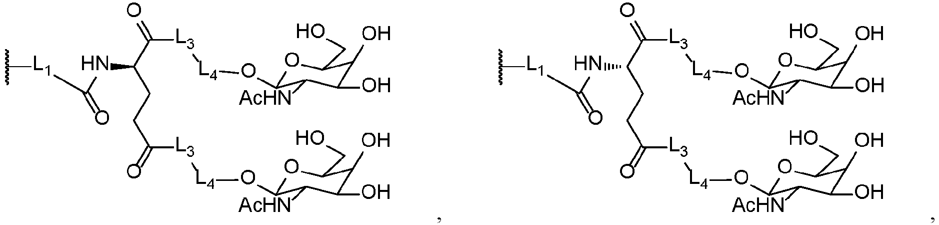 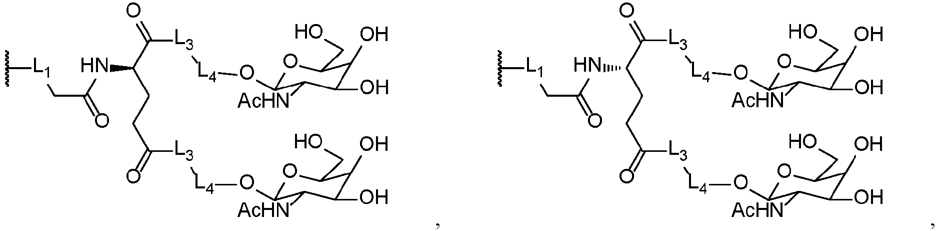 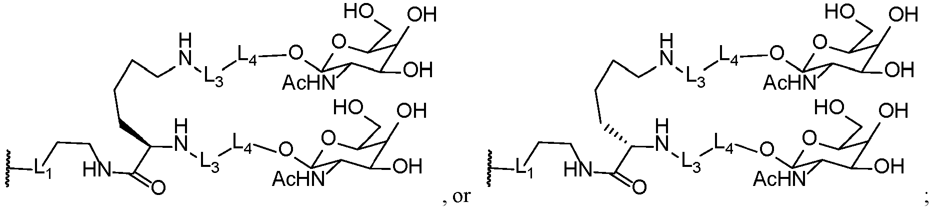
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
where e is 1, 2, 3, 4, or 5;
T is bond, -CH₂-, -C(O)-, -M-, -CH₂-M-, or -C(O)-M-;
where M is 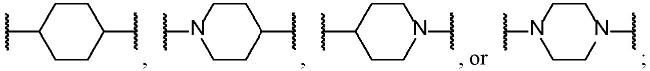
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

34. The compound, or the pharmaceutically acceptable salt, stereoisomer or isotope variant thereof according to the technical solution 33, having Formula (I): 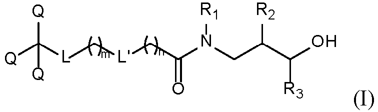 where,
Q is independently H, 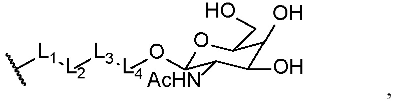 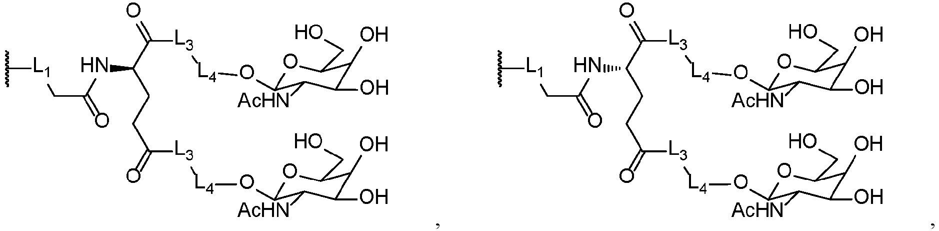 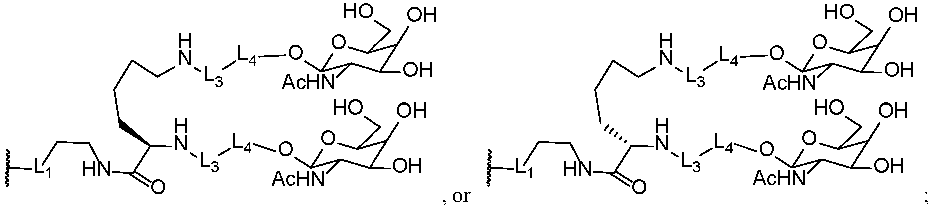
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond, -C(O)NH-, or -NHC(O)-;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
nis 0, 1,2, 3, 4, 5, 6, 7, 8,9, or 10.

35. The compound, or the pharmaceutically acceptable salt, stereoisomer or isotope variant thereof according to the technical solution 34, where,
Q is independently H or 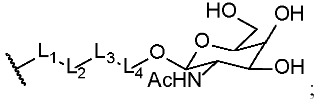
where L₁ is -CH₂O- or -NHC(O)-(CH₂NHC(O))ₐ-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O-;
L' is bond;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
nis0, 1, 2, 3, 4, 5, 6, 7, 8, 9, or 10.

36. The compound, or the pharmaceutically acceptable salt, stereoisomer or isotope variant thereof according to the technical solution 34, having Formula (I-1), Formula (I-2), or Formula (I-3): 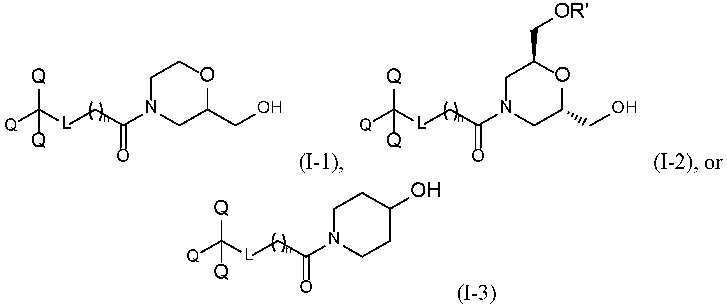 where,
Q is 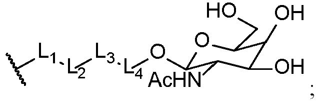
where L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1, 2, 3, 4, 5, 6, 7, or 8;
L is -CH₂O-;
R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl; and
n is 0, 1, 2, 3, 4, 5, 6, 7, 8, 9, or 10.

37. The compound, or the pharmaceutically acceptable salt, stereoisomer or isotope variant thereof according to the technical solution 34, where,
Q is independently H, 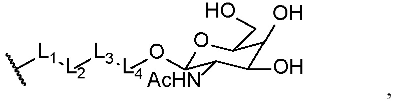 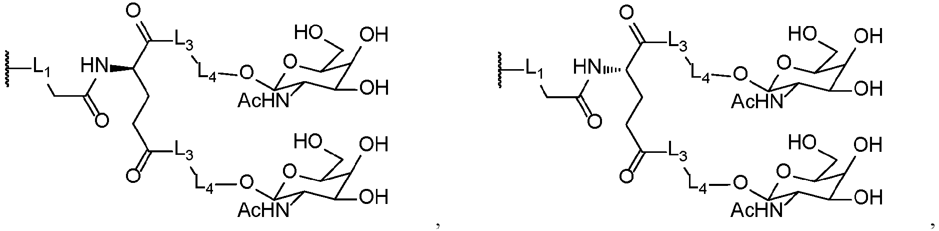 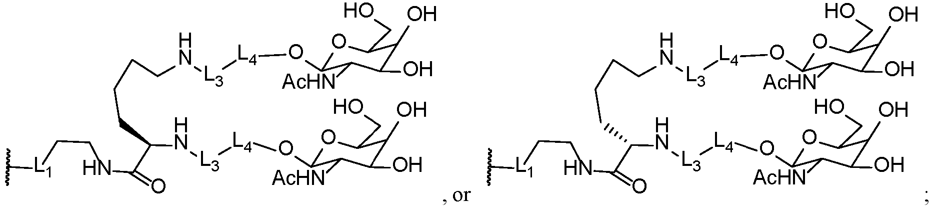
where L₁ is -CH₂O-, -CH₂O-CH₂CH₂O-, or -NHC(O)-(CH₂NHC(O))ₐ-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

38. The compound, or the pharmaceutically acceptable salt, stereoisomer or isotope variant thereof according to the technical solution 37, having Formula (II-1) or Formula (II-2): 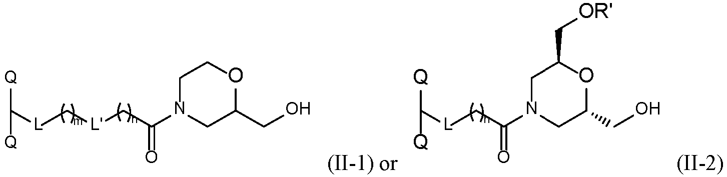 where,
Q is independently 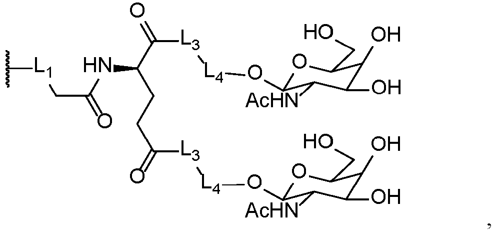 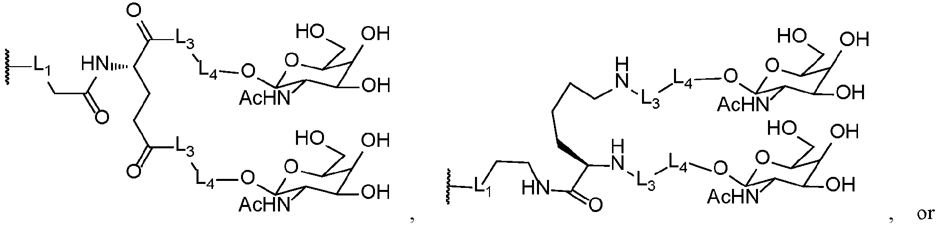 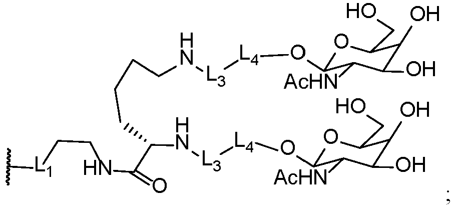
where L₁ is -CH₂O- or -CH₂O-CH₂CH₂O-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1, 2, 3, 4, 5, 6, 7, or 8;
L is -NHC(O)-;
L' is bond or -C(O)NH-;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

39. The compound, or the pharmaceutically acceptable salt, stereoisomer or isotope variant thereof according to the technical solution 34, where,
Q is independently H, 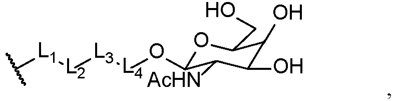 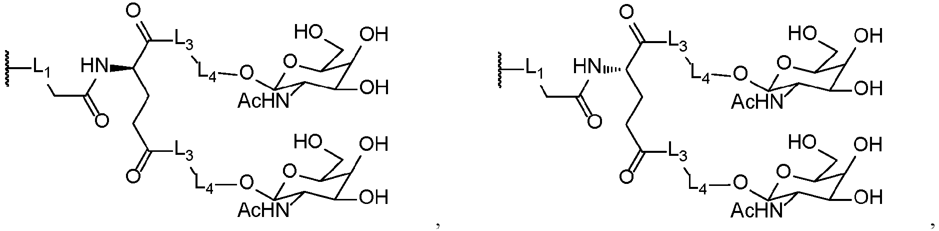 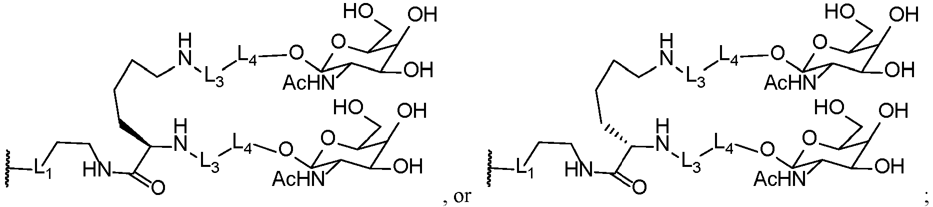
where L₁ is -CH₂-, -C(O)-, -CH₂O-, -CH₂O-CH₂CH₂O-, or -NHC(O)-(CH₂NHC(O))ₐ-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

40. The compound, or the pharmaceutically acceptable salt, stereoisomer or isotope variant thereof according to the technical solution 39, having Formula (II-2): 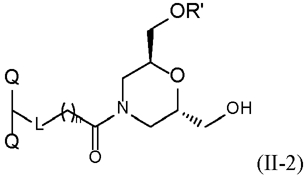 where,
Q is 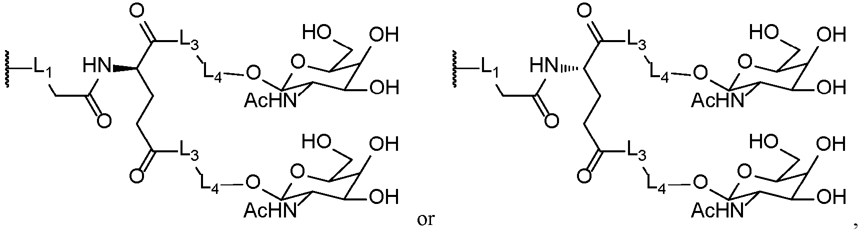 preferably 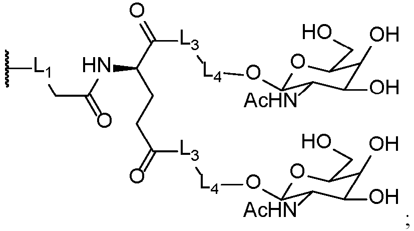
L₁ is -CH₂O-;
L₃ is -NHCH₂CH₂-;
L₄ is -(OCH₂CH₂)_{c}-,
c is 1, 2, or 3;
L is -NHC(O)-;
R' is H; and
n is 10 to 30, preferably 15 to 25, more preferably 8.

41. The compound, or the pharmaceutically acceptable salt, stereoisomer or isotope variant thereof according to the technical solution 39, having Formula (II-2): 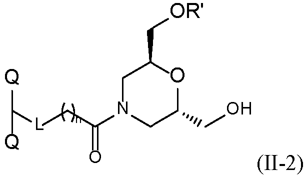 where,
Q is independently 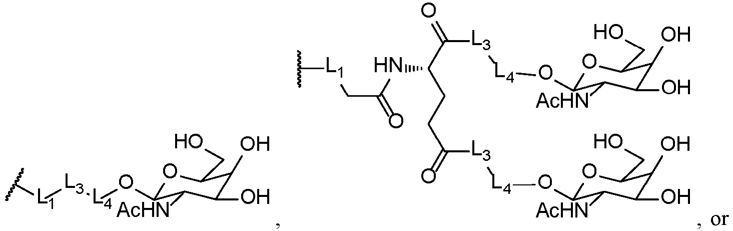 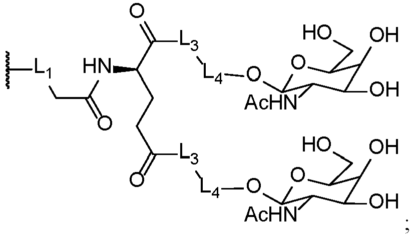 where L₁ is -CH₂- or -C(O)-;
L₃ is -(NHCH₂CH₂)_{b}-,
L₄ is -(OCH₂CH₂)_{c}-,
where b is 1, 2, 3, 4, or 5;
c is 1, 2, 3, 4, or 5;
L is -CH₂O- or -NHC(O)-;
R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl; and
nis0, 1, 2, 3, 4, 5, 6, 7, 8, 9, or 10.

42. The compound, or the pharmaceutically acceptable salt, stereoisomer or isotope variant thereof according to the technical solution 41, having Formula (II-3): 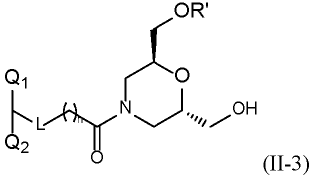 where,
Q₁ is 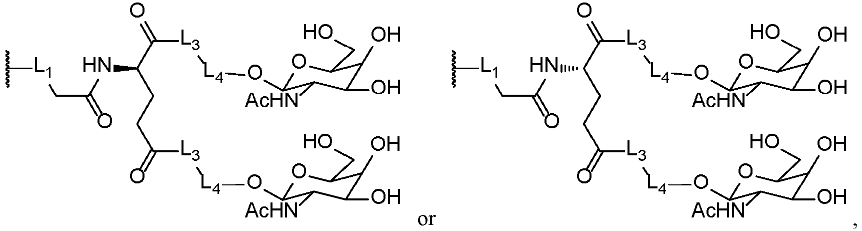 preferably 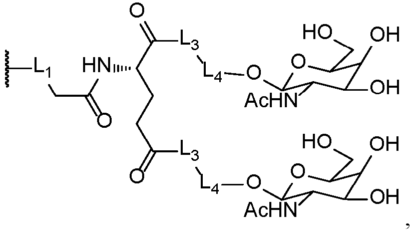 where L₁ is -CH₂-;
Q₂ is 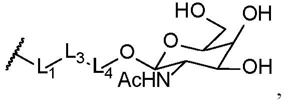 where L₁ is -C(O)-;
L₃ is -NHCH₂CH₂-;
L₄ is -(OCH₂CH₂)_{c}-,
c is 1, 2, or 3;
L is -NHC(O)-;
R' is H; and
n is 10 to 30, preferably 15 to 25, more preferably 8.

43. The compound, or the pharmaceutically acceptable salt, stereoisomer or isotope variant thereof according to the technical solution 33, where,
Q is independently H, 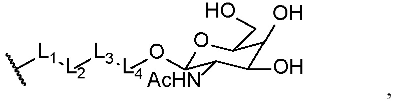 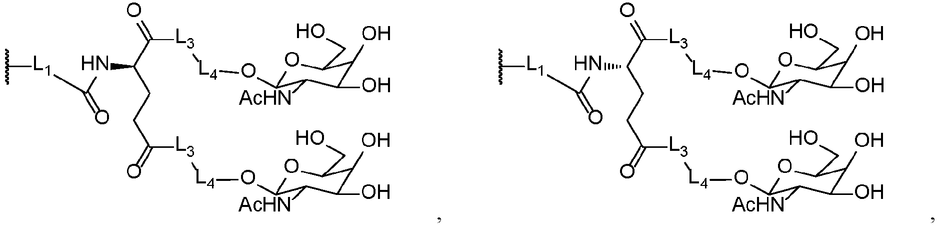 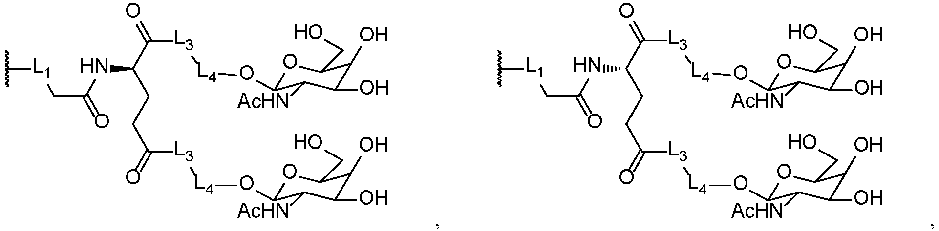 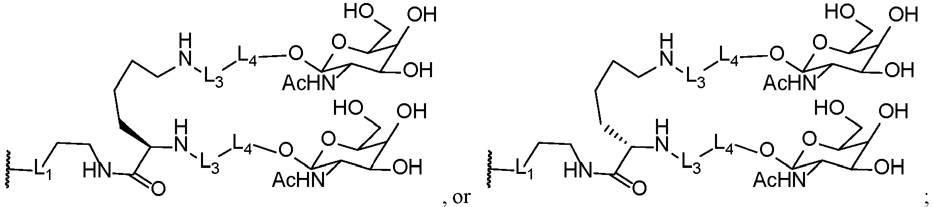
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
where the e is 1, 2, 3, 4, or 5;
T is bond, -CH₂-, -M-, -CH₂-M-, or -C(O)-M-;
where the M is 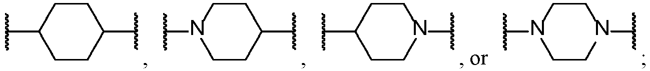
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

44. The compound, or the pharmaceutically acceptable salt, stereoisomer or isotope variant thereof according to the technical solution 43, where T is -M-, -CH₂-M-, or -C(O)-M-, and the M is 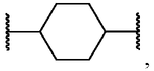 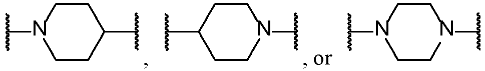
45. The compound, or the pharmaceutically acceptable salt, stereoisomer or isotope variant thereof according to the technical solution 43 or 44, where,
Q is independently H or 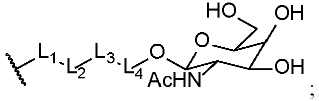
where L₁ is -CH₂O- or -NHC(O)-(CH₂NHC(O))ₐ-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
where a is 0, 1, 2, or 3;
b is 1, 2, 3, 4, or 5;
c is 1, 2, 3, 4, or 5;
the d is 1, 2, 3, 4, 5, 6, 7, or 8;
L is bond or -CH₂O-;
L' is bond or -O(CH₂CH₂O)ₑ-;
where the e is 1, 2, 3, 4, or 5;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to the technical solution 43 or 44.

46. The compound, or the pharmaceutically acceptable salt, stereoisomer or isotope variant thereof according to the technical solution 45, having Formula (III-1), Formula (III-2), or Formula (III-3): 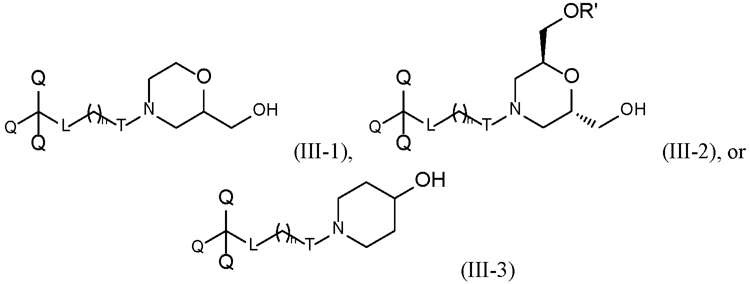 where,
Q is 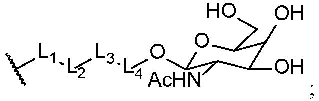
where L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1,2, 3, 4, 5, 6, 7, or 8;
L is bond or -CH₂O-;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
n is 0, 1, 2, 3, 4, 5, 6, 7, 8, 9, or 10; and
T is as defined according to the technical solution 43 or 44.

47. The compound, or the pharmaceutically acceptable salt, stereoisomer or isotope variant thereof according to the technical solution 43 or 44, where,
Q is independently H, 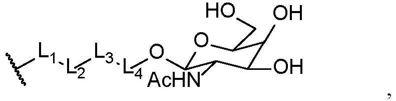 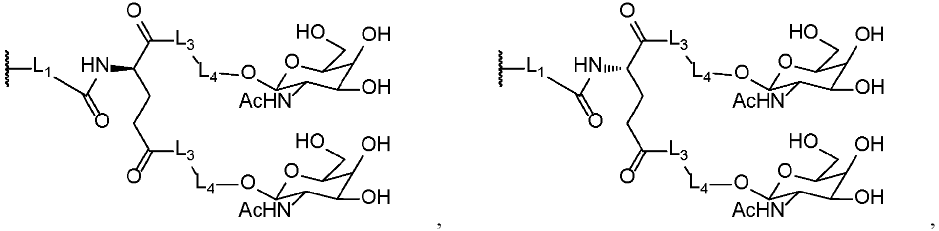 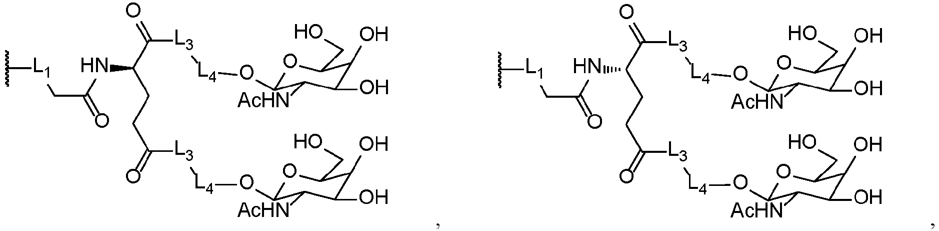 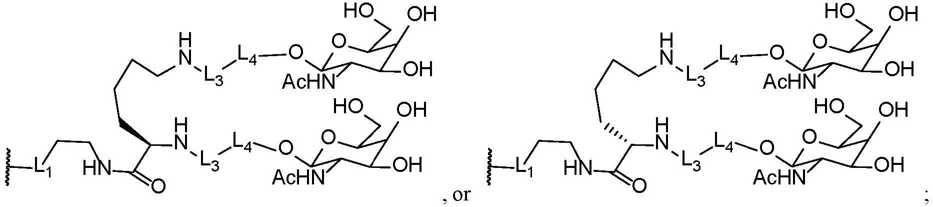
where L₁ is -CH₂-, -CH₂O-, or -C(O)-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1,2, 3, 4, 5, 6, 7, or 8;
L is bond or -NHC(O)-;
L' is bond;
R₁ and R₂ together form -CH₂CH₂O- or -CH₂CH(R)-O-, and R₃ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to the technical solution 43 or 44.

48. The compound, or the pharmaceutically acceptable salt, stereoisomer or isotope variant thereof according to the technical solution 43 or 44, having Formula (IV-1) or Formula (IV-2): 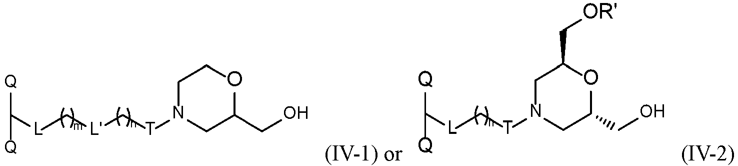 where,
Q is independently 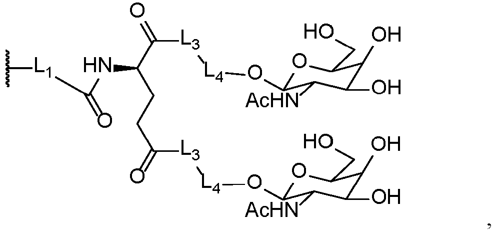 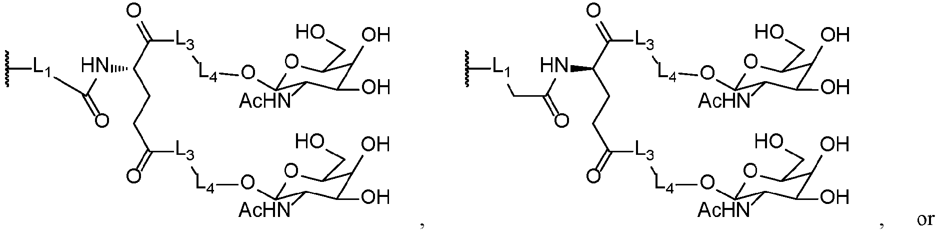 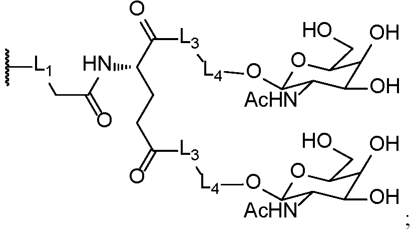
where L₁ is -CH₂-, -CH₂O-, or -C(O)-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
where b is 1, 2, 3, 4, or 5;
c is 1, 2, 3, 4, or 5;
d is 1,2, 3, 4, 5, 6, 7, or 8;
L is bond or -NHC(O)-;
L' is bond;
R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to the technical solution 43 or 44.

49. The compound, or the pharmaceutically acceptable salt, stereoisomer or isotope variant thereof according to the technical solution 33, having Formula (II-3): 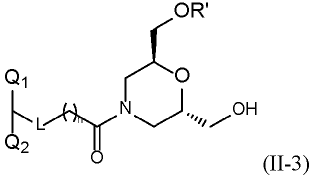 where,
Q₁ is 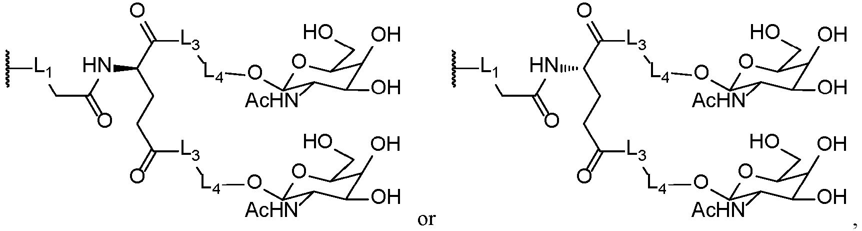 preferably 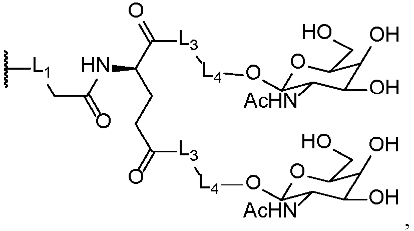 where L₁ is -CH₂-;
Q₂ is 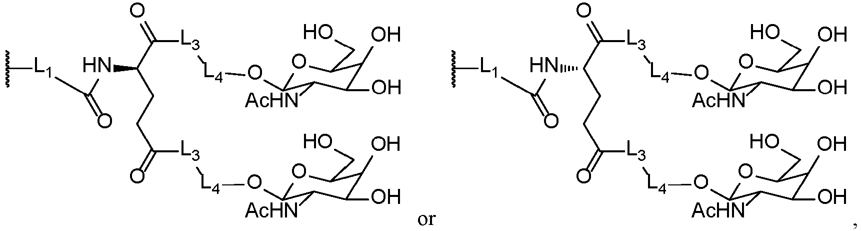 preferably 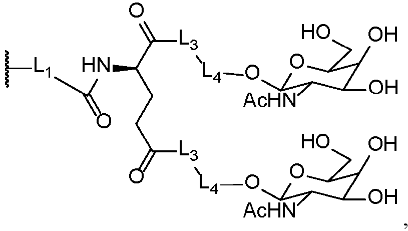 where L₁ is bond;
L₃ is -NHCH₂CH₂-;
L₄ is -(OCH₂CH₂)_{c}-,
c is 1, 2, or 3;
L is -NHC(O)-;
R' is H; and
n is 10 to 30, preferably 15 to 25, more preferably 8.

50. The compound, or the pharmaceutically acceptable salt, stereoisomer or isotope variant thereof according to the technical solution 33, where,
Q is independently H, 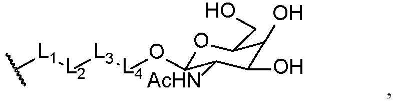 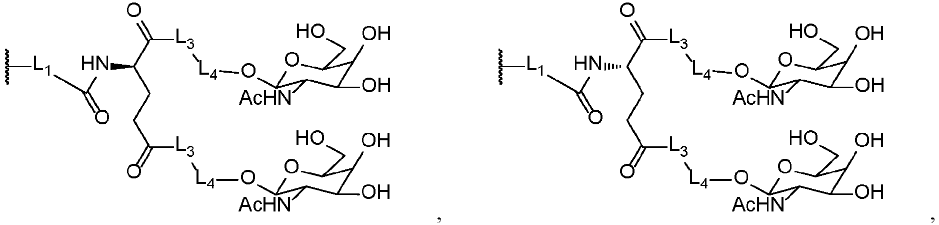 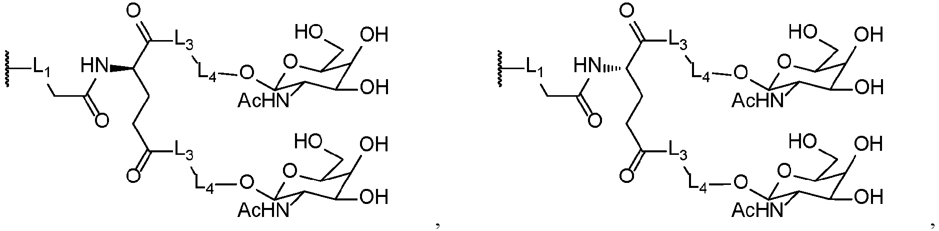 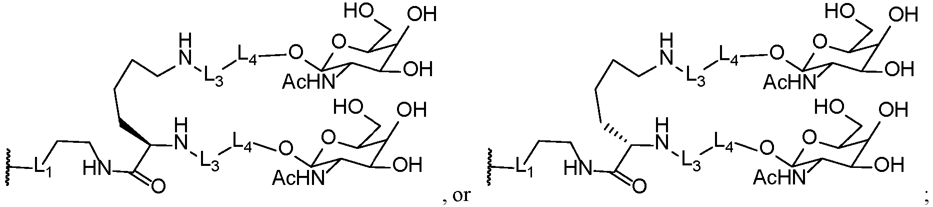
where L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is -O(CH₂CH₂O)ₑ-;
where the e is 1, 2, 3, 4, or 5;
T is bond, -CH₂-, -C(O)-, -M-, -CH₂-M-, or -C(O)-M-;
where the M is 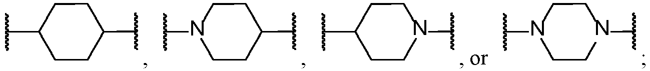
R₁ and R₂ together form -CH₂CH₂O- or -CH₂CH(R)-O-, and R₂ is H;
or R₁ and R₃ together form -C₁₋₂ alkylene-, and R₂ is H;
where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

51. The compound, or the pharmaceutically acceptable salt, stereoisomer or isotope variant thereof according to the technical solution 33, where the compound is selected from:

| Num ber | Structure |
|---|---|
| 1 | 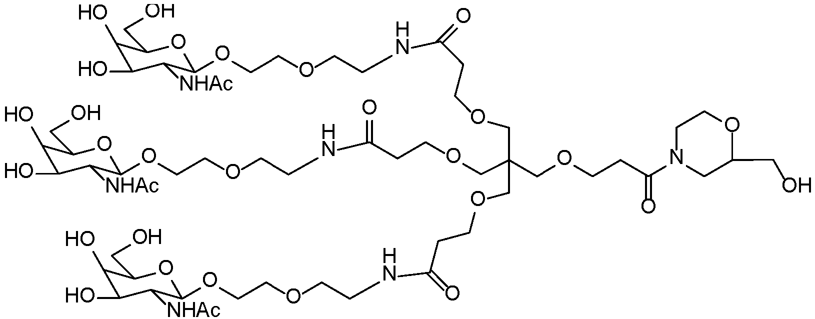 |
| 2 | 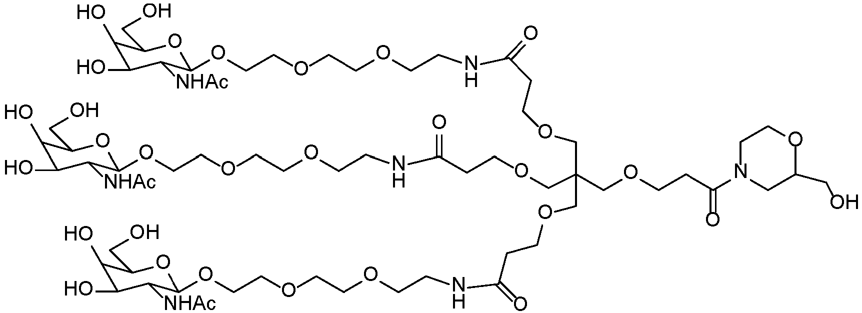 |
| 3 | 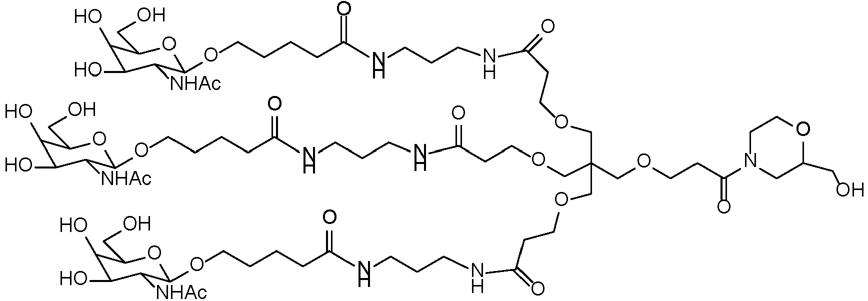 |
| 4 | 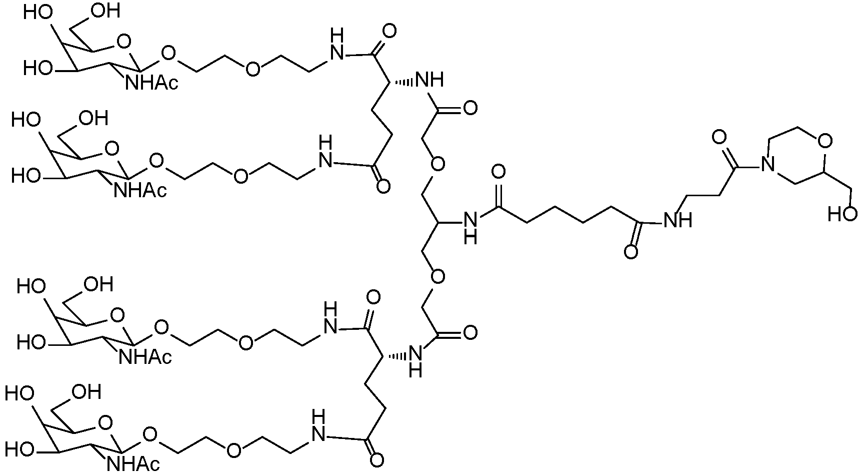 |
| 5 | 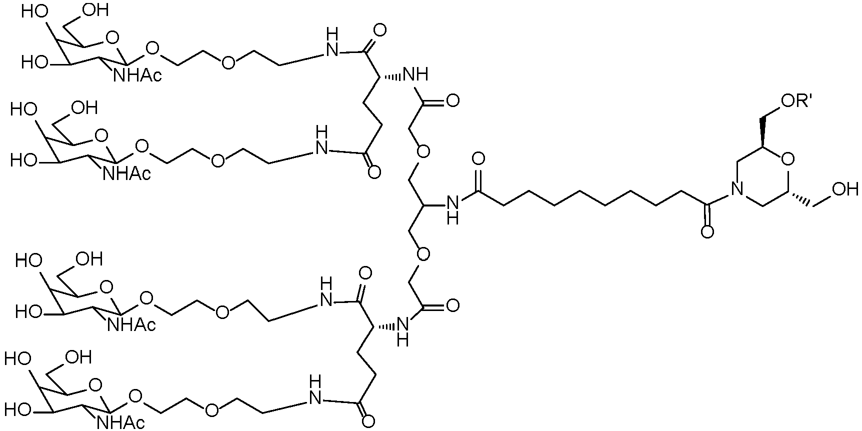 |
| 6 | 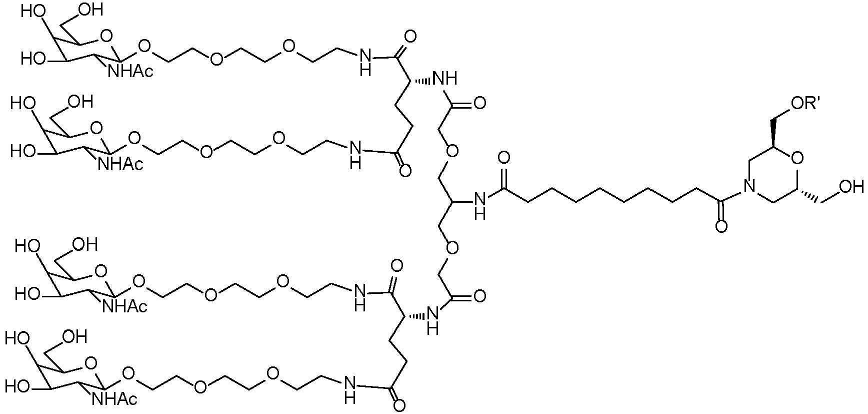 |
| 7 | 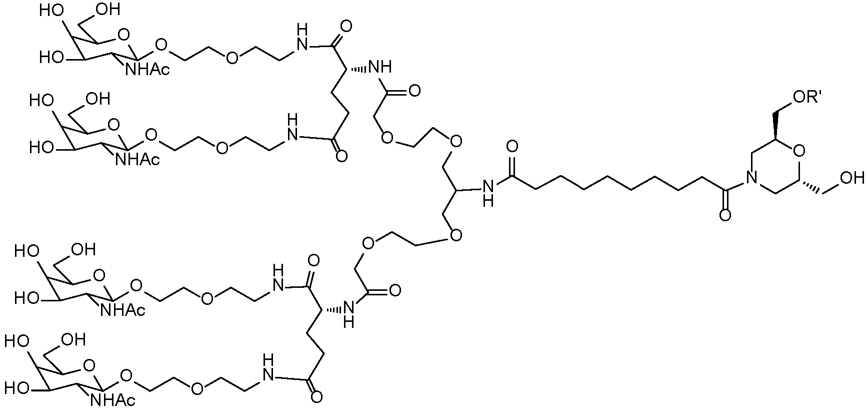 |
| 8 | 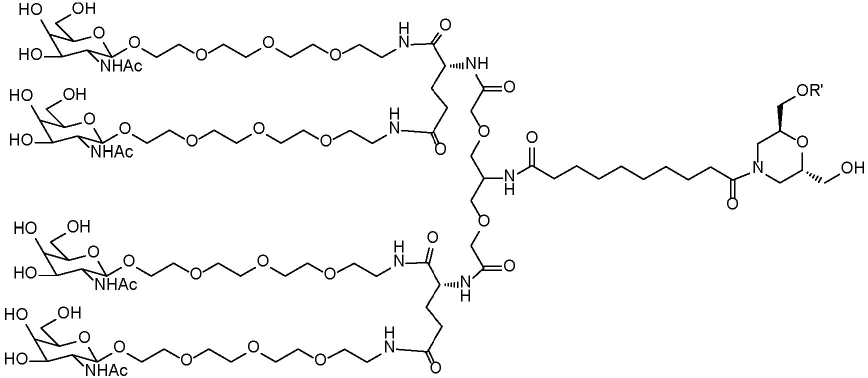 |
| 9 | 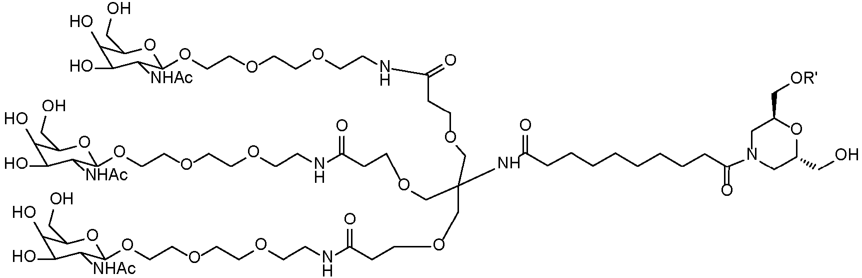 |
| 10 | 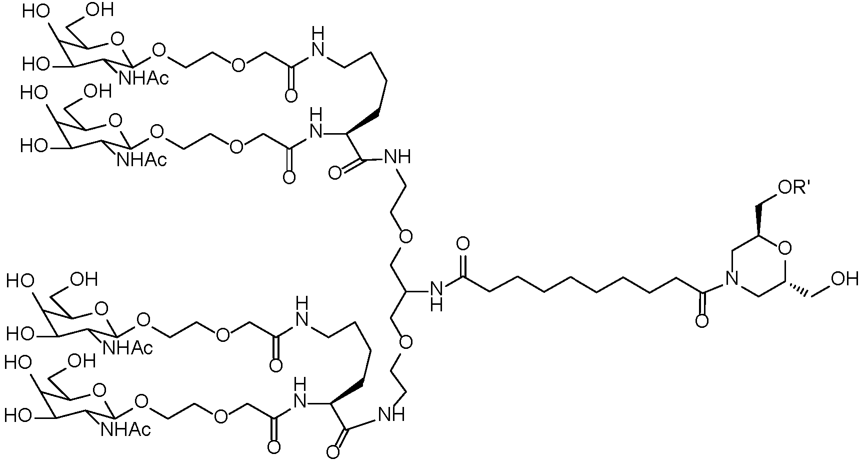 |
| 11 | 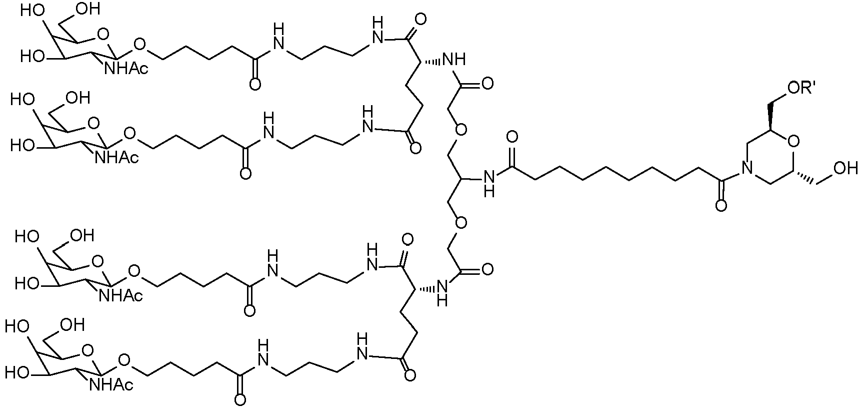 |
| 12 | 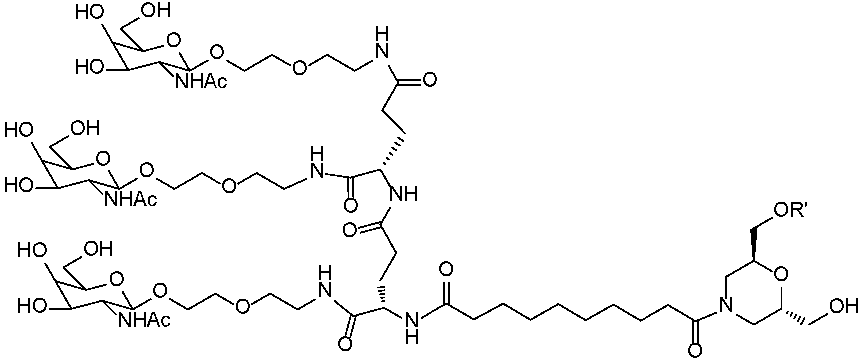 |
| 13 | 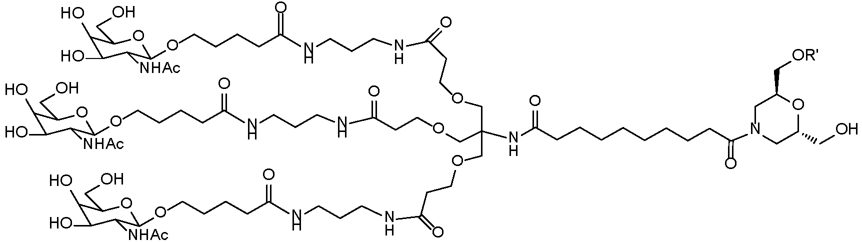 |
| 14 | 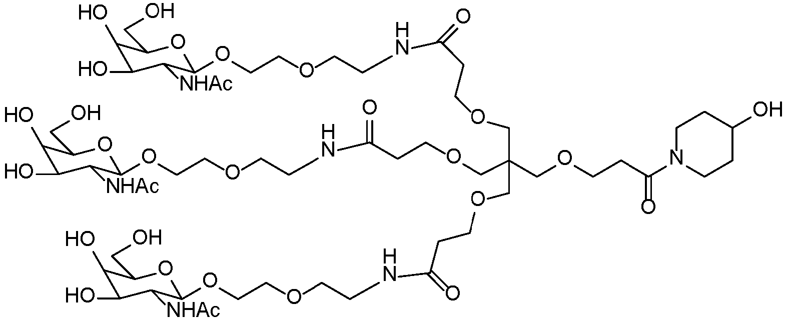 |
| 15 | 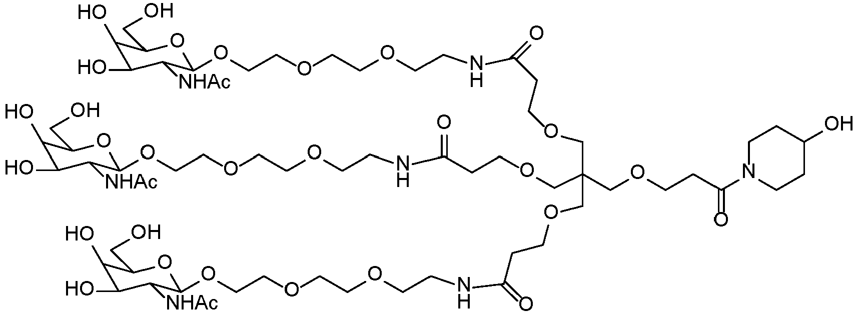 |
| 16 | 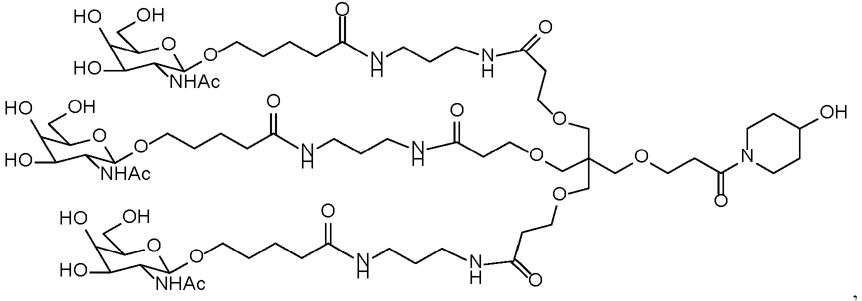 |

where R' is H, -C(O)CH₂CH₂C(O)OH, or 4,4'-dimethoxytriphenylmethyl.
52. The compound, or the pharmaceutically acceptable salt, stereoisomer or isotope variant thereof according to the technical solution 33, where the compound is selected from:

| Number | Structure |
|---|---|
| 17 | 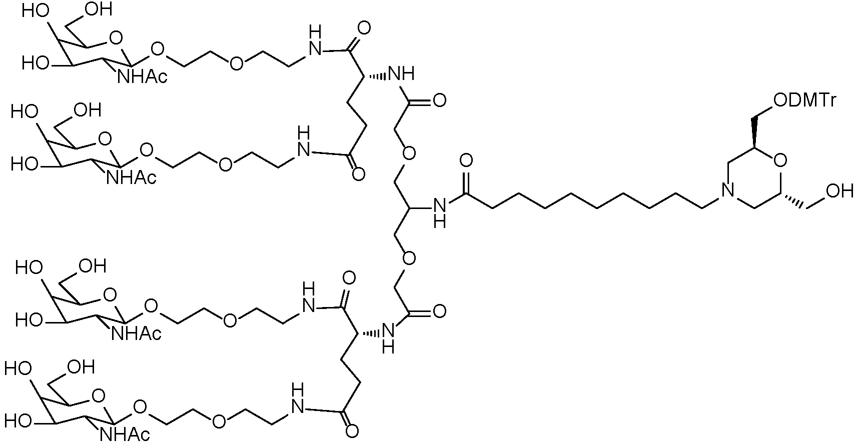 |
| 18 | 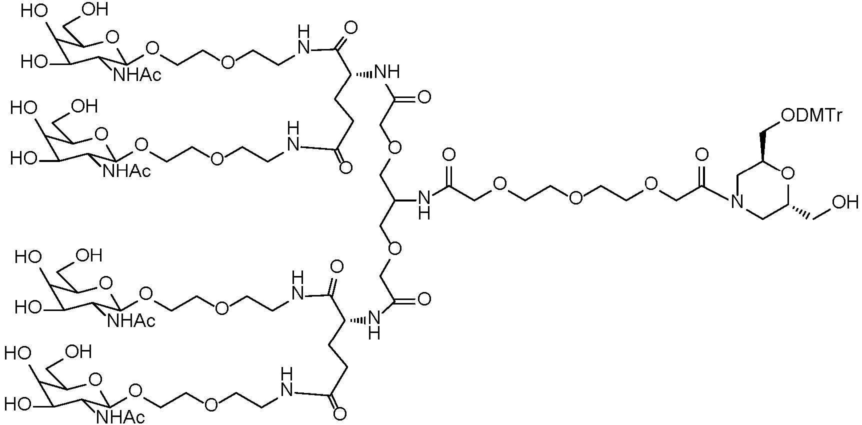 |
| 19 | 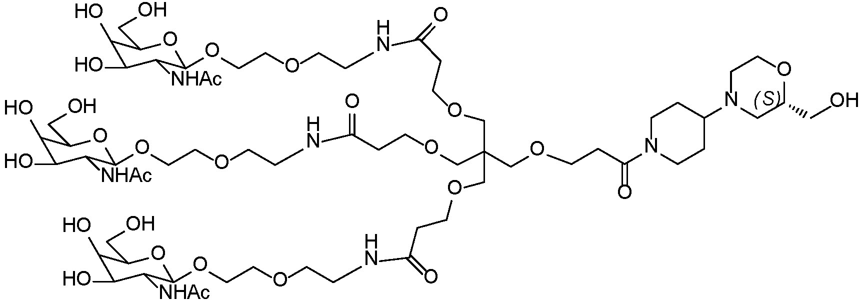 |
| 20 | 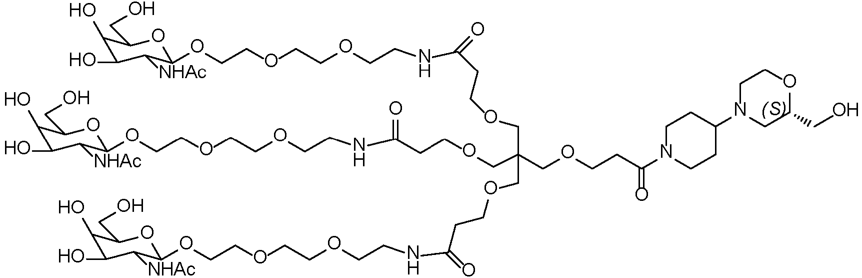 |
| 21 | 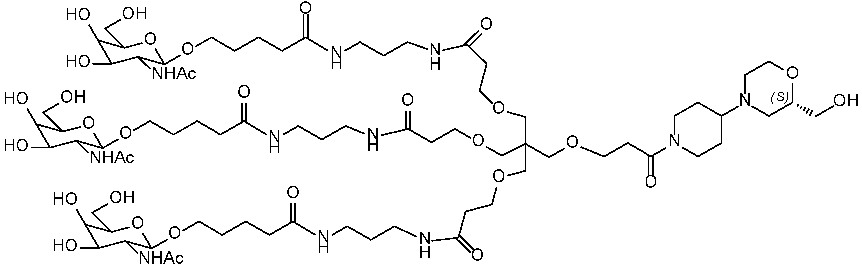 |
| 22 | 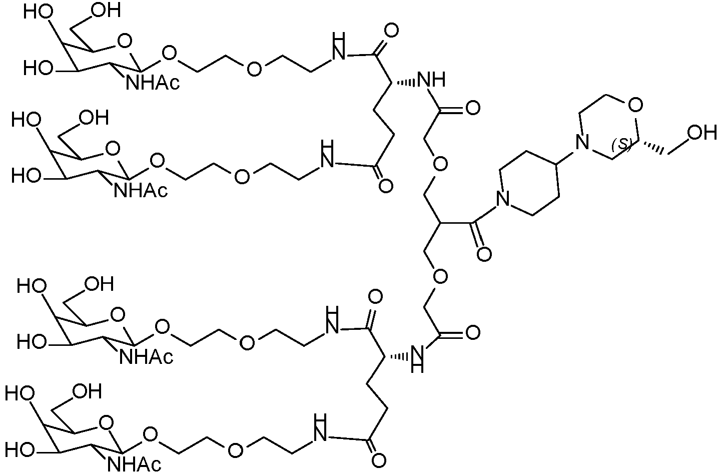 |
| 23-1 | 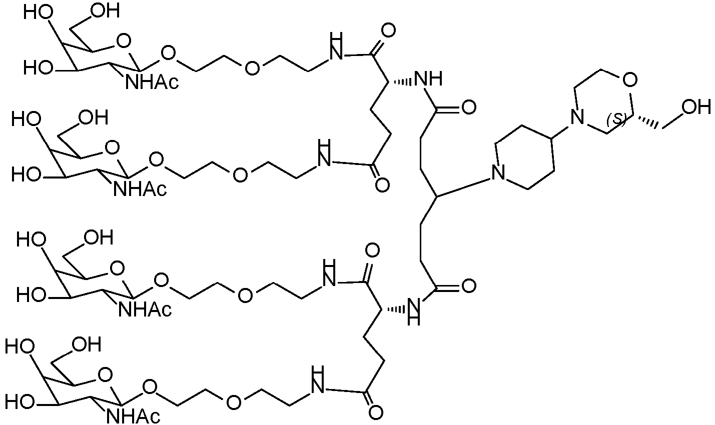 |
| 23-2 | 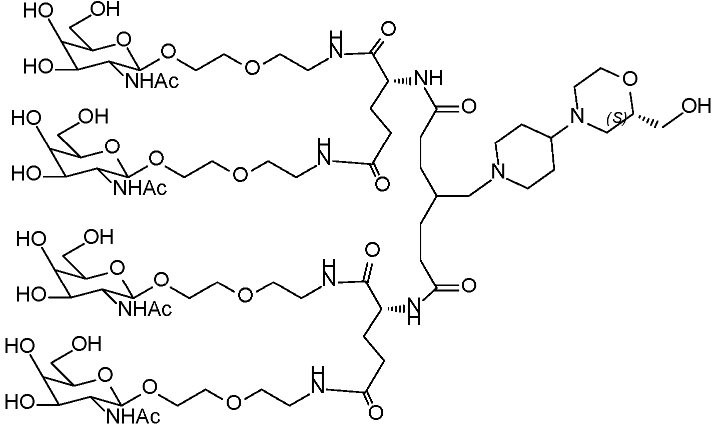 |
| 24 | 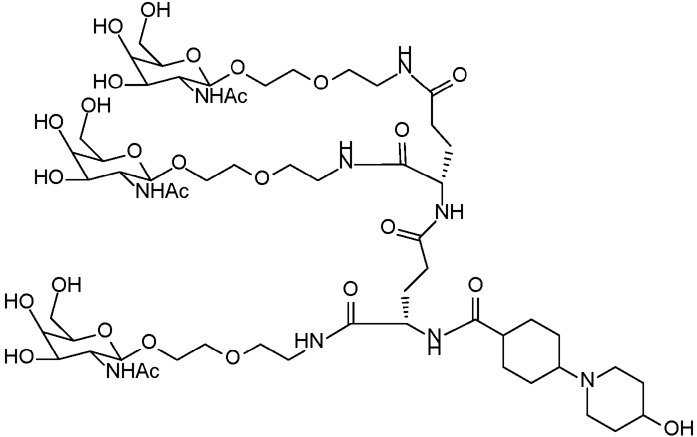 |
| 25 | 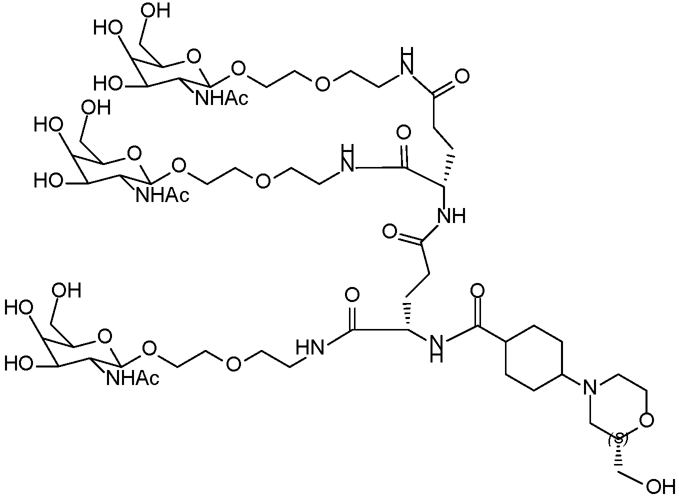 |
| 26 | 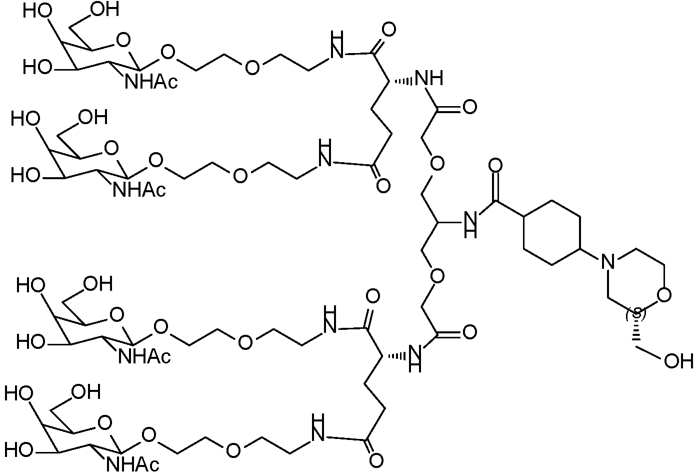 |
| 27 | 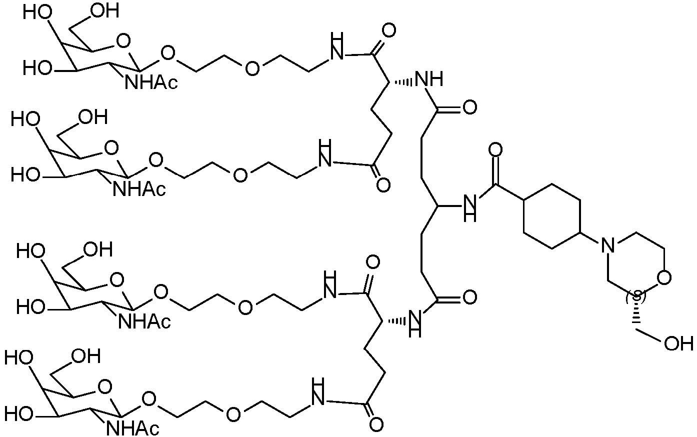 |
| 28 | 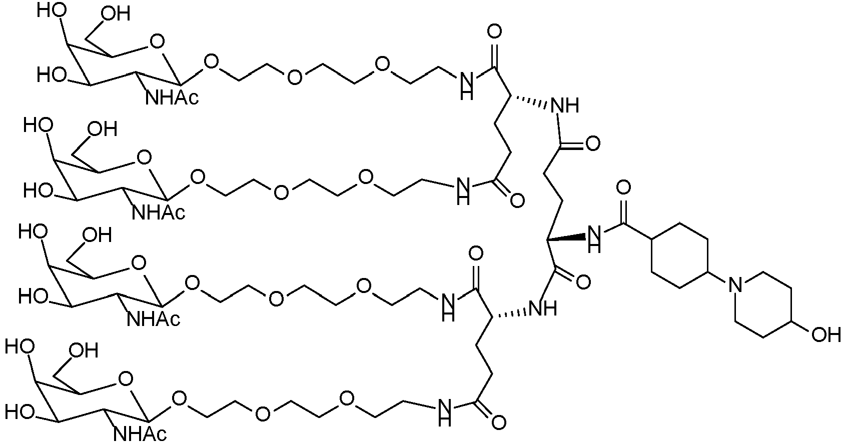 |
| 29 | 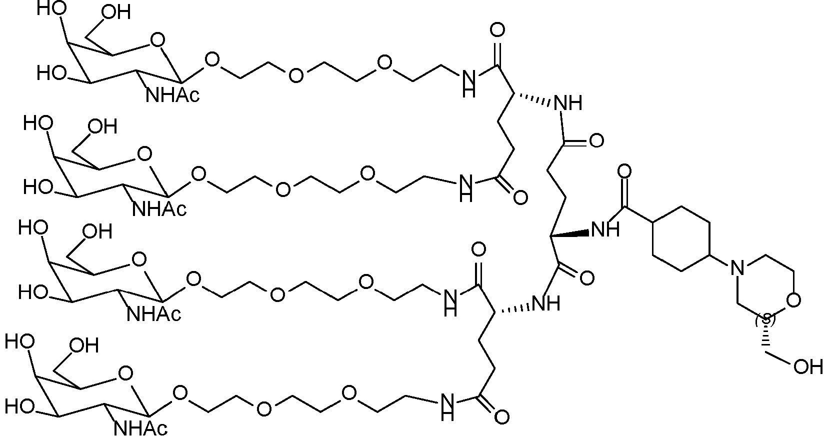 |
| 30 | 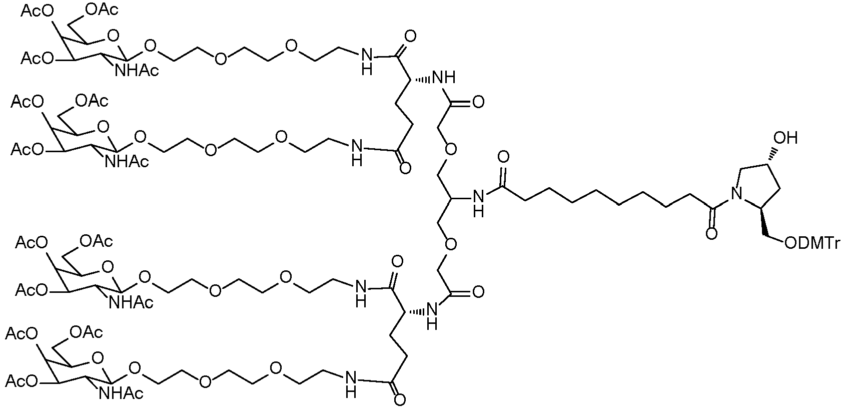 |
| 31 | 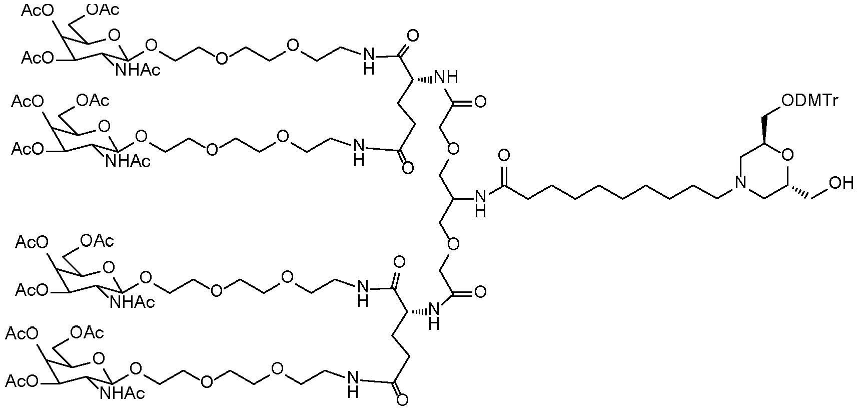 |
| 32 | 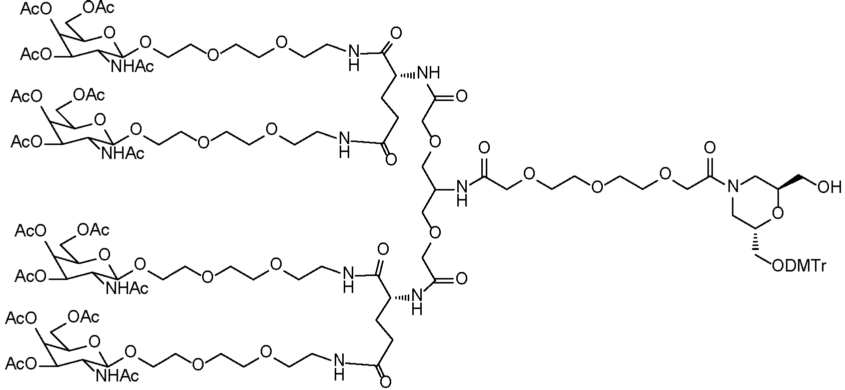 |
| 33 | 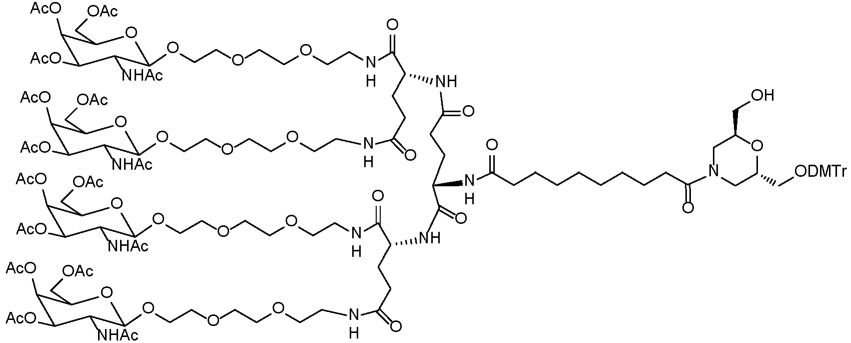 |

53. An intermediate compound, being a compound of Formula (V): 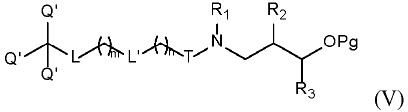 where,
Pg is a hydroxyl protective group, for example, selected from the following: 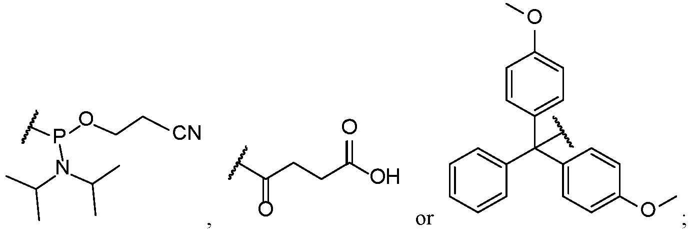
Q' is independently H, 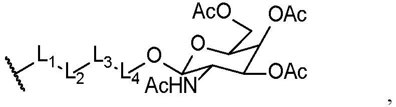 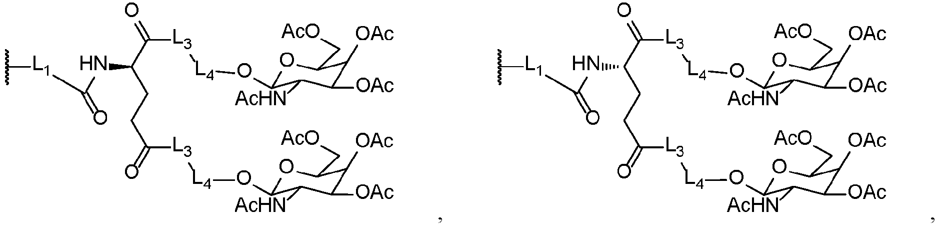 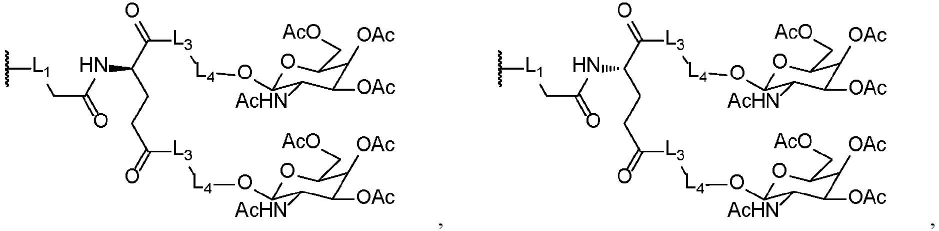 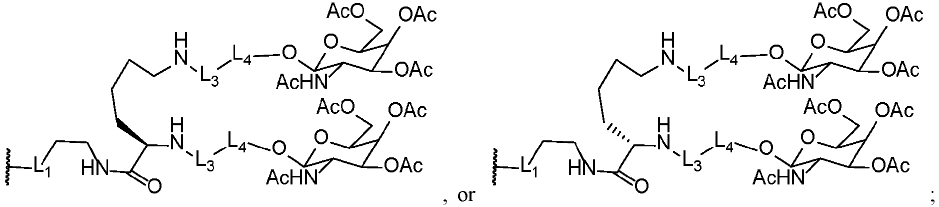 and
other groups are as defined according to any one of the technical solutions 33-50.

54. An intermediate compound, selected from:

| Num ber | Structure |
|---|---|
| 1a | 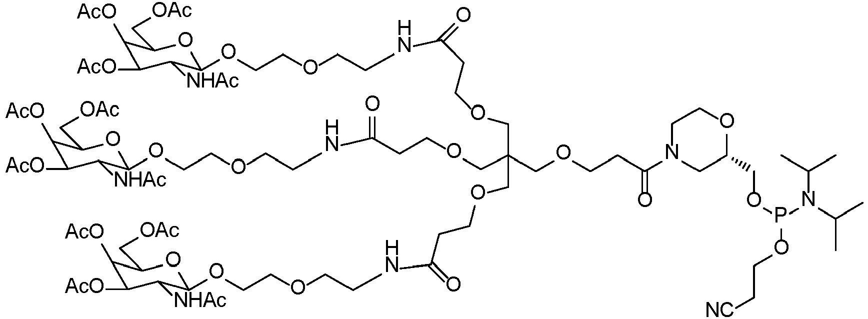 |
| 2a | 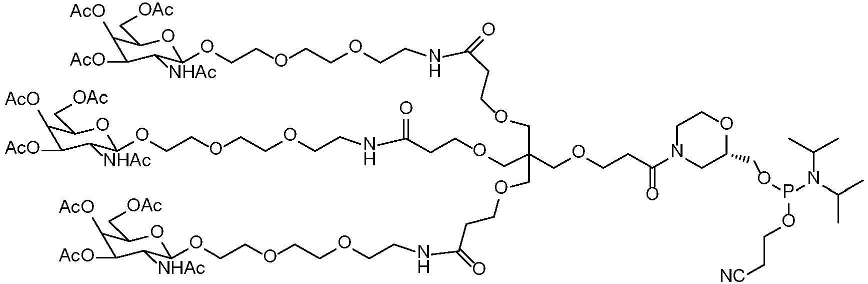 |
| 3a | 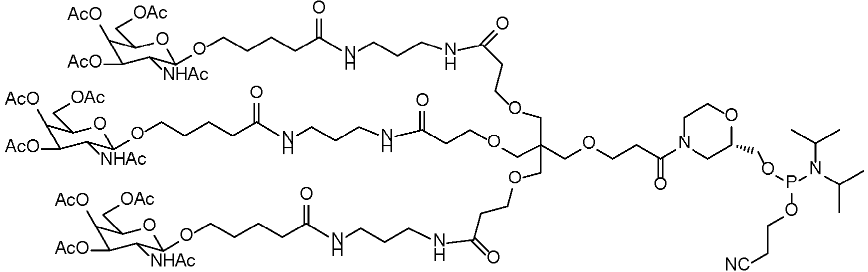 |
| 4a | 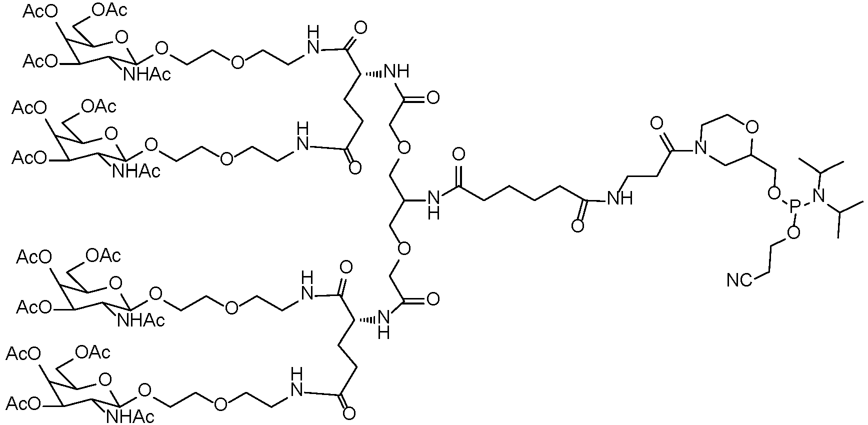 |
| 5a | 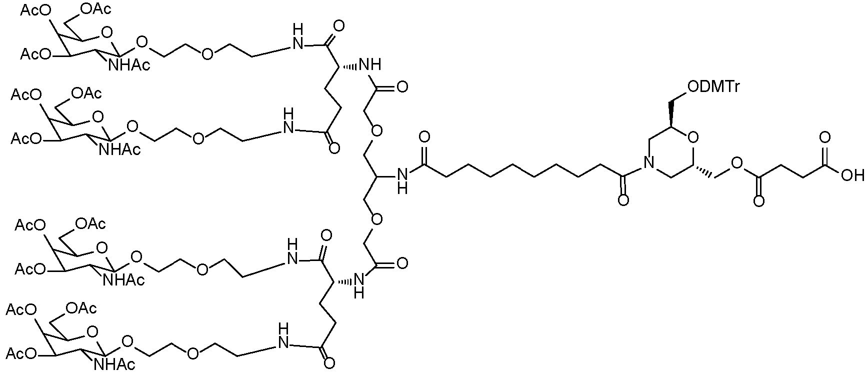 |
| 6a | 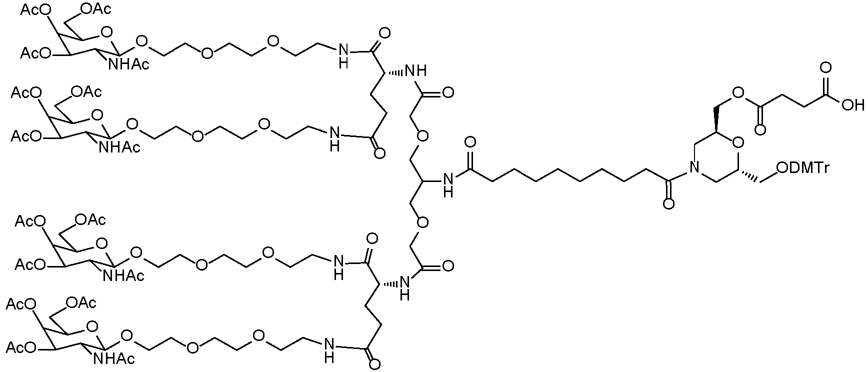 |
| 7a | 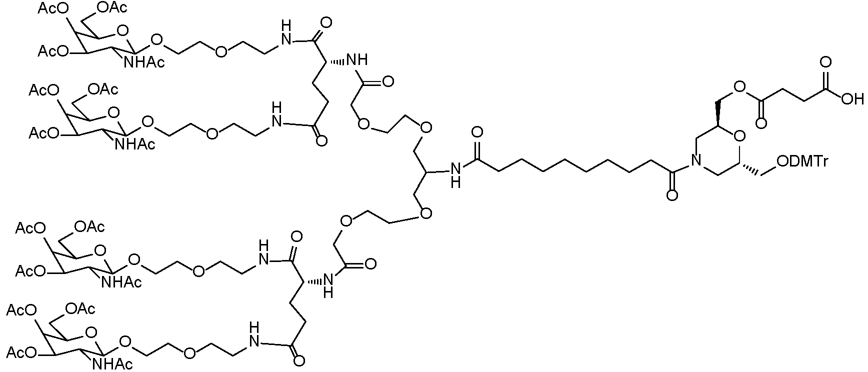 |
| 8a | 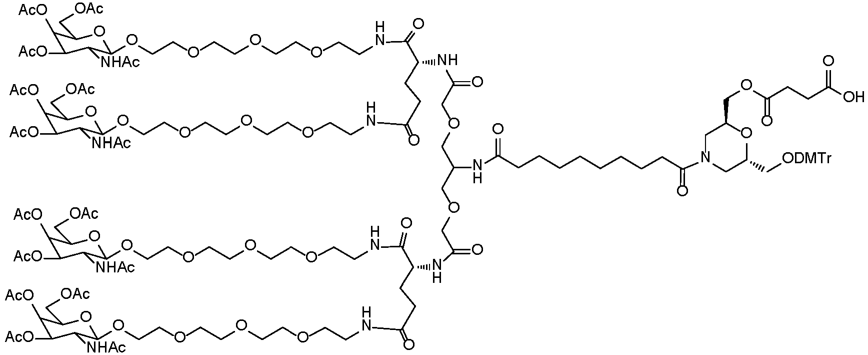 |
| 9a | 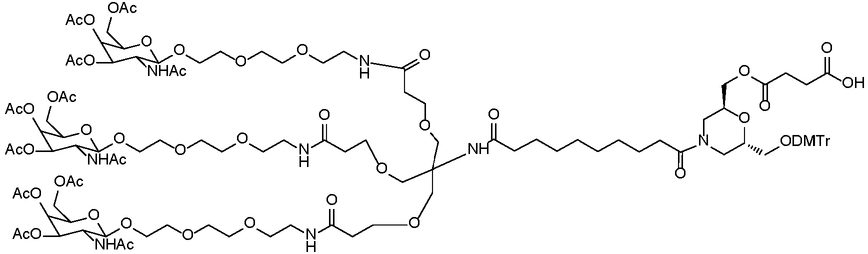 |
| 10a | 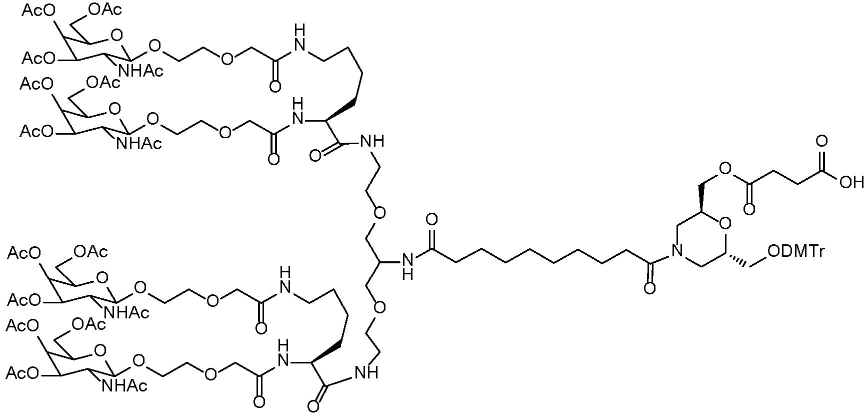 |
| 11a | 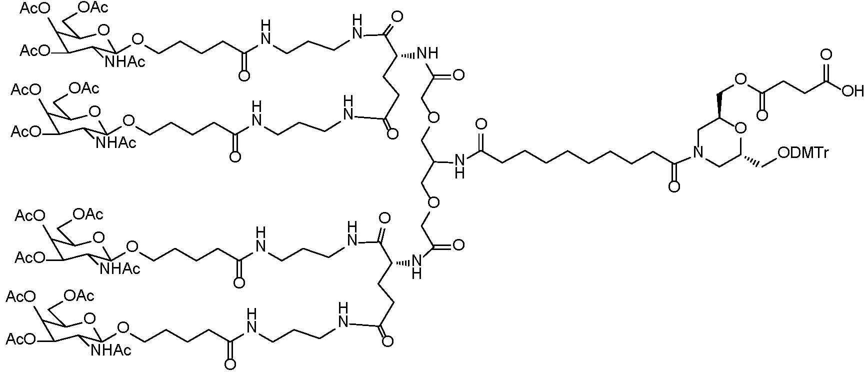 |
| 12a | 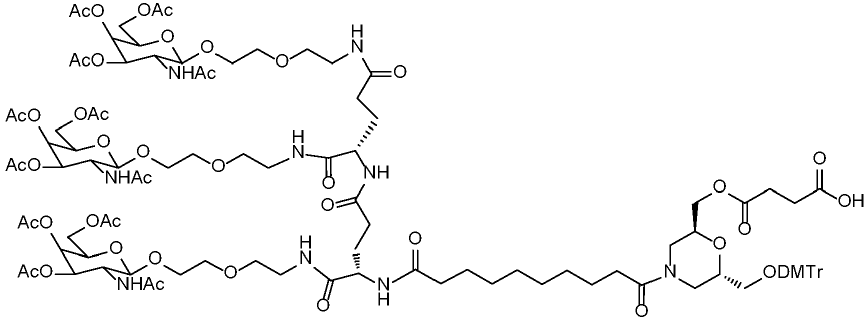 |
| 13a | 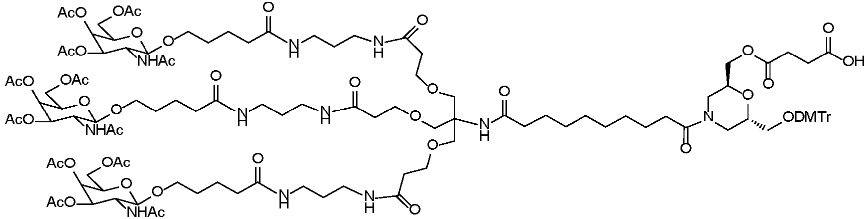 |
| 14a | 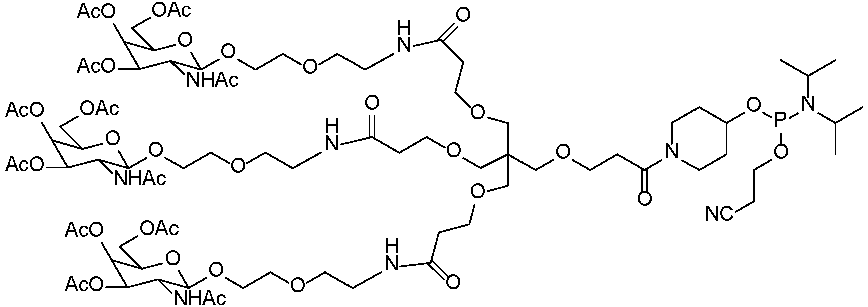 |
| 15a | 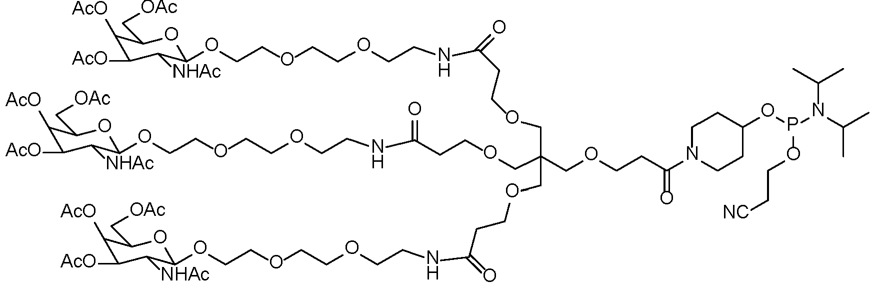 |
| 16a | 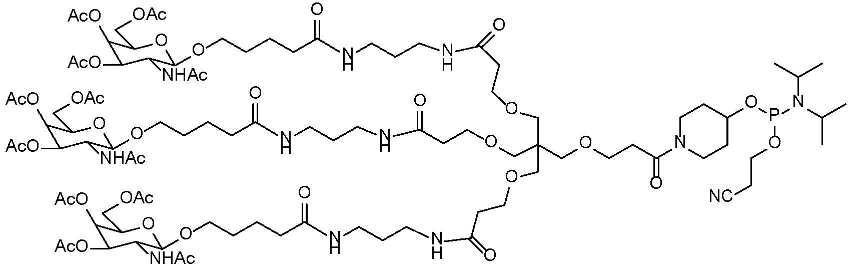 |

55. An intermediate compound, selected from:

| Num ber | Structure |
|---|---|
| 17a | 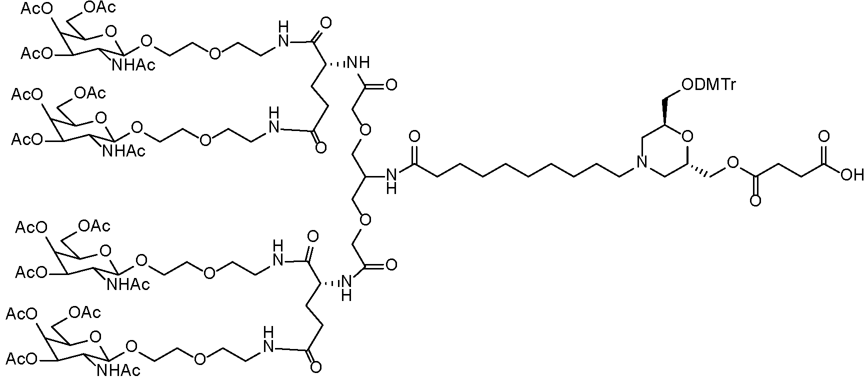 |
| 18a | 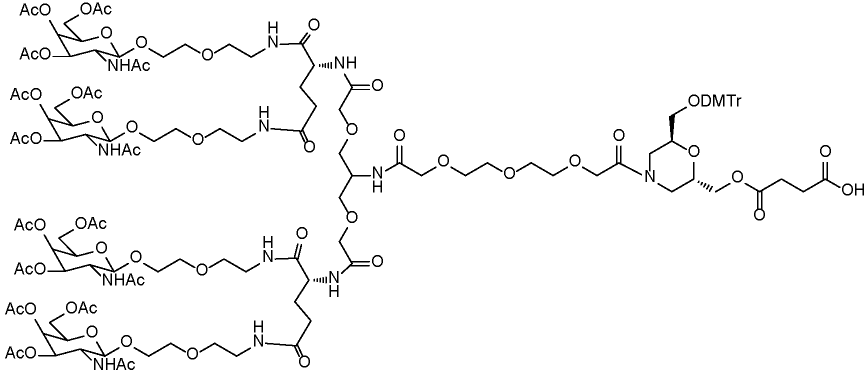 |
| 19a | 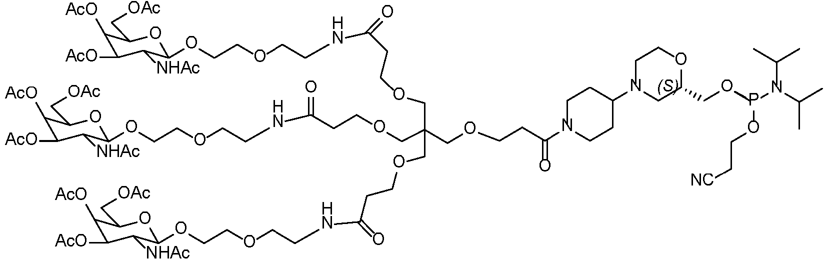 |
| 20a | 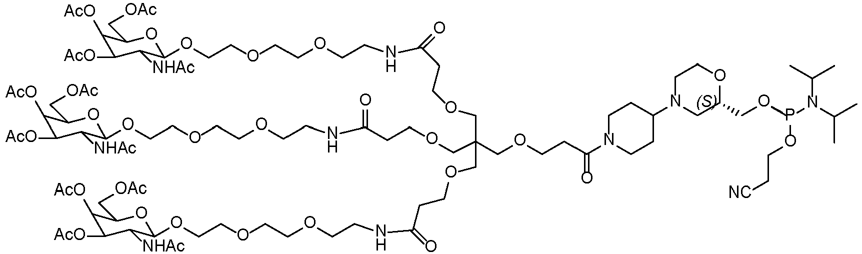 |
| 21a | 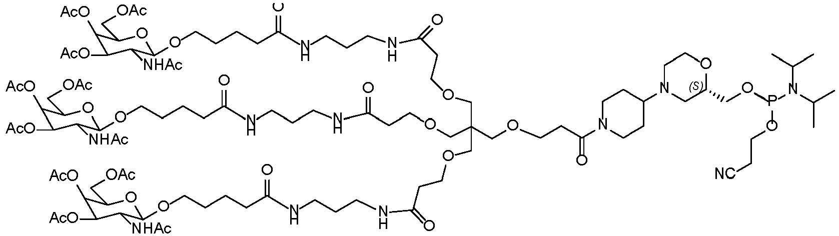 |
| 22a | 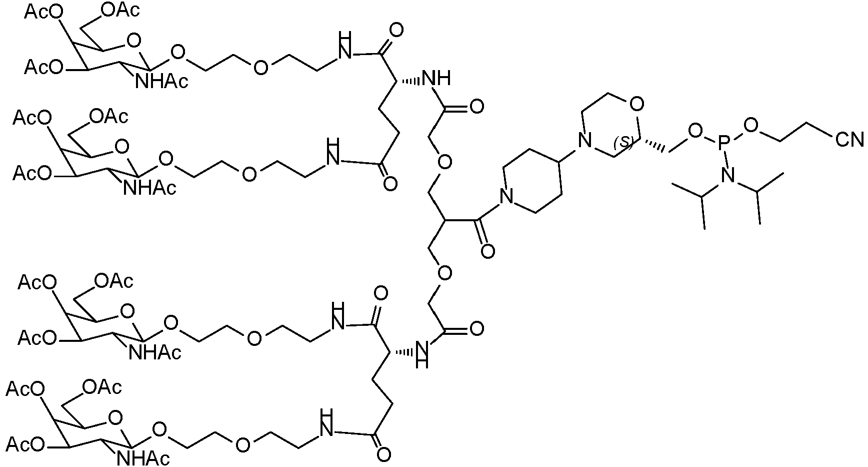 |
| 23 a-1 | 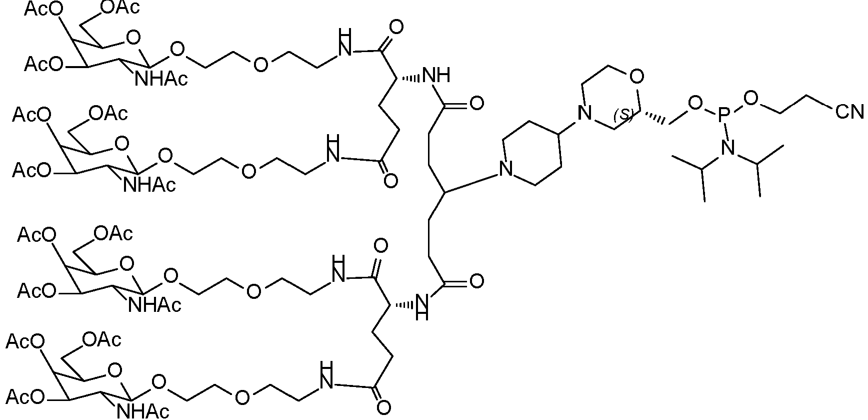 |
| 23 a-2 | 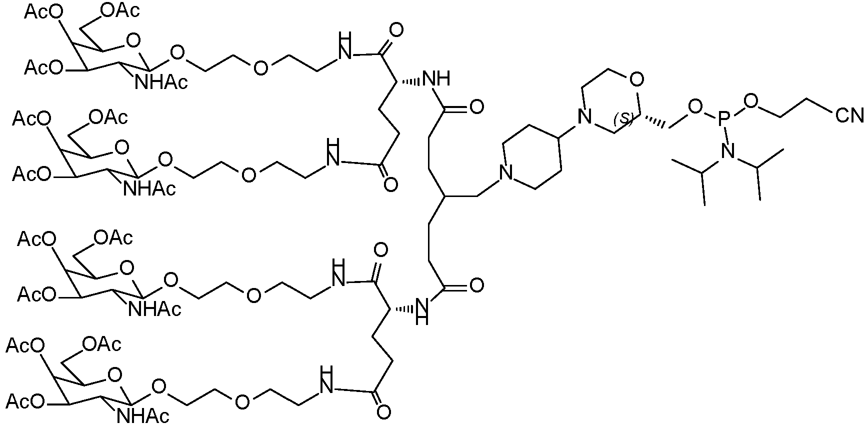 |
| 24a | 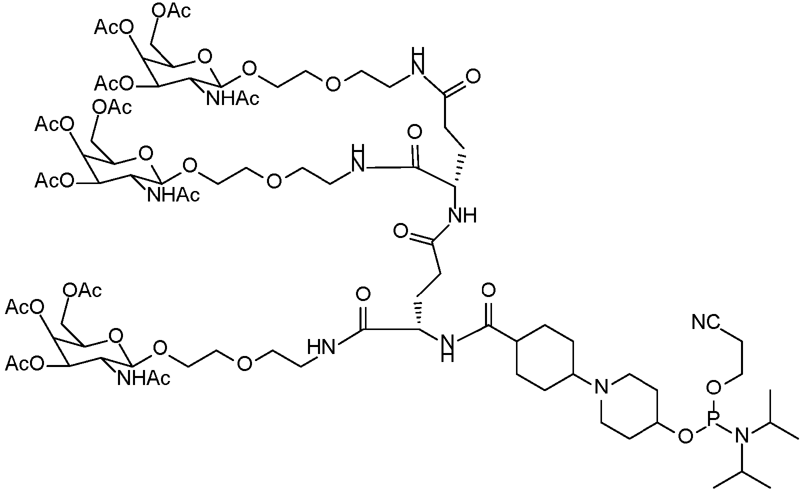 |
| 25a | 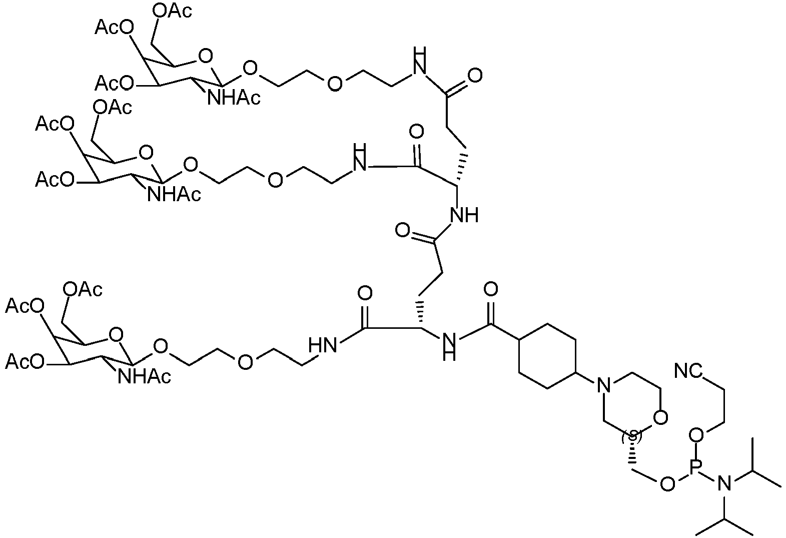 |
| 26a | 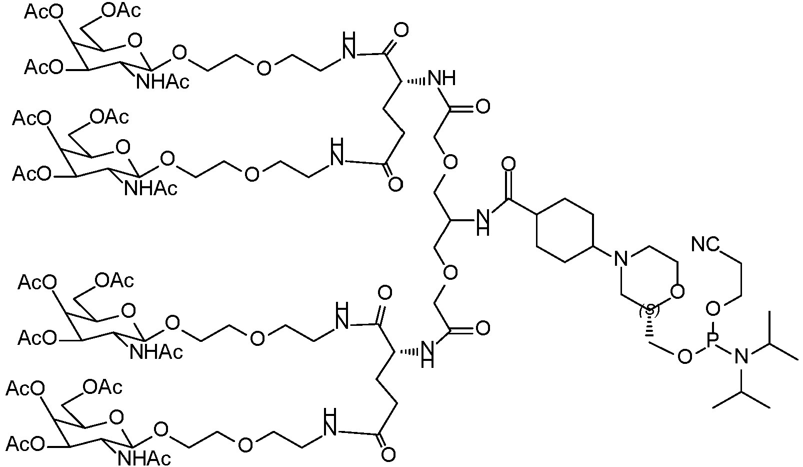 |
| 27a | 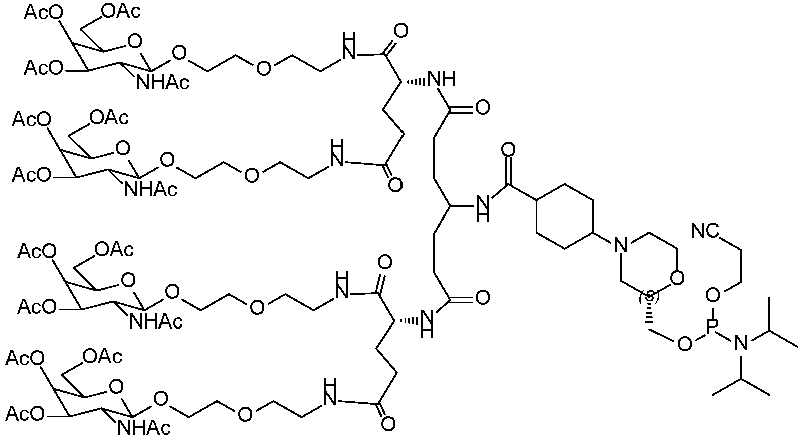 |
| 28a | 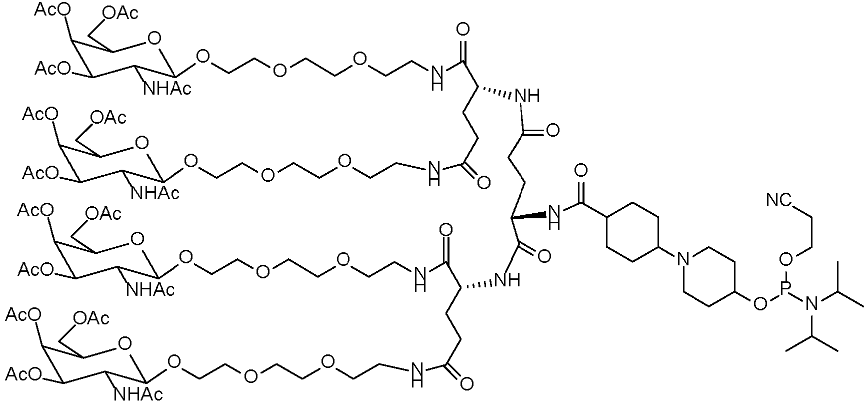 |
| 29a | 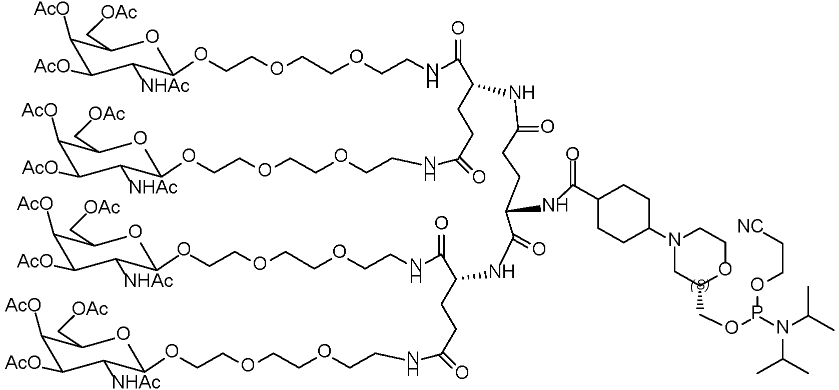 |
| 30a | 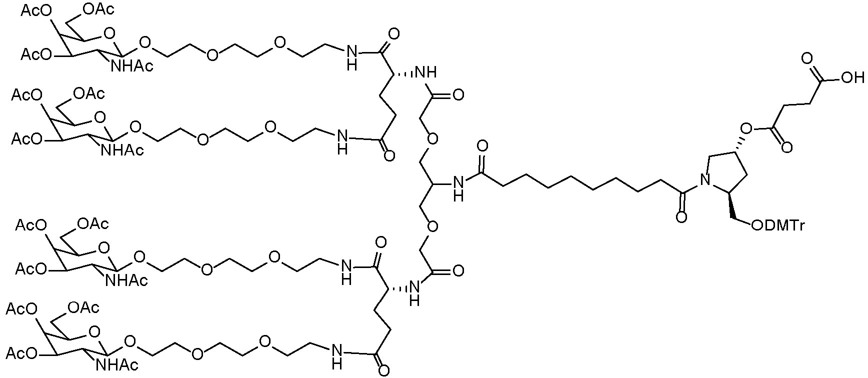 |
| 31a | 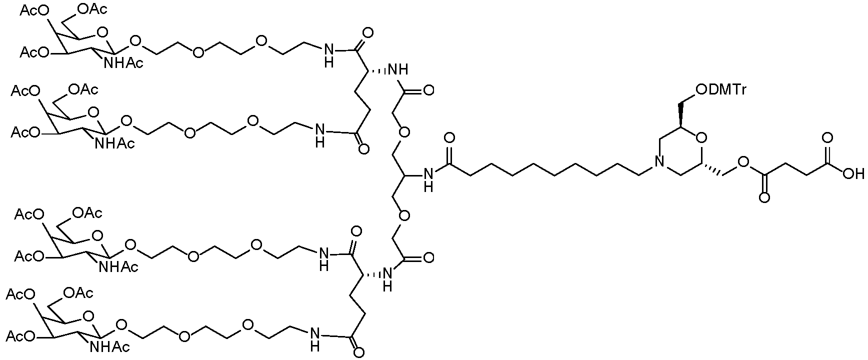 |
| 32a | 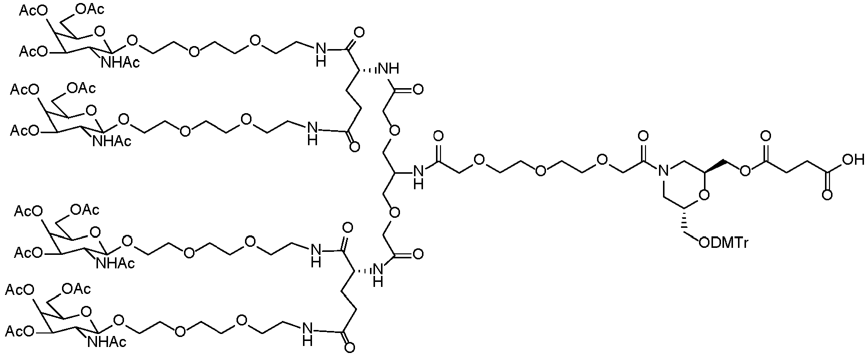 |
| 33a | 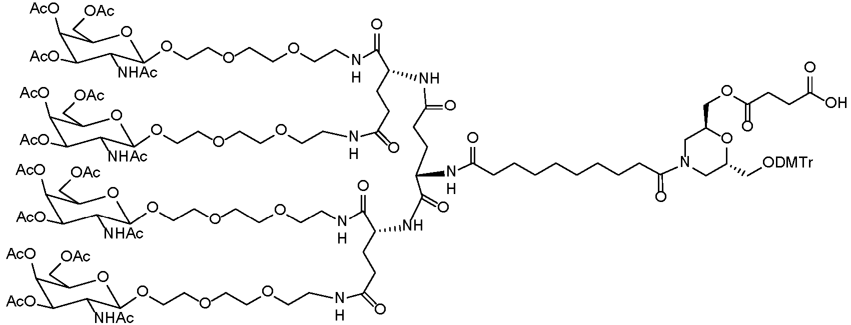 |

56. A method for preparing a compound of Formula (I), including treating a compound of Formula (V) under the condition of removing a protective hydroxyl group: 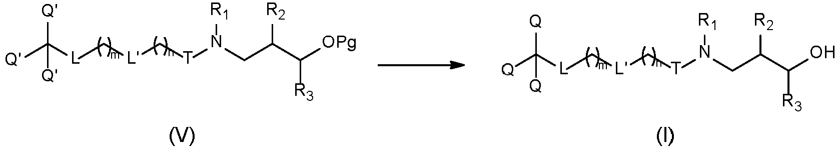 where various groups are as defined according to any one of the technical solutions 33-50.

### DESCRIPTION OF THE EMBODIMENTS

The following embodiments are merely used for illustrating the present invention and are not intended to limit the scope of the present invention.
CbzCl: Benzyl chloroformate
CbzOSu: N-(benzyloxycarbonyloxy)succinimide
DCI: Dicyclohexylcarbodiimide
DIEA/DIPEA: Diisopropylethylamine
DMAP: Dimethylaminopyridine
EDCl: 1-ethyl-(3-dimethylaminopropyl)carbodiimide hydrochloride
HATU: 2-(7-azabenzotriazole)-N,N,N',N'-tetramethyluronium hexafluorphosphate
HBTU: O-benzotriazole-tetramethyluronium hexafluorphosphate
HOBt: 1-hydroxybenzotriazole
TEBA: Benzyltriethylammonium bromide
TEBAC: Triethylbenzylammonium chloride
TMSOTf: Trimethylsilyl trifluoromethanesulfonate

### Sequence information used in the embodiments:

| **Double strand number** | **Sequence (5'->3')** |
|---|---|
| | **The first one is a sense strand, and the second one is an antisense strand.** |
| DR002247 | CmsAmsGmUmGfUmUfCfUfUmGmCmUmCmUmAmUmAmsAms-GL5 (SEQ ID NO: 1) |
| | UmsUfsAmUmAmGfAmGmCmAmAmGmAmAfCmAfCmUmGmsUmsUm (SEQ ID NO: 2) |
| DR002249 | CmsAmsGmUmGIUmUfCIUIUmGmCmUmCmUmAmUmAmsAms-GL13 (SEQ ID NO: 3) |
| | UmsUfsAmUmAmGfAmGmCmAmAmGmAmAfCmAfCmUmGmsUmsUm (SEQ ID NO: 2) |
| DR002234 | GL20-CmsAmGmUmGfUmUfCfUfUmGmCmUmCmUmAmUmsAmsAm (SEQ ID NO: 4) |
| | UmsUfsAmUmAmGfAmGmCmAmAmGmAmAfCmAfCmUmGmsUmsUm (SEQ ID NO: 2) |
| DR002235 | GL14-CmsAmGmUmGfUmUfCfUfUmGmCmUmCmUmAmUmsAmsAm (SEQ ID NO: 5) |
| | UmsUfsAmUmAmGfAmGmCmAmAmGmAmAfCmAfCmUmGmsUmsUm (SEQ ID NO: 2) |
| DR002238 | GL16-CmsAmGmUmGfUmUfCfUfUmGmCmUmCmUmAmUmsAmsAm (SEQ ID NO: 6) |
| | UmsUfsAmUmAmGfAmGmCmAmAmGmAmAfCmAfCmUmGmsUmsUm (SEQ ID NO: 2) |
| DR002240 | GL21-CmsAmGmUmGfUmUfCfUfUmGmCmUmCmUmAmUmsAmsAm (SEQ ID NO: 7) |
| | UmsUfsAmUmAmGfAmGmCmAmAmGmAmAfCmAfCmUmGmsUmsUm (SEQ ID NO: 2) |
| DR002242 | CmsAmsGmUmGIUmUfCIUIUmGmCmUmCmUmAmUmAmsAms-GL6 (SEQ ID NO: 8) |
| | UmsUfsAmUmAmGfAmGmCmAmAmGmAmAfCmAfCmUmGmsUmsUm (SEQ ID NO: 2) |
| DR002243 | CmsAmsGmUmGIUmUfCIUIUmGmCmUmCmUmAmUmAmsAms-GL8 (SEQ ID NO: 9) |
| | UmsUfsAmUmAmGfAmGmCmAmAmGmAmAfCmAfCmUmGmsUmsUm (SEQ ID NO: 2) |
| DR002244 | CmsAmsGmUmGIUmUfCIUIUmGmCmUmCmUmAmUmAmsAms-GL9 (SEQ ID NO: 10) |
| | UmsUfsAmUmAmGfAmGmCmAmAmGmAmAfCmAfCmUmGmsUmsUm (SEQ ID NO: 2) |
| DR002245 | CmsAmsGmUmGIUmUfCIUIUmGmCmUmCmUmAmUmAmsAms-GL7 (SEQ ID NO: 11) |
| | UmsUfsAmUmAmGfAmGmCmAmAmGmAmAfCmAfCmUmGmsUmsUm (SEQ ID NO: 2) |
| DR002246 | CmsAmsGmUmGfUmUfCfUfUmGmCmUmCmUmAmUmAmsAms-GL11 (SEQ ID NO: 12) |
| | UmsUfsAmUmAmGfAmGmCmAmAmGmAmAfCmAfCmUmGmsUmsUm (SEQ ID NO: 2) |
| DR002248 | CmsAmsGmUmGIUmUfCIUIUmGmCmUmCmUmAmUmAmsAms-GL12 (SEQ ID NO: 13) |
| | UmsUfsAmUmAmGfAmGmCmAmAmGmAmAfCmAfCmUmGmsUmsUm (SEQ ID NO: 2) |
| DR005649 | CmsAmsGmUmGIUmUfCIUIUmGmCmUmCmUmAmUmAmsAms-GL12 (SEQ ID NO: 14) |
| | UmsUfsAmUmAmGfAmGmCmAmAmGmAmAfCmAfCmUmGmsUmsUm (SEQ ID NO: 2) |
| DR005648 | CmsAmsGmUmGfUmUfCfUfUmGmCmUmCmUmAmUmAmsAms-GL5 (SEQ ID NO: 15) |
| | UmsUfsAmUmAmGfAmGmCmAmAmGmAmAfCmAfCmUmGmsUmsUm (SEQ ID NO: 2) |
| DR005651 | CmsAmsGmUmGIUmUfCIUIUmGmCmUmCmUmAmUmAmsAms-GL33 (SEQ ID NO: 16) |
| | UmsUfsAmUmAmGfAmGmCmAmAmGmAmAfCmAfCmUmGmsUmsUm (SEQ ID NO: 2) |
| DR002220 | CmsAmsGmUmGIUmUfCIUfUmGmCmUmCmUmAmUmAmAm-L96 (SEQ ID NO: 17) |
| | UmsUfsAmUmAmGfAmGmCmAmAmGmAmAfCmAfCmUmGmsUmsUm (SEQ ID NO: 2) |
| DR005728 | CmsAmsGmUmGIUmUfCIUIUmGmCmUmCmUmAmUmAmsAms-GL30 (SEQ ID NO: 18) |
| | UmsUfsAmUmAmGfAmGmCmAmAmGmAmAfCmAfCmUmGmsUmsUm (SEQ ID NO: 2) |
| DR005729 | CmsAmsGmUmGIUmUfCIUIUmGmCmUmCmUmAmUmAmsAms-GL31 (SEQ ID NO: 19) |
| | UmsUfsAmUmAmGfAmGmCmAmAmGmAmAfCmAfCmUmGmsUmsUm (SEQ ID NO: 2) |
| DR005730 | CmsAmsGmUmGIUmUfCIUIUmGmCmUmCmUmAmUmAmsAms-GL32 (SEQ ID NO: 20) |
| | UmsUfsAmUmAmGfAmGmCmAmAmGmAmAfCmAfCmUmGmsUmsUm (SEQ ID NO: 2) |
| DR005636 | IBs-AmsAmAmAmGmGmGmAmCfAfGfUmAmUmUmCmUmCmAmGmUms-IB-L96 (SEQ ID NO: 21) |
| | AmsCfsUmGfAmGfAmAfUmAfCmUfGmUfCmCfCmUfUmsUfsUm (SEQ ID NO: 22) |
| DR005679 | IBs-AmsAmGmGmGmAmCfAfGfUmAmUmUmCmUmCmAmGmUms-IB-L96 (SEQ ID NO: 23) |
| | AmsCfsUmGfAmGfAmAfUmAfCmUfGmUfCmCfCmUfUmsUfsUm (SEQ ID NO: 22) |
| DR005681 | IBs-AmsAmAmAmGmGmGmAmCfAfGfUmAmUmUmCmUmCmAmGmUms-IBs-GL6 (SEQ ID NO: 24) |
| | AmsCfsUmGfAmGfAmAfUmAfCmUfGmUfCmCfCmUfUmsUfsUm (SEQ ID NO: 22) |
| DR005685 | UmsGmsAmAmCmUmCfAfAfCmUmCmAmAmAmAmCmUmsUms-GL6 (SEQ ID NO: 25) |
| | AmsAfsGmUfUmUfUmGfAmGfUmUfGmAfGmUfUmCfAmsAfsGm (SEQ ID NO: 26) |
| DR005686 | GmsGmsAmUmCmAmCfAfAfAmAmCmUmUmCmAmAmUmsAms-GL6 (SEQ ID NO: 27) |
| | UmsAfsUmUfGmAfAmGfUmUfUmUfGmUfGmAfUmCfCmsAfsUm (SEQ ID NO: 28) |
| DR005687 | CmsAmsCmGmUmUmGfCfUfUmGmAmAmAmUmUmGmAmsAms-GL6 (SEQ ID NO: 29) |
| | UmsUfsCmAfAmUfUmUfCmAfAmGfCmAfAmCfGmUfGmsGfsAm (SEQ ID NO: 30) |
| DR005692 | IBs-UmsCmCmAmCmGmUmUmGfCfUfUmGmAmAmAmUmUmGmAmAms-IBs-GL6 (SEQ ID NO: 31) |
| | UmsUfsCmAfAmUfUmUfCmAfAmGfCmAfAmCfGmUfGmsGfsAm (SEQ ID NO: 30) |
| DR001479 | NAG37s-IBs-GmCmUmCmAmAmCmAmUfAfUfUmUmGmAmUmCmAmGmUmAms-IB (SEQ ID NO: 32) |
| | UmsAfsCmsUfGmAfUmCfAmAfAmUfAmUfGmUfUmGfAmGfsCm (SEQ ID NO: 33) |
| DR002221 | CmsUmsAmGmAmCmCfUmGfUmdTUmUmGmCmUmUmUmUmGmUm-L96 (SEQ ID NO: 34) |
| | AmsCfsAmAfAfAfGmCfAmAfAmAfCmAfGmGfUmCfUmAmGmsAmsAm (SEQ ID NO: 35) |
| DR005642 | CmsUmsAmGmAmCmCfUmGfUmdTUmUmGmCmUmUmUmUmGmsUms-GL12 (SEQ ID NO: 36) |
| | AmsCfsAmAfAfAfGmCfAmAfAmAfCmAfGmGfUmCfUmAmGmsAmsAm (SEQ ID NO: 35) |
| DR005656 | CmsUmsAmGmAmCmCfUmGfUmdTUmUmGmCmUmUmUmUmGmsUms-GL6 (SEQ ID NO: 37) |
| | AmsCfsAmAfAfAfGmCfAmAfAmAfCmAfGmGfUmCfUmAmGmsAmsAm (SEQ ID NO: 35) |
| DR002359 | GmsUmsUmUmUmGmCIUfUfUmUmGmUmAmAmCmUmsUmsAm-L96 (SEQ ID NO: 38) |
| | UmsAfsAmGfUmUfAmCfAmAfAmAfGmCfAmAfAmAfCmsAfsGm (SEQ ID NO: 39) |
| DR005672 | GmsUmsUmUmUmGmCfUfUfUmUmGmUmAmAmCmUmUmsAms-GL6 (SEQ ID NO: 40) |
| | UmsAfsAmGfUmUfAmCfAmAfAmAfGmCfAmAfAmAfCmsAfsGm (SEQ ID NO: 39) |
| DR005675 | GmsUmsUmUmUmGmCfUfUfUmUmGmUmAmAmCmUmUmAm-GL6 (SEQ ID NO: 41) |
| | UmsAfsAmGfUmUfAmCfAmAfAmAfGmCfAmAfAmAfCmsAfsGm (SEQ ID NO: 39) |
| DR005674 | UmsGmsUmUmUmUmGfCfUfUmUmUmGmUmAmAmCmUmsUms-GL6 (SEQ ID NO: 42) |
| | AmsAfsGmUfUmAfCmAfAmAfAmGfCmAfAmAfAmCfAmsGfsGm (SEQ ID NO: 43) |

Herein, meanings of various abbreviation are as follows:
Distribution of A, U, G and C represents a natural adenine ribonucleotide, a uracil ribonucleotide, a guanine ribonucleotide, and a cytosine ribonucleotide.
m represents that an adjacent nucleotide on the left side is a 2'-OCH₃ modified nucleotide. For example, Am, Um, Gm and Cm represent 2'-OCH₃ modified A, U, G, and C.
f represents that an adjacent nucleotide on the left side is a 2'-F modified nucleotide. For example, Af, Uf, Gf and Cf represent 2'-F modified A, U, G, and C, respectively.
"s" or "s-" represents that two nucleotides and/or delivery vectors that are adjacent to each other left and right are connected by thiophosphate.
L96 represents a GalNAc delivery vector having the following structure well known in the field, where 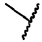 represents a position connected with siRNA by a phosphate group or a thiophosphate group, with reference to, for example, PCT publication No. WO2009073809 and No. WO2009082607. 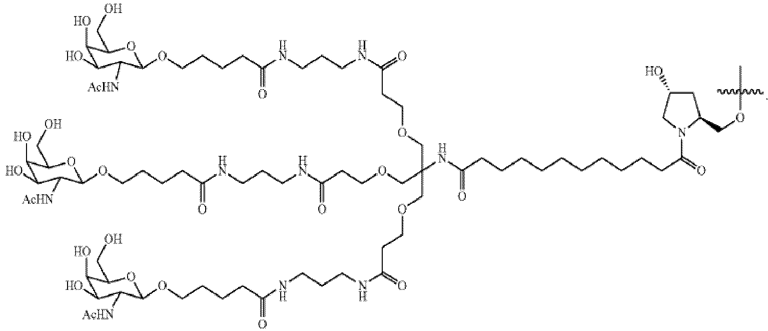
NAG37 represents a GalNAc delivery vector having the following structure well known in the field, where 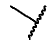 represents a position connected with siRNA by a phosphate group or a thiophosphate group, with reference to, for example, PCT publication No. WO2018044350. 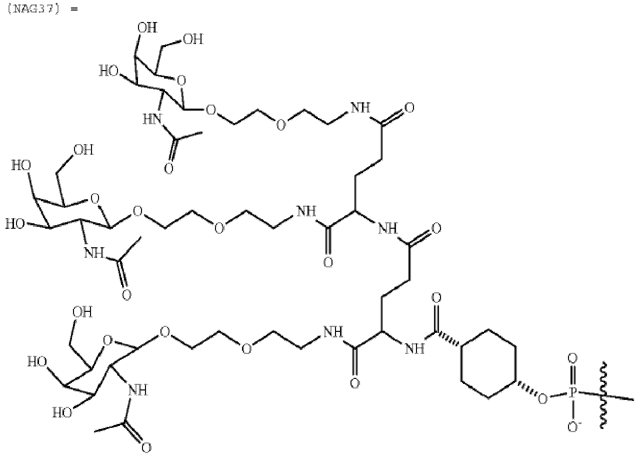
IB represents a reverse baseless deoxyribonucleotide, which can include the following three structures according to a position/connection mode in siRNA. The IB is well known in the field, with reference to , for example, F. Czaudema, Nucleic Acids Res., 2003, 31(11), 2705-16 and PCT publication No. WO2016011123 and No. WO2019051402. 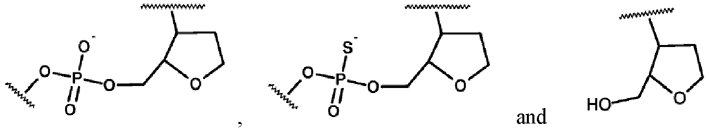
Structures of GL1-GL32 are as defined above.

### Example 1: Preparation of a compound E1

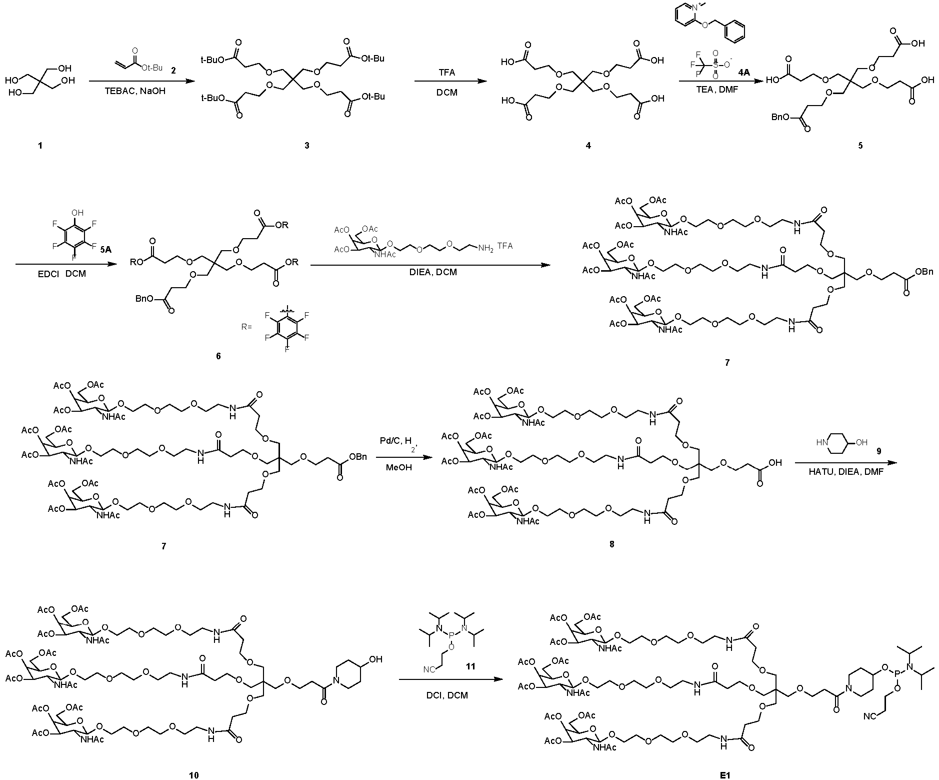

### 1. Preparation of a compound 3

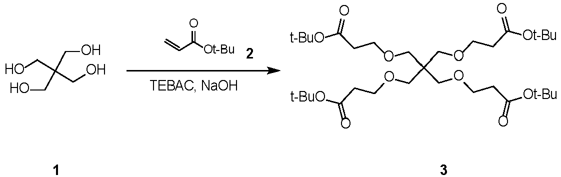

A compound 1 (100 g, 734 mmol) was added to water (176.28 mL) dissolved with sodium hydroxide (176.28 g, 4.40 mol) at 25°C, then TEBAC (83.65 g, 367 mmol) and a compound 2 (564.83 g, 4.407 mol) were added, and after nitrogen replacement was carried out for three times, a reaction solution was stirred at 25°C for 18 h. Thin layer chromatography (dichloromethane/methanol=10/1) showed that the compound 1 was completely consumed, and thin layer chromatography (petroleum ether/ethyl acetate=5/1) showed that a new product point was generated. The reaction solution was diluted with 1,000 mL of ice water and extracted with ethyl acetate (500 mL×3), an organic phase was dried with anhydrous sodium sulfate and then concentrated under vacuum to obtain a crude product, and the crude product was purified by column chromatography (petroleum ether/ethyl acetate=1/0 to 10/1) to obtain a colorless oily liquid compound 3 (about 56 g). ¹H NMR (400 MHz, CD₃OD) *δ* 3.59 (t, *J =* 6.4 Hz, 8 H), 3.36 (s, 8 H), 2.43 (t, *J =* 6.4 Hz, 8 H), 1.47 (s, 36 H).

### 2. Preparation of a compound 4

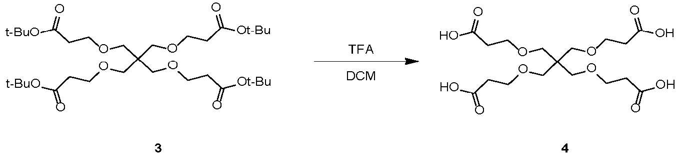

The compound 3 (56 g, 87.372 mmol) was dissolved in DCM (400 mL) at 25°C, TFA (100 mL) was slowly dropped, and a reaction solution was stirred at 25°C for 18 h. Thin layer chromatography (petroleum ether/ethyl acetate=5/1) showed that reactants were completely consumed and a new point was generated. The reaction solution was concentrated to dryness to obtain a crude pink oily liquid compound (about 20 g). ¹H NMR (400 MHz, CD₃OD) *δ* 3.63 (t, *J =* 6.0 Hz, 8 H), 3.36 (s, 8 H), 2.51 (t, *J =* 6.4 Hz, 8 H).

### 3. Preparation of a compound 5

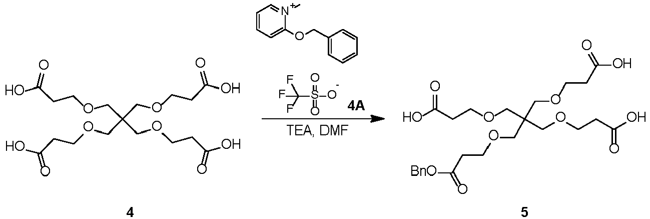

The compound 4 (4.2 g, 9.896 mmol) was dissolved in DMF (21 mL) at 25°C, TEA (1.376 mL, 9.896 mmol) and a compound 4A (3.46 g, 9.896 mmol) were sequentially added, heating was carried out to 83°C, and a reaction solution was stirred in a nitrogen atmosphere at 83°C for 24 h. Thin layer chromatography (dichloromethane/methanol=8/1) showed that the compound 4 was completely consumed and a new point was generated. LCMS showed an MS value of a compound 5. The reaction solution was concentrated under vacuum to obtain a crude product, and the crude product was purified by MPLC (dichloromethane/methanol=1/0 to 10/1) to obtain a yellow oily liquid compound 5 (about 6.6 g). ¹H NMR (400 MHz, CDCl₃) *δ* 7.32 - 7.37 (m, 5 H), 5.14 (s, 2 H), 3.60 - 3.70 (m, 8 H), 3.35 (s, 8 H), 2.53 - 2.65 (m, 8 H). LCMS: m/z=515.0 (M+H)⁺.

### 4. Preparation of a compound 6

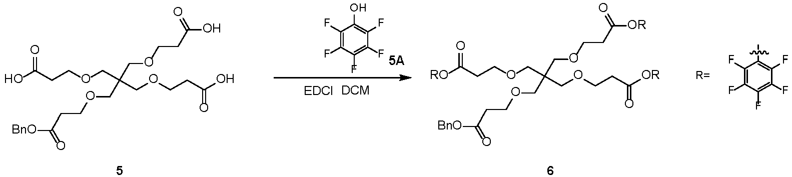

The compound 5 (6.6 g, 12.827 mmol) was dissolved in DCM (200 mL) at 25°C, then a compound 5A (11.81 g, 64.137 mmol) and EDCI (12.30 g, 64.137 mmol) were sequentially added, and a reaction solution was stirred at 25°C for 6 h. Thin layer chromatography (dichloromethane/methanol=8/1) showed that the compound 5 was completely consumed, and thin layer chromatography (petroleum ether/ethyl acetate=5/1) showed that a new product point was generated. LCMS showed an MS value of a compound 6. The reaction solution was concentrated under vacuum to obtain a crude product, and the crude product was purified by MPLC (petroleum ether/ethyl acetate=1/0 to 5/1) to obtain a colorless oily liquid compound 6 (about 4.9 g). ¹H NMR (400 MHz, CDCl₃) *δ* 7.32 - 7.37 (m, 5 H), 5.14 (s, 2 H), 3.74 (t, *J* = 6.0 Hz, 6 H), 3.68 (t, *J* = 6.4 Hz, 2 H), 3.36 - 3.45 (m, 8 H), 2.87 (t, *J* = 6.0 Hz, 6 H), 2.62 (t, *J* = 6.4 Hz, 2 H). LCMS: m/z=1013.2 (M+H)⁺.

### 5. Preparation of a compound 7

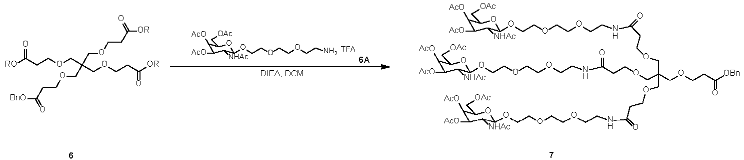

The compound 6 (3.0 g, 2.96 mmol) and a compound 6A(6.81 g, 11.8 mmol, prepared by a method of an intermediate 3-4 in Example 23) were dissolved in DCM (50.0 mL), DIEA (3.91 mL, 23.7 mmol) was added, nitrogen replacement was carried out for three times, and a reaction solution was enabled to undergo a reaction at 20°C for 16 h. Thin layer chromatography (first developing agent: dichloromethane:methanol=10/1; and second developing agent: petroleum ether:ethyl acetate=3:1) showed that raw materials disappeared and a new point was generated. The reaction solution was diluted with 150 ml dichloromethane, and washed with a citric acid aqueous solution (50.0 ml×3), a saturated sodium bicarbonate aqueous solution (50.0 ml×3) and a saturated salt solution (50.0 ml×3). An organic phase was dried with anhydrous sodium sulfate, filtered, concentrated under reduced pressure and purified by column chromatography (dichloromethane:methanol=100:1 to 8:1) to obtain a white solid product compound 7 (about 400 mg). ¹H NMR (400 MHz, CD₃OD) *δ* 7.29 - 7.39 (m, 5 H), 5.31 - 5.36 (m, 3 H), 5.16 (s, 2 H), 5.05 - 5.09 (m, 3 H), 4.61 - 4.68 (m, 3 H), 4.06 - 4.19 (m, 10 H), 4.00 - 4.05 (m, 3 H), 3.90 - 3.96 (m, 3 H), 3.58 - 3.76 (m, 33 H), 3.51 - 3.57 (m, 7 H), 3.35 - 3.41 (m, 8 H), 2.58 - 2.65 (m, 2 H), 2.42 (t, *J =* 6.0 Hz, 6 H), 2.14 (s, 9 H), 2.03 (s, 9 H), 1.92 - 1.96 (m, 18 H).

### 6. Preparation of a compound 8

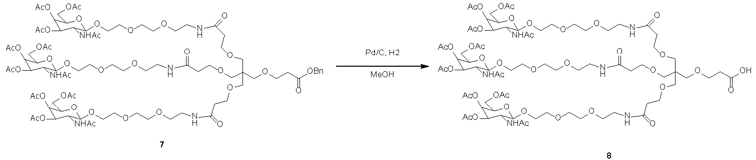

The compound 7 (5.00 g, 2.63 mmol) was dissolved in MeOH (60.0 mL) added with Pd/C (1.50 g) with a mass fraction of 10% at 20°C, H₂ replacement was carried out for three times, and stirring was carried out at 20°C and 15 psi for 14 h. Thin layer chromatography (dichloromethane:methanol=8/1) showed that raw materials disappeared and a new point was generated. A reaction solution was filtered with diatomite, and a filtrate was concentrated under reduced pressure to obtain a white solid compound 8 (about 4.40 g). ¹H NMR (400 MHz, CD₃OD) *δ* 5.34 (d, *J =* 2.8 Hz, 3 H), 5.04 - 5.11 (m, 3 H), 4.62 - 4.67 (m, 3 H), 4.08 - 4.20 (m, 9 H), 4.00 - 4.08 (m, 4 H), 3.91 - 3.98 (m, 3 H), 3.59 - 3.77 (m, 33 H), 3.53 - 3.59 (m, 7 H), 3.37 - 3.40 (m, 8 H), 2.49 - 2.53 (m, 2 H), 2.43 (t, *J =* 6.0 Hz, 6 H), 2.15 (s, 9 H), 2.03 (s, 9 H), 1.93 - 1.97 (m, 18 H).

### 7. Preparation of a compound 10

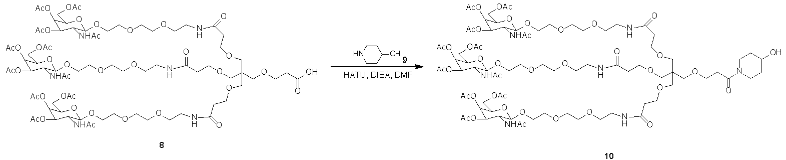

The compound 8 (1.50 g, 0.831 mmol), HATU (0.470 g, 1.24 mmol) and DIEA (0.412 mL, 2.492 mmol) were sequentially dissolved in anhydrous DMF (15.0 mL) at 20°C, nitrogen replacement was carried out for 3 times, stirring was carried out at 20°C for 0.5 h, and a compound 9 (0.130 g, 1.246 mmol) was added and stirred at 20°C for 2 h. LC/MS showed that raw materials completely disappeared and a main peak was generated. Thin layer chromatography (dichloromethane:methanol=8/1) showed that the raw materials disappeared and a new point was generated. A reaction solution was diluted with dichloromethane (100 mL), and sequentially washed with a saturated sodium bicarbonate aqueous solution (20.0 mL×3) and a saturated salt solution (20.0 mL×3). An organic phase was dried with anhydrous sodium sulfate and then concentrated under reduced pressure to obtain a crude product. The crude product was purified by column chromatography (dichloromethane:methanol=10/1 to 8/1) to obtain a brown solid product compound 10 (about 1.00 g). ¹H NMR (400 MHz, CD₃OD) *δ* 5.31 - 5.35 (m, 3 H), 5.04 - 5.10 (m, 3 H), 4.63 - 4.67 (m, 3 H), 4.08 - 4.21 (m, 9 H), 4.01 - 4.08 (m, 4 H), 3.91 - 3.98 (m, 3 H), 3.83 - 3.90 (m, 2 H), 3.69 - 3.77 (m, 4 H), 3.59 - 3.69 (m, 27 H), 3.53 - 3.58 (m, 6 H), 3.36 - 3.41 (m, 15 H), 2.62 - 2.68 (m, 2 H), 2.40 - 2.46 (m, 6 H), 2.15 (s, 9 H), 2.03 (s, 9 H), 1.80 - 1.98 (m, 20 H), 1.35 - 1.53 (m, 2 H). LCMS: m/z=945.5 (M/2+H)⁺.

### 8. Preparation of E1

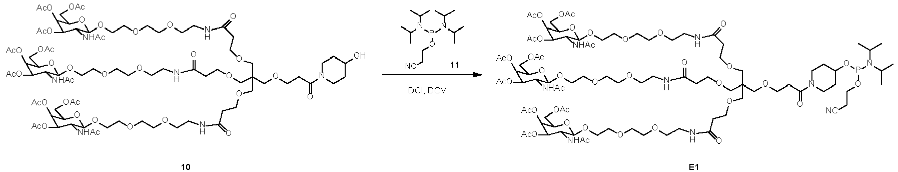

The compound 10 (1.00 g, 0.529 mmol) was dissolved in acetonitrile, spin-dried to remove water in the compound and then dissolved in DCM (10.0 mL) in a nitrogen atmosphere, DCI (0.03 g, 0.265 mmol) was added, and a compound 11 (0.32 g, 1.06 mmol) was added dropwise to the above solution and stirred at 20°C for 1 h. LCMS showed that raw materials disappeared and 40.0% of a product was generated. A reaction solution was cooled to 0°C, 6 ml of a saturated sodium bicarbonate aqueous solution and 6 ml of a saturated salt solution were added dropwise, and the mixed solution was diluted with 150 ml of dichloromethane and then subjected to liquid separation. An organic phase was washed with 50 ml of a mixed aqueous solution of saturated sodium bicarbonate and a saturated salt solution at 1:1. The organic phase was dried with anhydrous sodium sulfate, filtered and spin-dried to obtain a crude product. The crude product was diluted with 15 ml of dichloromethane, slowly added dropwise to 60 ml of methyl tert-butyl ether and then filtered, a filter cake was washed with methyl tert-butyl ether for three times, and the filter cake was dissolved in acetonitrile and then spin-dried to remove the methyl tert-butyl ether in the compound. A yellow solid E1 (615 mg, 0.252 mmol, 47.6%) was obtained.

LCMS (ESI): m/z=1103.3 ((M+59*2)/2+H)⁺;

¹H NMR (400 MHz, DMSO-*d*₆) *δ* 7.86 - 7.93 (m, 3H), 7.78 - 7.83 (m, 3H), 5.19 - 5.23 (m, 3H), 4.97 (dd, *J*=3.2, 11.2 Hz, 3H), 4.51 - 4.57 (m, 3H), 3.99 - 4.06 (m, 10H), 3.84 - 3.92 (m, 3H), 3.75 - 3.82 (m, 3H), 3.62 - 3.75 (m, 4H), 3.45 - 3.62 (m, 34H), 3.37 - 3.41 (m, 6H), 3.17 - 3.23 (m, 13H), 2.76 (t, *J=*6.0 Hz, 2H), 2.53 - 2.57 (m, 2H), 2.28 (t, *J*=6.4 Hz, 6H), 2.12 (s, 9H), 2.00 (s, 9H), 1.9-1.89 (s, 9H), 1.71 - 1.82 (m, 11H), 1.38 - 1.60 (m, 2H), 1.09 - 1.17 (m, 12H).

³¹PNMR (400 MHz, DMSO-*d*₆) *δ* -145.22.

### Example 2: Preparation of a compound E2

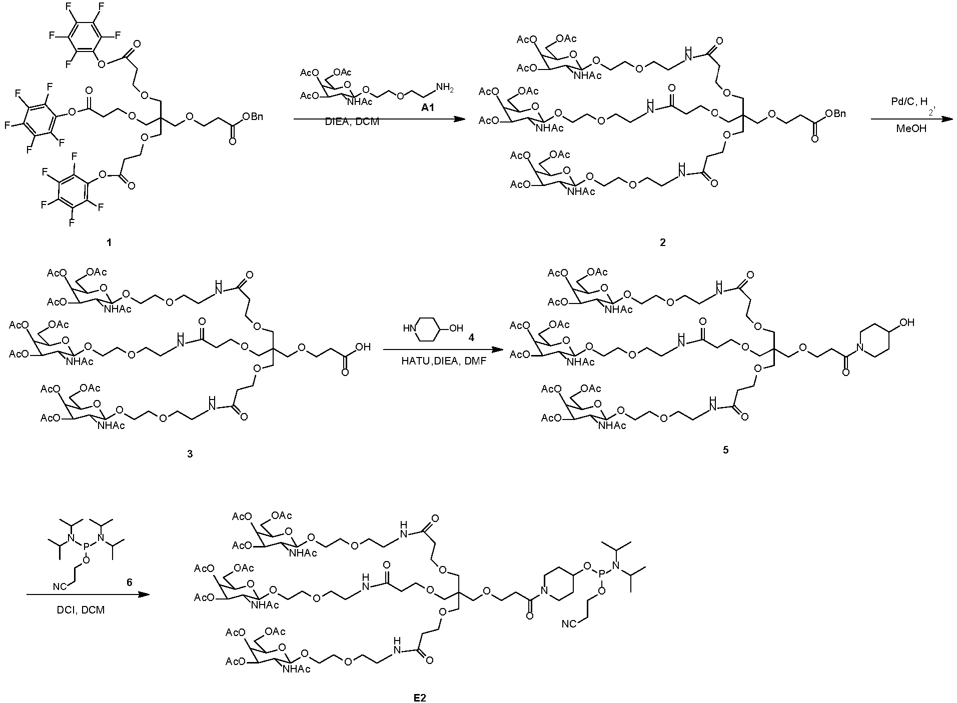

### 1. Preparation of a compound 2

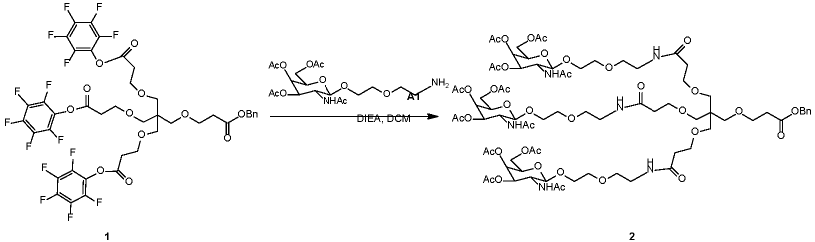

A compound 1 (2.60 g, 2.57 mmol, prepared by a method of the compound 6 in Example 1) was dissolved in DCM (60.0 mL) at room temperature, DIEA (2.12 mL, 12.8 mmol) and a compound A1 (4.46 g, 10.3 mmol, prepared by a method of an intermediate 2-3 in Example 22) were sequentially added to a resulting solution, and nitrogen replacement was carried out for 3 times. A reaction mixture was stirred at 25°C for 18 h. Liquid chromatography-mass spectrometry detected an MS response of a product. Thin layer chromatography (dichloromethane/methanol=10/1) showed that raw materials completely disappeared and a new point was generated. A reaction solution was concentrated under reduced pressure, and a resulting crude product was purified by column chromatography (dichloromethane/methanol=100/7 to 100/15) to obtain a white solid 2 (about 4.1 g). ¹H NMR (400 MHz, CD₃OD) *δ* 7.28-7.41 (m, 5H), 5.31-5.35 (m, 3H), 5.15 (s, 2H), 5.07-5.11 (m, 3H), 4.61-4.65 (m, 3H), 4.07-4.19 (m, 9H), 4.00-4.04 (m, 3H), 3.91-3.94 (m, 3H), 3.64-3.76 (m, 8H), 3.56-3.64 (m, 13H), 3.52-3.56 (m, 6H), 3.34-3.45 (m, 10H), 2.60-2.64 (m, 2H), 2.41-2.46 (m, 6H), 2.14 (s, 9H), 2.03 (s, 9H), 1.94 (d, *J =* 6.80 Hz, 18H). LCMS: m/z=883.0 (M/2+H)⁺.

### 2. Preparation of a compound 3

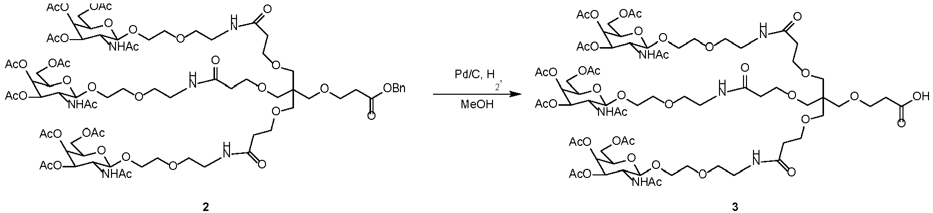

The compound 2 (4.10 g, 2.33 mmol) was dissolved in MeOH (100 mL) at room temperature, wet Pd/C (0.50 g, 4.70 mmol) with a mass fraction of 10% was added to a resulting solution, hydrogen replacement was carried out for 3 times, and a reaction mixture was stirred in a hydrogen atmosphere (14.696 psi) at 25°C for 18 h. Liquid chromatography-mass spectrometry detected an MS response of a product. Thin layer chromatography (dichloromethane/methanol=10/1, with phosphomolybdic acid as a color developing agent) showed that raw materials were completely consumed and a new point was generated. A reaction solution was filtered, and a filtrate was concentrated under reduced pressure to obtain a white solid 3 (about 3.87 g). ¹H NMR (400 MHz, CD₃OD) *δ* 5.30-5.36 (m, 3H), 5.07-5.09 (m, 3H), 4.60-4.66 (m, 3H), 4.06-4.22 (m, 9H), 4.01-4.06 (m, 3H), 3.92-3.96 (m, 3H), 3.69-3.75 (m, 3H), 3.61-3.67 (m, 14H), 3.48-3.60 (m, 7H), 3.32-3.45 (m, 16H), 2.49-2.51 (m, 2H), 2.43-2.47 (m, 6H), 2.15 (s, 9H), 2.03 (s, 9H), 1.95 (d, *J* = 5.20 Hz, 18H). LCMS: m/z=837.9 (M/2+H)⁺.

### 3. Preparation of a compound 5

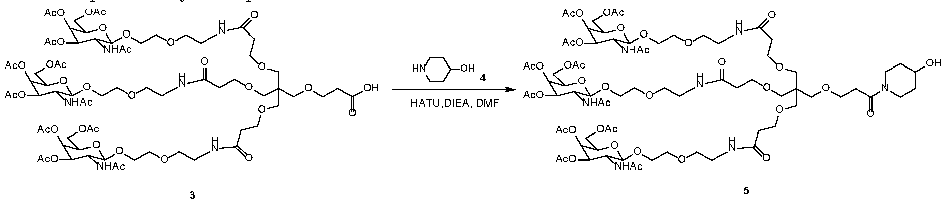

The compound 3 (1.54 g, 0.920 mmol) was dissolved in DMF (15.0 mL) at 25°C, HATU (0.52 g, 1.38 mmol) and DIEA (0.456 mL, 2.76 mmol) were sequentially added, a reaction solution was stirred at 25°C for 30 min, then a compound 4 was added, and the reaction solution was stirred at 25°C for 2.5 h. LCMS showed that the compound 3 was completely consumed and an MS value of a compound 5 was obtained. The reaction solution was diluted with 50 mL of DCM, and washed with a saturated NaHCO₃ solution (20 mL×3). An organic phase was washed with a saturated salt solution (20 mL×3), and then the organic phase was dried with anhydrous Na₂SO₄ and spin-dried to obtain a crude product. The obtained crude product was purified by column chromatography (dichloromethane:methanol=1/0 to 5/1) to obtain a white solid compound 5 (about 1.26 g). ¹H NMR (400 MHz, CD₃OD) *δ* 5.32 - 5.36 (m, 3 H), 5.06 (dd, *J =* 3.2, 11.2 Hz, 3 H), 4.61 - 4.66 (m, 3 H), 4.06 - 4.20 (m, 9 H), 4.01-4.05 (m, 3 H), 3.91-3.97 (m, 3 H), 3.70 - 3.76 (m, 3 H), 3.60 - 3.67 (m, 14 H), 3.50 - 3.57 (m, 7 H), 3.33 - 3.40 (m, 18 H), 2.66 (t, *J =* 6.0 Hz, 2 H), 2.44 (t, *J =* 6.0 Hz, 6 H), 2.15 (s, 9 H), 2.03 (s, 9 H), 1.92-1.98 (m, 18 H), 1.27 - 1.50 (m, 4 H). LCMS: m/z=879.5 (M/2+H)⁺.

### 4. Preparation of E2

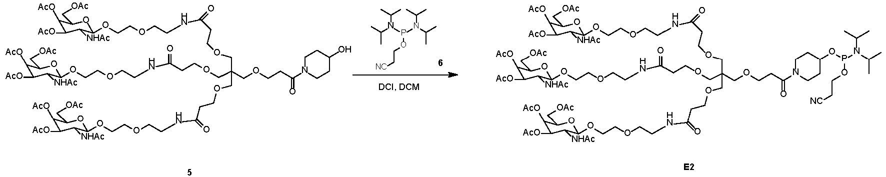

The compound 5 (1.26 g, 0.717 mmol) was dissolved in DCM (10.0 mL) at room temperature, a compound 6 (0.43 g, 1.43 mmol) and DCI (0.08 g, 0.717 mmol) were added to a resulting solution, nitrogen replacement was carried out for 3 times, and a reaction mixture was stirred at 25 °C for 1 h. Liquid chromatography-mass spectrometry detected an MS response of a product. A reaction solution was cooled to 0°C in an ice salt bath, 6 mL of a saturated NaHCO₃ solution and 6 mL of a saturated salt solution were added to the reaction solution, and 50 mL of DCM was added for extraction. Then, an organic phase was washed with a mixed solution (20 mL×3) of saturated NaHCO₃ and a saturated salt solution at 1:1, dried with Na₂SO₄ and spin-dried to obtain a crude product. The crude product was dissolved in 15 mL of DCM. A crude product solution was slowly dropped to MTBE in a dry ice bath and then filtered to obtain a white solid E2 (640 mg, yield: 45.60%, purity: 77.02%).

¹H NMR (400 MHz, DMSO-*d*₆) *δ* 7.79-7.91 (m, 6H), 5.20-5.23 (m, 3H), 4.96-4.98 (m, 3H), 4.51-4.56 (m, 3H), 4.03 (s, 10H), 3.65-3.92 (m, 11H), 3.46-3.61 (m, 21H), 3.34-3.42 (m, 8H), 3.19-3.22 (m, 15H), 2.74-2.76 (m, 2H), 2.52-2.54 (m, 2H), 2.27-2.29 (m, 6H), 2.10 (s, 9H), 2.00 (s, 9H), 1.89 (s, 9H), 1.77 (s, 9H), 1.12-1.16 (m, 12H).

³¹PNMR (400 MHz, DMSO-*d*₆) *δ* -145.243.

LCMS: m/z=1037.4 (M/2+CH₃COOH)⁺.

### Example 3: Preparation of a compound E3

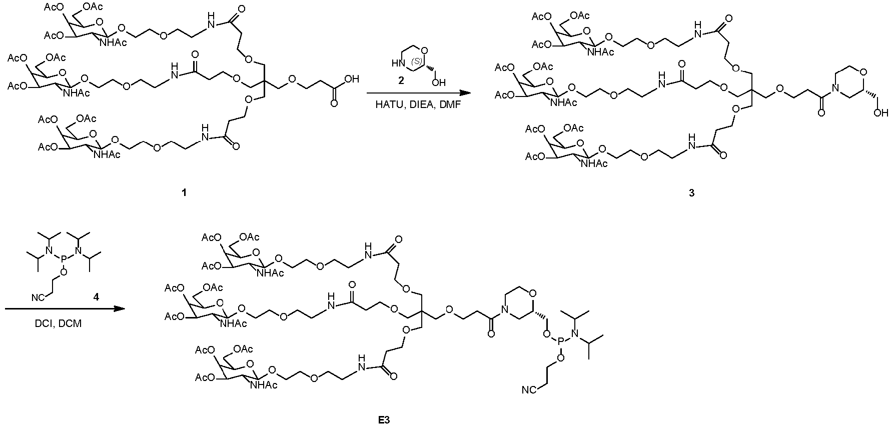

### 1. Preparation of a compound 3

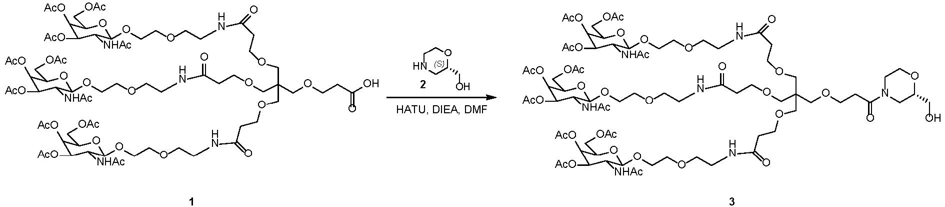

A compound 2 (0.17 g, 1.43 mmol) was dissolved in DMF (10.0 mL) at room temperature, a compound 1 (1.20 g, 0.717 mmol, prepared by a method of the compound 3 in Example 2), DIEA (0.178 mL, 1.08 mmol) and HATU (0.41 g, 1.08 mmol) were added to a resulting solution, and nitrogen replacement was carried out for 3 times. A reaction mixture was stirred at 25°C for 1 h. Liquid chromatography-mass spectrometry detected an MS response of a product. Thin layer chromatography (dichloromethane/methanol=8/1) showed that raw materials completely disappeared and a new point was generated. A reaction solution was concentrated under reduced pressure, and a resulting crude product was purified by column chromatography (dichloromethane/methanol=10/1 to 8/1) to obtain a white solid 3 (about 0.96 g). LCMS: m/z=887.4 (M/2+H)⁺.

### 2. Preparation of E3

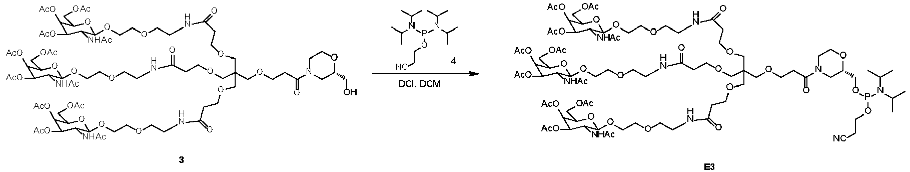

The compound 3 (0.96 g, 0.542 mmol) was dissolved in DCM (10.0 mL) at room temperature, a compound 4 (0.33 g, 1.08 mmol) and DCI (0.03 g, 0.271 mmol) were added to a resulting solution, nitrogen replacement was carried out for 3 times, and a reaction mixture was stirred at 25 °C for 1 h. Liquid chromatography-mass spectrometry detected an MS response of a product. A reaction solution was cooled to 0°C in an ice salt bath, 6 mL of a saturated NaHCO₃ solution and 6 mL of a saturated salt solution were sequentially added to the reaction solution, and 50 mL of DCM was added for extraction. Then, an organic phase was washed with a mixed solution (20 mL×3) of saturated NaHCO₃ and a saturated salt solution at 1:1, dried with Na₂SO₄ and spin-dried to obtain a crude product. The crude product was dissolved in 15 mL of DCM. A crude product solution was slowly dropped to MTBE in a dry ice bath and then filtered to obtain a white solid E3 (520 mg, yield: 48.67%, purity: 85.29%).

LCMS (ESI): m/z=1045.3 (M/2+CH₃COOH)⁺;

¹H NMR (400 MHz, DMSO-*d*₆) *δ* 7.75-7.92 (m, 6H), 5.19-5.22 (m, 3H), 4.96-4.99 (m, 3H), 4.52-4.57 (m, 3H), 4.01-4.04 (m, 10H), 3.73-3.92 (m, 11H), 3.46-3.61 (m, 21H), 3.37-3.41 (m, 8H), 3.19-3.22 (m, 15H), 2.75-2.78 (m, 3H), 2.27-2.32 (m, 6H), 2.06-2.13 (m, 11H), 1.99-2.02 (m, 9H), 1.86-1.90 (m, 9H), 1.76-1.79 (m, 9H), 1.12-1.15 (m, 12H).

³¹PNMR (400 MHz, DMSO-*d*₆) *δ* -147.960.

### Example 4: Preparation of a compound E4

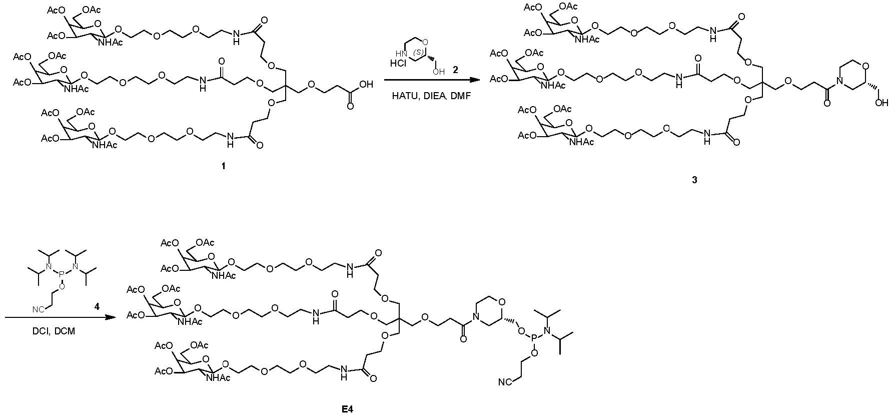

### 1. Preparation of a compound 3

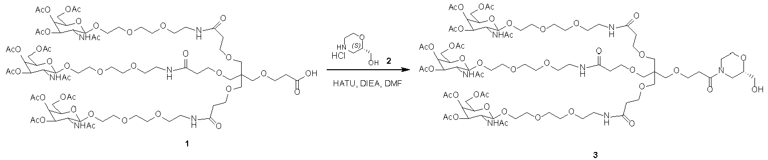

A compound 1 (1.35 g, 0.748 mmol, prepared by a method of the compound 8 in Example 1), HATU (0.43 g, 1.12 mmol) and DIEA (0.371 mL, 2.24 mmol) were sequentially dissolved in anhydrous DMF (15.0 mL) at 22°C, nitrogen replacement was carried out for 3 times, stirring was carried out at 22°C for 0.5 h, and a compound 2 (42.0 mg, 0.415 mmol) was added and stirred at 22°C for 2 h. LC/MS showed that raw materials completely disappeared and a main peak was generated. Thin layer chromatography (dichloromethane:methanol=8/1) showed that the raw materials disappeared and a new point was generated. A reaction solution was diluted with dichloromethane (100 mL), and sequentially washed with a saturated sodium bicarbonate aqueous solution (20.0 mL×3) and a saturated salt solution (20.0 mL×3). An organic phase was dried with anhydrous sodium sulfate and then concentrated under reduced pressure to obtain a crude product. The crude product was purified by column chromatography (dichloromethane:methanol=100/1 to 8/1) to obtain a white solid compound 3 (about 950 mg). ¹H NMR (400 MHz, CD₃OD) *δ* 5.34 (d, *J =* 2.8 Hz, 3 H), 5.05 - 5.10 (m, 3 H), 4.61 - 4.68 (m, 3 H), 4.08 - 4.21 (m, 9 H), 4.01-1.20 (m, 4 H), 3.89 - 4.00 (m, 5 H), 3.59 - 3.77 (m, 33 H), 3.56 (t, *J =* 5.6 Hz, 7 H), 3.34 - 3.42 (m, 15 H), 2.62 - 2.70 (m, 2 H), 2.43 (t, *J =* 6.0 Hz, 6 H), 2.14 (s, 9 H), 2.03 (s, 9 H), 1.92 - 1.96 (m, 18 H). LCMS: m/z=953.3 (M/2+H)⁺.

### 2. Preparation of E4

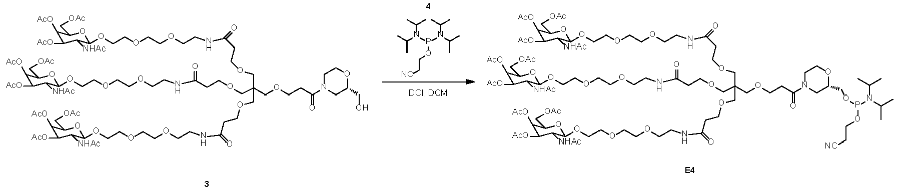

The compound 3 (1.00 g, 0.525 mmol) was dissolved in acetonitrile, spin-dried to remove water in the compound and then dissolved in DCM (10.0 mL) in a nitrogen atmosphere, a compound 4 (0.32 g, 1.05 mmol) and DCI (0.03 g, 0.262 mmol) were slowly added dropwise, and a reaction solution was enabled to undergo a reaction at 20°C for 1 h. LC/MS showed that raw materials disappeared. The reaction solution was cooled to 0°C, 6 ml of a saturated sodium bicarbonate aqueous solution and 6 ml of a saturated salt solution were added dropwise, and the mixed solution was diluted with 100 ml of dichloromethane and then subjected to liquid separation. An organic phase was washed with 50 ml of a mixed aqueous solution of saturated sodium bicarbonate and a saturated salt solution at 1:1. The organic phase was dried with anhydrous sodium sulfate, filtered and spin-dried to obtain a crude product. The crude product was diluted with 15 ml of dichloromethane, slowly added dropwise to 60 ml of methyl tert-butyl ether and then filtered, a filter cake was washed with methyl tert-butyl ether for three times, and the filter cake was dissolved in acetonitrile and then spin-dried to remove the methyl tert-butyl ether in the compound. A yellow solid product E4 (712 mg, 0.339 mmol, 64.4%) was obtained.

LCMS (ESI): m/z=1111.5 ((M+59*2)/2+H)⁺;

¹H NMR (400 MHz, DMSO-*d*₆) *δ* 7.86 - 7.94 (m, 3 H), 7.76 - 7.84 (m, 3 H), 5.19 - 5.26 (m, 3 H), 4.92 - 5.00 (m, 3 H), 4.51 - 4.57 (m, 3 H), 3.98 - 4.08 (m, 10 H), 3.82 - 3.93 (m, 5 H), 3.73 - 3.81 (m, 5 H), 3.45 - 3.63 (m, 36 H), 3.38 - 3.41 (m, 6 H), 3.15 - 3.26 (m, 15 H), 2.75-2.83 (m, 2 H), 2.52 - 2.54 (m, 2 H), 2.28 (t, *J* = 6.4 Hz, 6 H), 2.10 (s, 9 H), 2.00 (s, 9 H), 1.89 (s, 9 H), 1.77 (s, 9 H), 1.07-1.17 (m, 12 H).

³¹PNMR (400 MHz, DMSO-*d*₆) *δ* -147.928, -148.145.

### Example 5: Preparation of a compound E5

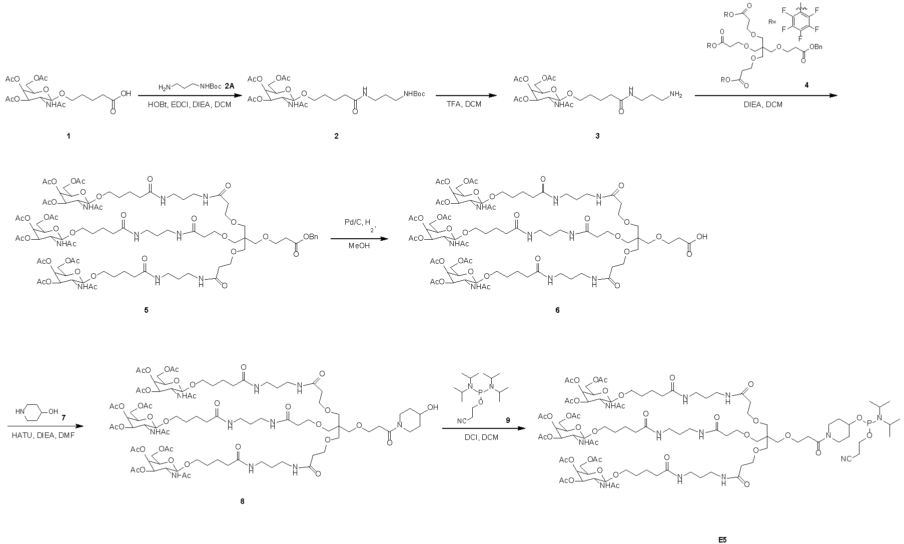

### 1. Preparation of a compound 2

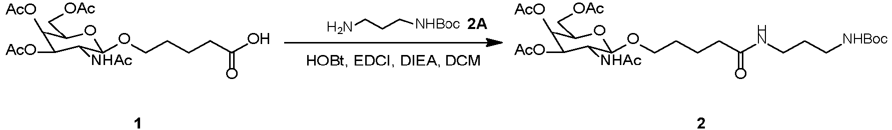

A compound 1 (10 g, 22.349 mmol, prepared by a method of an intermediate 1-3 in Example 25) was dissolved in dichloromethane (300 mL) at 25°C, then a compound 2A (4.67 g, 26.819 mmol), HOBt (3.62 g, 26.819 mmol), EDCI (5.14 g, 26.819 mmol) and DIEA (11.082 mL, 67.048 mmol) were sequentially added, and a reaction solution was stirred at 25°C for 18 h. Thin layer chromatography (dichloromethane/methanol=10/1) showed that the compound 1 was completely consumed and a new point was generated. LCMS showed an MS value of a compound 2. The reaction solution was diluted with 300 mL of dichloromethane, and washed with a saturated citric acid aqueous solution (30 mL×3). An organic phase was washed with a saturated sodium bicarbonate solution (50 mL×3), and then the organic phase was dried with anhydrous sodium sulfate and spin-dried to obtain a white solid compound 2 (about 11.5 g). ¹H NMR (400 MHz, CD₃OD) *δ* 5.33 - 5.37 (m, 1 H), 5.07 (dd, *J=* 3.2, 11.2 Hz, 1 H), 4.54 - 4.62 (m, 1 H), 4.11 - 4.26 (m, 3 H), 4.00 - 4.06 (m, 1 H), 3.85 - 3.93 (m, 1 H), 3.48 - 3.60 (m, 1 H), 3.18 - 3.24 (m, 2 H), 3.05-3.11 (m, 2 H), 2.20 (t, *J =* 7.2 Hz, 2 H), 2.16 (s, 3 H), 2.04 (s, 3 H), 1.92-1.98 (m, 6 H), 1.55 - 1.75 (m, 6 H), 1.45 (s, 9 H). LCMS: m/z=604.1 (M+H)⁺.

### 2. Preparation of a compound 3

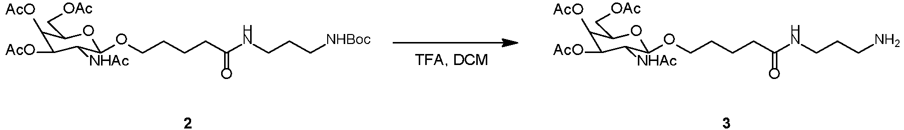

The compound 2 (10 g, 16.565 mmol) was dissolved in dichloromethane (100 mL) at 25°C, TFA (10 mL) was slowly dropped, and a reaction solution was stirred at 25°C for 18 h. Thin layer chromatography (dichloromethane/methanol=10/1) showed that reactants were completely consumed and a new point was generated. The reaction solution was concentrated to dryness to obtain a crude yellow oily liquid compound 3 (about 20 g). ¹H NMR (400 MHz, CD₃OD) *δ* 5.32 - 5.36 (m, 1 H), 5.04 (dd, *J =* 3.2, 11.2 Hz, 1 H), 4.50 - 4.55 (m, 1 H), 3.97 - 4.16 (m, 4 H), 3.85 - 3.92 (m, 1 H), 3.48 - 3.57 (m, 1 H), 3.26 - 3.30 (m, 2 H), 2.90-2.98 (m, 2 H), 2.24 (t, *J =* 7.2 Hz, 2 H), 2.14 (s, 3 H), 2.02 (s, 3 H), 1.92-1.96 (m, 6 H), 1.80 - 1.88 (m, 2 H), 1.63 - 1.73 (m, 2 H), 1.57 - 1.62 (m, 2 H).

### 3. Preparation of a compound 5

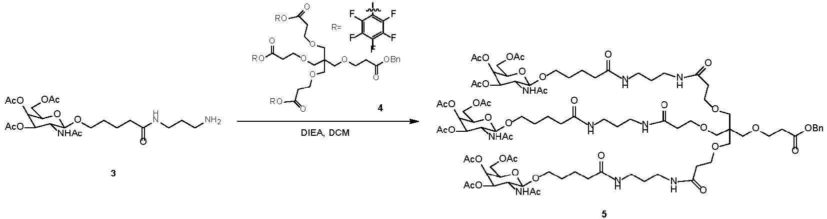

A compound 4 (4.37 g, 4.315 mmol, prepared by a method of the compound 6 in Example 1) was dissolved in dichloromethane (150 mL) at 25°C, then the compound 3 (8.7 g, 17.277 mmol) and DIEA (6 mL, 36.302 mmol) were sequentially added, and a reaction solution was stirred at 25°C for 18 h. Thin layer chromatography (dichloromethane/methanol=10/1) showed that the compound 4 was completely consumed, and thin layer chromatography (petroleum ether/ethyl acetate=5/1) showed that a new product point was generated. LCMS showed an MS value of a compound 5. The reaction solution was diluted with 100 mL of dichloromethane, and washed with a saturated citric acid aqueous solution (30 mL×3). An organic phase was washed with a saturated sodium bicarbonate solution (30 mL×3), and then the organic phase was dried with anhydrous sodium sulfate and spin-dried to obtain a crude product. The crude product was purified by MPLC (petroleum ether/ethyl acetate=1/0 to 10/1) to obtain a white solid compound 5 (about 7.1 g). ¹H NMR (400 MHz, CD₃OD) *δ* 7.30 - 7.40 (m, 5 H), 5.31 - 5.36 (m, 3 H),5.15(s, 2 H), 5.06 (dd, *J=* 3.2, 11.2 Hz, 3 H), 4.52 - 4.58 (m, 3 H), 4.10 - 4.32 (m, 12 H), 3.83 - 3.90 (m, 3 H), 3.67 - 3.73 (m, 1 H), 3.62 - 3.66 (m, 2 H), 3.56 - 3.61 (m, 6 H), 3.48-3.55 (m, 3 H), 3.33-3.36 (m, 5 H), 3.17-3.24 (m, 13 H), 2.60 (t, *J =* 6.0 Hz, 2 H), 2.39 (t, *J* = 6.0 Hz, 6 H), 2.20 (t, *J* = 7.2 Hz, 6 H), 2.14 (s, 9 H), 2.02 (s, 9 H), 1.91-1.96 (m, 18 H), 1.62 - 1.73 (m, 13 H), 1.52 - 1.62 (m, 6 H). LCMS: m/z=987.6 (M/2+H)⁺.

### 4. Preparation of a compound 6

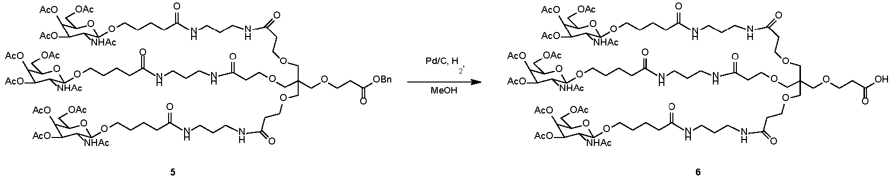

The compound 5 (7.1 g, 3.602 mmol) was dissolved in methanol (100 mL) at 25°C, Pd/C (0.5 g, 4.698 mmol) with a mass fraction of 10% was added, and a reaction solution was stirred in a hydrogen atmosphere (14.696 psi) at 25°C for 18 h. Thin layer chromatography (dichloromethane/methanol=8/1) showed that the compound 5 was completely consumed and a new point was generated. The reaction solution was filtered, and a filtrate was spin-dried to obtain a compound 6 (about 6.7 g).

### 5. Preparation of a compound 8

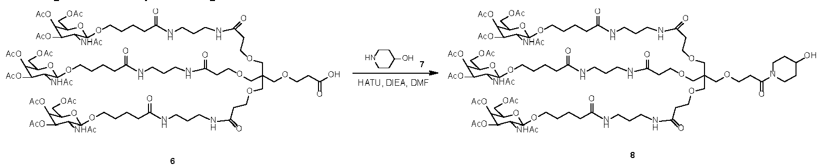

The compound 6 (1.50 g, 0.797 mmol), HATU (0.450 g, 1.196 mmol) and DIEA (0.395 mL, 2.392 mmol) were added to DMF (15.0 mL), nitrogen replacement was carried out for three times, a reaction was carried out at 20°C for 30 min, and then a compound 7 (0.120 g, 1.19 mmol) was added. A reaction solution was enabled to undergo to a reaction at 20°C for 2 h. Thin layer chromatography (dichloromethane:methanol=8/1) showed that raw materials disappeared and a new point was generated. The reaction solution was diluted with dichloromethane (100 mL), and sequentially washed with a saturated sodium bicarbonate aqueous solution (30.0 mL×3) and a saturated salt solution (30.0 mL×3). An organic phase was dried with anhydrous sodium sulfate and then concentrated under reduced pressure to obtain a crude product. The crude product was purified by column chromatography (dichloromethane:methanol=100/1 to 8/1) to obtain a yellow solid compound 8 (about 1.00 g). ¹H NMR (400 MHz, CD₃OD) *δ* 5.31 - 5.36 (m, 3 H), 5.06 (dd, *J =* 3.2, 11.2 Hz, 3 H), 4.50 - 4.58 (m, 3 H), 4.07 - 4.23 (m, 10 H), 3.98-4.06 (m, 4 H), 3.84-3.92 (m, 5 H), 3.60 - 3.65 (m, 8 H), 3.50 - 3.56 (m, 3 H), 3.33 - 3.36 (m, 8 H), 3.18 - 3.26 (m, 13 H), 2.60 - 2.68 (m, 2 H), 2.41 (t, *J =* 6.0 Hz, 6 H), 2.21 (t, *J* = 7.2 Hz, 6 H), 2.14 (s, 9 H), 2.03 (s, 9 H), 1.90-1.98 (m, 20 H), 1.56 - 1.74 (m, 20 H). LCMS: m/z=983.1 (M/2+H)⁺.

### 6. Preparation of E5

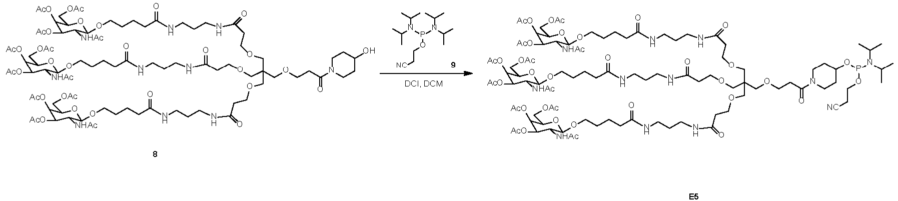

The compound 8 (1 g, 0.509 mmol) was dissolved in dichloromethane (10 mL) at 25°C, DCI (0.03 g, 0.255 mmol) and a compound 9 (0.31 g, 1.018 mmol) were sequentially added, and a reaction solution was stirred in a nitrogen atmosphere at 25 °C for 2 h. LCMS showed that the compound 8 was completely consumed and an MS value of E5 was obtained. The reaction solution was cooled to 0°C in an ice salt bath, 6 mL of a saturated sodium bicarbonate solution and 6 mL of a saturated salt solution were added to the reaction solution, and 50 mL of dichloromethane was added. Then, an organic phase was washed with a mixed solution (20 mL×3) of saturated sodium bicarbonate and a saturated salt solution at 1:1, dried with anhydrous sodium sulfate and spin-dried to obtain a crude product. The crude product was dissolved in 15 mL of dichloromethane. A crude product solution was slowly dropped to MTBE in a dry ice bath to precipitate a white solid and then filtered to obtain a white solid E5 (722 mg,, 0.222 mmol, 43.61%).

¹H NMR (400 MHz, DMSO-*d*₆) *δ* 7.80 - 7.85 (m, 6 H), 7.70 - 7.76 (m, 3 H), 5.19 - 5.23 (m, 3 H), 4.93 - 5.00 (m, 3 H), 4.45 - 4.51 (m, 3 H), 3.99 - 4.05 (m, 10 H), 3.83 - 3.90 (m, 3 H), 3.64 - 3.74 (m, 6 H), 3.56 - 3.62 (m, 2 H), 3.47 - 3.55 (m, 9 H), 3.36 - 3.44 (m, 4 H), 3.21 (s, 8 H), 2.98 - 3.07 (m, 13 H), 2.76 (t, *J =* 6.0 Hz, 2 H), 2.53 - 2.58 (m, 2 H), 2.26 (t, *J =* 6.0 Hz, 6 H), 2.10 (s, 9 H), 2.04 (t, *J =* 7.2 Hz, 6 H), 2.00 (s, 9 H), 1.89 (s, 9 H), 1.77 (s, 9 H), 1.38 - 1.55 (m, 22 H), 1.06 - 1.17 (m, 12 H).

³¹PNMR (400 MHz, DMSO-*d*₆) *δ* -145.243.

LCMS: m/z=1041.7 ((M-83)/2+H)⁺.

### Example 6: Preparation of a compound E6

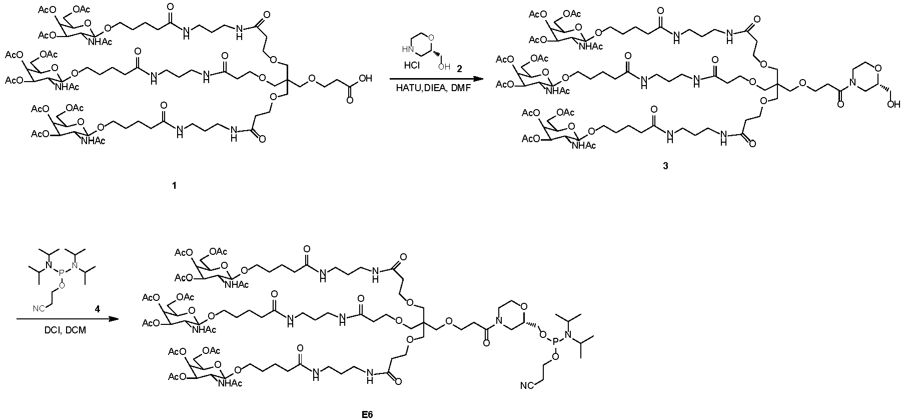

### 1. Preparation of a compound 3

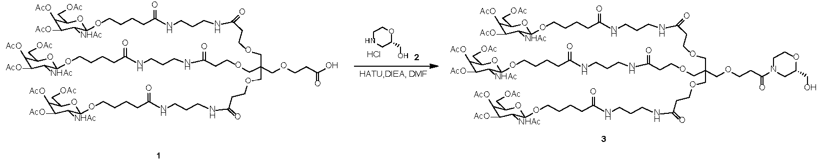

A compound 1 (1.50 g, 0.797 mmol, prepared by a method of the compound 6 in Example 5), HATU (0.450 g, 1.19 mmol) and DIEA (0.395 mL, 2.39 mmol) were added to DMF (15.0 mL), nitrogen replacement was carried out for three times, a reaction was carried out at 20°C for 30 min, and then a compound 2 (0.180 g, 1.19 mmol) was added. A reaction solution was enabled to undergo to a reaction at 20°C for 2 h. Thin layer chromatography (dichloromethane:methanol=8/1) showed that raw materials disappeared and a new point was generated. The reaction solution was diluted with dichloromethane (100 mL), and sequentially washed with a saturated sodium bicarbonate aqueous solution (30.0 mL×3) and a saturated salt solution (30.0 mL×3). An organic phase was dried with anhydrous sodium sulfate and then concentrated under reduced pressure to obtain a crude product. The crude product was purified by column chromatography (dichloromethane:methanol=100/1 to 8/1). A yellow solid compound 3 (about 800 mg) was obtained. ¹H NMR (400 MHz, CD₃OD) *δ* 5.33 (d, *J*=3.2 Hz, 3 H), 5.02 - 5.10 (m, 3 H), 4.55 (d, *J*=8.4 Hz, 3 H), 4.07 - 4.20 (m, 9 H), 3.99 - 4.06 (m, 4 H), 3.94 - 3.98 (m, 1 H), 3.85-3.90 (m, 3 H), 3.48 - 3.68 (m, 15 H), 3.34 (s, 8 H), 3.18 - 3.27 (m, 13 H), 2.62 - 2.69 (m, 2 H), 2.40 (t, *J =* 6.0 Hz, 6 H), 2.21 (t, *J =* 7.2 Hz, 6 H), 2.14 (s, 9 H), 2.03 (s, 9 H), 1.91 - 1.96 (m, 18 H), 1.54 - 1.74 (m, 20 H). LCMS: m/z=990.7 (M/2+H)⁺.

### 2. Preparation of E6

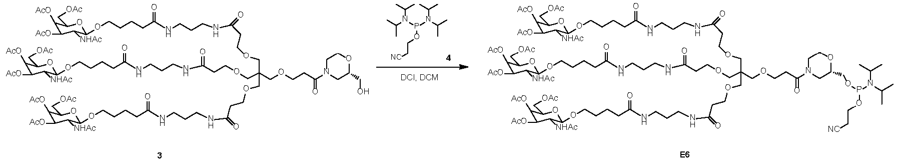

The compound 3 (800 mg, 0.404 mmol) was dissolved in dichloromethane (10 mL) at 25°C, DCI (23.86 mg, 0.202 mmol) and a compound 4 (0.31 g, 1.018 mmol) were sequentially added, and a reaction solution was stirred in a nitrogen atmosphere at 25°C for 2 h. LCMS showed that the compound 3 was completely consumed and an MS value of E6 was obtained. The reaction solution was cooled to 0°C in an ice salt bath, 6 mL of a saturated sodium bicarbonate solution and 6 mL of a saturated salt solution were added to the reaction solution, and 50 mL of dichloromethane was added. Then, an organic phase was washed with a mixed solution (20 mL×3) of saturated sodium bicarbonate and a saturated salt solution at 1:1, dried with anhydrous sodium sulfate and spin-dried to obtain a crude product. The crude product was dissolved in 15 mL of dichloromethane. A crude product solution was slowly dropped to MTBE in a dry ice bath to precipitate a white solid and then filtered to obtain a white solid E6 (595 mg,, 0.117 mmol, 29.07%).

¹H NMR (400 MHz, DMSO-*d*₆) *δ* 7.79 - 7.86 (m, 6 H), 7.70 - 7.77 (m, 3 H), 5.21 (d, *J =* 3.2 Hz, 3 H), 4.96 (dd, *J =* 3.2, 11.2 Hz, 3 H), 4.48 (d, *J =* 8.0 Hz, 3 H), 4.02 (s, 9 H), 3.80 - 3.92 (m, 5 H), 3.66 - 3.79 (m, 6 H), 3.47 - 3.65 (m, 13 H), 3.36 - 3.45 (m, 5 H), 3.21 (s, 8 H), 2.96 - 3.07 (m, 13 H), 2.74 - 2.81 (m, 2 H), 2.54 - 2.56 (m, 2 H), 2.26 (t, *J =* 6.0 Hz, 6 H), 2.10 (s, 9 H), 2.04 (t, *J =* 7.2 Hz, 6 H), 2.00 (s, 9 H), 1.89 (s, 9 H), 1.77 (s, 9 H), 1.41 - 1.53 (m, 18 H), 1.14 (d, *J=* 6.4 Hz, 12 H).

³¹PNMR (400 MHz, DMSO-*d*₆) *δ* -147.942.

LCMS: m/z=1049.4 (M/2+H)⁺.

### Example 7: Preparation of a compound E7

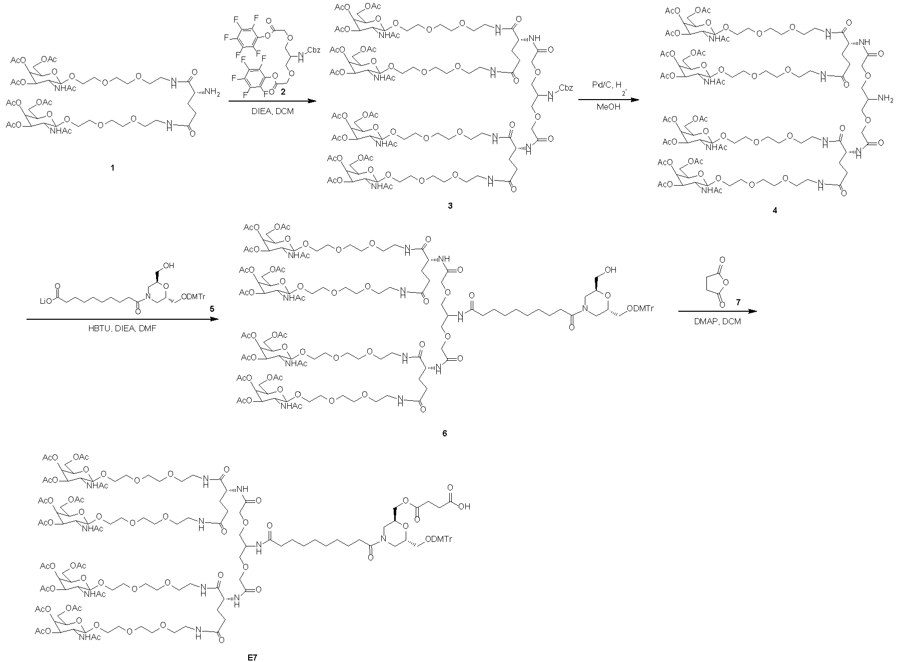

### 1. Preparation of a compound 3

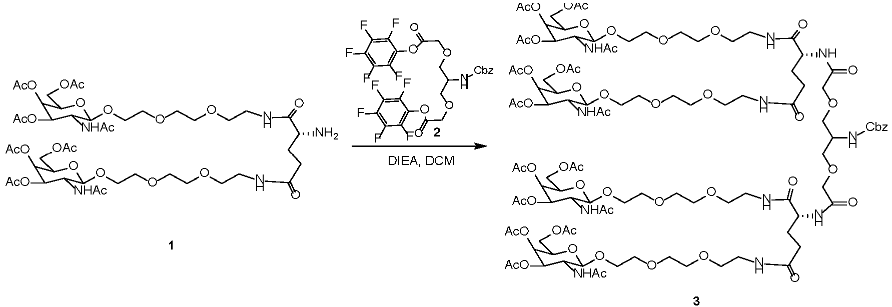

A compound 1 (2.00 g, 1.87 mmol, prepared by a method of an intermediate 3-3 in Example 24) was dissolved in DCM (20.0 mL) at room temperature, DIEA (0.135 mL, 0.814 mmol) and a compound 2 (0.550 g, 0.814 mmol) were sequentially added to a resulting solution, and nitrogen replacement was carried out for 3 times. A reaction mixture was stirred at 25°C for 16 h. Liquid chromatography-mass spectrometry detected an MS response of a product. Thin layer chromatography (dichloromethane/methanol=5/1) showed that raw materials disappeared and a new point was generated. A reaction solution was concentrated under reduced pressure, and a resulting crude product was purified by column chromatography (dichloromethane/methanol=5/1) to obtain a white solid 3 (about 780 mg). ¹H NMR (400 MHz, CD₃OD) *δ* 7.28-7.42 (m, 5H), 5.30-5.34 (m, 4H), 5.04-5.14 (m, 6H), 4.63-4.67 (m, 4H), 4.36-4.44 (m, 2H), 4.00-4.20 (m, 23H), 3.91-3.95 (m, 4H), 3.69-3.77 (m, 9H), 3.52-3.67 (m, 32H), 3.34-3.43 (m, 9H), 2.29-2.31 (m, 4H), 2.14 (s, 12H), 2.03 (s, 12H), 1.92-1.96 (m, 24H). LCMS: m/z=1221.6 (M/2+H)⁺.

### 2. Preparation of a compound 4

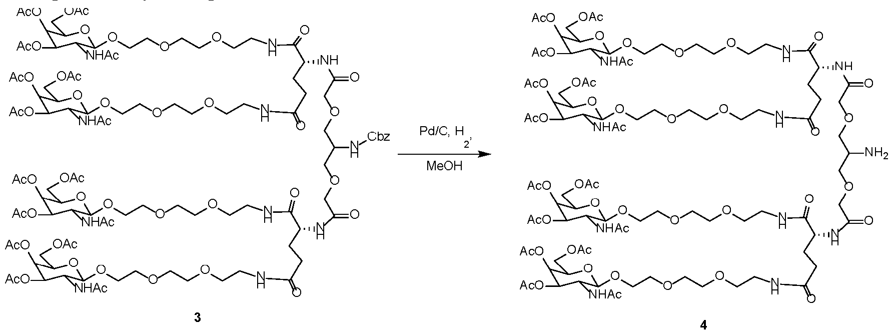

The compound 3 (1.10 g, 0.451 mmol) was dissolved in MeOH (10.0 mL) at room temperature, wet Pd/C (0.050 g, 0.451 mmol) with a mass fraction of 10% was added to a resulting solution, hydrogen replacement was carried out for 3 times, and a reaction mixture was stirred in a hydrogen atmosphere (14.696 psi) at 25°C for 18 h. Liquid chromatography-mass spectrometry detected an MS response of a product. Thin layer chromatography (dichloromethane/methanol=10/1, with phosphomolybdic acid as a color developing agent) showed that raw materials were completely consumed and a new point was generated. A reaction solution was filtered, and a filtrate was concentrated under reduced pressure to obtain a white solid 4 (about 840 mg). ¹H NMR (400 MHz, CD₃OD) *δ* 5.32-5.34 (m 4H), 5.06-5.10 (m, 4H), 4.63-4.65 (m, 4H), 4.38-4.40 (m, 2H), 3.99-4.20 (m, 20H), 3.90-3.97 (m, 4H), 3.69-3.76 (m, 6H), 3.50-3.68 (m, 36H), 3.35-3.44 (m, 11H), 2.28-2.38 (m, 4H), 2.15 (s, 12H), 2.03 (s, 12H), 1.90-1.94 (m, 24H). LCMS: m/z=1154.7 (M/2+H)⁺.

### 3. Preparation of a compound 6

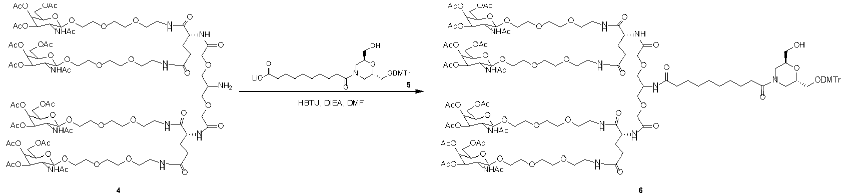

A compound 5 (232 mg, 0.364 mmol, prepared by a method of a compound Int-6 in Example 13) was dissolved in DCM (10.0 mL) at room temperature, HBTU (207 mg, 0.546 mmol), DIEA (0.181 mL, 1.09 mmol) and the compound 4 (840 mg, 0.364 mmol) were sequentially added to a resulting solution, and nitrogen replacement was carried out for 3 times. A reaction mixture was stirred at 25°C for 1 h. Liquid chromatography-mass spectrometry detected that raw materials disappeared. Thin layer chromatography (dichloromethane/methanol=5/1) showed that the raw materials disappeared and a new point was generated. A reaction solution was concentrated under reduced pressure, and a resulting crude product was purified by column chromatography (dichloromethane/methanol=8/1 to 5/1) to obtain a white solid 6 (about 620 mg). ¹H NMR (400 MHz, CD₃OD) *δ* 7.41-7.43 (m, 2H), 7.23-7.34 (m, 7H), 6.83-6.90 (m, 4H), 5.31-5.35 (m, 4H), 5.01-5.12 (m, 4H), 4.63-4.65 (m, 4H), 4.41-4.45 (m, 2H), 4.31-4.33 (m, 1H), 3.99-4.22 (m, 22H), 3.87-3.97 (m, 6H), 3.58-3.81 (m, 45H), 3.34-3.43 (m, 10H), 2.19-2.40 (m, 10H), 2.14 (s, 12H), 2.02 (s, 12H), 1.92-1.96 (mz, 24H), 1.48-1.63 (m, 4H), 1.28-1.38 (m, 8H). LCMS: m/z=1460.0 (M/2+H)⁺.

### 4. Preparation of E7

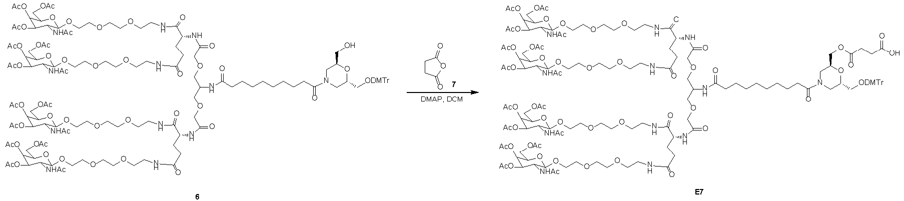

The compound 6 (300 mg, 0.103 mmol) was dissolved in DCM (10.0 mL) at room temperature, DIEA (0.102 mL, 0.618 mmol), a compound 7 (10.3 mg, 0.103 mmol) and DMAP (12.6 mg, 0.103 mmol) were sequentially added to a resulting solution, and nitrogen replacement was carried out for 3 times. A reaction mixture was stirred at 25°C for 2 h. Liquid chromatography-mass spectrometry detected that raw materials disappeared. A reaction solution was concentrated under reduced pressure, and a resulting crude product was prepared and separated by preparative high performance liquid chromatography (preparative-HPLC, column: Waters Xbridge BEH C18 100*30mm*10um; mobile phase: water-ACN; B%: 17%-57%, 5 min) to obtain a white solid E7 (53.0 mg, yield: 17.08%, purity: 78.94%).

¹H NMR (400 MHz, CD₃OD) *δ* 7.41-7.45 (m, 2H), 7.17-7.34 (m, 7H), 6.85-6.89 (m, 4H), 5.32-5.36 (m, 4H), 5.03-5.13 (m, 4H), 4.63-4.67 (m, 4H), 4.38-4.47 (m, 2H), 4.32-4.34 (m, 1H), 4.01-4.26 (m, 22H), 3.88-4.00 (m, 6H), 3.77-3.81 (m, 7H), 3.49-3.76 (m, 45H), 3.33-3.47 (m, 10H), 2.56-2.62 (m, 2H), 2.45-2.55 (m, 3H), 2.21-2.38 (m, 7H), 2.14 (s, 12H), 2.05-2.11 (m, 2H), 2.02 (s, 12H), 1.92-1.96 (m, 24H), 1.47-1.68 (m, 4H), 1.28-1.34 (m, 8H).

MS: m/z=3022.36 (M+H)⁺.

### Example 8: Preparation of a compound E8

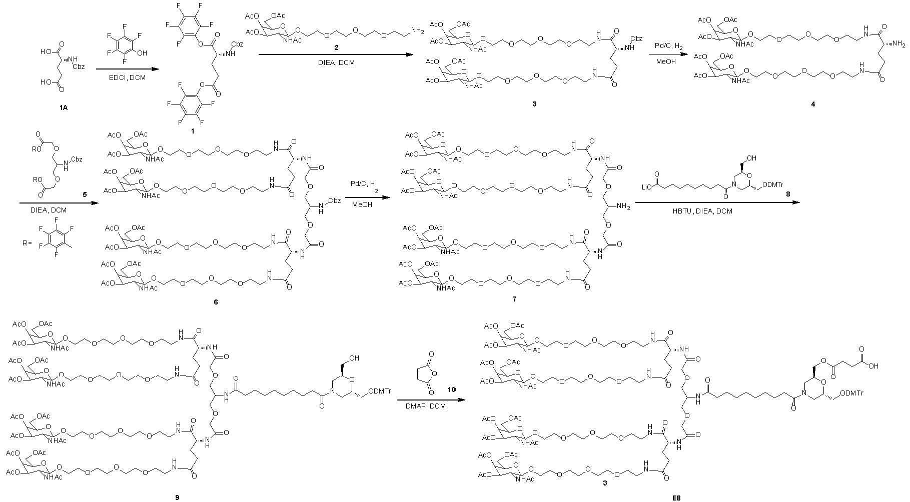

### 1. Preparation of a compound 1

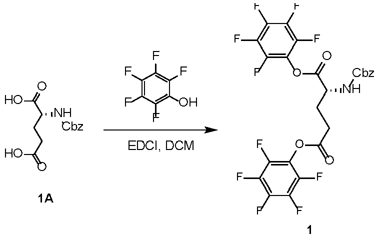

A compound 1A (5.00 g, 17.8 mmol) was added to DCM (200 mL) at 25°C, then a compound pentafluorophenol (8.18 g, 44.4 mmol) and EDCI (10.2 g, 53.3 mmol) were sequentially added, and a reaction solution was stirred at 25°C for 14 h. Thin layer chromatography (petroleum ether/ethyl acetate=5/1) showed that a new point was generated. The reaction solution was concentrated under vacuum to obtain a crude product, and the crude product was purified by column chromatography (petroleum ether/ethyl acetate=1/0 to 5/1) to obtain a white solid compound 1 (about 9.00 g). ¹H NMR (400 MHz, CDCl₃) *δ* 7.34-7.38 (m, 5H), 5.39-5.41 (m, 1H), 5.19 (s, 2H), 4.87-4.92 (m, 1H), 2.86-2.97 (m, 2H), 2.55-2.58 (m, 1H), 2.28-2.34 (m, 1H).

### 2. Preparation of a compound 3

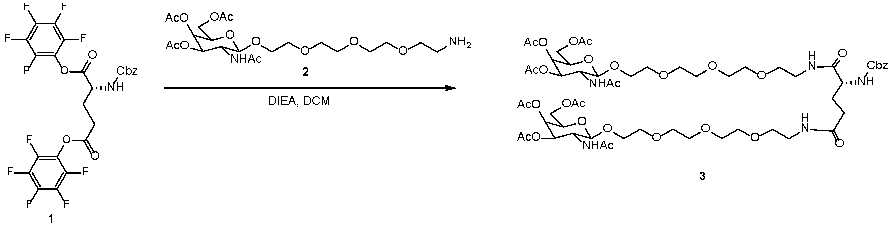

The compound 1 (800 mg, 1.30 mmol) was dissolved in DCM (20.0 mL), DIEA (421 mg, 3.26 mmol) and a compound 2 (2,076 mg, 3.26 mmol, prepared by a method of an intermediate 5 in Example 24) were added, and a reaction solution was stirred at 25°C for 18 h. LC/MS detected an MS response of a product. A point plate (DCM:MeOH=10:1) showed that raw materials were completely reacted. The reaction solution was directly concentrated, filtered with a normal-phase silica gel column and eluted with MeOH and DCM at 0:1 to 1:10 to obtain a light yellow solid compound 3 (about 1.3 g). LCMS: m/z=1290.4 (M+H)⁺.

### 3. Preparation of a compound 4

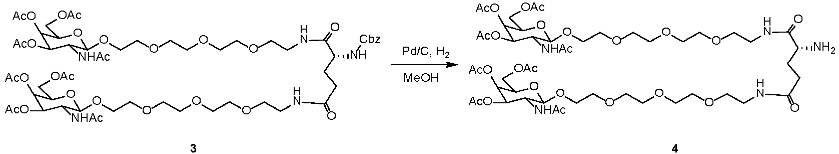

The compound 3 (1.30 g, 1.01 mmol) was dissolved in MeOH (30 mL), Pd/C (0.20 g, 1.88 mmol) with a mass fraction of 10% was added, hydrogen replacement was carried out for 3 times, and a reaction solution was enabled to undergo a reaction in an H₂ atmosphere (15 psi) at 25 °C for 18 h. LC/MS detected an MS response of a product. The reaction solution was filtered to remove the Pd/C and then washed with methanol, and filtrates were combined and concentrated to obtain a yellow oily compound 4 (about 1.08 g). LCMS: m/z=1156.9 (M+H)⁺.

### 4. Preparation of a compound 6

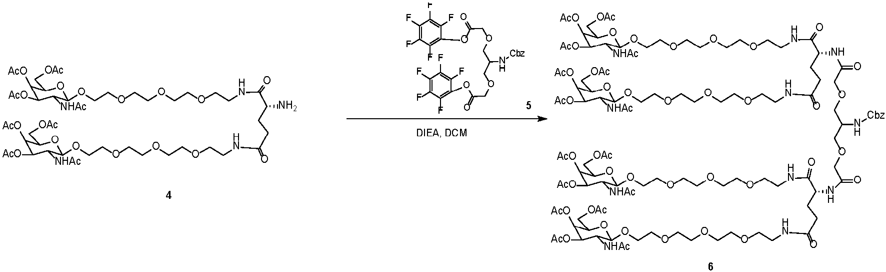

The compound 4 (1,080 mg, 0.934 mmol) was dissolved in DCM (20 mL), a compound 5 (302.2 mg, 0.449 mmol, prepared by a method of the compound 2 in Example 7) and DIEA (0.17 mL, 0.934 mmol) were added, and a reaction solution was stirred at 25°C for 18 h. LC/MS detected an MS response of a product. TLC (DCM:MeOH=10:1) showed that a new point was generated. The reaction solution was directly concentrated and filtered with a column (from DCM:MeOH=1:0 to DCM:MeOH=10:1) to obtain a compound 6 (about 820 mg). LCMS: m/z=1309.5 (M/2+H)⁺.

### 5. Preparation of a compound 7

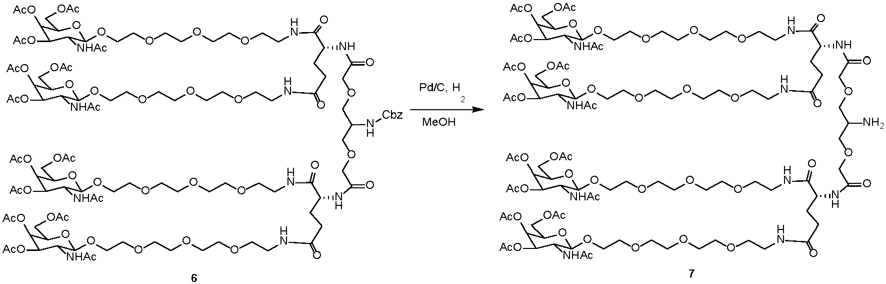

The compound 6 (800 mg, 0.306 mmol) was dissolved in MeOH (10 mL), Pd/C (100 mg, 0.940 mmol) with a mass fraction of 10% was added, hydrogen replacement was carried out for 3 times, and a reaction solution was enabled to undergo a reaction in an H₂ atmosphere (15 psi) at 25 °C for 18 h. LC/MS detected an MS response of a product. The reaction solution was filtered to remove the Pd/C and then washed with methanol, and filtrates were combined and concentrated to obtain a white solid compound 7 (about 700 mg). LCMS: m/z=1242.4 (M/2+H)⁺.

### 6. Preparation of a compound 9

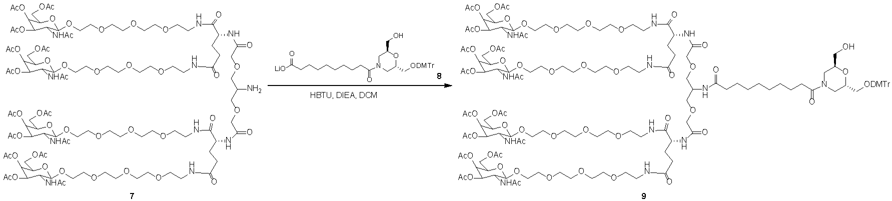

The compound 7 (700 mg, 0.282 mmol) was dissolved in DCM (20 mL), an HBTU reagent (160 mg, 0.423 mmol), DIEA (0.140 mL, 0.846 mmol) and a compound 8 (180 mg, 0.282 mmol, prepared by a method of a compound Int-6 in Example 13) were sequentially added, and a reaction solution was stirred at 25°C for 18 h. LC/MS detected an MS response of a product. TLC (DCM:MeOH=10:1) showed that raw materials were completely reacted and a new point was generated. The reaction solution was washed with 20 ml of a saturated sodium bicarbonate aqueous solution and extracted with dichloromethane (20 ml×2). Organic phases were combined, concentrated and filtered with a normal-phase column (DCM:MeOH=1:0 to 10:1) to obtain a white solid compound 9 (about 750 mg). LCMS: m/z=1399.1 [(M-DMTr)/2+H]⁺.

### 7. Preparation of E8

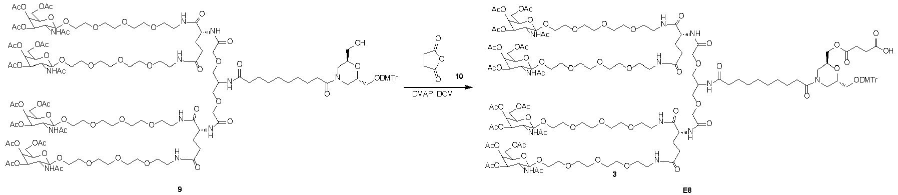

The compound 9 (500 mg, 0.161 mmol) was dissolved in DCM (5 mL), a compound 10 (96.9 mg, 0.968 mmol), DIEA (0.160 mL, 0.968 mmol) and DMAP (19.7 mg, 0.161 mmol) were added, and a reaction solution was stirred at 25°C for 18 h. LCMS detected an MS response of a product. The reaction solution was directly spin-dried, dissolved in 5 ml of acetonitrile and treated by preparative-HPLC (chromatographic column: Waters Xbridge BEH C18 100*30mm*10um; mobile phase: water-acetonitrile; B%: 20%-50%, 11 min; flow rate: 50 ml/min) to obtain a white solid compound E8. LCMS (ESI): m/z=1449.0 [(M-DMTr)/2+H]⁺; ¹H NMR (400 MHz, CD₃OD) δ 7.43(d, *J* = 8.0 Hz, 2H), 7.30-7.32 (m, 6H), 7.20-7.25 (m, 1H), 6.85-6.89 (m, 4H), 5.33 (d, *J =* 3.2 Hz, 4H), 5.06 (dd, *J* = 11.2, 3.2 Hz, 4H), 4.66 (d, *J* = 8.8 Hz, 4H), 4.33-4.43 (m, 4H), 4.09-4.17 (m, 12H), 4.01-4.04 (m, 8H), 3.89-3.94 (m, 4H), 3.79 (d, *J =* 2.0 Hz, 6H), 3.71-3.76 (m, 9H), 3.60-3.66 (m, 48H), 3.52-3.58 (m, 8H), 3.37-3.40 (m, 8H), 2.54-2.60 (m, 4H), 2.26-2.31 (m, 8H), 2.14 (s, 12H), 2.02 (s, 12H), 1.96-2.00 (m, 2H), 1.94 (d, *J=* 4.0 Hz, 24H), 1.53-1.61 (m, 4H), 1.29-1.38 (m, 10H).

### Example 9: Preparation of a compound E9

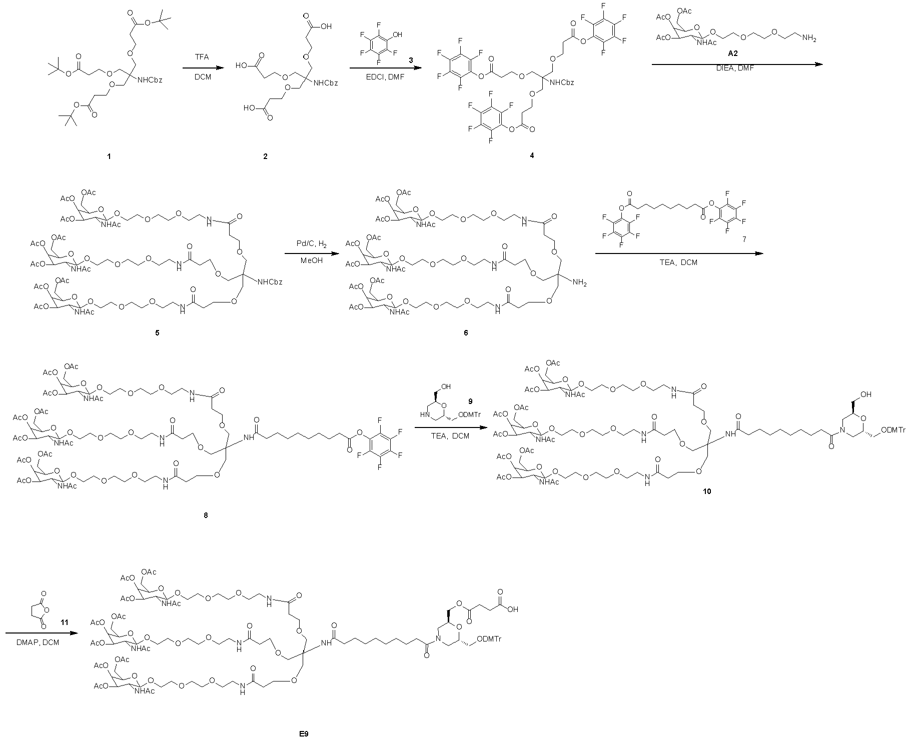

### 1. Preparation of a

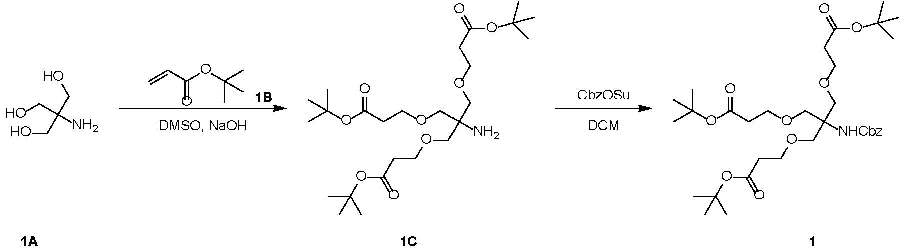

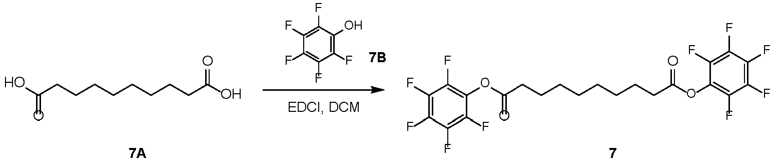

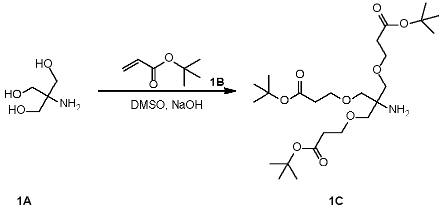

At room temperature, a compound 1A (10 g, 82.6 mmol), a compound 1B (42.3 g, 330 mmol) and NaOH (5 M, 1.65 mL) were sequentially added to DMSO (300 mL) to carry out a reaction at 24°C for 12 h. Thin layer chromatography (dichloromethane:methanol=10/1) showed that a new point was generated. A reaction solution was diluted with water (1,000 mL) and then extracted with dichloromethane (500 ml×3). An organic phase was dried with anhydrous sodium sulfate and then concentrated under reduced pressure to obtain a colorless oily compound 1C (45 g). ¹H NMR (400 MHz, CDCl₃) δ 3.62 - 3.70 (m, 6 H) 3.25 - 3.49 (m, 6 H), 2.41 - 2.52 (m, 6 H), 1.45 (s, 27 H).

### 2. Preparation of a compound 1

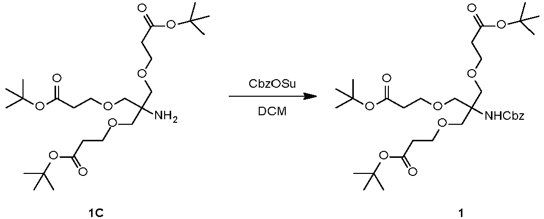

At room temperature, the compound 1C (30.0 g, 59.3 mmol), CbzOSu (17.8 g, 71.2 mmol) and DIEA (11.5 g, 89 mmol) were sequentially added to DCM (300 mL). A reaction was carried out at 25°C for 14 h. Thin layer chromatography (petroleum ether/ethyl acetate=3/1) showed that a new point was generated. A reaction solution was diluted with water (500 mL) and then extracted with dichloromethane (200 ml×3). An organic phase was concentrated under reduced pressure, and a crude product was purified by column chromatography (petroleum ether/ethyl acetate=30/1 to 2/1) to obtain a colorless oily compound 1 (about 19.0 g). ¹H NMR (400 MHz, CDCl₃) δ 7.29 - 7.39 (m, 5 H), 5.30 (s, 1 H), 5.04 (s, 2 H), 3.57 - 3.72 (m, 12 H), 2.44 (t, *J =* 6.4 Hz, 6 H), 1.44 (s, 27 H).

### 3. Preparation of a compound 2

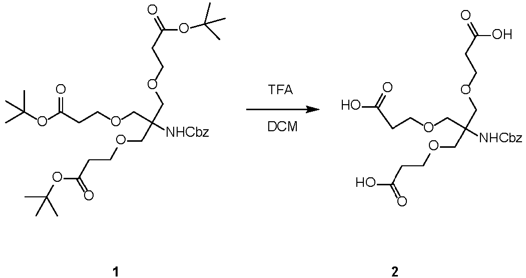

The compound 1 (8.00 g, 21.083 mmol) was dissolved in DCM (30 mL) at room temperature, and TFA (10 mL, 130.591 mmol) was added and stirred at 25°C for 15 h. Liquid chromatography-mass spectrometry showed that raw materials were completely reacted and a product was generated. A reaction solution was directly concentrated under reduced pressure to obtain a crude colorless oily compound 2 (about 7.27 g). ¹H NMR (400 MHz, CD₃OD) *δ* 7.26-7.38 (m, 5H), 5.03 (s, 2H), 3.59-3.70 (m, 12H), 2.51 (t, *J =* 6.0 Hz, 6H). LCMS: m/z=472.1 (M+H)⁺.

### 4. Preparation of a compound 4

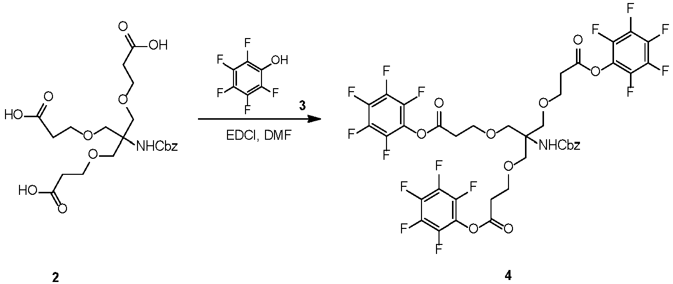

The compound 2 (1.00 g, 2.121 mmol) was dissolved in DMF (30 mL) at room temperature, and EDCI (2.44 g, 12.726 mmol) and a compound 3 (2.34 g, 12.726 mmol) were added and stirred at 25°C for 2 h. Thin layer chromatography (petroleum ether/ethyl acetate=5/1) showed that raw materials disappeared and a new point was generated. A reaction solution was concentrated under reduced pressure to obtain a crude product, and the crude product was purified by column chromatography (petroleum ether/ethyl acetate=20/3 to 5/1). A white solid 4 (about 1.24 g) was obtained. ¹H NMR (400 MHz, CD₃OD) *δ* 7.26-7.38 (m, 5H), 4.98 (s, 2H), 3.80(t, *J =* 5.6 Hz, 6H), 3.72 (s, 6H), 2.93 (t, *J =* 5.2 Hz, 6H).

### 5. Preparation of a compound 5

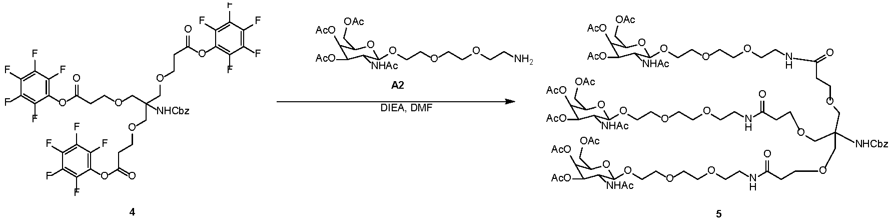

At room temperature, the compound 4 (1.24 g, 1.279 mmol) was dissolved in DMF (40 mL), and a compound A2 (1.84 g, 3.837 mmol, prepared by a method of an intermediate 3-4 in Example 23) and DIEA (2.114 mL, 12.789 mmol) were added and stirred at 25°C for 15 h. Thin layer chromatography (dichloromethane:methanol=10/1, with phosphomolybdic acid as a color developing agent)) showed that raw materials disappeared and a new point was generated. A reaction solution was concentrated under reduced pressure, and a crude product was purified by column chromatography (dichloromethane/methanol=25/2 to 10/1). A white solid 5 (about 1.60 g) was obtained. ¹H NMR (400 MHz, CD₃OD) *δ* 7.27-7.41 (m, 5H), 5.34 (d, *J =* 3.6 Hz, 3H), 5.07 (dd, *J =* 3.2, 11.2 Hz, 3H), 5.04 (s, 2H), 4.64 (d, *J =* 8.4 Hz, 3H), 4.06-4.19 (m, 9H), 4.00-4.05 (m, 3H), 3.90-3.96 (m, 3H), 3.70-3.75 (m, 3H), 3.59-3.70 (m, 30H), 3.51-3.56 (m, 6H), 3.34-3.39 (m, 6H), 2.43 (t, *J =* 6.0 Hz, 6H), 2.14 (s, 9H), 2.03 (s, 9H), 1.95 (s, 9H), 1.93 (s, 9H).

### 6. Preparation of a compound 6

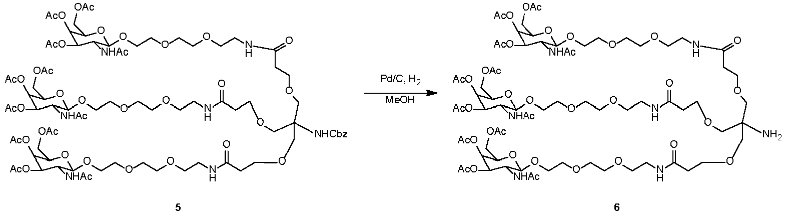

The compound 5 (1.6 g, 0.864 mmol) was dissolved in MeOH (20 mL) at room temperature, Pd/C (0.3 g, 2.819 mmol) with a mass fraction of 10% was added, H₂ replacement was carried out for 3 times, and stirring was carried out at 25°C for 15 h. Thin layer chromatography (dichloromethane:methanol=10/1) showed that raw materials disappeared and a new point was generated. Liquid chromatography-mass spectrometry showed that the raw materials were completely reacted and a product was generated. A reaction solution was filtered with diatomite, and a filtrate was concentrated under reduced pressure to obtain a colorless transparent solid 6 (about 1.40 g). ¹H NMR (400 MHz, CD₃OD) *δ* 5.34 (d, *J =* 2.8 Hz, 3H), 5.07 (dd, *J =* 3.2, 11.2 Hz, 3H), 4.65 (d, *J =* 8.4 Hz, 3H), 4.06-4.20 (m, 9H), 4.01-4.06 (m, 3H), 3.90-3.98 (m, 3H), 3.72-3.76 (m, 3H), 3.52-3.70 (m, 30H), 3.36-3.41 (m, 12H), 2.46 (t*, J =* 6.0 Hz, 6H), 2.15 (s, 9H), 2.03 (s, 9H), 1.95 (s, 9H), 1.94 (s, 9H). LCMS: m/z=860.1 (M/2+H)⁺.

### 7. Preparation of a compound 7

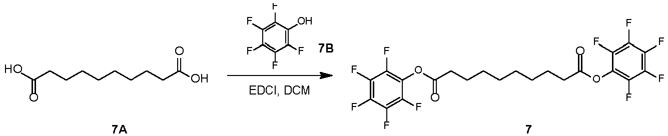

A compound 7A (20 g, 98.888 mmol) was added to DCM (500 mL) at 25°C, then a compound 7B (45.51 g, 247.3219 mmol) and EDCI (56.87 g, 296.663 mmol) were sequentially added, and a reaction solution was stirred at 25°C for 18 h. Thin layer chromatography (petroleum ether/ethyl acetate=1/1) showed that reactants were completely consumed, and thin layer chromatography (petroleum ether/ethyl acetate=5/1) showed that a new product point was generated. The reaction solution was concentrated under vacuum to obtain a crude product, and the crude product was purified by MPLC to obtain a white solid compound 7 (about 48.7 g). ¹H NMR (400 MHz, CDCl₃) *δ* 2.68 (t, *J =* 7.6 Hz, 4 H), 1.72-1.87 (m, 4 H), 1.34 - 1.51 (m, 8 H).

### 8. Preparation of a compound 8

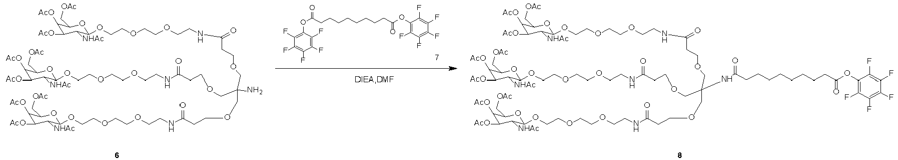

The compound 6 (1.00 g, 0.582 mmol) was dissolved in DMF (20 mL) at room temperature, and the compound 7 (0.93 g, 1.745 mmol) and DIEA (0.481 mL, 2.909 mmol) were added to carry out a reaction at 25°C for 15 h. Thin layer chromatography (dichloromethane:methanol=10:1) showed that raw materials disappeared and a new point was generated. A reaction solution was concentrated under reduced pressure, and a crude product was purified by column chromatography (dichloromethane:methanol=25/2 to 10/1) to obtain a colorless oily liquid 8 (about 809 mg). ¹H NMR (400 MHz, CD₃OD) δ 5.34 (d, *J =* 2.8 Hz, 3H), 5.07 (dd, *J* = 3.2, 11.2 Hz, 3H), 4.65 (d, *J =* 8.4 Hz, 3H), 4.06-4.18 (m, 9H), 4.01-4.05 (m, 3H), 3.90-3.98 (m, 3H), 3.61-3.76 (m, 35H), 3.54-3.58 (m, 6H), 3.36-3.41 (m, 6H), 2.73 (t, *J* = 7.2 Hz, 2H), 2.45 (t, *J* 6.0 Hz, 6H), 2.14 (s, 9H), 2.03 (s, 9H), 1.90-1.97 (m, 18H), 1.70-1.84 (m, 2H), 1.52-1.65 (m, 2H), 1.30-1.35 (m, 8H). ¹H NMR: RW0008-15-P1A (400 MHz, CD₃OD).

### 9. Preparation of a compound 10

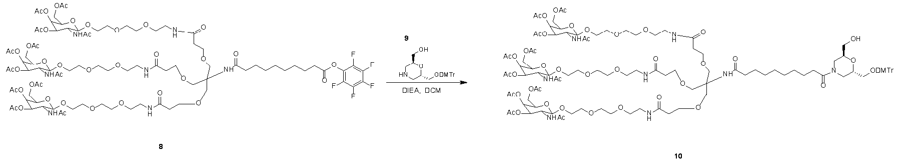

The compound 8 (350 mg, 0.169 mmol) was dissolved in DCM (2.00 mL) at room temperature, and a compound 9 (76.1 mg, 0.169 mmol) and DIEA (0.112 mL, 0.677 mmol) were added to carry out a reaction for 12 h. Thin layer chromatography (dichloromethane/methanol=10/1) showed that raw materials disappeared and a new point was generated. A reaction solution was diluted with water (10 mL) and then extracted with dichloromethane (10×3). An organic phase was dried with anhydrous sodium sulfate and filtered, and a filtrate was concentrated under reduced pressure to obtain a colorless oily compound 10 (about 388 g). ¹H NMR (400 MHz, METHANOL-d₄) δ 7.43 (d, *J =* 8.4 Hz, 2H), 7.20-7.35 (m, 7H), 6.82-6.92 (m, 4H), 5.34 (d, *J =* 2.8 Hz, 3H), 5.07 (dd, *J* = 3.6, 11.2 Hz, 3H), 4.65 (d, *J =* 8.4 Hz, 3H), 4.06-4.17 (m, 9H), 3.99-4.05 (m, 3H), 3.90-3.96 (m, 4H), 3.79 (d, *J =* 2.8 Hz, 6H), 3.58-3.75 (m, 39H), 3.53-3.57 (m, 7H), 3.38 (t, J=5.6 Hz, 8H), 3.22-3.24 (m, 1H), 2.45 (t, *J =* 6.0 Hz, 6H), 2.15-2.20 (m, 2H), 2.13-2.15 (m, 9H), 2.02-2.04 (m, 9H), 1.91-1.96 (m, 18H), 1.49-1.61 (m, 4H), 1.27-1.35 (m, 8H).

### 10. Preparation of E9

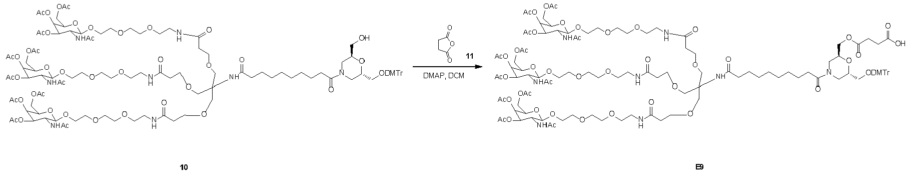

The compound 10 (388 mg, 0.166 mmol) was dissolved in DCM (4 mL) at room temperature, and DIEA (0.165 mL, 0.997 mmol), DMAP (5.08 mg, 0.042 mmol) and a compound 11 (99.79 mg, 0.997 mmol) were added to carry out a reaction at 25°C for 3 h. LCMS showed that raw materials were completely reacted and a product was generated. Dichloromethane (30 mL) was added for dilution, and an organic phase with washed with a saturated NaCl solution (10 mL×3) for 3 times and then dried with anhydrous sodium sulfate. A reaction solution was concentrated under reduced pressure, and a resulting crude product was prepared and separated by preparative high performance liquid chromatography (preparative-HPLC, column: Welch Xtimate C18 150*25mm*5um; mobile phase: water-ACN; B%: 20%-50%, 11 min) to obtain a white solid E9 (78.0 mg, yield: 19.28%, purity: 76.01%). LCMS: m/z=1216.1 (M/2+H)⁺; ¹H NMR (400 MHz, CD₃OD) *δ* 7.43 (d, *J* = 8.0 Hz, 2H), 7.22-7.35 (m, 7H), 6.80-6.92 (m, 4H), 5.34 (d, *J =* 3.2 Hz, 3H), 5.08 (dd, *J =* 3.2, 11.2 Hz, 3H), 4.65 (d, *J =* 8.4 Hz, 3H), 4.05-4.18 (m, 10H), 4.00-4.04 (m, 3H), 3.90-3.97 (m, 4H), 3.79 (d, *J =* 2.4 Hz, 6H), 3.58-3.75 (m, 39H), 3.55 (t, *J*=5.2 Hz, 7H), 3.32-3.42 (m, 8H), 3.20-3.24 (m, 1H), 2.56 (dd, *J*=5.6, 18.8 Hz, 4H), 2.44 (t, *J =* 6.0 Hz, 6H), 2.16-2.21 (m, 2H), 2.14 (s, 9H), 2.02 (s, 9H), 1.90-1.98 (m, 18H), 1.48-1.61 (m, 4H), 1.31 (d, *J =* 13.2 Hz, 8H).

### Example 10: Preparation of a compound E10

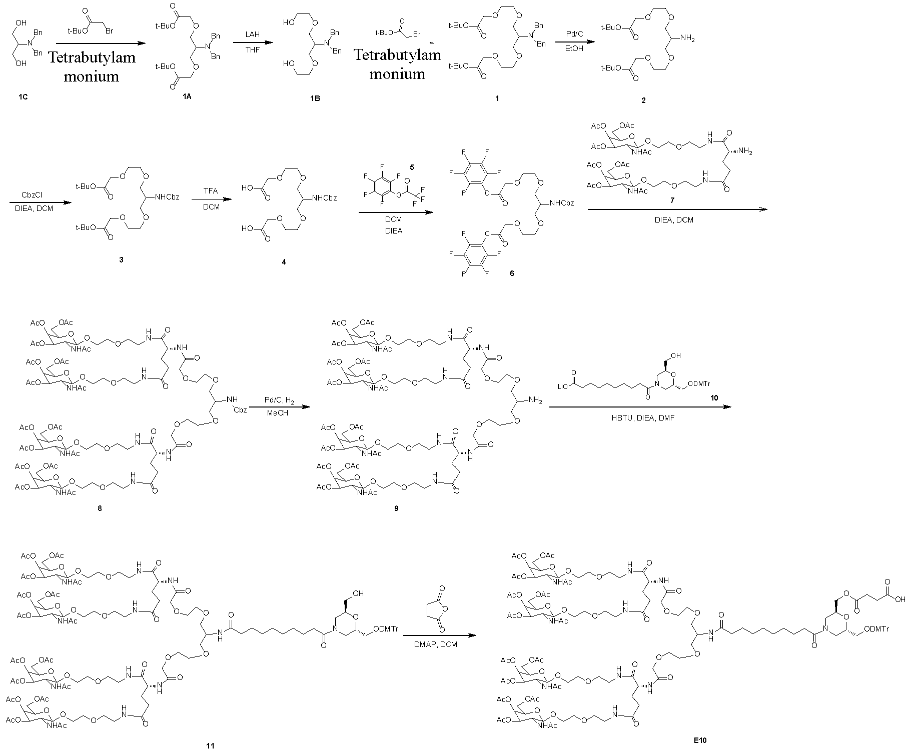

### 1. Preparation of a compound 1A

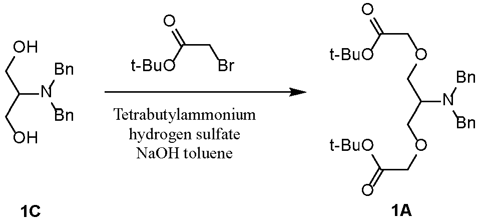

A compound 1C (20 g, 73.8 mmol) was dissolved in anhydrous toluene (200 mL), tetrabutylammonium hydrogen sulfate (3.75 g, 11.0 mmol), tert-butyl bromoacetate (85.9 g, 442.8 mmol) and 30% NaOH (160 mL) were sequentially added, and a reaction solution was stirred at 45°C for 18 h. A TCL plate (PE:EA=1:1, UV254 nm) showed that raw materials were completely consumed and a new point was generated. The reaction solution was cooled to room temperature and subjected to standing for layering and liquid separation, an alkaline aqueous phase on a lower layer was extracted with methyl tert-butyl ether (200 ml×2), and organic phases were combined, concentrated and filtered with a normal-phase column (silica gel, PE:EA=1:0 to PE:EA=3:1) to obtain a compound 1A (about 28.5 g).

### 2. Preparation of a compound 1B

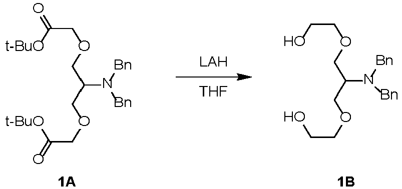

LiAlH₄ (1.67 g, 44.0 mmol) was added to THF (100 mL) under stirring, nitrogen replacement was carried out, and then cooling was carried out to 0°C in an ice bath. The compound 1A (10.0 g, 20.0 mmol) was dissolved in 20 ml of THF and then slowly added dropwise to a resulting solution with a syringe at 0°C, and a reaction solution was stirred at 0°C for 2 h. LC/MS detected that raw materials were completely consumed and an MS response of a product was obtained. 2 ml of water, 2 ml of a 15% NaOH aqueous solution and 6 ml of water were sequentially added dropwise to the reaction solution under stirring and then fully stirred, an anhydrous sodium sulfate powder was added, filtration was carried out after stirring was carried out for 15 min, and an organic phase was concentrated to obtain a colorless oily compound 1B (about 6.60 g). LCMS: m/z=360.3 (M+H)⁺.

### 3. Preparation of a compound 1

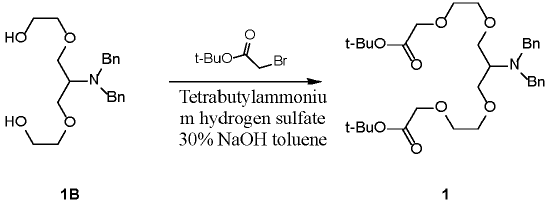

The compound 1B (6.60 g, 18.4 mmol) was dissolved in anhydrous toluene (100 mL), tetrabutylammonium hydrogen sulfate (1.25 g, 3.67 mmol), tert-butyl bromoacetate (21.5 g, 110 mmol) and 30% NaOH (80 mL) were sequentially added, and a reaction solution was stirred at 45°C for 18 h. LC/MS detected an MS response of a product. A TCL plate (PE:EA=3:1, UV254 nm) showed that raw materials were completely consumed and a new point was generated. The reaction solution was cooled to room temperature and subjected to standing for layering and liquid separation, an alkaline aqueous phase on a lower layer was extracted with methyl tert-butyl ether (50 ml×2), and organic phases were combined, concentrated and filtered with a normal-phase column (silica gel, PE:EA=1:0 to PE:EA=3:1) to obtain a compound 1 (about 9.6 g). LCMS: m/z=589.9 (M+H)⁺.

### 4. Preparation of a compound 2

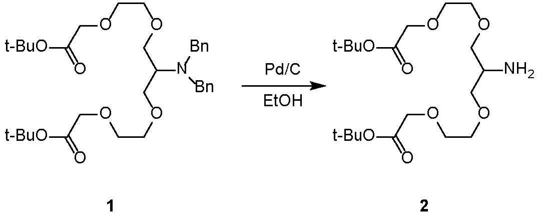

The compound 1 (9.60 g, 16.4 mmol) was dissolved in EtOH (50 mL), Pd/C (1 g, 9.40 mmol) with a mass fraction of 10% was added, hydrogen replacement was carried out for 3 times, and a reaction solution was enabled to undergo a reaction in an H₂ atmosphere (15 psi) at 45°C for 18 h. LC/MS detected an MS response of a product. The reaction solution was filtered to remove the palladium on carbon and then concentrated to obtain a compound 2 (about 6.60 g). ¹H NMR (400 MHz, CD₃OD) δ 4.04 (s, 4H), 3.68-3.70 (m, 4H), 3.62-3.64 (m, 4H), 3.50-3.54 (m, 2H), 3.40-3.44 (m, 2H), 3.06-3.12 (m, 1H), 1.48 (s, 18H). LCMS: m/z=408.7 (M+H)⁺.

### 5. Preparation of a compound 3

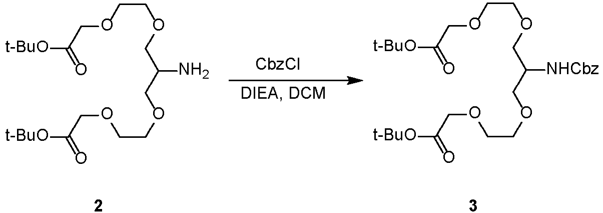

The compound 2 (6.60 g, 16.4 mmol) was dissolved in DCM (100 mL), DIEA (5.41 mL, 32.7 mmol) was added first, then benzyl chloroformate (2.53 mL, 18.0 mmol) was added under stirring, and a reaction solution was stirred at 25°C for 18 h. LC/MS detected an MS response of a product. TCL (PE:EA=3:1, PMA) showed that raw materials were completely reacted and a new point was generated. The reaction solution was directly concentrated and filtered with a normal-phase column (PE:EA=1:0 to 3:1) to obtain a colorless oily compound 3 (about 7.5 g). ¹H NMR (400 MHz, CD₃OD) δ 7.29-7.35 (m, 5H), 5.08 (s, 2H), 4.02 (s, 4H), 3.87-3.92 (m, 1H), 3.63-3.67 (m, 8H), 3.55-3.56 (m, 4H), 1.47 (s, 18H). LCMS: m/z=542.3 (M+H)⁺.

### 6. Preparation of a compound 4

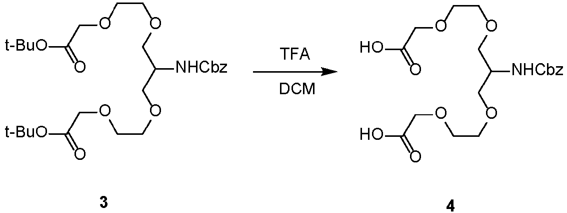

The compound 3 (3.7 g, 6.88 mmol) was dissolved in DCM (18 mL), TFA (6 mL) was added, and a reaction solution was stirred at 25°C for 18 h. LC/MS detected an MS response of a product. The reaction solution was directly concentrated to obtain a yellow oily compound 4 (about 4.3 g). ¹H NMR (400 MHz, CD₃OD) δ 7.28-7.35 (m, 5H), 5.08 (s, 2H), 4.12 (s, 4H), 3.87-3.93 (m, 1H), 3.63-3.68 (m, 8H), 3.55-3.56 (m, 4H). LCMS: m/z=430.1 (M+H)⁺.

### 7. Preparation of a compound 6

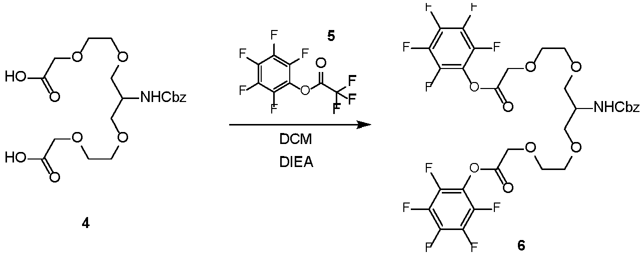

The compound 4 (1 g, 2.33 mmol) was dissolved in DCM (30 mL), DIEA (4.06 mL, 23.3 mmol) and a compound 5 (1.96 g, 6.99 mmol) were added, and a reaction solution was stirred at 25°C for 1 h. LC/MS detected an MS response of a product. TLC (PE:EA=3:1) showed that raw materials were completely reacted and a new point was generated. The reaction solution was directly concentrated and filtered with a normal-phase column (PE:EA=1:0 to PE:EA=3:1) to obtain a white solid compound 6 (about 900 mg). LCMS: m/z=762.6 (M+H)⁺.

### 8. Preparation of a compound 8

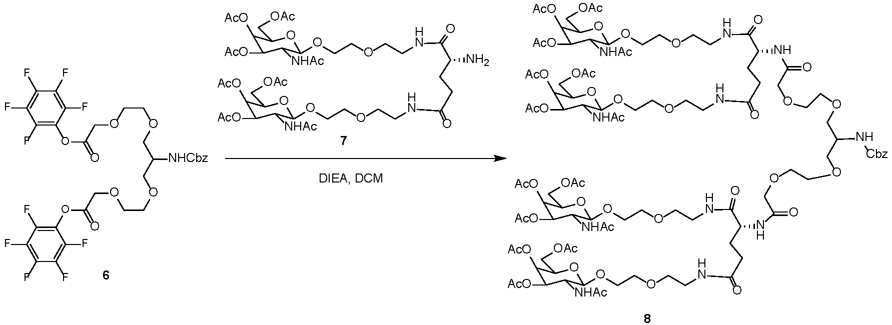

The compound 6 (1,132 mg, 1.16 mmol) was dissolved in DCM (10 mL), DIEA (0.26 mL, 1.58 mmol) and a compound 7 (400 mg, 0.53 mmol, prepared by a method of a compound 6 in Example 12) were added, and a reaction solution was stirred at 25°C for 18 h. TLC (DCM:MeOH=10:1) showed that raw materials were completely reacted and a new point was generated. LC/MS detected an MS response of a product. The reaction solution was directly concentrated and filtered with a normal-phase column (DCM:MeOH=1:0 to 10:1) to obtain a colorless oily compound 8 (about 1,100 mg). LCMS:. m/z=1177.4 (M/2+H)⁺.

### 9. Preparation of a compound 9

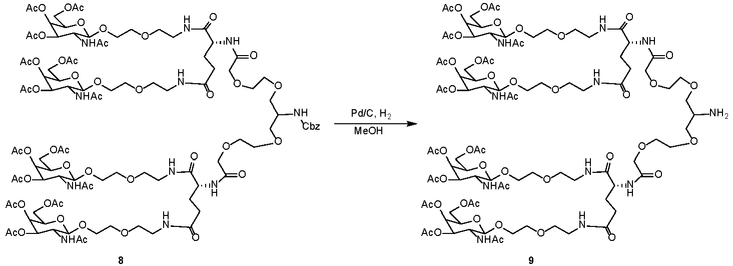

The compound 8 (1,100 mg, 0.467 mmol) was dissolved in MeOH (20 mL), Pd/C (100 mg, 0.940 mmol) with a mass fraction of 10% was added, hydrogen replacement was carried out for 3 times, and a reaction solution was enabled to undergo a reaction in an H₂ atmosphere (15 psi) at 25°C for 18 h. LC/MS detected an MS response of a product. The reaction solution was filtered to remove the Pd/C and then washed with methanol, and filtrates were combined and concentrated to obtain a white solid compound 9 (about 960 mg). LCMS: m/z=1110.3 (M/2+H)⁺.

### 10. Preparation of a compound 11

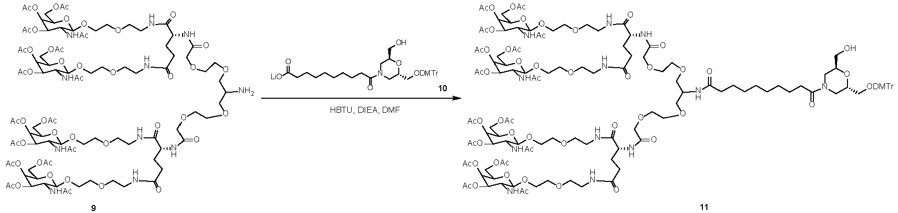

The compound 9 (960 mg, 0.433 mmol) was dissolved in DMF (20 mL), an HBTU reagent (246 mg, 0.649 mmol), DIEA (0.214 mL, 1.30 mmol) and a compound 10 (277 mg, 0.433 mmol, prepared by a method of a compound Int-6 in Example 13) were sequentially added, and a reaction solution was stirred at 25°C for 18 h. LC/MS detected an MS response of a product. TLC (DCM:MeOH=10:1) showed that raw materials were completely reacted and a new point was generated. The reaction solution was washed with 20 ml of a saturated sodium bicarbonate aqueous solution and extracted with dichloromethane (20 ml×2). Organic phases were combined, concentrated and filtered with a normal-phase column (DCM:MeOH=1:0 to 10:1) to obtain a white solid compound 11 (about 1,060 mg). LCMS: m/z=1266.9 [(M-DMTr)/2+H]⁺.

### 11. Preparation of E10

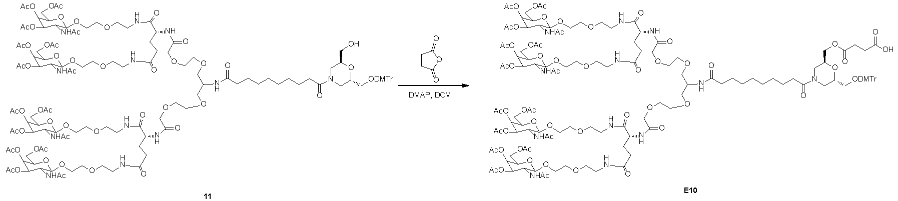

The compound 11 (500 mg, 0.161 mmol) was dissolved in DCM (5 mL), tetrahydrofuran-2,5-dione (96.86 mg, 0.968 mmol), DIEA (0.160 mL, 0.968 mmol) and DMAP (19.7 mg, 0.161 mmol) were added, and a reaction solution was stirred at 25°C for 18 h. LCMS detected an MS response of a product. The reaction solution was directly spin-dried, dissolved in 5 ml of acetonitrile and treated by preparative-HPLC (chromatographic column: Waters Xbridge BEH C18 100*30mm*10um; mobile phase: water-acetonitrile; B%: 20%-50%, 11 min; flow rate: 50 ml/min) to obtain a white solid E10 (56 mg, 0.018 mmol, 10.9%) LCMS (ESI): m/z=1317.0 [(M-DMTr)/2+H]⁺; ¹H NMR (400 MHz, CD₃OD) δ 7.43(d, *J* = 8.0 Hz, 2H), 7.27-7.32 (m, 6H), 7.18-7.24 (m, 1H), 6.85-6.89 (m, 4H), 5.34 (d, *J =* 3.6 Hz, 4H), 5.07-5.12 (m, 4H), 4.62-4.67 (m, 4H), 4.42-4.48 (m, 2H), 4.20-4.24 (m, 2H), 3.98-4.17 (m, 22H), 3.90-3.94 (m, 4H), 3.79 (d, *J =* 2.0 Hz, 6H), 3.69-3.74 (m, 12H), 3.62-3.63 (m, 14H), 3.52-3.55 (m, 8H), 3.35-3.40 (m, 8H), 3.20 (s, 2H), 2.48-2.60 (m, 4H), 2.28-2.34 (m, 5H), 2.20-2.28 (m, 2H), 2.14 (s, 12H), 2.06-2.11 (m, 2H), 2.02 (s, 12H), 1.97-1.99 (m, 2H), 1.94 (d, *J =* 5.2 Hz, 24H), 1.53-1.59 (m, 4H), 1.28-1.38 (m, 12H).

### Example 11: Preparation of a compound E11

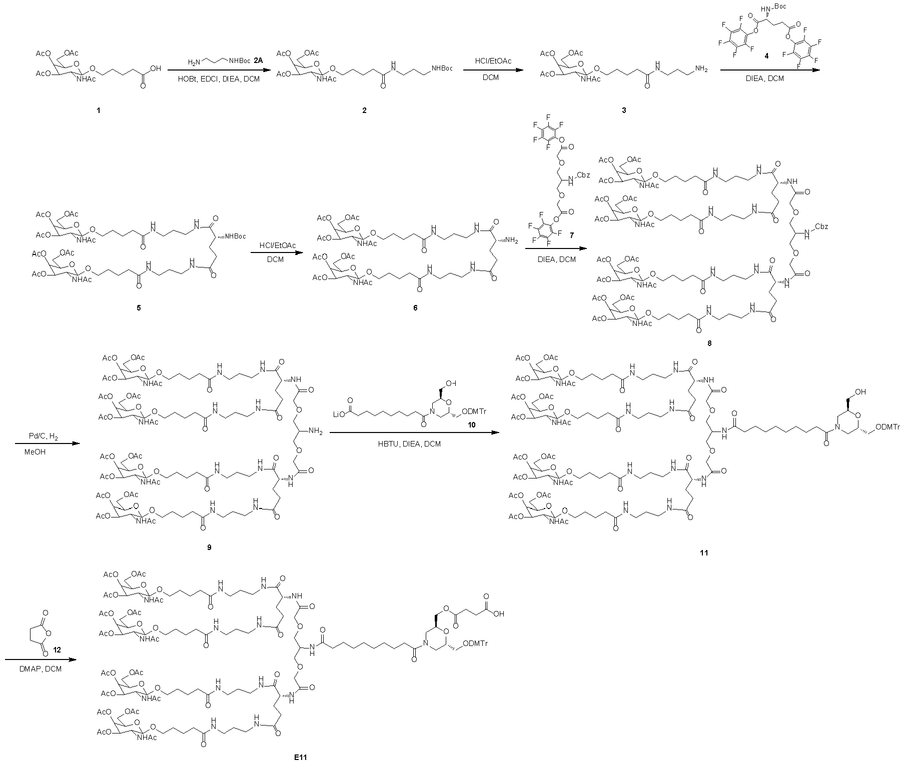

### 1. Preparation of a compound 2

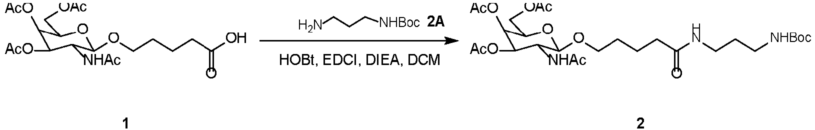

A compound 1 (5.00 g, 11.2 mmol, prepared by a method of an intermediate 1-3 in Example 25) was dissolved in DCM (100 mL) at room temperature, HOBt (1.81 g, 13.4 mmol), EDCI (2.57 g, 13.4 mmol), DIEA (2.77 mL, 16.8 mmol) and a compound 2A (2.34 g, 13.4 mmol) were sequentially added to a resulting solution, and nitrogen replacement was carried out for 3 times. A reaction mixture was stirred at 25°C for 16 h. Liquid chromatography-mass spectrometry detected an MS response of a product. Thin layer chromatography (dichloromethane/methanol=10/1) showed that raw materials were completely consumed and a new point was generated. A reaction solution was diluted with dichloromethane (300 mL), and sequentially washed with a saturated citric acid solution (30.0 mL), sodium bicarbonate (30.0 mL), a salt solution (30.0 mL) and water. An organic phase was dried with sodium sulfate and filtered, and a filtrate was spin-dried to obtain a crude light yellow oily compound 2 (about 6.80 g), which was directly used in a next reaction. ¹H NMR (400 MHz, CD₃OD) *δ* 5.33-5.35 (m, 1H), 5.05-5.07 (m, 1H), 4.52-4.61 (m, 1H), 3.98-4.22 (m, 4H), 3.85-3.93 (m, 1H), 3.52-3.56 (m, 1H), 3.17-3.26 (m, 2H), 3.06-3.10 (m, 2H), 2.19-2.25 (m, 2H), 2.12-2.19 (m, 3H), 2.04 (s, 3H), 1.90-1.96 (m, 6H), 1.56-1.74 (m, 6H), 1.45 (s, 9H). LCMS: m/z=604.4 (M+H)⁺.

### 2. Preparation of a compound 3

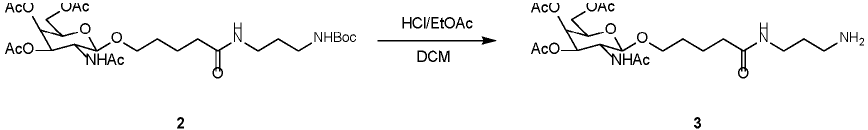

The compound 2 (6.80 g, 11.3 mmol) was dissolved in DCM (50.0 mL) at room temperature, HCl/EtOAc (20.0 mL) was added to a resulting solution, and a reaction mixture was stirred at 25°C for 1 h. Thin layer chromatography (dichloromethane/methanol=10/1, with phosphomolybdic acid as a color developing agent) showed that raw materials were completely consumed and a new point was generated. A reaction solution was concentrated under reduced pressure to obtain a crude white solid 3 (about 6.90 g), which was directly used in a next reaction without purification.

### 3. Preparation of a compound 5

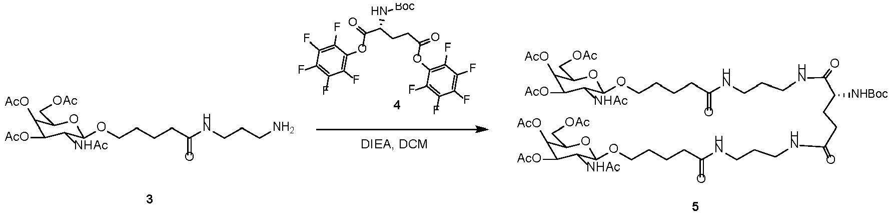

The compound 3 (6.80 g, 13.5 mmol) was dissolved in DCM (20.0 mL) at room temperature, DIEA (2.23 mL, 13.5 mmol) and a compound 4 (3.61 g, 6.08 mmol) were sequentially added to a resulting solution, and nitrogen replacement was carried out for 3 times. A reaction mixture was stirred at 25°C for 18 h. Thin layer chromatography (dichloromethane/methanol=10/1) showed that raw materials were completely consumed and a new point was generated. A reaction solution was concentrated under reduced pressure, and a resulting crude product was purified by column chromatography (dichloromethane/methanol=12/1 to 10/1) to obtain a white solid 5 (about 6.00 g). ¹H NMR (400 MHz, CD₃OD) *δ* 5.3-5.34 (m, 2H), 5.04-5.08 (m, 2H), 4.54-4.58 (m, 2H), 3.97-4.21 (m, 9H), 3.81-3.92 (m, 2H), 3.51-3.53 (m, 2H), 3.14-3.28 (m, 9H), 2.25-2.32 (m, 2H), 2.19-2.23 (m, 4H), 2.14 (s, 6H), 2.03 (s, 6H), 1.94 (d, *J =* 9.2 Hz, 12H), 1.62-1.75 (m, 9H), 1.56-1.62 (m, 4H), 1.44 (s, 9H).

### 4. Preparation of a compound 6

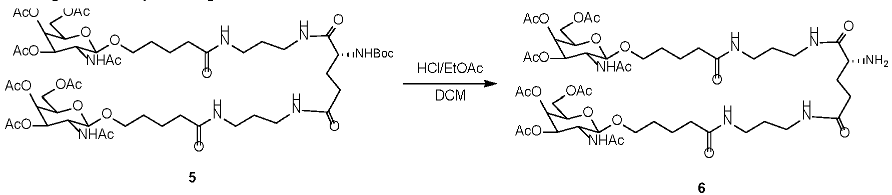

The compound 5 (1.0 g, 0.821 mmol) was dissolved in DCM (10.0 mL) at room temperature, HCl/EtOAc (1.00 mL) was added to a resulting solution, and a reaction mixture was stirred at 25°C for 16 h. Thin layer chromatography (dichloromethane/methanol=10/1, with phosphomolybdic acid as a color developing agent) showed that raw materials were completely consumed and a new point was generated. A reaction solution was concentrated under reduced pressure to obtain a crude white solid 6 (about 1.57 g). The crude product was directly used in a next reaction without purification.

### 5. Preparation of a compound 8

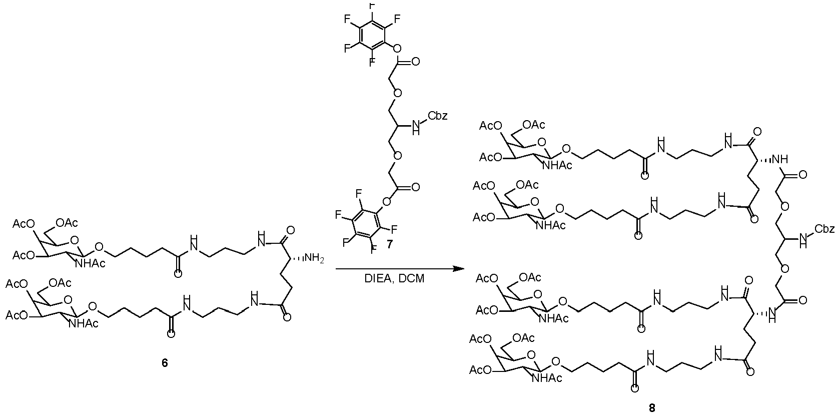

The compound 6 (1.57 g, 0.772 mmol) was dissolved in DCM (20.0 mL) at room temperature, DIEA (0.255 mL, 1.544 mmol) and a compound 7 (0.21 g, 0.309 mmol) were sequentially added to a resulting solution, and nitrogen replacement was carried out for 3 times. A reaction mixture was stirred at 25°C for 16 h. Liquid chromatography-mass spectrometry detected an MS response of a product. Thin layer chromatography (dichloromethane/methanol=5/1) showed that raw materials disappeared and a new point was generated. A reaction solution was concentrated under reduced pressure, and a resulting crude product was purified by column chromatography (dichloromethane/methanol=5/1) to obtain a white solid 8 (about 780 mg). ¹H NMR (400 MHz, CD₃OD) *δ* 7.25-7.42 (m, 5H), 5.30-5.33 (m, 4H), 5.04-5.06 (m, 4H), 4.54-4.56 (m, 4H), 4.30-4.40 (m, 2H), 3.96-4.19 (m, 21H), 3.83-3.90 (m, 4H), 3.67-3.76 (m, 4H), 3.50-3.52 (m, 4H), 3.11-3.27 (m, 18H), 2.25-2.33 (m, 4H), 2.18-2.20 (m, 8H), 2.14 (s, 12H), 2.02 (s, 12H), 1.94 (d, *J* = 9.20 Hz, 24H), 1.56-1.70 (m, 24H). LCMS: m/z=1272.5 (M/2+H)⁺.

### 6. Preparation of a compound 9

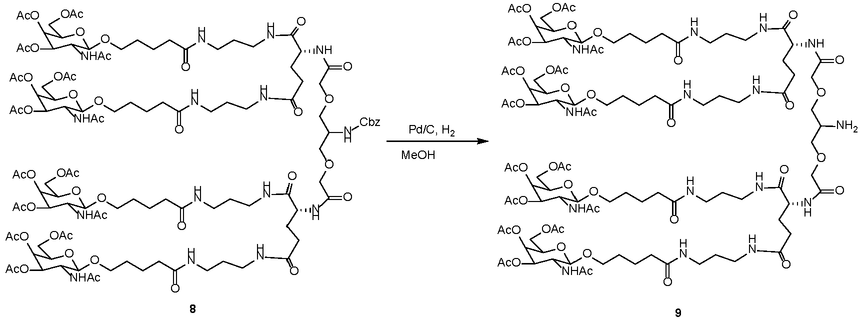

The compound 8 (380 mg, 0.150 mmol) was dissolved in MeOH (10.0 mL) at room temperature, wet Pd/C (100 mg, 0.940 mmol) with a mass fraction of 10% was added to a resulting solution, hydrogen replacement was carried out for 3 times, and a reaction mixture was stirred in a hydrogen atmosphere (14.696 psi) at 25°C for 18 h. Liquid chromatography-mass spectrometry detected an MS response of a product. Thin layer chromatography (dichloromethane/methanol=10/1, with phosphomolybdic acid as a color developing agent) showed that raw materials were completely consumed and a new point was generated. A reaction solution was filtered, and a filtrate was concentrated under reduced pressure to obtain a white solid 9 (about 330 mg). ¹H NMR (400 MHz, CD₃OD) *δ* 5.31-5.35 (m, 4H), 5.04-5.06 (m, 4H), 4.53-4.56 (m, 4H), 4.34-4.36 (m, 2H), 3.99-4.16 (m, 21H), 3.85-3.90 (m, 4H), 3.51-3.56 (m, 4H), 3.18-3.22 (m, 18H), 2.28-2.33 (m, 4H), 2.19-2.22 (m, 8H), 2.14 (s, 12H), 2.03 (s, 12H), 1.94 (d, *J =* 8.8 Hz, 24H), 1.58-1.71 (m, 24H). LCMS: m/z=1204.9 (M/2+H)⁺.

### 7. Preparation of a compound 11

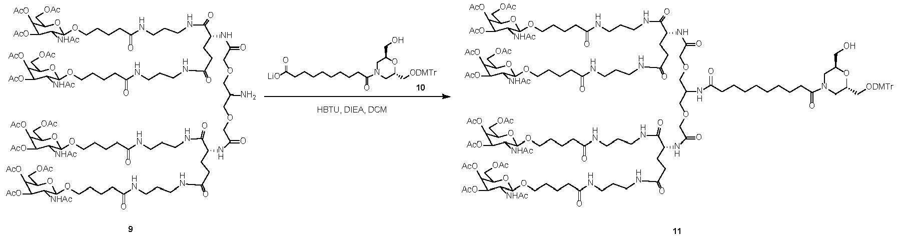

The compound 9 (170 mg, 0.071 mmol) was dissolved in DCM (10.0 mL) at room temperature, a compound 10 (45.2 mg, 0.071 mmol, prepared by a method of a compound Int-6 in Example 13), DIEA (0.035 mL, 0.212 mmol), HBTU (40.2 mg, 0.106 mmol) and DMAP (8.63 mg, 0.071 mmol) were added to a resulting solution, and nitrogen replacement was carried out for 3 times. A reaction mixture was stirred at 25°C for 1 h. Liquid chromatography-mass spectrometry detected that raw materials disappeared. Thin layer chromatography (dichloromethane:methanol=5/1) showed that the raw materials disappeared and a new point was generated. A reaction solution was concentrated under reduced pressure, and a resulting crude product was purified by column chromatography (dichloromethane/methanol=8/1 to 5/1) to obtain a white solid 11 (about 340 mg).

### 8. Preparation of E11

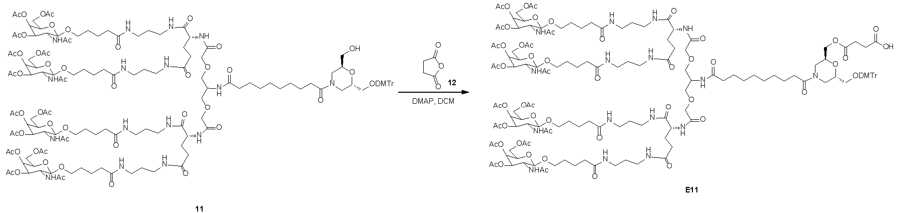

The compound 11 (340 mg, 0.112 mmol) was dissolved in DCM (10.0 mL) at room temperature, DIEA (14.5 mg, 0.112 mmol), a compound 12 (11.3 mg, 0.112 mmol) and DMAP (6.84 mg, 0.056 mmol) were sequentially added to a resulting solution, and nitrogen replacement was carried out for 3 times. A reaction mixture was stirred at 25°C for 1 h. Liquid chromatography-mass spectrometry detected that raw materials disappeared. A reaction solution was concentrated under reduced pressure, and a resulting crude product was prepared and separated by preparative high performance liquid chromatography (preparative-HPLC, column: Waters Xbridge BEH C18 100*30mm*10um; mobile phase: water-ACN; B%: 19%-49%, 3 min) to obtain a white solid E11 (72.3 mg, yield: 20.59%, purity: 80.47%). ¹H NMR (400 MHz, CD₃OD) *δ* 7.41-7.43 (m, 2H), 7.29-7.31 (m, *J =* 8.8 Hz, 6H), 7.17-7.26 (m, 1H), 6.83-6.90 (m, 4H), 5.31-5.34 (m, 4H), 5.02-5.08 (m, 4H), 4.50-4.56 (m, 4H), 4.32-4.40 (m, 3H), 4.20-4.25 (m, 1H), 4.09-4.19 (m, 10H), 4.05-4.09 (m, 7H), 3.95-4.04 (m, 6H), 3.83-3.90 (m, 5H), 3.76-3.80 (m, 6H), 3.58-3.75 (m, 8H), 3.48-3.52 (m, 4H), 3.18-3.23 (m, 15H), 2.56-2.62 (m, 2H), 2.48-2.54 (m, 2H), 2.24-2.35 (m, 8H), 2.18-2.20 (m, 8H), 2.14 (s, 12H), 2.09-2.11 (m, 2H), 2.02 (s, 12H), 1.90-1.98 (m, 28H), 1.55-1.72 (m, 28H), 1.28-1.32 (m, 8H).

### Example 12: Preparation of a compound E12

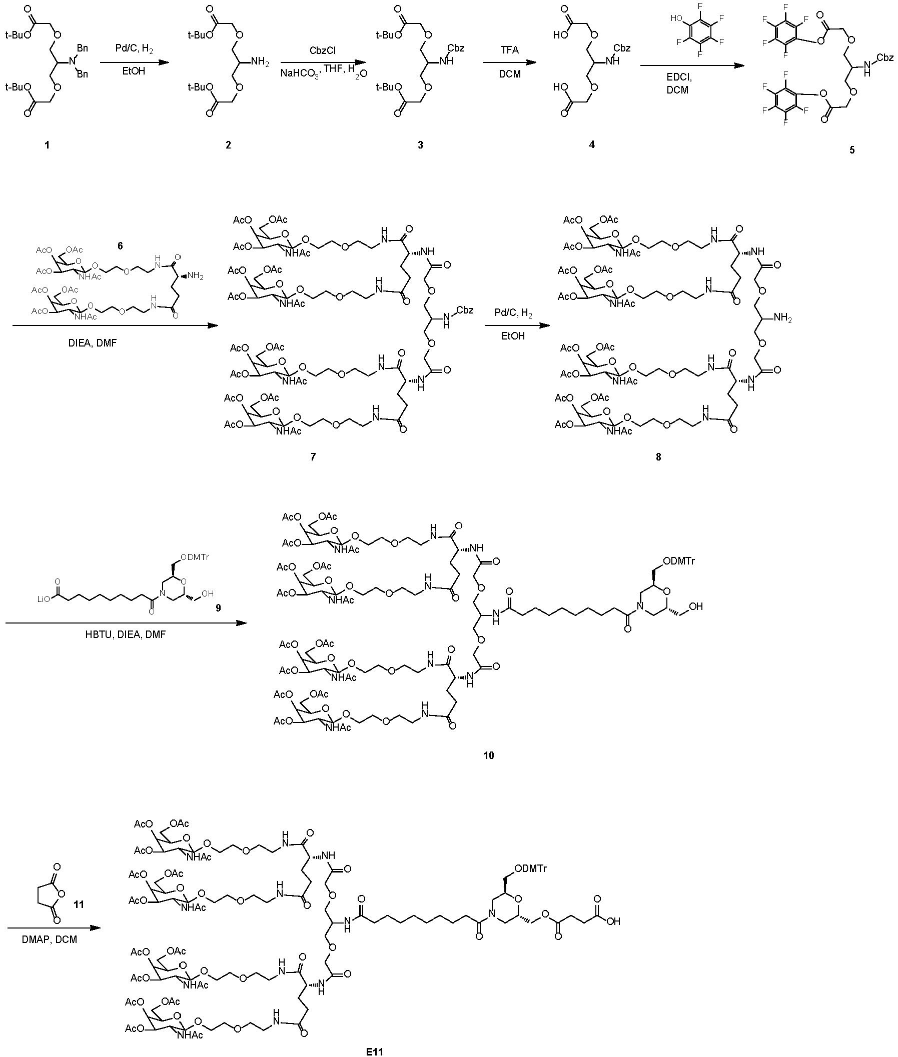

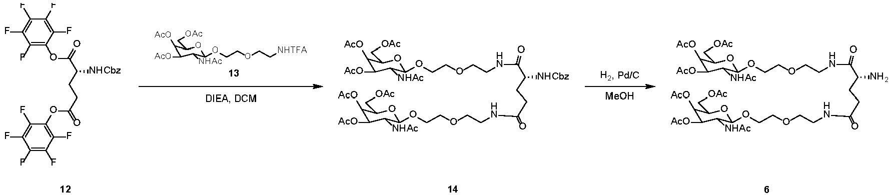

### 1. Preparation of a compound 14

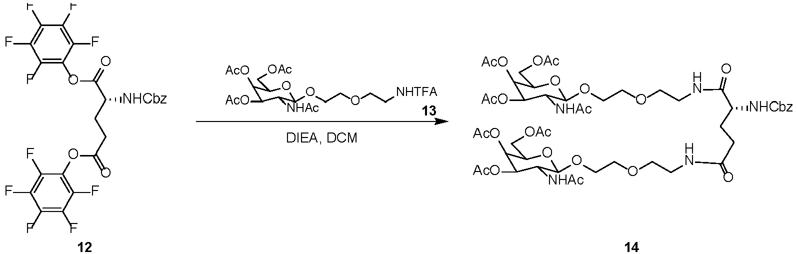

A compound 13 (9.29 g, 21.4 mmol, prepared by a method of an intermediate 2-3 in Example 22) was dissolved in anhydrous dichloromethane (100 mL) at room temperature, DIEA (15.4 mL, 92.9 mmol) and a compound 12 (5.70 g, 9.29 mmol, prepared by a method of the compound 1 in Example 8) were sequentially added to a resulting solution, and nitrogen replacement was carried out for 3 times. A reaction mixture was stirred at 25°C for 16 h. Liquid chromatography-mass spectrometry detected an MS response of a product. Thin layer chromatography (dichloromethane/methanol=10/1) showed that raw materials were completely consumed and a new point was generated. A reaction solution was diluted with dichloromethane (300 mL), sequentially washed with a citric acid aqueous solution (50.0 mL) and a saturated salt solution (30.0 mL×3), and then extracted. An organic phase was collected, dried with anhydrous sodium sulfate and filtered. A filtrate was concentrated under reduced pressure, and a resulting crude product was purified by column chromatography (dichloromethane/methanol=12/1 to 10/1) to obtain a white solid compound 14 (about 9.6 g). ¹H NMR (400 MHz, CD₃OD) *δ* 7.24-7.44 (m, 5H), 5.32-5.34 (m, 2H), 5.01-5.17 (m, 4H), 4.60-4.70 (m, 2H), 3.99-4.21 (m, 9H), 3.87-3.96 (m, 2H), 3.67-3.76 (m, 2H), 3.60-3.64 (m, 4H), 3.47-3.57 (m, 4H), 3.33-3.37 (m, 6H), 2.27-2.39 (m, 2H), 2.10 (s, 6H), 2.02 (s, 6H), 1.92-1.96 (m, 12H). LCMS: m/z=1114.7 (M+H)⁺.

### 2. Preparation of a compound 6

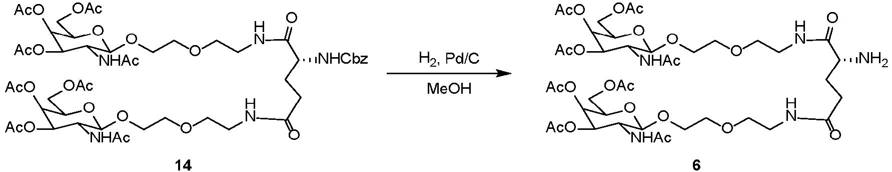

The compound 14 (5.00 g, 4.49 mmol) was dissolved in methanol (100 mL) at room temperature, wet Pd/C (1.00 g, 9.40 mmol) with a mass fraction of 10% was added to a resulting solution, hydrogen replacement was carried out for 3 times, and a reaction mixture was stirred in a hydrogen atmosphere (14.696 psi) at 25°C for 18 h. Thin layer chromatography (dichloromethane/methanol=10/1, with phosphomolybdic acid as a color developing agent) showed that raw materials were completely consumed and a new point was generated. A reaction solution was filtered, and a filtrate was concentrated under reduced pressure to obtain a white solid compound 6 (about 4.2 g). ¹H NMR (400 MHz, CH₃OD) *δ* 5.32-5.34 (m, 2H), 5.07-5.11 (m, 2H), 4.631-4.63 (m, 2H), 4.01-4.17 (m, 9H), 3.92-3.96 (m, 2H), 3.68-3.75 (m, 2H), 3.61-3.66 (m, 4H), 3.54-3.56 (m, 4H), 3.32-3.43 (m, 6H), 2.25-2.34 (m, 2H), 2.15 (s, 6H), 2.03 (s, 6H), 1.92-1.96 (m, 12H).

### 3. Preparation of a compound 2

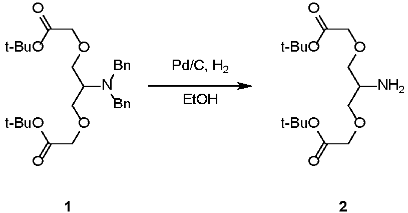

A compound 1 (10.0 g, 20.0 mmol) was dissolved in ethanol (100 mL), Pd/C (1.00 g, 9.39 mmol) with a mass fraction of 10% was added, hydrogen replacement was carried out for 3 times, and a reaction solution was enabled to undergo a reaction in an H₂ atmosphere (15 psi) at 45°C for 18 h. Liquid chromatography-mass spectrometry detected an MS response of a product. The reaction solution was filtered with diatomite to remove the Pd/C and then washed with ethanol (10.0 ml×3) for three times. Filtrates were combined and concentrated to obtain a yellow oily product 2. LCMS: m/z=320.3 (M+H)⁺.

### 4. Preparation of a compound 3

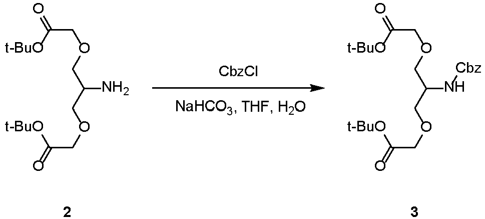

The compound 2 (1.00 g, 3.17 mmol) was dissolved in tetrahydrofuran (10.0 mL) and H₂O (10.0 mL) at 25°C, and sodium bicarbonate (798 mg, 9.51 mmol) and CbzCl (1.18 g, 4.75 mmol) were added. A reaction solution was stirred at 25°C for 1 h. Liquid chromatography-mass spectrometry showed that raw materials were completely reacted and an MS value of a target product appeared. Thin layer chromatography (petroleum ether: ethyl acetate=3 :1) showed that the raw materials were completely reacted and a new point was generated. 150.0 ml of an ethyl acetate solution was added to the reaction solution, and a mixed solution was washed with a salt solution (15.0 ml×3) for three times. An organic phase was dried with anhydrous sodium sulfate and then concentrated under reduced pressure to obtain a crude product. The crude product was purified by column chromatography (petroleum ether:ethyl acetate=3:1) to obtain a colorless oily compound 3 (about 770 mg). ¹H NMR (400 MHz, CD₃OD) δ 7.25-7.39 (m, 5H), 5.08 (s, 2H), 4.00 (s, 4H), 3.88-3.93 (m, 1H), 3.62 (d, *J =* 5.6 Hz, 4H), 1.47 (s, 18H). LCMS: m/z=454.3 (M+H)⁺.

### 5. Preparation of a compound 4

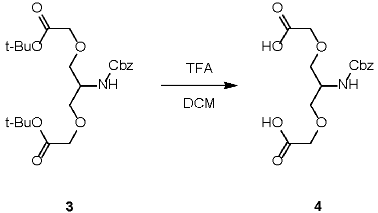

The compound 3 (770 mg, 1.71 mmol) was dissolved in anhydrous dichloromethane (6.00 mL) at 25°C, and TFA (3.00 mL, 39.1 mmol) was added. A reaction solution was stirred at 25°C for 12 h. Liquid chromatography-mass spectrometry showed that raw materials were completely reacted and an MS value of a target product was generated. Thin layer chromatography (petroleum ether:ethyl acetate=3:1) showed that the raw materials were completely reacted. The reaction solution was directly concentrated under reduced pressure to obtain a colorless oily product 4 (about 770 mg). ¹H NMR (400 MHz, CD₃OD) δ 7.24-7.39 (m, 5H), 5.08 (s, 2H), 4.10 (s, 4H), 3.90-3.95 (m, 1H), 3.57-3.68 (m, 4H). LCMS: m/z=342.1 (M+H)⁺.

### 6. Preparation of a compound 5

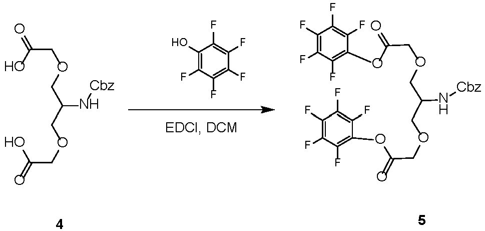

The compound 4 (770 mg, 2.25 mmol) was dissolved in anhydrous dichloromethane (6.00 mL) at 25°C, and pentafluorophenol (913 mg, 4.96 mmol) and EDCI (432 mg, 2.25 mmol) were added. A mixed solution was stirred at 25°C for 1 h. Liquid chromatography-mass spectrometry showed that an MS value of a product was generated. Thin layer chromatography (petroleum ether:ethyl acetate=3:1) showed that a new point was generated. A reaction solution was concentrated under reduced pressure to obtain a crude product, and the crude product was purified by column chromatography (petroleum ether:ethyl acetate=3:1) to obtain a colorless oily compound 5 (about 500 mg). ¹H NMR (400 MHz, CD₃OD) δ 7.20-7.44 (m, 5H), 5.08 (s, 2H), 4.58 (s, 4H), 3.95-4.05 (m, 1H), 3.71-3.81 (m, 4H). LCMS: m/z=674.0 (M+H)⁺.

### 7. Preparation of a compound 7

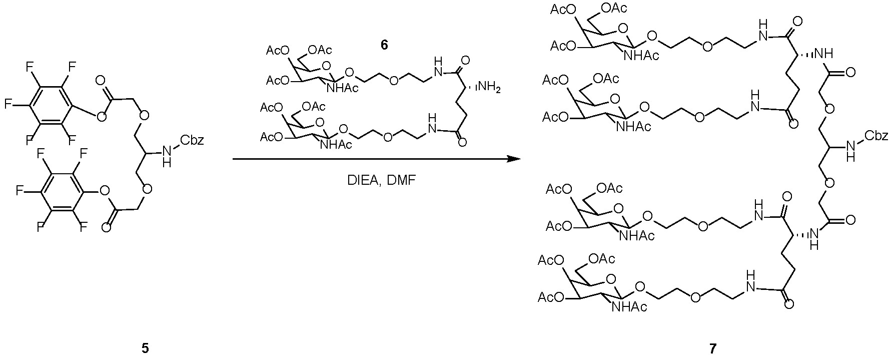

The compound 5 (500 mg, 0.742 mmol) and a compound 6 (2.72 g, 1.86 mmol) were dissolved in DMF (6.00 mL) at 25°C, and DIEA (0.123 mL, 0.742 mmol) was added. A mixed solution was stirred at 25°C for 12 h. Liquid chromatography-mass spectrometry showed that raw materials were completely reacted and a product was generated. Thin layer chromatography (petroleum ether:ethyl acetate=3:1, T1) showed that the raw materials were completely reacted. A reaction solution was concentrated under reduced pressure to obtain a crude product. The crude product was purified by column chromatography (dichloromethane:methanol=10:1, T2) to obtain a yellow solid compound 7 (about 860 mg). ¹H NMR (400 MHz, CD₃OD) δ 7.25-7.45 (m, 5H), 5.34 (d, *J* = 3.2 Hz, 4H), 5.07-5.11 (m, 4H), 4.60-4.71 (m, 4H), 4.35-4.44 (m, 2H), 4.13-4.16 (m, 6H), 4.10 (d, *J =* 3.6 Hz, 2H), 4.08 (d, *J =* 3.6 Hz, 2H), 4.00-4.06 (m, 9H), 3.88-3.96 (m, 4H), 3.67-3.75 (m, 15H), 3.61-3.62 (m, 8H), 3.47-3.56 (m, 9H), 3.34-3.40 (m, 8H), 2.25-2.35 (m, 4H), 2.14 (s, 12H), 2.01-2.04 (m, 13H), 1.94 *(d, J=* 7.6 Hz, 25H). LCMS: m/z=1133.3 (M/2+H)⁺.

### 8. Preparation of a compound 8

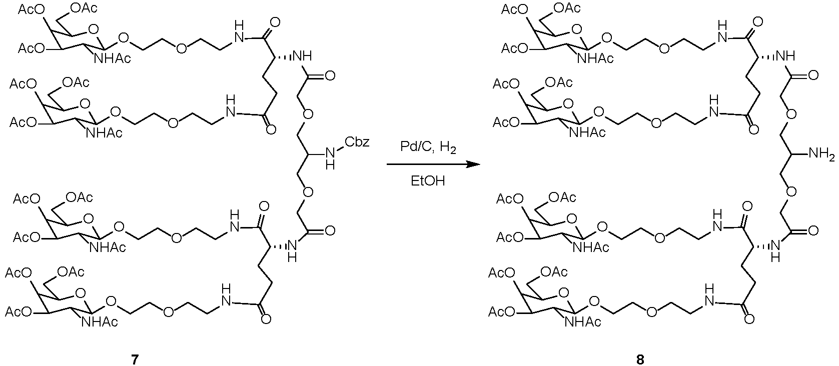

The compound 7 (350 mg, 0.155 mmol) was dissolved in ethanol (6.00 mL) at 25°C, Pd/C (500 mg, 4.70 mmol) with a mass fraction of 10% was added, and a mixed solution was stirred at 25°C for 12 h. Liquid chromatography-mass spectrometry showed that raw materials were completely reacted. A reaction solution was filtered with diatomite, and a filtrate was concentrated under reduced pressure to obtain a yellow oily compound 8 (about 200 mg). ¹H NMR (400 MHz, CD₃OD) δ 5.34 (s, 4H), 5.07-5.11 (m, 4H), 4.65 (dd, *J =* 9.6, 11.2 Hz, 4H), 4.36-4.45 (m, 2H), 4.12-4.20 (m, 8H), 4.00-4.12 (m, 13H), 3.91-3.95 (m, 5H), 3.69-3.74 (m, 5H), 3.62-3.63 (m, 12H), 3.53-3.56 (m, 10H), 3.36-3.42 (m, 8H), 2.29-2.40 (m, 4H), 2.15 (s, 12H), 2.03 (s, 12H), 1.90-1.97 (m, 24H). LCMS: m/z=1066.3 (M/2+H)⁺.

### 9. Preparation of a compound 10

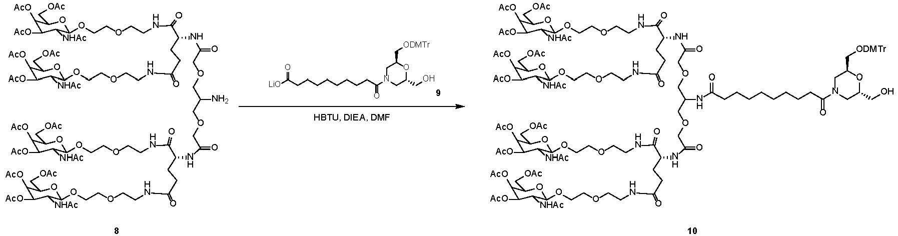

The compound 8 (450 mg, 0.211 mmol) was dissolved in DMF (6.00 mL) at 25°C, HBTU (104 mg, 0.274 mmol) and DIEA (0.105 mL, 0.633 mmol) were added, and a compound 9 (135 mg, 0.211 mmol, prepared by a method of a compound Int-6 in Example 13) was added at last. A mixed solution was stirred at 25°C for 12 h. Thin layer chromatography (dichloromethane:methanol=10:1) showed that a new point was generated. Dichloromethane (100 ml) was added to the mixed solution, and the mixed solution was washed with saturated sodium bicarbonate (5 ml×3) and a salt solution (5 ml×3) for three times, respectively. An organic phase was dried with anhydrous sodium sulfate and then concentrated under reduced pressure to obtain a crude product. The crude product was purified by column chromatography (dichloromethane:methanol=10:1)) to obtain a yellow oily compound 10 (about 350 mg). LCMS: m/z=1371.8 (M/2+H)⁺.

### 10. Preparation of E12

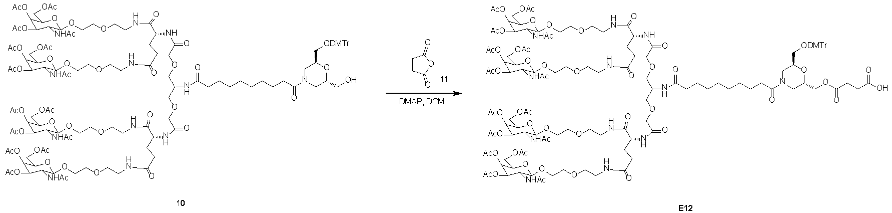

The compound 10 (350 mg, 0.127 mmol) was dissolved in anhydrous dichloromethane (5.00 mL) at 25°C, a compound 11 (76.5 mg, 0.765 mmol) was added, and DIEA (0.126 mL, 0.765 mmol) and DMAP (3.89 mg, 0.032 mmol) were sequentially added to a mixed solution. A reaction solution was stirred at 25°C for 3 h. Liquid chromatography-mass spectrometry showed that raw materials were completely reacted and a product was generated. 200 ml of dichloromethane was added to the mixed solution, and the mixed solution was washed with a saturated salt solution (15.0 ml×3) for three times. An organic phase was dried with anhydrous sodium sulfate and then concentrated under reduced pressure to obtain a crude product. The crude product was separated by high performance liquid chromatography (chromatographic column: Waters Xbridge BEH C18 100*30mm*10um; mobile phase: water-ACN; gradient: 17%-57%/11 min; flow rate: 50 ml/min) to obtain a white solid E12 (35 mg, 0.012 mmol, 9.65%).

LCMS (ESI): m/z=1422.0 (M/2+H)⁺;

¹H NMR (400 MHz, CD₃OD) δ 7.42-7.44 (m, 2H), 7.19-7.35 (m, 7H), 6.81-6.92 (m, 4H), 5.32-5.34 (m, 4H), 5.06-5.12 (m, 4H), 4.59-4.68 (m, 4H), 4.40-4.42 (m, 2H), 4.30-4.34 (m, 1H), 4.18-4.28 (m, 2H), 4.11-4.18 (m, 8H), 3.99-4.11 (m, 14H), 3.82-3.99 (m, 7H), 3.78-3.79 (m, 6H), 3.67-3.75 (m, 8H), 3.65-3.66 (m, 1H), 3.60-3.62 (m, 10H), 3.52-3.54 (m, 8H), 3.47-3.49 (m, 1H), 3.37-3.38 (m, 7H), 3.11-3.18 (m, 2H), 2.50-2.61 (m, 4H), 2.21-2.41 (m, 10H), 2.14 (s, 12H), 2.02 (s, 12H), 1.93-1.94 (m, 24H), 1.49-1.66 (m, 4H), 1.29-1.31 (m, 8H).

### Example 13: Preparation of a compound E13

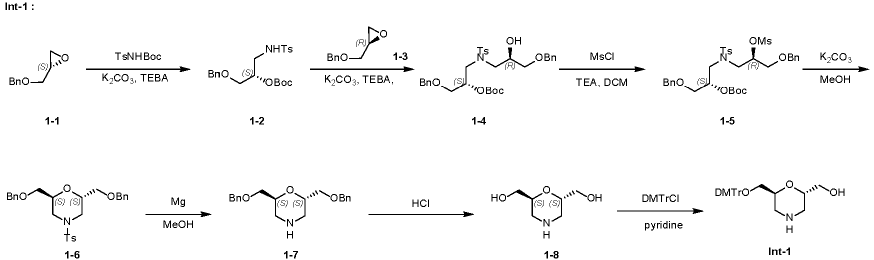

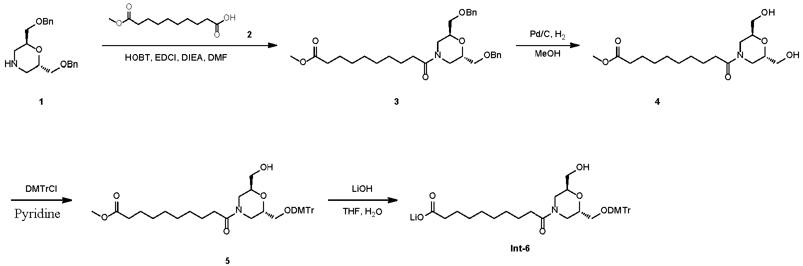

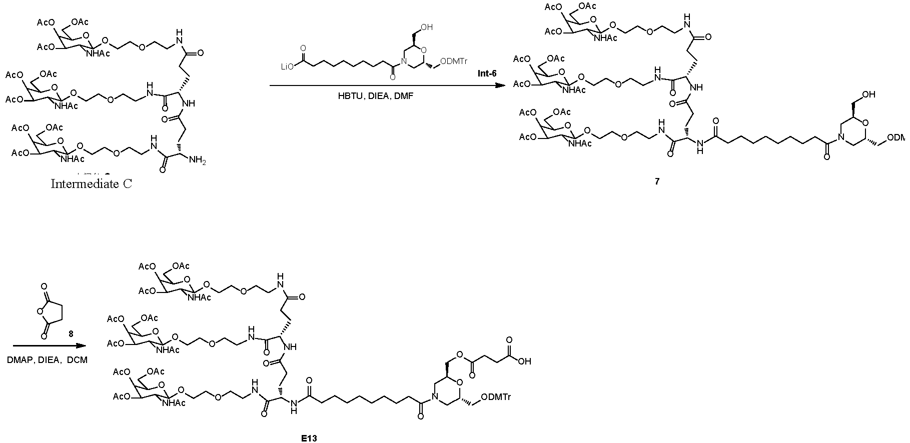

### 1. Preparation of a compound 1-2

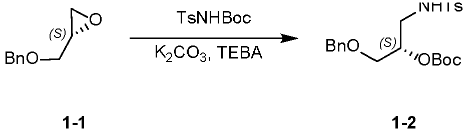

At room temperature, a compound 1-1 (38.0 g, 231 mmol), TsNHBoc (75.4 g, 278 mmol), K₂CO₃ (6.40 g, 46.3 mmol) and TEBA (5.27 g, 23.1 mmol) were added to a three-necked flask. A reaction was carried out at 95°C for 2 h, and then cooling was carried out to 25°C. TLC (PE/EA=2/1, UV 254 nm) showed that the reaction was completed. A reaction solution was diluted with water (500 mL) and then extracted with dichloromethane (200 mL×3). An organic phase was concentrated under reduced pressure, and a crude product was purified by column separation (PE/EA=10/1 to 3/1) to obtain a colorless oily compound 1-2 (about 40.0 g). ¹H NMR (400 MHz, CDCl₃) δ 7.72 (d, *J =* 8.4 Hz, 2 H), 7.28 - 7.39 (m, 7 H), 4.72 - 4.86 (m, 2 H), 4.42 - 4.56 (m, 2 H), 3.52 - 3.61 m, 2 H), 3.14 - 3.39 (m, 2 H), 2.43 (s, 3 H), 1.47 (s, 9 H).

### 2. Preparation of a compound 1-4

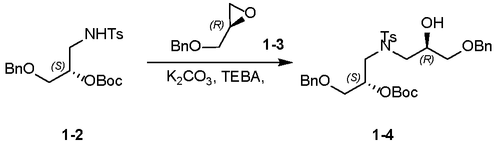

The compound 1-2 (79.0 g, 181 mmol), a compound 1-3 (4.21 g, 30.5 mmol) and TEBA (3.47 g, 15.2 mmol) were enabled to undergo a reaction at 95°C for 3 h. TLC (PE/EA=2/1, UV 254 nm) showed that the reaction was completed. A reaction solution was diluted with water (500 mL) and then extracted with dichloromethane (200 mL×3). An organic phase was concentrated under reduced pressure, and a crude product was purified by column separation (PE/EA=10/1 to 3/1) to obtain a colorless oily compound 1-4 (about 40.0 g). ¹H NMR (400 MHz, CDCl₃) δ 7.64 - 7.77 (m, 2 H), 7.27 - 7.38 (m, 13 H), 5.05 - 5.16 (m, 1 H), 4.50 - 4.59 (m, 4 H), 4.03 - 4.11 (m, 1 H), 3.62 - 3.76 (m, 2 H), 3.44 - 3.58 (m, 3 H), 3.25 - 3.33 (m, 2 H), 3.15 - 3.20 (m, 1 H), 2.42 (s, 3 H), 1.46 (s, 9 H).

### 3. Preparation of a compound 1-5

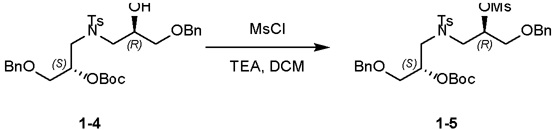

In an ice bath, the compound 1-4 (77.0 g, 128 mmol) and Et₃N (28.6 mL, 205 mmol) were sequentially added to dichloromethane (800 mL), and methanesulfonyl chloride (24.2 g, 212 mmol) was slowly added dropwise. A reaction was carried out at 25°C for 2 h. LCMS showed that the reaction was completed. Water (800 mL) was added to a reaction solution for washing, and an organic phase was concentrated under reduced pressure to obtain a colorless oily compound 1-5 (about 90.0 g). LCMS (ESI): m/z=700.1 (M+Na)⁺; ¹H NMR (400 MHz, CDCl₃) δ 7.68 (d*, J =* 8.4 Hz, 2 H), 7.28 - 7.39 (m, 12 H), 5.10 - 5.20 (m, 1 H), 4.93 - 5.02 (m, 1 H), 4.52 - 4.61 (m, 4 H), 3.72 - 3.88 (m, 2 H), 3.52 - 3.67 (m, 4 H), 3.27 - 3.36 (m, 1 H), 3.15 - 3.22 (m, 1 H), 3.04 (s, 3 H), 2.43 (s, 3 H), 1.43 (s, 9 H). LCMS: m/z=700.1 (M+H)⁺.

### 4. Preparation of a compound 1-6

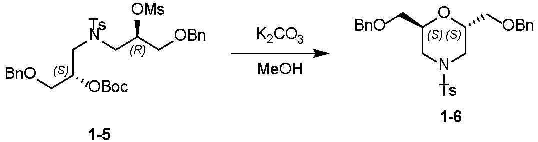

At room temperature, the compound 1-5 (90.0 g, 133 mmol) and potassium carbonate (91.8 g, 664 mmol) were added to MeOH (900 mL). A reaction was carried out at 66°C for 2 h. TLC (PE/EA=2/1, UV 254 nm) showed that a new point was generated. An organic phase was concentrated under reduced pressure, water (200 mL) was added to a reaction solution for dilution, and extraction was carried out with dichloromethane (200 mL×3). An organic phase was concentrated under reduced pressure, and a crude product was purified by column separation (PE/EA=30/1 to 2/1) to obtain a white solid compound 1-6 (about 64.0 g). ¹H NMR (400 MHz, CDCl₃) δ 7.62 (d, *J =* 8.4 Hz, 2 H), 7.27 - 7.39 (m, 12 H), 4.48 - 4.62 (m, 4 H), 3.93 - 4.08 (m, 2 H), 3.55 - 3.69 (m, 4 H), 2.84 - 3.10 (m, 4 H), 2.44 (s, 3 H).

### 5. Preparation of a compound 1-7

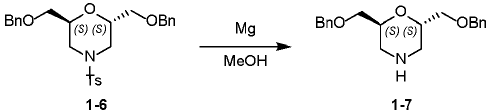

At room temperature, the compound 1-6 ( 69.0 g, 143 mmol) and magnesium chips (54.7 g, 2.28 mol) were added to MeOH (400 mL) to carry out a reaction at 66°C for 1 h. TLC (DCM/MeOH= 10/1, UV 254 nm) showed that raw materials were completely reacted and a new point was generated. A reaction solution was diluted with water (3,000 mL) and a saturated ammonium chloride aqueous solution (3,000 mL), and then extracted with dichloromethane (1,000×3). An organic phase was washed with saturated sodium bicarbonate (300 mL×3), and the organic phase was concentrated under reduced pressure to obtain a colorless oily compound 1-7 (about 32.0 g). ¹H NMR (400 MHz, CDCl₃) δ 7.27 - 7.40 (m, 10 H), 4.57 (s, 4 H), 3.90 - 4.00 (m, 2 H), 3.59 - 3.69 (m, 4 H), 2.77 - 3.04 (m, 4 H).

### 6. Preparation of a compound 1-8

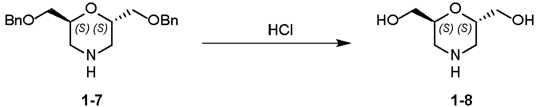

At room temperature, the compound 1-7 (8.00 g, 24.4 mmol) was added to HCl (100 mL, 12 M) to carry out a reaction at 50°C for 2 h. A reaction solution was concentrated under reduced pressure to obtain a colorless oily compound 1-8 (about 3.60 g, HCl salt). ¹H NMR (400 MHz, CDCl₃) δ 4.12 - 4.22 (m, 2 H), 3.78 (d, *J =* 4.4 Hz, 4 H), 3.17 - 3.30 (m, 4 H).

### 7. Preparation of a compound Int-1

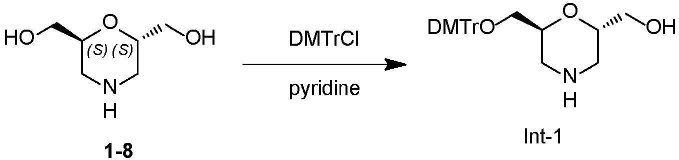

At room temperature, the compound 1-8 (8.29 g, 24.5 mmol) was added to pyridine (50 mL, 618 mmol), and then DMTrCl (4.77 g, 12.3 mmol) was added. The reaction system was enabled to undergo a reaction at 25°C for 18 h. TLC (DCM/MeOH=10/1, UV 254 nm) showed that a product was generated. A reaction solution was concentrated under reduced pressure, diluted with saturated ammonium chloride (200 mL) and extracted with DCM (100 mL×3). An organic phase was concentrated under reduced pressure. A crude product was separated by column chromatography (DCM/MeOH=99/1 to 10/1) to obtain a yellow solid Int-1 (about 2.6 g, 5.78 mmol, 23.6%). ¹H NMR (400 MHz, CDCl₃) δ 7.38 - 7.50 (m, 2 H), 7.25 - 7.36 (m, 6 H), 7.18 - 7.25 (m, 1 H), 6.87 (d, *J =* 8.8 Hz, 4 H), 4.26 - 4.40 (m, 1 H), 3.94 - 4.05 (m, 1 H), 3.85 (d, *J =* 4.4 Hz, 2 H) 3.73 - 3.82 (m, 6 H), 3.34 - 3.41 (m, 1 H), 3.23 - 3.30 (m, 3 H), 3.05 - 3.23 (m, 2 H).

### 8. Preparation of a compound 3

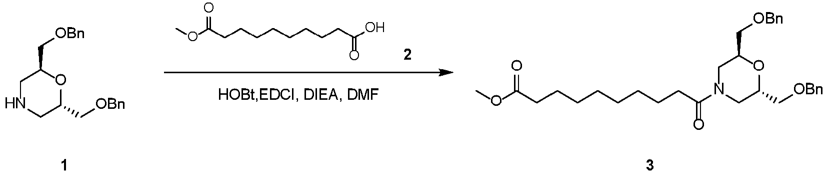

A compound 1 (1.00 g, 3.05 mmol) was dissolved in anhydrous N,N-dimethylformamide (10.0 mL) at 25°C, HOBt (0.54 g, 3.97 mmol), EDCI (0.76 g, 3.97 mmol) and DIEA (1.51 mL, 9.16 mmol) were sequentially added, and then a compound 2 (0.79 g, 3.66 mmol) was added. A mixed solution was stirred at 25°C for 2 h. Liquid chromatography-mass spectrometry showed that raw materials were completely reacted and a product was generated. Thin layer chromatography (dichloromethane:methanol=10:1) showed that the raw materials were completely reacted and a product was generated. Dichloromethane (200 ml) was added to the mixed solution, and the mixed solution was washed with a saturated citric acid solution (20 ml×3), a saturated sodium bicarbonate solution (20 ml×3) and a saturated salt solution (20 ml×3) for three times, respectively. An organic phase was dried with anhydrous sodium sulfate and then concentrated under reduced pressure to obtain a crude product. The crude product was purified by column chromatography (dichloromethane: methanol=10:1) to obtain a yellow oily compound 3 (about 1.00 g). ¹H NMR (400 MHz, CD₃OD) δ 7.21-7.41 (m, 10H), 4.47-4.60 (m, 4H), 3.88-4.01 (m, 2H), 3.75 (dd, *J =* 3.2, 13.2 Hz, 1H), 3.63-3.65 (m, 3H), 3.40-3.63 (m, 7H), 2.27-2.34 (m, 4H), 1.50-1.60 (m, 4H), 1.28-1.34 (m, 8H).

LCMS: m/z=526.8 (M+H)⁺.

### 9. Preparation of a compound 4

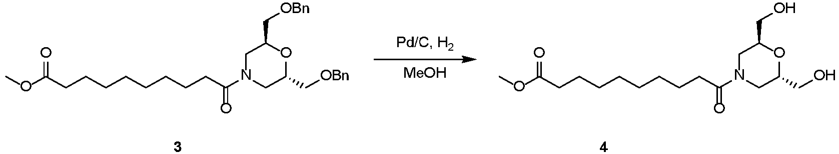

The compound 3 (380 mg, 0.20 mmol) was dissolved in anhydrous methanol (10.0 mL) at 25°C, and Pd/C (500 mg, 4.70 mmol) with a mass fraction of 10% was added. A reaction solution was stirred at 50°C for 12 h. Liquid chromatography-mass spectrometry showed that a product was generated. The reaction solution was filtered with diatomite, and a filtrate was concentrated under reduced pressure to obtain a yellow oily compound 4 (about 600 mg). ¹H NMR (400 MHz, CD₃OD) δ 3.79-3.89 (m, 2H), 3.73 (dd, *J =* 3.6, 13.2 Hz, 1H), 3.64-3.68 (m, 5H), 3.57-3.62 (m, 2H), 3.48-3.56 (m, 2H), 3.38-3.47 (m, 1H), 1.58-1.62 (m, 5H), 1.34 (d, *J =* 2.8 Hz, 11H), 1.30-1.37 (m, 1H). LCMS: m/z=346.5 (M+H)⁺.

### 10. Preparation of a compound 5

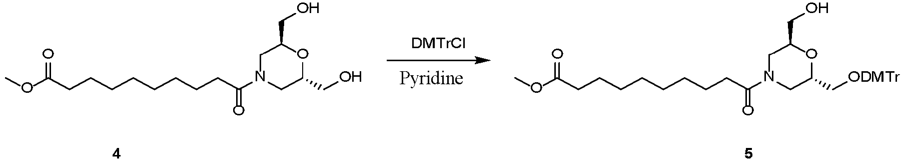

The compound 4 (600 mg, 1.74 mmol) was dissolved in pyridine (3.00 mL) at 25°C, and DMTrCl (883 mg, 2.61 mmol) was added. A reaction solution was stirred at 25°C for 1 h. Liquid chromatography-mass spectrometry showed that a product was generated. Thin layer chromatography (dichloromethane:methanol=10:1) showed that raw materials were completely reacted and a product was generated. Dichloromethane (200 ml) was added to a mixed solution, and the mixed solution was washed with a saturated sodium bicarbonate solution (20 ml×3) and a salt solution (20 ml×3) for three times, respectively. An organic phase was dried with anhydrous sodium sulfate and then concentrated under reduced pressure to obtain a crude product. The crude product was purified by column chromatography (petroleum ether: ethyl acetate=3:1) to obtain a colorless liquid compound 5 (about 475 mg). ¹H NMR (400 MHz, CD₃OD) δ 7.41-7.43 (m, 2H), 7.16-7.34 (m, 7H), 6.83-6.87 (m, 4H), 3.88-4.04 (m, 2H), 3.77-4.09 (m, 7H), 3.61-3.68 (m, 4H), 3.43-3.58 (m, 3H), 3.33-3.38 (m, 1H), 3.14-3.20 (m, 1H), 3.05 (dd, *J =* 8.4, 13.2 Hz, 1H), 2.20-2.42 (m, 4H), 1.51-1.60 (m, 4H), 1.25-1.39 (m, 8H). LCMS: m/z=648.3 (M+H)⁺.

### 11. Preparation of a compound Int-6

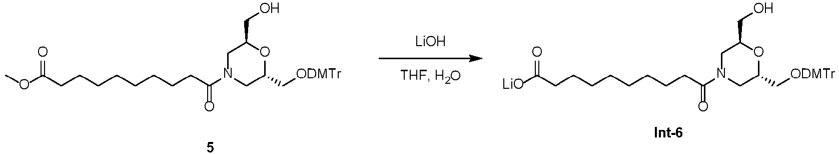

The compound 5 (475 mg, 0.73 mmol) was dissolved in tetrahydrofuran (8.00 mL) and H₂O (2.00 mL) at 25°C, and lithium hydroxide monohydrate (36.9 mg, 0.89 mmol) was added. A reaction solution was stirred at 25°C for 12 h. Thin layer chromatography (dichloromethane:methanol=10:1) showed that raw materials were completely reacted and a product was generated. The reaction solution was concentrated under reduced pressure and then directly freeze-dried on a freeze dryer to obtain a white solid product compound 6 (about 430 mg).

### 12. Preparation of a compound 7

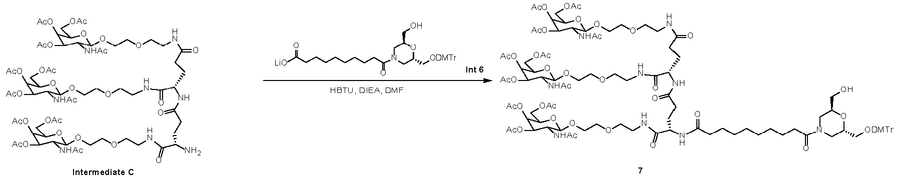

The compound Int-6 (100 mg, 0.156 mmol) was dissolved in anhydrous N,N-dimethylformamide (3.00 mL) at 25°C, and an HBTU reagent (89.0 mg, 0.234 mmol), DIEA (0.078 mL, 0.47 mmol) and an intermediate C (254 mg, 0.156 mmol, prepared by a method of an intermediate 2-2 in Example 26) were sequentially added. A reaction solution was stirred at 25°C for 12 h. Thin layer chromatography (dichloromethane:methanol=10:1) showed that a new point was generated. Dichloromethane (200 ml) was added to a mixed solution, and the mixed solution was washed with a saturated sodium bicarbonate solution (20 ml×3) for three times and washed with a salt solution (20 ml×3) for three times. An organic phase was dried with anhydrous sodium sulfate and then concentrated under reduced pressure to obtain a crude product. The crude product was purified by column chromatography (dichloromethane: methanol=10:1) to obtain a yellow oily product compound 7 (about 140 mg). ¹H NMR (400 MHz, CD₃OD) δ 7.43 (d*, J =* 8.4 Hz, 2H), 7.18-7.34 (m, 7H), 6.81-6.91 (m, 4H), 5.34 (d, *J =* 2.8 Hz, 3H), 5.09-5.11 (m, 3H), 4.62-4.66 (m, 3H), 4.27-4.35 (m, 2H), 4.07-4.17 (m, 9H), 4.00-4.07 (m, 4H), 3.89-3.98 (m, 5H), 3.79 (d, *J =* 2.4 Hz, 6H), 3.69-3.75 (m, 5H), 3.60-3.64 (m, 9H), 3.52-3.56 (m, 10H), 3.43-3.50 (m, 4H), 3.39-3.40 (m, 1H), 2.25-2.40 (m, 8H), 2.13-2.15 (m, 9H), 2.02 (s, 9H), 1.94-1.96 (m, 18H), 1.48-1.66 (m, 4H), 1.21-1.36 (m, 11H).

### 13. Preparation of E13

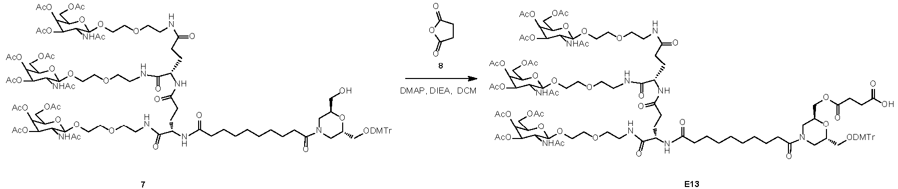

The compound 7 (400 mg, 0.187 mmol) was dissolved in anhydrous dichloromethane (5.00 mL), DIEA (0.18 mL, 1.12 mmol) and DMAP (5.71 mg, 0.047 mmol) were sequentially added, and a compound 8 (112 mg, 1.12 mmol) was added at last. A mixed solution was stirred at 25°C for 3 h. Thin layer chromatography (dichloromethane:methanol=10:1) showed that a new point was generated. Dichloromethane (200 ml) was added to the mixed solution, the mixed solution was washed with a saturated salt solution (15.0 ml×3) for three times, and an organic phase was dried with anhydrous sodium sulfate and then concentrated under reduced pressure to obtain a crude product. The crude product was separated by high performance liquid chromatography (chromatographic column: Welch Xtimate C18 150*25mm*5um; mobile phase: water-ACN; gradient: 27%-57%/11 min; flow rate: 25 ml/min) to obtain a white solid compound E13 (74.8 mg, 0.033 mmol, yield: 17.9%). LCMS (ESI): m/z=1149.5 (M/2+H)⁺ ; ¹H NMR (400 MHz, CD₃OD) δ 7.42-7.44 (m, 2H), 7.18-7.34 (m, 7H), 6.83-6.90 (m, 4H), 5.33-5.34 (m, 3H), 5.08-5.11 (m, 3H), 4.61-4.68 (m, 3H), 4.27-4.36 (m, 2H), 4.19-4.27 (m, 1H), 4.10-4.19 (m, 7H), 4.08 (s, 2H), 4.00-4.07 (m, 4H), 3.90-3.94 (m, 4H), 3.83-3.89 (m, 1H), 3.79 (d, *J*=2.4 Hz, 6H), 3.70-3.72 (m, 4H), 3.53-3.54 (m, 15H), 3.44-3.48 (m, 3H), 3.35-3.40 (m, 4H), 3.10-3.23 (m, 2H), 2.53-2.63 (m, 4H), 2.22-2.40 (m, 8H), 2.11-2.16 (m, 9H), 2.05-2.10 (m, 2H), 2.02 (s, 9H), 1.90-1.97 (m, 19H), 1.48-1.65 (m, 4H), 1.29-1.32 (m, 8H).

### Example 14: Preparation of a compound E14

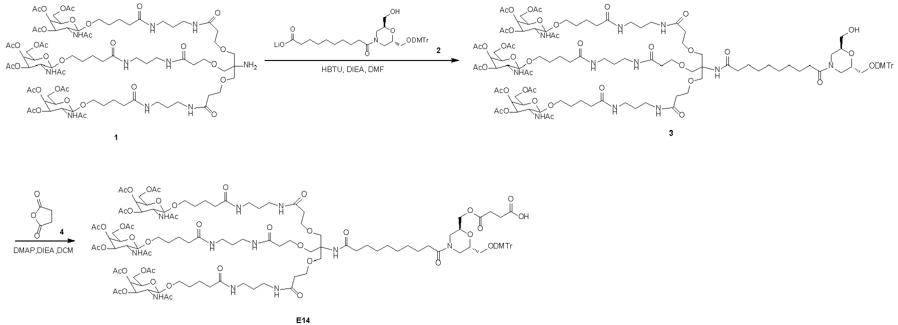

### 1. Preparation of a compound 3

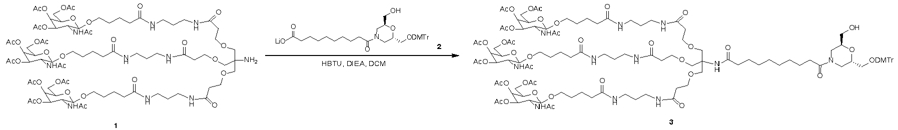

A compound 1 (800 mg, 0.446 mmol, prepared by a method of an intermediate 1-2 in Example 27) was dissolved in DCM (5 mL) at room temperature, and a compound 2 (252.28 mg, 0.398 mmol, prepared by a method of the compound Int-6 in Example 13), HBTU (253.69 mg, 0.669 mmol) and DIEA (0.221 mL, 1.338 mmol) were added to carry out a reaction at 25°C for 15 h. Thin layer chromatography (dichloromethane/methanol=8/1) showed that raw materials disappeared and a new point was generated. Liquid chromatography-mass spectrometry showed that the raw materials were completely reacted and a product was generated. Dichloromethane (20 mL) was added for diluting a reaction solution, and an organic phase was sequentially washed with a saturated NaHCO₃ solution (10 mL×3) and a saturated NaCl solution (10 mL×3) for 3 times and then dried with anhydrous sodium sulfate. The reaction solution was concentrated under reduced pressure to obtain a crude product, and the crude product was purified by column chromatography (dichloromethane/methanol=8/1) to obtain a white solid 3 (about 612 mg). ¹H NMR (400 MHz, CD₃OD) *δ* 7.43 (d, *J =* 8.4 Hz, 2H), 7.30 (d, *J* = 7.6 Hz, 5H), 7.17-7.26 (m, 2H), 6.81-6.90 (m, 4H), 5.33 (d, *J*=3.2Hz, 3H), 5.06 (dd, *J =* 3.2, 11.2 Hz, 3H), 4.55 (d, *J =* 8.4 Hz, 3H), 4.04-4.18 (m, 10H), 3.97-4.04 (m, 4H), 3.82-3.96 (m, 5H), 3.79 (d, *J =* 2.8 Hz, 6H), 3.59-3.70 (m, 15H), 3.49-3.56 (m, 5H), 3.17-3.27 (m, 15H), 2.42 (t*, J =* 6.0 Hz, 6H), 2.16-2.24 (m, 8H), 2.12-2.15 (m, 9H), 2.01-2.03 (m, 9H), 1.94 (d, *J*=7.53 Hz, 18H), 1.64-1.71 (m, 12H), 1.52-1.62 (m, 10H), 1.24-1.34 (m, 8H). LCMS: m/z=1203.5 ((M+H)/2)⁺.

### 2. Preparation of a compound E14

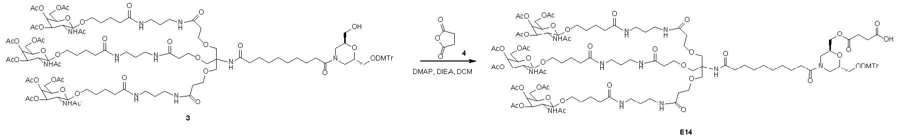

The compound 3 (612 mg, 0.254 mmol) was dissolved in DCM (5 mL) at room temperature, and DIEA (0.252 mL, 1.524 mmol), DMAP (7.76 mg, 0.063 mmol) and a compound 4 (152.49 mg, 1.524 mmol) were added to carry out a reaction at 25°C for 3 h. Liquid chromatography-mass spectrometry showed that raw materials were completely reacted and a product was generated. Dichloromethane (30 mL) was added for diluting a reaction solution, and an organic phase with washed with a saturated NaCl solution (30 mL×3) for 3 times and then dried with anhydrous sodium sulfate. The reaction solution was concentrated under reduced pressure, and a resulting crude product was separated by preparative high performance liquid chromatography (preparative-HPLC, column: Waters Xbridge BEH C18 100*30mm*10um; mobile phase: water-ACN; B%: 17%-57%, 12 min) to obtain a white solid E14 (160.1 mg, yield: 25.1%, purity: 73.34%).

1H NMR (400 MHz, CD₃OD) *δ* 7.43 (d, *J =* 8.0 Hz, 2H), 7.31 (d, *J =* 8.8 Hz, 5H), 7.17-7.27 (m, 2H), 6.85-6.90 (m, 4H), 5.33 (d, *J =* 3.2 Hz, 3H), 5.06 (dd, *J =* 3.6, 11.2 Hz, 3H), 4.56 (d, *J =* 8.4 Hz, 3H), 4.05-4.20 (m, 10H), 3.94-4.03 (m, 5H), 3.80-3.94 (m, 5H), 3.79 (d, *J =* 2.4 Hz, 6H), 3.57-3.72 (m, 15H), 3.40-3.57 (m, 4H), 3.18-3.26 (m, 15H), 2.48-2.61 (m, 4H), 2.42 (t, *J =* 6.0 Hz, 6H), 2.16-2.25 (m, 8H), 2.14 (s, 9H), 2.02 (s, 9H), 1.92-1.96 (m, 18H), 1.62-1.71 (m, 12H), 1.51-1.62 (m, 10H), 1.30 (d, *J* = 13.2 Hz, 8H).

LCMS: m/z=1283.5 ((M +59)/ 2)⁺.

### Example 15: Preparation of a compound E15

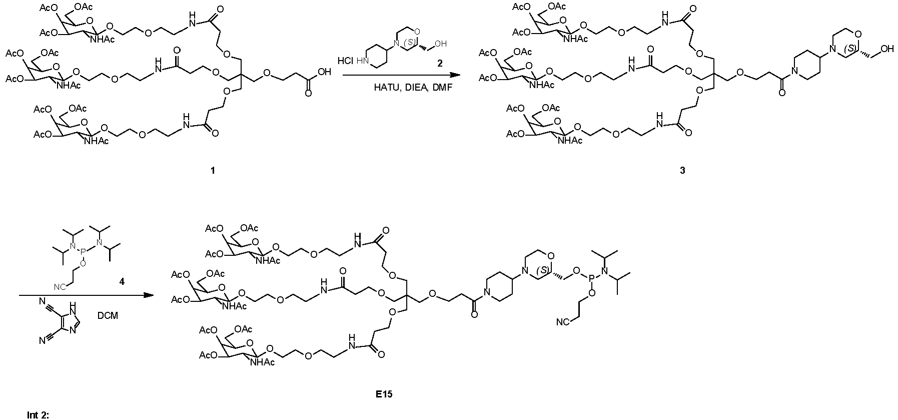

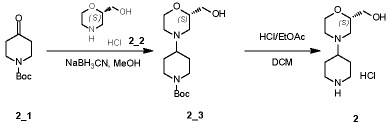

### 1. Preparation of a compound 2_3

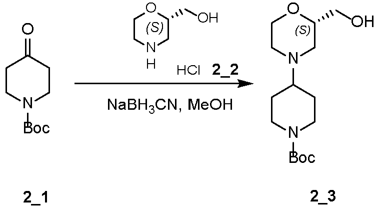

A compound 2_1 ( 11.6 g, 58.59 mmol) and a compound 2_2 (4.5 g, 29.29 mmol) were dissolved in MeOH (100 mL). A reaction solution was enabled to undergo to a reaction at 20°C for 2 h. Then, sodium cyanoborohydride (7.37 g, 117.180 mmol) was added to reactants. The reaction solution was enabled to undergo to a reaction at 20°C for 16 h. TLC (DCM/MeOH=10/1) showed that a new point was generated. 60 mL of DCM and 40 mL of water were added to the reaction solution, the reaction solution was subjected to liquid separation, and an aqueous phase was extracted with DCM (55 mL*1). Combined organic phases were washed with a saturated salt solution (50 mL*1), dried with anhydrous sodium sulfate, filtered and spin-dried to obtain a crude product. The crude product was purified by column chromatography (DCM/MeOH=1/0 to 10/1) to obtain a white solid compound 2_3 (about 2.6 g). ¹H NMR (400 MHz, MeOD-*d*4) δ 4.11 (br d, *J*=13.6 Hz, 2H), 3.86-3.93 (m, 1H), 3.42-3.77 (m, 5H), 2.92 (br d, *J*=11.2 Hz, 1H), 2.69-2.86 (m, 3H), 2.24- 2.19-2.51 (m, 2H), 2.00-2.12 (m, 1H), 1.79-1.97 (m, 2H), 1.39-1.52 (m, 10H).

### 2. Preparation of a compound 2

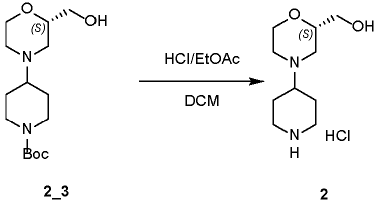

The compound 2_3 (6.5 g, 21.6 mmol) was dissolved in DCM (50 mL), and HCl/EtOAc (20 mL) was added. A reaction solution was enabled to undergo to a reaction at 20°C for 1 h. TLC (DCM/MeOH=10/1) showed a new point was generated. The reaction solution was spin-dried to obtain a crude white solid compound 2 (about 5.1 g). ¹ H NMR (400 MHz, MeOD-*d*4) δ 4.14-4.22 (m, 1H), 3.90-4.04 (m, 2H), 3.53-3.73 (m, 7H), 2.98-3.24 (m, 4H), 2.42-2.54 (m, 2H), 2.03-2.17 (m, 2H).

### 3. Preparation of a compound 3

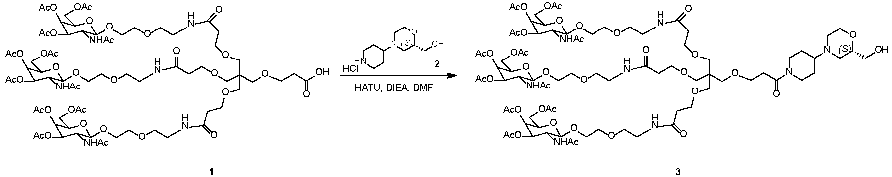

A compound 1 (1,500 mg, 0.896 mmol), HATU (511 mg, 1.34 mmol) and DIEA (0.741 mL, 4.48 mmol) were dissolved in DMF (15.0 mL). A reaction solution was enabled to undergo to a reaction at 20°C for 30 min. Then, the compound 2 (269 mg, 1.34 mmol) was added, and the reaction solution was enabled to undergo to a reaction at 20°C for 2 h. LCMS showed that an MS value of a product was generated. TLC (DCM/MeOH=5/1) showed that a new point was generated. 15 mL of DCM and 20 mL of a saturated sodium bicarbonate aqueous solution were added to the reaction solution, the reaction solution was subjected to liquid separation, and an aqueous phase was extracted with DCM (15.0 mL×1). Combined organic phases were washed with a saturated salt solution (25 mL×1), dried with anhydrous sodium sulfate, filtered and spin-dried to obtain a crude product. The crude product was purified by column chromatography (DCM/MeOH=1/0 to 5/1) to obtain a yellow solid product compound 3 (about 850 mg). ¹H NMR (400 MHz, DMSO-*d*₆) δ 7.79-7.95 (m, 6H), 5.21 (d, *J =* 3.2 Hz, 3H), 4.98 (dd, *J =* 3.2, 11.2 Hz, 3H), 4.65 (s, 1H), 4.55 (d, *J =* 8.4 Hz, 3H), 4.37 (d, *J =* 12.0 Hz, 1H), 4.03 (s, 9H), 3.72-3.95 (m, 8H), 3.45-3.66 (m, 18H), 3.37-3.42 (m, 8H), 3.08-3.27 (m, 16H), 2.93-3.06 (m, 1H), 2.76-2.89 (m, 1H), 2.62-2.75 (m, 1H), 2.53-2.59 (m, 3H), 2.29 (t, *J =* 6.4 Hz, 6H), 2.12 (s, 9H), 2.00 (s, 9H), 1.89 (s, 9H), 1.77 (s, 9H), 1.23-1.30 (m, 6H). LCMS: m/z=929.1 (M/2+H)⁺.

### 4. Preparation of E15

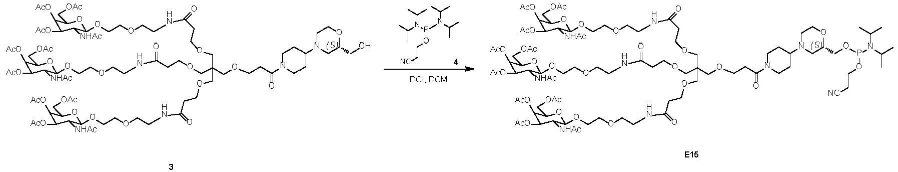

The compound 3 (850 mg, 0.458 mmol) was dissolved in acetonitrile, spin-dried to remove water in the compound and then dissolved in DCM (10.0 mL) in a nitrogen atmosphere, DCI (27.0 mg, 0.229 mmol) and a compound 4 (276 mg, 0.916 mmol) were slowly added dropwise, and a reaction solution was enabled to undergo a reaction at 20°C for 1 h. LCMS showed that raw materials disappeared. The reaction solution was cooled to 0°C, 6 ml of a saturated sodium bicarbonate aqueous solution and 6 ml of a saturated salt solution were added dropwise, and the mixed solution was diluted with 100 ml of dichloromethane and then subjected to liquid separation. An organic phase was washed with 50 ml of a mixed aqueous solution of saturated sodium bicarbonate and a saturated salt solution at 1:1. The organic phase was dried with anhydrous sodium sulfate, filtered and spin-dried to obtain a crude product. The crude product was diluted with 15 ml of dichloromethane, slowly added dropwise to 60 ml of methyl tert-butyl ether and then filtered, a filter cake was washed with methyl tert-butyl ether for three times, and the filter cake was dissolved in acetonitrile and then spin-dried to remove the methyl tert-butyl ether in the compound. A yellow solid product E15 (555 mg, 0.269 mmol, 58.9%) was obtained.

LCMS (ESI): m/z=1087.0 ((M+59*2)/2+H)⁺;

¹H NMR (400 MHz, DMSO-*d*₆) *δ* 7.85 - 7.90 (m, 3 H), 7.75-7.84 (m, 3 H), 5.20 - 5.24 (m, 3 H), 4.98 (dd, *J =* 3.6, 11.2 Hz, 3 H), 4.51 - 4.58 (m, 3 H), 3.98-4.07 (m, 10 H), 3.83 - 3.91 (m, 4 H), 3.69 - 3.82 (m, 6 H), 3.47 - 3.62 (m, 23 H), 3.35-3.42 (m, 8 H), 3.15 - 3.26 (m, 15 H), 2.95 - 3.02 (m, 1 H), 2.73 - 2.84 (m, 3 H), 2.63-2.65 (m, 1 H), 2.36 - 2.42 (m, 2 H), 2.29 (t, J=6.4 Hz, 6 H), 2.10-2.13 (m, 1 H), 2.10 (s, 9 H), 2.00 (s, 9 H), 1.89 (s, 9 H), 1.69 - 1.79 (m, 11 H), 1.24 -1.39 (m, 2 H), 1.03 - 1.17 (m, 12 H).

³¹P NMR (400 MHz, DMSO-*d*₆) *δ* -147.834.

### Example 16: Preparation of a compound E16

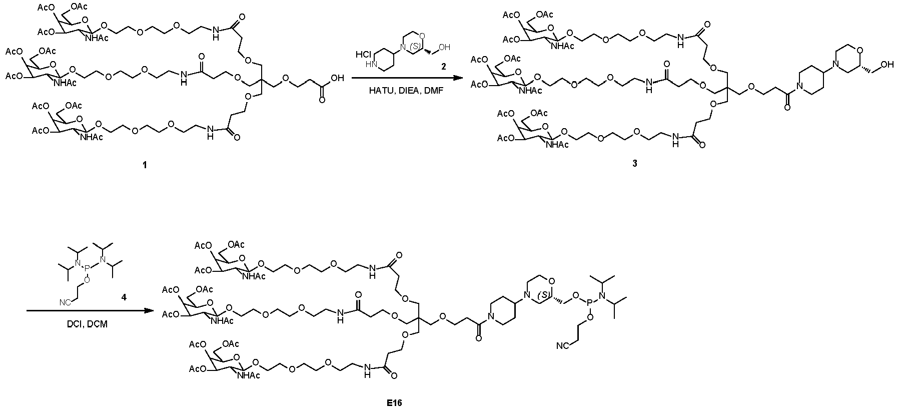

### 1. Preparation of a compound 3

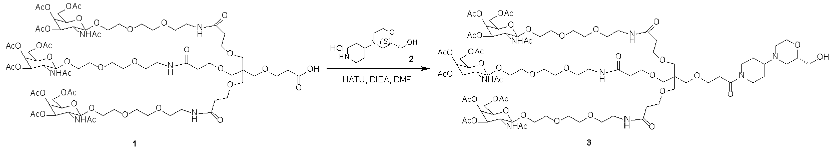

A compound 1 (1.35 g, 0.748 mmol, prepared by a method of the compound 8 in Example 1), HATU (0.430 g, 1.12 mmol) and DIEA (0.371 mL, 2.24 mmol) were added to DMF (2.00 mL), nitrogen replacement was carried out for three times, a reaction was carried out at 20°C for 0.5 h, and then a compound 2 (0.270 g, 1.12 mmol, prepared by a method of the compound 2 in Example 15) was added to carry out a reaction at 20°C for 1.5 h. LC/MS showed that raw materials completely disappeared and a main peak was generated. Thin layer chromatography (dichloromethane/methanol=8/1) showed that the raw materials disappeared and a new point was generated. A reaction solution was diluted with dichloromethane (100 mL), and sequentially washed with a saturated sodium bicarbonate aqueous solution (20.0 mL×3) and a saturated salt solution (20.0 mL×3). An organic phase was dried with anhydrous sodium sulfate and then concentrated under reduced pressure to obtain a crude product. The crude product was purified by column chromatography (dichloromethane:methanol=100/1 to 8/1) to obtain a brown solid compound 3 (about 850 mg). ¹H NMR (400 MHz, CD₃OD) *δ* 5.32 - 5.36 (m, 3 H), 5.05 - 5.11 (m, 3 H), 4.63 - 4.67 (m, 3 H), 4.08 - 4.20 (m, 10 H), 4.01 - 4.07 (m, 4 H), 3.91 - 3.97 (m, 4 H), 3.69 - 3.77 (m, 4 H), 3.60 - 3.68 (m, 29 H), 3.53 - 3.59 (m, 9 H), 3.35 - 3.41 (m, 15 H), 2.62 - 2.72 (m, 3 H), 2.48 - 2.55 (m, 1 H), 2.43 (t, *J =* 6.0 Hz, 6 H), 2.27 - 2.37 (m, 1 H), 2.15 (s, 9 H), 2.03 (s, 9 H), 1.91 - 1.97 (m, 20 H), 1.29 - 1.39 (m, 2 H). LCMS: m/z=994.9 (M/2+H)⁺.

### 2. Preparation of E16

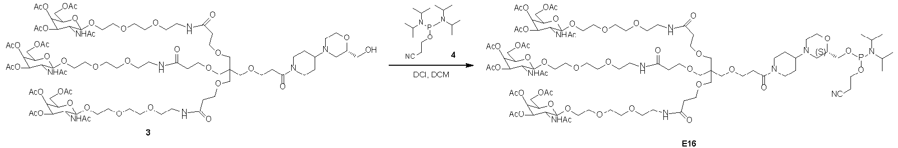

The compound 3 (850 mg, 0.428 mmol) was dissolved in acetonitrile, spin-dried to remove water and then dissolved in DCM (10.0 mL), DCI (25.2 mg, 0.214 mmol) was added, a compound 4 (257 mg, 0.855 mmol) was added in a nitrogen atmosphere, nitrogen replacement was carried out for three times, and a reaction was carried out at 20°C for 1 h. LCMS showed that raw materials disappeared. A reaction solution was cooled to 0°C, 6 ml of a saturated sodium bicarbonate aqueous solution and 6 ml of a saturated salt solution were added dropwise, and the mixed solution was diluted with 100 ml of dichloromethane and then subjected to liquid separation. An organic phase was washed with 50 ml of a mixed aqueous solution of saturated sodium bicarbonate and a saturated salt solution at 1:1. The organic phase was dried with anhydrous sodium sulfate, filtered and spin-dried to obtain a crude product. The crude product was diluted with 15 ml of dichloromethane, slowly added dropwise to 60 ml of methyl tert-butyl ether and then filtered, a filter cake was washed with methyl tert-butyl ether for three times, and the filter cake was dissolved in acetonitrile and then spin-dried to remove the methyl tert-butyl ether in the compound. A yellow solid product E16 (591 mg, 0.270 mmol, 63.17%) was obtained.

LCMS (ESI): m/z=2246.3 (M/+59+H)⁺; LCMS: m/z=1053.7 ((M-83)/2+H)⁺.

¹H NMR (400 MHz, DMSO-*d*₆) *δ* 7.87 - 7.93 (m, 3 H), 7.78 - 7.84 (m, 3 H), 5.19 - 5.24 (m, 3 H), 4.93 - 5.01 (m, 3 H), 4.53 - 4.58 (m, 3 H), 3.96 - 4.08 (m, 10 H), 3.83 - 3.92 (m, 4 H), 3.69 - 3.82 (m, 6 H), 3.44 - 3.64 (m, 37 H), 3.37 - 3.41 (m, 6 H), 3.14 - 3.26 (m, 15 H), 2.92 - 3.00 (m, 1 H), 2.72 - 2.84 (m, 3 H), 2.63 - 2.68 (m, 1 H), 2.35 - 2.44 (m, 2 H), 2.28 (t, J = 6.0 Hz, 6 H), 2.16 - 2.22 (m, 1 H), 2.10 (s, 9 H), 2.00 (s, 9 H), 1.89 (s, 9 H), 1.69 - 1.80 (m, 11 H), 1.20-1.40 (m, 2 H), 1.00 - 1.17 (m, 12 H).

³¹P NMR (400 MHz, DMSO-*d*₆) *δ* -147.815.

### Example 17: Preparation of a compound E17

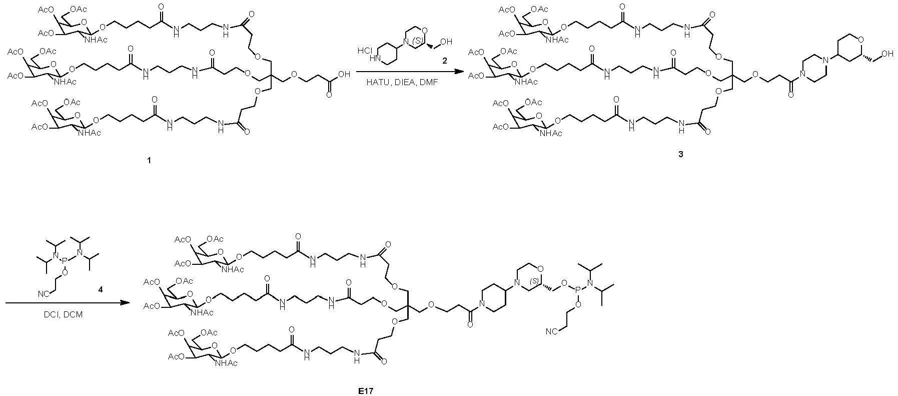

### 1. Preparation of a compound 3

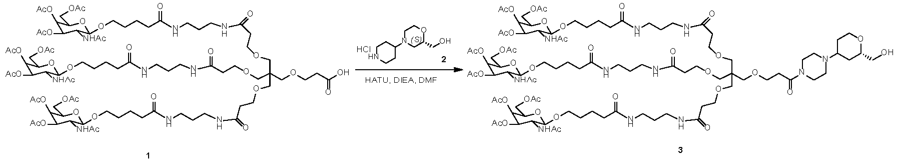

A compound 1 (1,500 mg, 0.797 mmol, prepared by a method of the compound 6 in Example 5), HATU (454 mg, 1.19 mmol) and DIEA (0.659 mL, 3.98 mmol) were dissolved in DMF (20 mL). A reaction solution was stirred at 20°C for 0.5 h. Then, a compound 2 (239 mg, 1.196 mmol, prepared by a method of the compound 2 in Example 15) was added. The reaction solution was stirred at 20°C for 2 h. LCMS showed that an MS value of a product was generated. TLC (DCM/MeOH=5/1) showed that a new point was generated. 15 mL of DCM and 20 mL of a saturated sodium bicarbonate aqueous solution were added to the reaction solution, the reaction solution was subjected to liquid separation, and an aqueous phase was extracted with DCM (15 mL×1). Combined organic phases were washed with a saturated salt solution (25 mL×1), dried with anhydrous sodium sulfate, filtered and spin-dried to obtain a crude product. The crude product was purified by column chromatography (DCM/MeOH=1/0 to 5/1) to obtain a yellow oily product compound 3 (about 840 mg). LCMS: m/z=1032.7 (M/2+H)⁺.

### 2. Preparation of E17

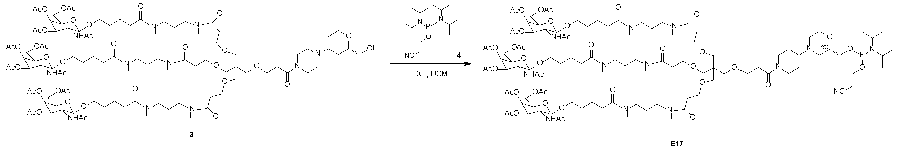

The compound 3 (890 mg, 0.431 mmol) was dissolved in acetonitrile, spin-dried to remove water in the compound and then dissolved in DCM (10.0 mL) in a nitrogen atmosphere, DCI (25.4 mg, 0.216 mmol) was added, a compound 4 (260 mg, 0.863 mmol) was slowly added dropwise, and a reaction solution was enabled to undergo a reaction at 20°C for 1 h. LCMS showed that raw materials disappeared. The reaction solution was cooled to 0°C, 6 ml of a saturated sodium bicarbonate aqueous solution and 6 ml of a saturated salt solution were added dropwise, and the mixed solution was diluted with 100 ml of dichloromethane and then subjected to liquid separation. An organic phase was washed with 50 ml of a mixed aqueous solution of saturated sodium bicarbonate and a saturated salt solution at 1:1. The organic phase was dried with anhydrous sodium sulfate, filtered and spin-dried to obtain a crude product. The crude product was diluted with 15 ml of dichloromethane, slowly added dropwise to 60 ml of methyl tert-butyl ether and then filtered, a filter cake was washed with methyl tert-butyl ether for three times, and the filter cake was dissolved in acetonitrile and then spin-dried to remove the methyl tert-butyl ether in the compound. A yellow solid product E17 (708 mg, 0.313 mmol, 72.51%) was obtained.

LCMS (ESI): m/z=2246.3 ((M+59*2)/2+H)⁺;

¹H NMR (400 MHz, DMSO-*d*₆) *δ* 7.78 - 7.86 (m, 6 H) 7.71 - 7.76 (m, 3 H), 5.21 (d, *J =* 3.2 Hz, 3 H), 4.96 (dd, *J =* 3.2, 11.2 Hz, 3 H), 4.48 (d, *J =* 8.4 Hz, 3 H), 3.99 - 4.06 (m, 10 H), 3.82 - 3.91 (m, 4 H), 3.65 - 3.79 (m, 6 H), 3.37 - 3.58 (m, 18 H), 3.21 (s, 8 H), 3.00 - 3.09 (m, 13 H), 2.86 - 2.99 (m, 2 H), 2.73 - 2.86 (m, 3 H), 2.62 - 2.69 (m, 1 H), 2.54 - 2.59 (m, 2 H), 2.26 (t, *J =* 6.0 Hz, 6 H), 2.15 - 2.20 (m, 1 H), 2.10 (s, 9 H), 2.04 (t, *J*=6.8 Hz, 6 H), 1.99 (s, 9 H), 1.89 (s, 9 H), 1.68-1.78 (m, 11 H), 1.41 - 1.55 (m, 20 H), 1.03 - 1.17 (m, 12 H).

³¹P NMR (400 MHz, DMSO-*d*₆) *δ* -147.834.

### Example 18: Preparation of a compound E18

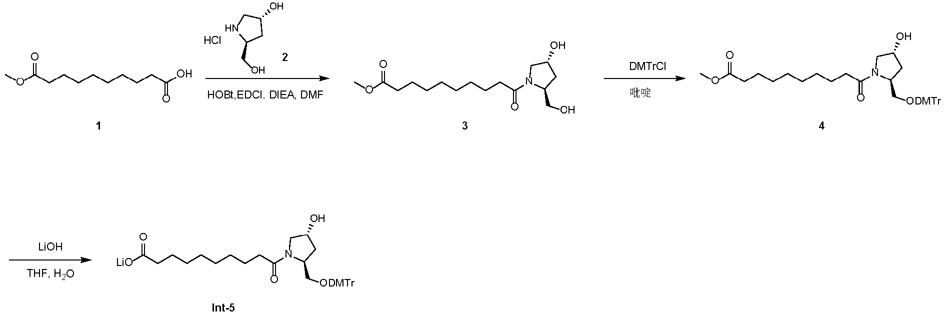

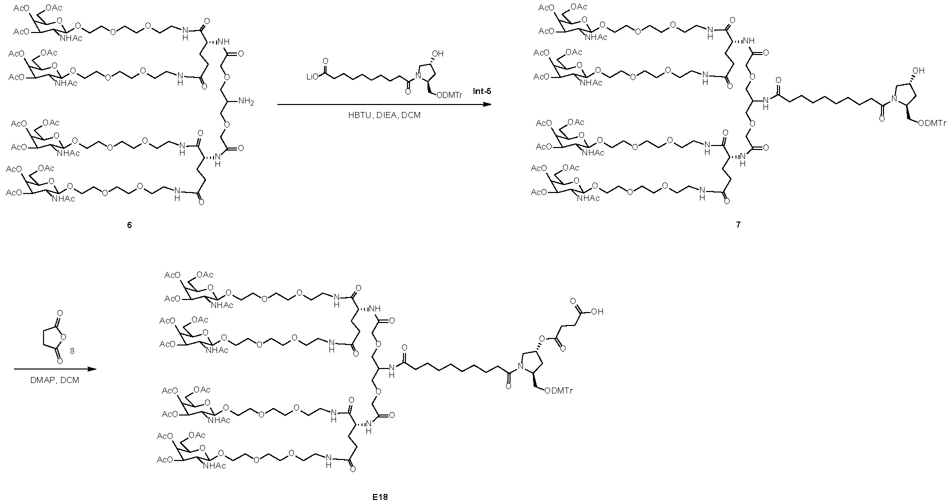

### 1. Preparation of a compound 3

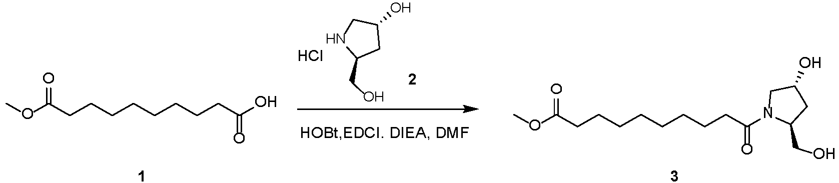

A compound 1 (1.42 g, 6.58 mmol) and a compound 2 (1.0 g, 6.58 mmol) were added to DMF (10.0 mL) at 25°C, and then HOBt (1.22 g, 9.02 mmol), EDCI (1.73 g, 9.02 mmol) and DIEA (3.44 mL, 20.8 mmol) were sequentially added. A mixed solution was stirred at 25°C for 3 h. LCMS showed that a product was generated, and TLC (DCM/MeOH=10/1, PMA) showed that a new point was generated. DCM (150 ml) was added to a reaction solution, and the reaction solution was sequentially washed with a saturated citric acid solution (20.0 ml×3), a sodium bicarbonate solution (20.0 ml×3) and a saturated salt solution (15 ml×3) for three times. An organic phase was dried with anhydrous sodium sulfate and then concentrated under reduced pressure to obtain a crude compound. The crude product was purified by TLC (DCM/MeOH=10/1 to 5/1) to obtain a yellow oily compound 3 (1.10 g, 3.49 mmol, yield: 50.3%).

LCMS: m/z=316.1 (M+H)⁺.

### 2. Preparation of a compound 4

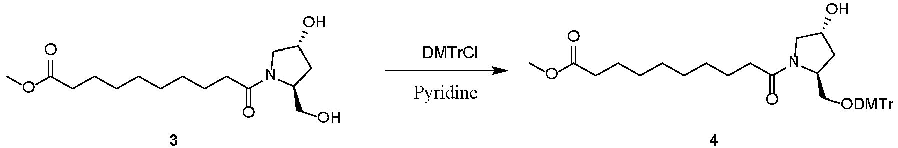

The compound 3 (1.10 g, 3.49 mmol) was dissolved in pyridine (20.0 mL) at 25°C, and a DCM solution (10.0 mL) of DMTrCl (1.18 g, 3.488 mmol) was added. A mixed solution was stirred at 25°C for 2 h. LCMS showed that a product was generated. DCM (200 ml) was added to the mixed solution, and the mixed solution was washed with saturated sodium bicarbonate (20.0 ml×3) and a saturated salt solution (20 ml×3) for three times, respectively. An organic phase was dried with anhydrous sodium sulfate and then concentrated under reduced pressure to obtain a crude product. The crude product was purified by TLC (DCM/MeOH=10/1 to 5/1) to obtain a yellow oily compound 4 (560 mg, 0.928 mmol, yield: 26.6%), which was characterized by LCMS.

LCMS: m/z=640.2 (M+H)⁺.

### 3. Preparation of a compound Int-5

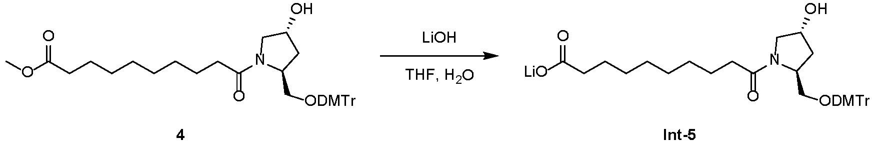

The compound 4 (560 mg, 0.906 mmol) was added to THF (3.00 mL) and H₂O (1.50 mL), and lithium hydroxide (57.05 mg, 1.360 mmol) was added to carry out a reaction at 20°C for 12 h. TLC (dichloromethane:methanol=8:1) showed that raw materials disappeared and a new point was generated. A reaction solution was spin-dried, added to 5 mL of water and then freeze-dried to obtain a white solid lithium salt product compound Int-5 (630 mg, 1.033 mmol, 113.99%).

### 4. Preparation of a compound 7

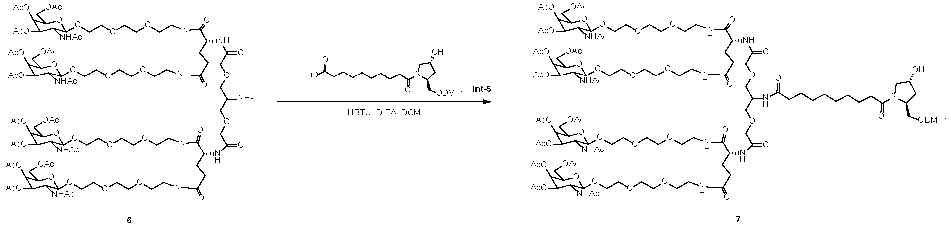

A compound 6 (prepared by a method of the compound 4 in Example 7, 190 mg, 0.08 mmol) was dissolved in DCM (5.00 mL) at 25°C, and then the Int-5 (50.2 mg, 0.08 mmol), HBTU (39.0 mg, 0.10 mmol) and DIEA (0.04 mL, 0.25 mmol) were sequentially added. A mixed solution was stirred at 25°C for 12 h. LCMS showed that a product was generated. After dichloromethane (60.0 ml) was added to a reaction solution, the reaction solution was washed with a saturated sodium bicarbonate solution (10.0 ml×3) and a saturated salt solution (10 ml×3) for three times. An organic phase was dried with anhydrous sodium sulfate and then concentrated under reduced pressure to obtain a crude compound 7 (270 mg, crude product). The product was directly used in a next step.

LCMS: m/z=1445.2 (M/2+H)⁺.

### 5. Preparation of a compound E18

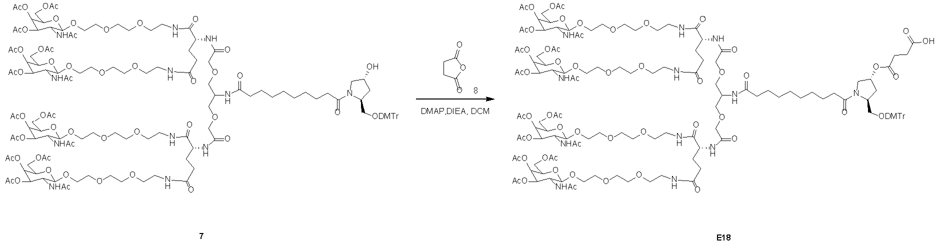

The compound 7 (270 mg, crude product) and a compound 8 (56.0 mg, 0.56 mmol) were added to DCM (5.00 mL) at 25°C, and then DIEA (0.093 mL, 0.56 mmol) and DMAP (2.85 mg, 0.023 mmol) were sequentially added. A mixed solution was stirred at 25°C for 12 h. LCMS showed that raw materials disappeared. Dichloromethane (100 ml) was added to a reaction solution, and the reaction solution was sequentially washed with a saturated sodium bicarbonate solution (15.0 ml×3) and a saturated salt solution (15.0 ml×3) for three times. An organic phase was dried with anhydrous sodium sulfate and then concentrated under reduced pressure to obtain a crude product. The crude product was separated by prep-HPLC (chromatographic column: Waters Xbridge BEH C18 150*25mm*5um; mobile phase: TEAA-ACN; gradient: 20%-53%/15 min; flow rate: 15 ml/min) to obtain a white solid product E18 (74 mg, 0.025 mmol, 26.49%). ¹H NMR (400 MHz, CD₃OD) *δ* 7.38 (d, *J =* 7.2 Hz, 2H), 7.17-7.32 (m, 7H), 6.78-6.94 (m, 4H), 5.34 (d, *J =* 2.80 Hz, 4H), 5.05-5.11 (m, 4H), 4.65 (dd, *J* = 3.2, 8.8 Hz, 4H), 4.08-4.45 (m, 14H), 3.88-4.08 (m, 13H), 3.75-3.83 (m, 7H), 3.66-3.74 (m, 8H), 3.58-3.66 (m, 24H), 3.48-3.58 (m, 9H), 3.32-3.48 (m, 11H), 3.11-3.21 (m, 24H), 3.07 (d, *J =* 7.2 Hz, 1H), 2.51-2.59 (m, 2H), 2.44-2.51 (m, 2H), 2.19-2.40 (m, 9H), 2.14 (s, 12H), 2.05-2.12 (m, 3H), 2.02 (s, 13H), 1.95 (s, 12H), 1.92 (s, 13H), 1.61 (s, 3H), 1.16-1.38 (m, 44H).

### Example 19: Preparation of a compound E19

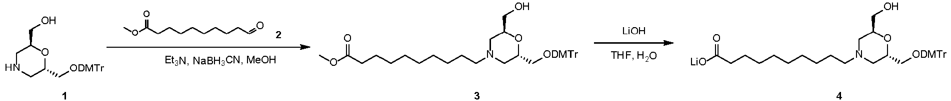

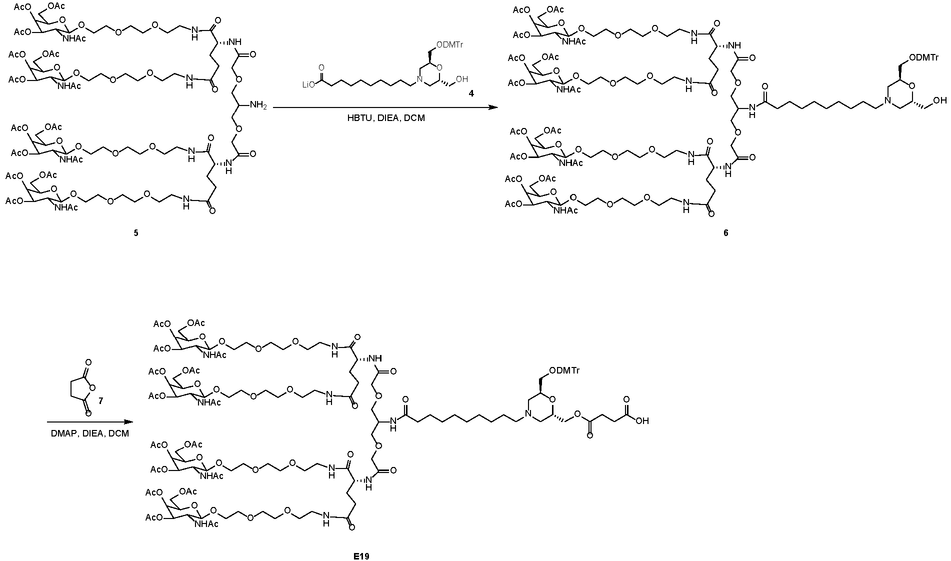

### 1. Preparation of a compound 3

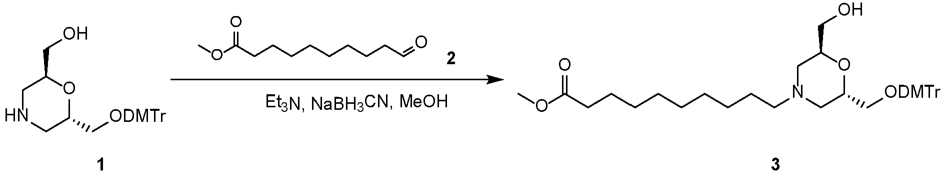

A compound 2 (630 mg, 3.15 mmol) and a compound 1 (1.42 g, 3.15 mmol) were dissolved in MeOH (20.0 mL) at 25°C, Et₃N (0.874 mL, 6.29 mmol) was added, and a reaction solution was stirred at 25°C for 12 h. NaBH₃CN (593 mg, 9.44 mmol) was added, and the reaction solution was stirred at 25°C for 24 h. LCMS showed an MS value of a compound 3. Thin layer chromatography (DCM/MeOH=10/1) showed that reactants were completely consumed and a new point was generated. The reaction solution was diluted with 100 mL of dichloromethane, washed with a saturated NaHCOs solution (10.0 mL×3) and then washed with a saturated NaCl solution. An organic phase was dried with anhydrous Na₂SO₄ and spin-dried to obtain a crude product. The crude product was purified by MPLC (DCM/MeOH=1/0 to 10/1) to obtain a yellow oily liquid compound 3 (970 mg, yield: 48.65%). ¹H NMR (400 MHz, CD₃OD) *δ* 7.38 - 7.47 (m, 2 H), 7.16 - 7.34 (m, 7 H), 6.85 (dd, *J =* 8.8, 1.6 Hz, 4 H), 3.93 - 4.01 (m, 1 H), 3.77 (s, 6 H), 3.64 - 3.71 (m, 2 H), 3.64 (s, 3 H), 3.60 - 3.60 (m, 1 H), 3.52 - 3.59 (m, 1 H), 3.38 (dd, *J=* 9.2, 6.0 Hz, 1 H), 3.16 - 3.23 (m, 1 H), 2.35 - 2.53 (m, 3 H), 2.14 - 2.32 (m, 5 H), 1.53 - 1.64 (m, 2 H), 1.40 - 1.49 (m, 2 H), 1.26 - 1.36 (m, 10 H).

### 2. Preparation of a compound 4

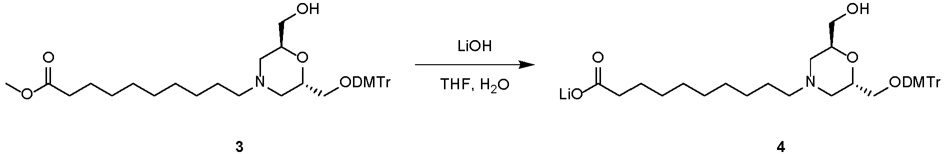

The compound 3 (570 mg, 0.899 mmol) was dissolved in a mixed solvent of THF (4.00 mL) and H₂O (2.00 mL) at 25°C, LiOH (25.9 mg, 1.08 mmol) was added, and a reaction solution was stirred to carry out a reaction at 40°C for 18 h. Thin layer chromatography (DCM/MeOH=10/1) showed that reactants were completely consumed and a new point was generated. The reaction solution was spin-dried, dissolved in 1.00 mL of acetonitrile and 4.00 mL of water and then freeze-dried to obtain a white solid product, which was confirmed as a compound 4 (510 mg, yield: 90.63%) by LCMS.

### 3. Preparation of a compound 6

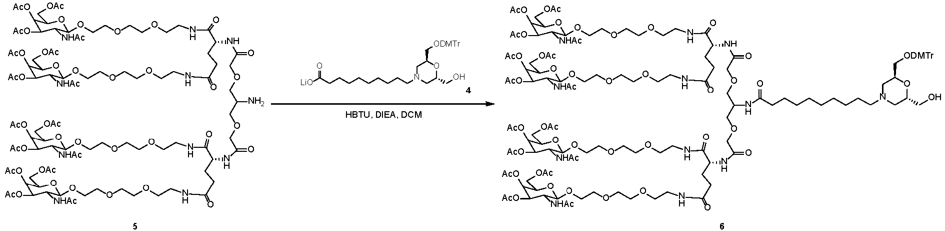

The compound 4 (54.4 mg, 0.087 mmol) was dissolved in DCM (10.0 mL) at 25°C, HBTU (41.2 mg, 0.109 mmol) and DIEA (0.086 mL, 0.522 mmol) were sequentially added, a reaction solution was stirred at 25°C for 0.5 h, then a compound 5 (prepared by a method of the compound 4 in Example 7, 200 mg, 0.087 mmol) was added, and a reaction solution was stirred at 25°C for 17.5 h. The reaction solution was diluted with DCM (20.0 mL) and washed with a saturated NaHCO₃ solution (5.00 mL×3). An organic phase was washed with a saturated salt solution (5.00 mL×3), and then the organic phase was dried with anhydrous Na₂SO₄ and spin-dried to obtain a crude product of a compound 6 (270 mg). The crude product was directly used in a next step without purification.

### 4. Preparation of a compound E19

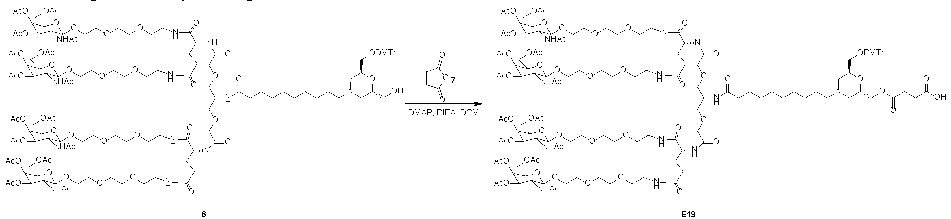

The compound 6 (270 mg, 0.093 mmol) was dissolved in DCM (10.0 mL) at 25°C, DIEA (0.092 mL, 0.557 mmol), DMAP (4.54 mg, 0.037 mmol) and a compound 7 (55.7 mg, 0.557 mmol) were sequentially added, and a reaction solution was stirred at 25°C for 18 h. LCMS showed an MS value of a product. The reaction solution was spin-dried to obtain a crude product, and the crude product was separated by a reversed-phase column (chromatographic column: Waters Xbridge BEH C18 100*25mm*5um; mobile phase: TEAA-ACN; B%: 20%-60%, 18 min; flow rate: 15 ml/min) to obtain a white solid compound E19 (64.0 mg, yield: 22.92%, purity: 95.34%).

¹H NMR (400 MHz,CD₃OD) *δ* 7.41 - 7.47 (m, 2 H), 7.17 - 7.35 (m, 7 H), 6.83 - 6.90 (m, 4 H), 5.32 - 5.36 (m, 4 H), 5.05 - 5.11 (m, 4 H), 4.64 - 4.68 (m, 4 H), 4.28 - 4.45 (m, 4 H), 4.00 - 4.21 (m, 21 H), 3.86 - 3.99 (m, 6 H), 3.79 (s, 6 H), 3.67 - 3.77 (m, 8 H), 3.59 - 3.67 (m, 24 H), 3.55 (q, *J =* 5.2 Hz, 8 H), 3.34 - 3.50 (m, 10 H), 3.08 (q*, J =* 7.2 Hz, 17 H), 2.56 - 2.63 (m, 2 H), 2.42 - 2.51 (m, 4 H), 2.21 - 2.36 (m, 10 H), 2.12 - 2.16 (m, 12 H), 2.06 - 2.12 (m, 2 H), 2.00 - 2.05 (m, 13 H), 1.98 (s, 1 H), 1.58 - 1.66 (m, 2 H), 1.41 - 1.49 (m, 2H), 1.21 - 1.35 (m, 35 H).

### Example 20: Preparation of a compound E20

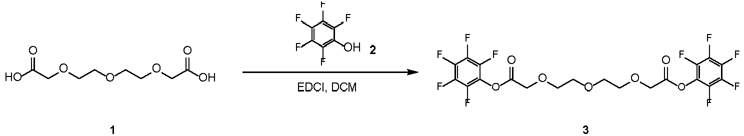

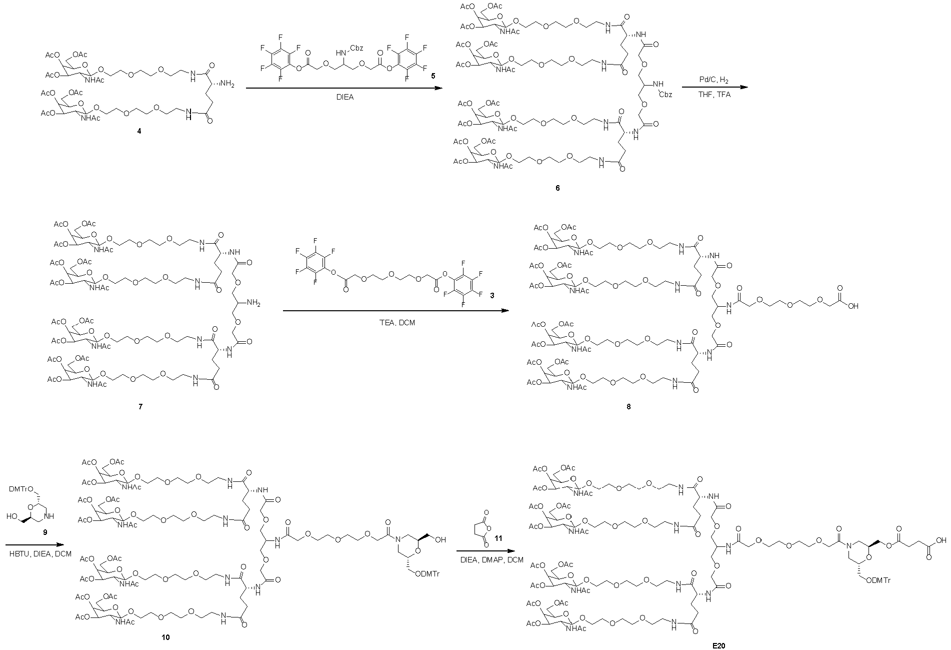

### 1. Preparation of a compound 3

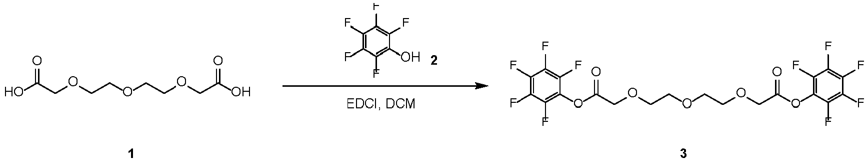

A compound 1 (1.60 g, 7.20 mmol) was dissolved in DCM (60 mL) at 25°C, then a compound 2 (3.31 g, 18.0 mmol) and EDCI (4.14 g, 21.6 mmol) were sequentially added, and a reaction solution was stirred at 25°C for 18 h. LCMS showed an MS value of a product, and thin layer chromatography (PE/EA=5/1) showed that a new point was generated. The reaction solution was concentrated under vacuum to obtain a crude product, and the crude product was purified by MPLC (PE/EA=1/0 to 5/1) to obtain a yellow oily compound 3 (1.70 g, yield: 42.59%). ¹H NMR (400 MHz, CDCl₃) *δ* 4.54 - 4.57 (m, 4 H), 3.83 - 3.87 (m, 4 H), 3.74 - 3.79 (m, 4 H).

### 2. Preparation of a compound 6

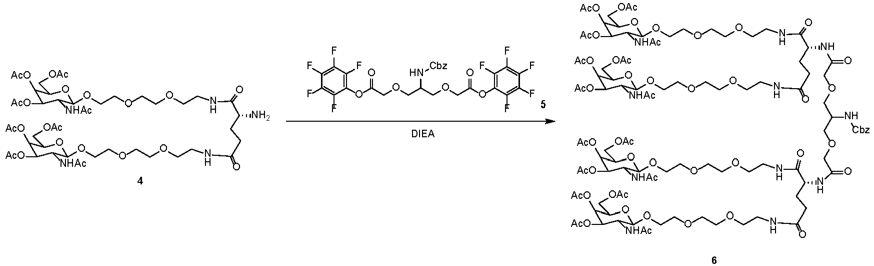

A compound 4 (prepared by a method of an intermediate 3-3 in Example 24, 2.30 g, 2.15 mmol) was dissolved in DCM (20.0 mL) at 25°C, then a compound 5 (0.690 g, 1.03 mmol) and DIEA (0.508 mL, 3.08 mmol) were sequentially added, and a reaction solution was stirred at 25°C for 10 h. LCMS showed that a target compound was generated in a reaction, and thin layer chromatography (DCM/MeOH=8/1) showed that reactants were completely consumed and a new point was generated. The reaction solution was directly spin-dried and filtered by a column with DCM/MeOH as an eluting agent, a product was eluted when MeOH was 10% to 15%, and a white solid compound 6 (1.93 g, yield: 77.09%) was obtained. ¹H NMR (400 MHz, CD₃OD) *δ* 7.29 - 7.40 (m, 5 H), 5.31 - 5.36 (m, 4 H), 5.05 - 5.13 (m, 6 H), 4.62 - 4.68 (m, 4 H), 4.36 - 4.43 (m, 2 H), 4.00 - 4.20 (m, 22 H), 3.89 - 3.96 (m, 4 H), 3.67 - 3.76 (m, 9 H), 3.49 - 3.66 (m, 34 H), 3.34 - 3.42 (m, 8 H), 2.27 - 2.35 (m, 4 H), 2.12 - 2.16 (m, 12 H), 2.01 - 2.04 (m, 12 H), 1.90 - 1.96 (m, 24 H).

### 3. Preparation of a compound 7

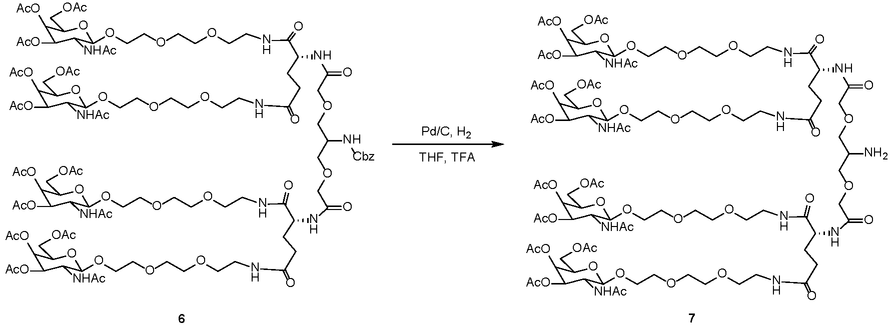

The compound 6 (930 mg, 0.381 mmol) was dissolved in THF (10.0 mL) at 25°C, TFA (47.8 mg, 0.419 mmol) and Pd/C 10% (100 mg, 0.940 mmol) were sequentially added, and a reaction solution was stirred to carry out a reaction in a hydrogen atmosphere (14.696 psi) at 25°C for 18 h. Thin layer chromatography (DCM/MeOH=8/1) showed that reactants were completely consumed and a new point was generated. The reaction solution was filtered, and a filtrate was spin-dried to obtain a white solid compound 7 (830 mg, yield: 94.44%). ¹H NMR (400 MHz, CD₃OD) *δ* 5.33 - 5.36 (m, 4 H), 5.09 (d, *J =* 3.6 Hz, 2 H), 5.06 (d, *J =* 2.4 Hz, 2 H), 4.60 - 4.67 (m, 4 H), 4.36 - 4.43 (m, 2 H), 4.00 - 4.19 (m, 22 H), 3.92 - 3.98 (m, 4 H), 3.69 - 3.77 (m, 9 H), 3.55 - 3.67 (m, 34 H), 3.35 - 3.45 (m, 8 H), 2.34 - 2.43 (m, 4 H), 2.15 (s, 12 H), 2.03 (s, 12 H), 1.93 - 1.97 (m, 24 H).

### 4. Preparation of a compound 8

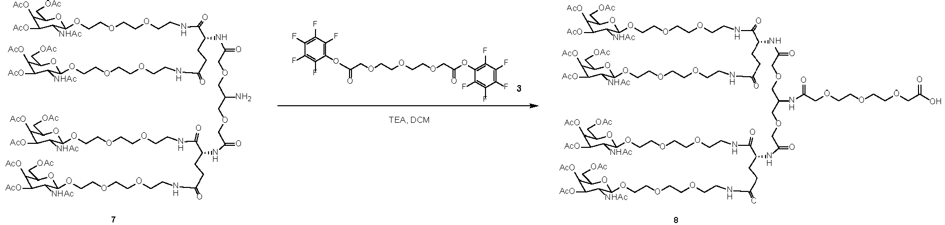

The compound 3 was dissolved in DCM (10.0 mL) at 25°C, then the compound 7 (400 mg, 0.173 mmol) and TEA (0.144, 1.04 mmol) were sequentially added, and a reaction solution was stirred at 25°C for 18 h. LCMS showed an MS value of a product. TLC (DCM/MeOH=10/1) showed that raw materials were completely reacted and a new point was generated. The reaction solution was concentrated under vacuum to obtain a crude product, and the crude product was purified by MPLC (DCM/MeOH=1/0 to 5/1) to obtain a white solid compound 8 (340 mg, yield: 78.09%). ¹H NMR (400 MHz,CD₃OD) *δ* 5.32 - 5.36 (m, 4 H), 5.06 - 5.12 (m, 4 H), 4.64 - 4.69 (m, 4 H), 4.38 - 4.47 (m, 3 H), 4.03 - 4.18 (m, 22 H), 3.90 - 3.96 (m, 6 H), 3.68 - 3.75 (m, 16 H), 3.61 - 3.66 (m, 24 H), 3.53 - 3.58 (m, 8 H), 3.35 - 3.43 (m, 8 H), 2.27 - 2.34 (m, 4 H), 2.13 - 2.17 (m, 12 H), 2.08 - 2.12 (m, 2 H), 2.02 - 2.04 (m, 12 H), 1.97 - 2.01 (m, 2 H), 1.93 - 1.96 (m, 23 H).

### 5. Preparation of a compound 10

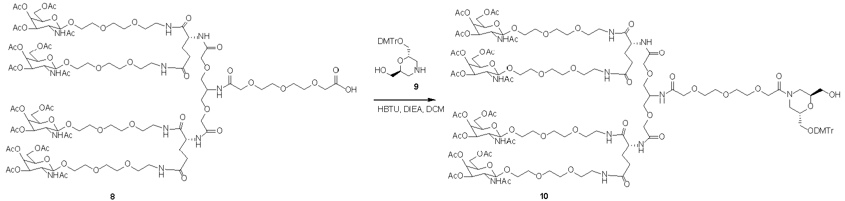

The compound 8 (310 mg, 0.123 mmol) was dissolved in DCM (10 mL) at 25°C, HBTU (140 mg, 0.370 mmol) and DIEA (0.122 mL, 0.741 mmol) were sequentially added, a reaction solution was stirred at 25°C for 0.5 h, then a compound 9 (166 mg, 0.370 mmol) was added, and the reaction solution was stirred at 25°C for 17.5 h. LCMS showed that an MS value of a product was obtained and raw materials were completely consumed. The reaction solution was spin-dried to obtain a crude product. The crude product was separated by a reversed-phase column preparative chromatography (chromatographic column: 01-Waters Xbridge BEH C18 19*150mm; mobile phase: TEAA-ACN; B%: 20%-58%, 15 min; flow rate: 15 ml/min) to obtain a white solid compound 10 (170 mg, yield: 46.80%). ¹H NMR (400 MHz,CD₃OD) *δ* 7.41 - 7.45 (m, 2 H), 7.25 - 7.34 (m, 7 H), 6.87 (t, *J =* 8.0 Hz, 4 H), 5.34 (d, *J =* 3.2 Hz, 4 H), 5.08 (ddd, *J =* 11.2, 3.2, 1.6 Hz, 4 H), 4.65 (dd, *J =* 8.4, 3.2 Hz, 4 H), 4.58 (br s, 4 H), 4.39 - 4.44 (m, 3 H), 4.00 - 4.19 (m, 24 H), 3.93 (dt, *J =* 11.2, 4.4 Hz, 4 H), 3.79 (s, 6 H), 3.69 - 3.76 (m, 11 H), 3.58 - 3.67 (m, 34 H), 3.51 - 3.57 (m, 9 H), 3.34 - 3.41 (m, 8 H), 3.15 (q, *J =* 7.2 Hz, 11 H), 2.27 - 2.34 (m, 4 H), 2.14 (s, 12 H), 2.06 - 2.12 (m, 2 H), 2.02 (s, 12 H), 1.96 - 2.00 (m, 2 H), 1.94 (d, *J =* 6.8 Hz, 24 H), 1.29 (t, *J =* 7.2 Hz, 15 H).

### 6. Preparation of a compound E20

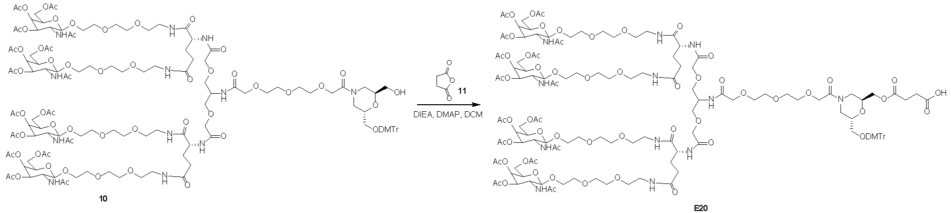

The compound 10 (170 mg, 0.058 mmol) was dissolved in DCM (6.00 mL) at 25°C, DIEA (0.057 mL, 0.347 mmol), DMAP (2.82 mg, 0.023 mmol) and a compound 11 (34.7 mg, 0.347 mmol) were sequentially added, and a reaction solution was stirred at 25°C for 18 h. LCMS showed an MS value of a product. The reaction solution was spin-dried to obtain a crude product, and the crude product was separated by a reversed-phase column (chromatographic column: C18; mobile phase: H₂O (0.1%TEA)-ACN; B%: 30%-70%, 20 min; flow rate: 20 ml/min) to obtain a white solid compound E20 (96 mg, yield: 54.61%, purity: 90.93%). ¹H NMR (400 MHz,CD₃OD) *δ* 7.44 (br d, *J* = 8.0 Hz, 2 H), 7.19 - 7.34 (m, 7 H), 6.88 (br dd, *J* = 8.0, 6.4 Hz, 4 H), 5.34 (d, *J =* 3.2 Hz, 4 H), 5.05 - 5.11 (m, 4 H), 4.65 (dd, *J =* 8.4, 2.8 Hz, 4 H), 4.38 - 4.45 (m, 3 H), 4.20 - 4.31 (m, 3 H), 4.01 - 4.19 (m, 24 H), 3.90 - 3.95 (m, 4 H), 3.79 (s, 6 H), 3.45 - 3.77 (m, 53 H), 3.34 - 3.42 (m, 9 H), 3.18 (q*, J =* 7.2 Hz, 5 H), 2.57 - 2.63 (m, 2 H), 2.48 - 2.54 (m, 2 H), 2.27 - 2.34 (m, 4 H), 2.08 - 2.20 (m, 13 H), 2.02 (s, 13 H), 1.94 (d, *J =* 5.6 Hz, 23 H), 1.30 (t, *J =* 7.2 Hz, 8 H).

### Example 21: Preparation of a compound E21

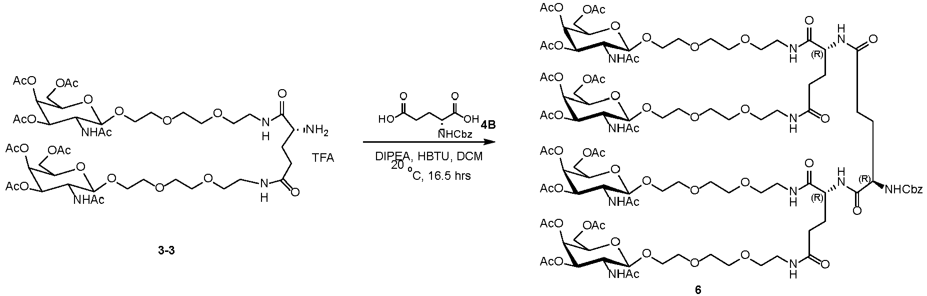

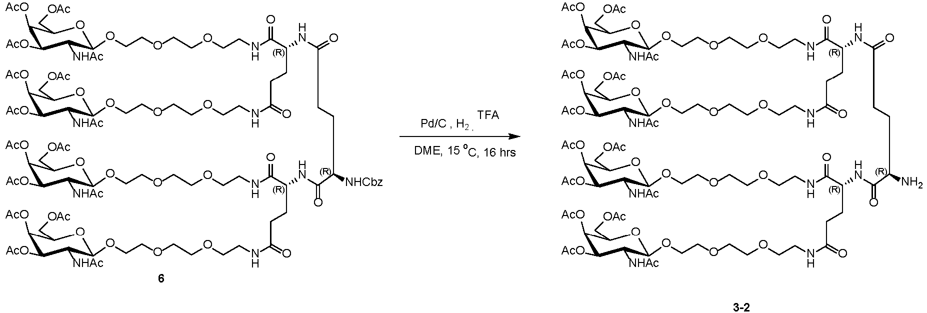

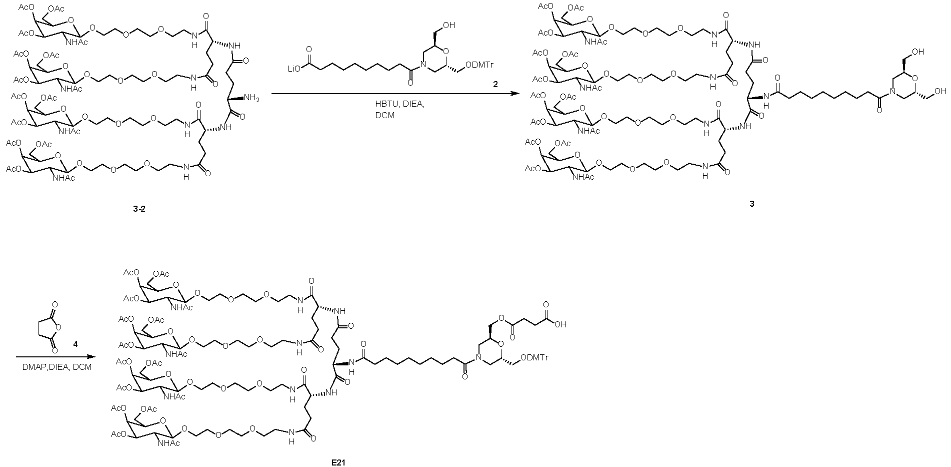

### 1. Preparation of a compound 6

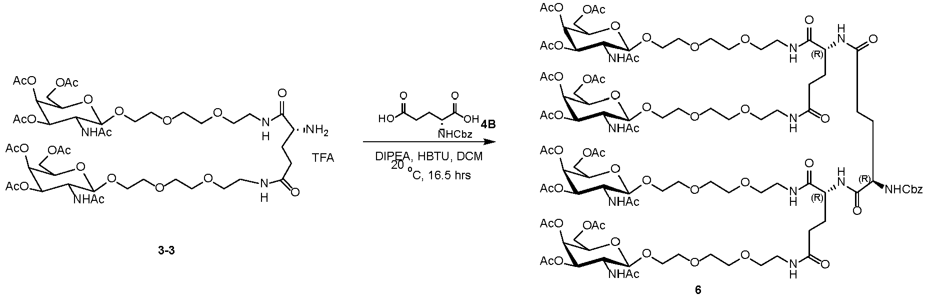

DIPEA (16.0 g, 124 mmol, 21.6 mL, 6.60 eq) was added at one time to a DCM solution of a compound 4B (5.30 g, 18.8 mmol, 1.00 eq) and a compound 3-3 (49.0 g, 41.4 mmol, 2.20 eq, TFA salt, prepared by a method of an intermediate 3-3 in Example 24) in a nitrogen atmosphere at 25°C. The solution was stirred at 25°C for 30 min. HBTU (17.8 g, 47.1 mmol, 2.50 eq) was added to the solution at 25°C. A mixture was stirred at 25°C for 16 h. LCMS detected a new compound. The mixture was concentrated under vacuum at 40°C. A residue was dissolved in DCM (500 mL), and 0.5 M HCl (200 mL×2) was added to the mixture at 20°C. The mixture was extracted with DCM (3×500 mL), cleaned with saturated NaHCOs (3×500 mL) and a salt solution (3×500 mL), dried with Na₂SO₄ and concentrated under vacuum. The residue was purified by column chromatography (SiO₂, DCM: MeOH=100/1 to 8/1) and prep-MPLC (column: 800 g Agela C18; mobile phase: [water-ACN]; 15-45%, 25 min; 45%, 10 min) to obtain a brown oily compound 6 (45.0 g, crude product).

### 2 Preparation of an intermediate 3-2

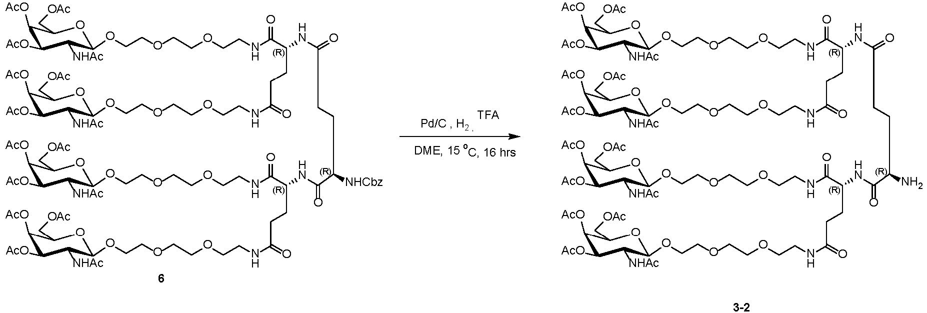

TFA (1.77 g, 15.54 mmol, 1.15 mL, 1.00 eq) and Pd/C (3.70 g, purity: 10%) were added to a DME solution (740 mL) of the compound 6 (37.0 g, 15.5 mmol, 1.00 eq). A suspension was subjected to degasification, and hydrogen replacement was carried out for three times. A mixture was stirred at 15°C in H₂ (15 Psi) for 16 h. LCMS showed a complete reaction. Filtration was carried out with diatomite, and a filtrate was concentrated under reduced pressure to remove a solvent so as to obtain a white solid intermediate 3-2 (77.2 g, crude product, TFA salt).

¹H NMR: ET53345-86-P1A (400 MHz, DMSO)

*δ* 8.9 (m, 1H), 8.40-8.20 (m, 6H), 7.83-7.81 (m, 6H), 5.32 (d, *J =* 3.2 Hz, 1H), 5.21 (d, *J =* 2.8 Hz, 4H), 4.96 (dd, *J =* 3.2 Hz, 2H), 4.55 (d, *J* =8.4 Hz, 4H), 4.35 (m, 1H), 4.20 (m, 1H), 4.02 (s, 12H), 3.86-3.77 (m, 10H), 3.61-3.49 (m, 42H), 3.42-3.19 (m, 10H), 2.15- 2.10 (m, 18H), 1.99 (s, 12H), 1.89 (s, 14H), 1.77-1.74 (m, 21H).

### 3. Preparation of a compound 3

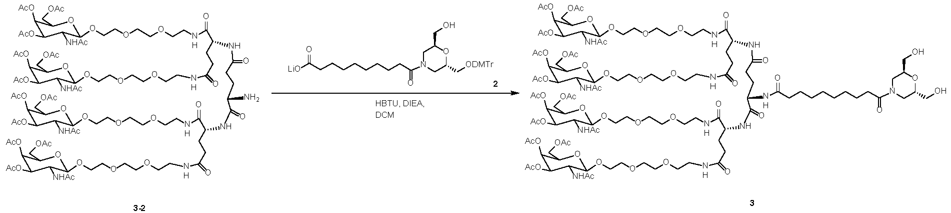

The intermediate 3-2 (500 mg, 0.222 mmol) was dissolved in DCM (10.0 mL), HBTU (127 mg, 0.334 mmol), DIEA (0.110 mL, 0.667 mmol) and a compound 2 (142 mg, 0.222 mmol) were sequentially added, and a reaction solution was stirred at 25°C for 18 h. LCMS detected an MS response of a product. Thin layer chromatography (DCM/MeOH=10/1) showed that raw materials were completely reacted and a new point was generated. The reaction solution was washed with a saturated sodium bicarbonate aqueous solution (20.0 mL) and extracted with dichloromethane(10.0 mL×2). Organic phases were combined, concentrated and filtered with a normal-phase column (DCM/MeOH=1:0 to 10:1) to obtain a white solid 3 (545 mg, yield: 85.56%).

### 4. Preparation of a compound E21

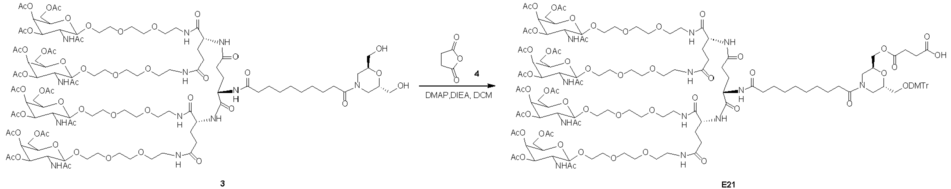

The compound 3 (545 mg, 0.190 mmol) was dissolved in DCM (20.0 mL), a compound 4 (114 mg, 1.14 mmol), DIEA (0.189 mL, 1.14 mmol) and DMAP (23.3 mg, 0.190 mmol) were added, and a reaction solution was stirred at 25°C for 18 h. LCMS detected an MS response of a product. The reaction solution was directly spin-dried, dissolved in of acetonitrile (5.00 mL) and treated by prep-HPLC (chromatographic column: Waters Xbridge BEH C18 100*30mm*10um; mobile phase: water-acetonitrile; B%: 17%-57%, 12 min; flow rate: 50 ml/min) to obtain a white solid compound E21 (50.0 mg, yield: 7.47%, purity: 84.29%). LCMS (ESI): m/z=2962.3 (M+H)⁺; ¹H NMR (400 MHz, CD₃OD) *δ* 7.43(d, *J =* 8.0 Hz, 2H), 7.27-7.32 (m, 6H), 7.18-7.25 (m, 1H), 6.85-6.88 (m, 4H), 5.32-5.34 (m, 4H), 5.06-5.09 (m, 4H), 4.64-4.67 (m, 4H), 4.32-4.39 (m, 2H), 4.02-4.21 (m, 20H), 3.83-3.99 (m, 6H), 3.79 (d*, J =* 2.0 Hz, 6H), 3.69-3.74 (m, 5H), 3.58-3.67 (m, 28H), 3.51-3.57 (m, 6H), 3.35-3.41 (m, 8H), 3.14-3.23 (m, 2H), 2.51-2.60 (m, 4H), 2.30-2.40 (m, 8H), 2.20-2.23 (m, 2H), 2.14 (s, 12H), 2.06-2.09 (m, 2H), 2.02-2.03 (m, 12H), 1.94 (d, *J =* 5.2 Hz, 24H), 1.54-1.59 (m, 4H), 1.29-1.38 (m, 12H).

### Example 22: Preparation of an intermediate 2-3

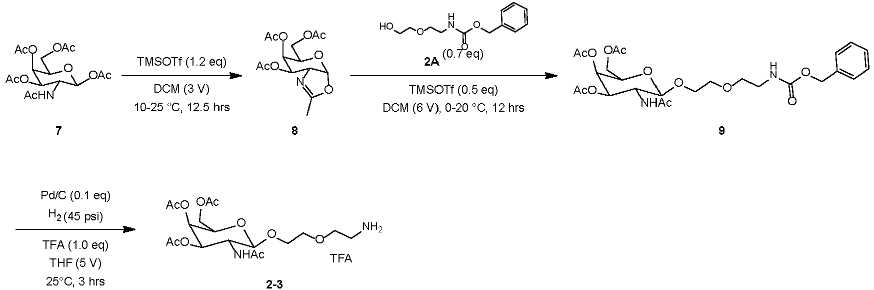

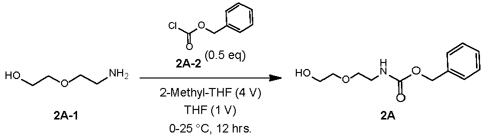

### 1. Preparation of a compound 8

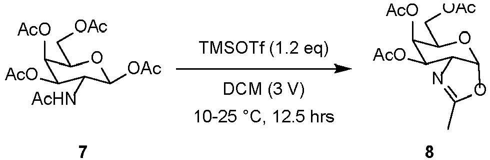

TMSOTf (68.5 g, 308 mmol, 55.7 mL) was added dropwise to a DCM solution (300 mL) of a compound 7 (100 g, 256 mmol) at 10-15°C for 0.5 h. Then, the compounds were stirred at 25°C for 12 h. TLC (ethyl acetate) showed that the compound 7 (R_{f} =0.3) was completely consumed and a compound 8 (R_{f}=0.4) was formed. The compounds were cooled to 0°C and slowly poured to a NaHCOs aqueous solution (100 g dissolved in 1.00 L of water) at 0-5°C. DCM (200 mL×3) was used to separate an organic phase and extract an aqueous phase. Organic layers were combined, washed with a salt solution (50.0 mL), dried with Na₂SO₄ and concentrated under vacuum to obtain a yellow oily compound 8 (about 83.0 g). ¹H NMR: (400 MHz, DMSO-*d₆*). *δ* 6.03 (d, *J* = 7.2 Hz, 2H), 5.24-5.22 (m, 1H), 4.89-4.86 (m, 1H), 4.26-4.24 (m, 1H), 4.13-4.05 (m, 2H), 4.04-3.94 (m, 1H), 2.06 (s, 3H), 1.99 (d, *J =* 3.2 Hz, 6H), 1.98 (s, 3H).

### 2. Preparation of a compound 2A

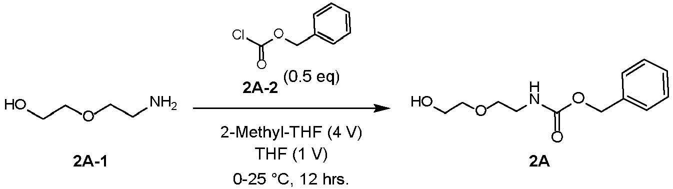

Two reactions were carried out in parallel.

A compound 2A-2 (365 g, 2.14 mol, 304 mL) in THF (450 mL) was added dropwise to a 2-methyl-THF solution (1.80 L) of a compound 2A-1 (450 g, 4.28 mol, 428 mL) at 0°C. After the addition, the compounds were stirred in a nitrogen atmosphere at 25°C for 12 h. TLC (ethyl acetate, compound 2A-2 R_{f}=0.9, compound 2A R_{f}=0.3) showed that the compound 2A-2 was completely consumed and a compound 2A was formed. The two reactions were combined. HCl/EtOAc (1 M, 900 mL) was added to a reaction mixture and stirred for 30 min. Filtration was carried out with celite to remove a white solid, and a filtrate was concentrated under vacuum to obtain a colorless oily compound 2A (about 990 g). ¹H NMR: (400 MHz, CDCl₃). *δ* 7.26-7.21 (m, 5H), 5.56 (s, 1H), 5.00 (s, 2H), 3.62-3.59 (m, 2H), 3.45-3.23 (m, 4H), 3.29 (s, 2H).

### 3. Preparation of a compound 9

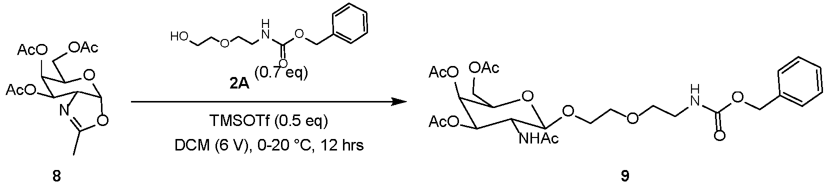

TMSOTf (28.0 g, 126 mmol, 22.8 mL) was added dropwise to a mixture of the compound 8 (83.0 g, 252 mmol) and the compound 2A (42.2 g, 176 mmol) in DCM (500 mL) at 0-10°C, and the compounds were stirred at 20°C for 12 h. TLC (petroleum ether/ethyl acetate =0/1) showed that the compound 8 was completely consumed and a compound 9 (R_{f}=0.3) was formed. LCMS (compound 9: RT=0.744 min) showed that the compound 8 was completely consumed. A reaction mixture was poured to a saturated NaHCO₃ aqueous solution (200 mL) at 0-5°C, washed with a salt solution (200 mL), dried with Na₂SO₄, filtered and concentrated under vacuum. A residue was purified by column chromatography (SiO₂, petroleum ether/ethyl acetate=1/0 to 0/1). A crude product was ground with petroleum ether/ethyl acetate=1/1 (100 mL) at 25°C for 30 min to obtain a white solid compound 9 (about 59.0 g). ¹H NMR: (400 MHz, DMSO-*d₆*). *δ* 7.82 (d, *J =* 9.2 Hz, 1H), 7.40-7.28 (m, 5H), 5.23 (d, *J* = 3.2 Hz, 1H), 5.26-4.98 (m, 3H), 4.56 (d, *J =* 8.4 Hz, 1H), 4.05 (s, 3H), 3.93-3.86 (m, 1H), 3.81-3.76 (m, 1H), 3.63-3. 48 (m, 3H), 3.43-3.40 (m, 2H), 3.35-3.15 (m, 2H), 2.11 (s, 3H), 2.01 (s, 3H), 1.91 (s, 3H), 1.79 (s, 3H).

### 4. Preparation of an intermediate 2-3 (TFA salt)

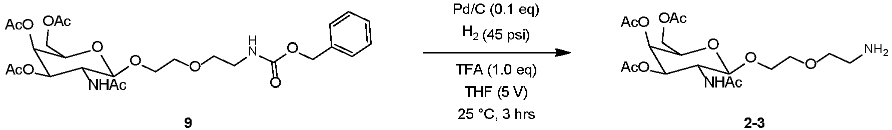

The compound 9 (95.0 g, 167 mmol) and TFA (19.0 g, 167 mmol, 12.4 mL) were added to Pd/C (9.50 g, content: 10%) of THF (475 mL) in an argon atmosphere. A reaction mixture was subjected to argon replacement for three times, followed by hydrogen replacement for three times. Then, the mixture was stirred at 25°C in an H₂ atmosphere (45 psi) for 3 h. TLC (dichloromethane/methanol=10/1) showed that the compound 9 (R_{f}=0.5) was completely consumed and an intermediate 2-3 (TFA salt) (R_{f}=0.2) was formed. The reaction mixture was concentrated under reduced pressure to obtain a white-like solid intermediate 2-3 (TFA salt) (55.53g, yield: 57.9%, purity: 95.7%). ¹H NMR: (400 MHz, CDCl₃). *δ* 8.00-7.97 (m, 5H), 5.29 (d, *J =* 3.2 Hz, 1H), 5.07 (d, *J =* 3.6 Hz, 1H), 4.63 (d, *J =* 8.4 Hz, 1H), 4.10 (s, 3H), 3.97-3.95 (m, 1H), 3.89-3.88 (m, 1H), 3.70-3.63 (m, 5H), 3.04 (s, 2H), 2.17 (s, 3H), 2.07 (s, 3H), 1.96 (s, 3H), 1.85 (s, 3H).

### Example 23: Preparation of an intermediate 3-4

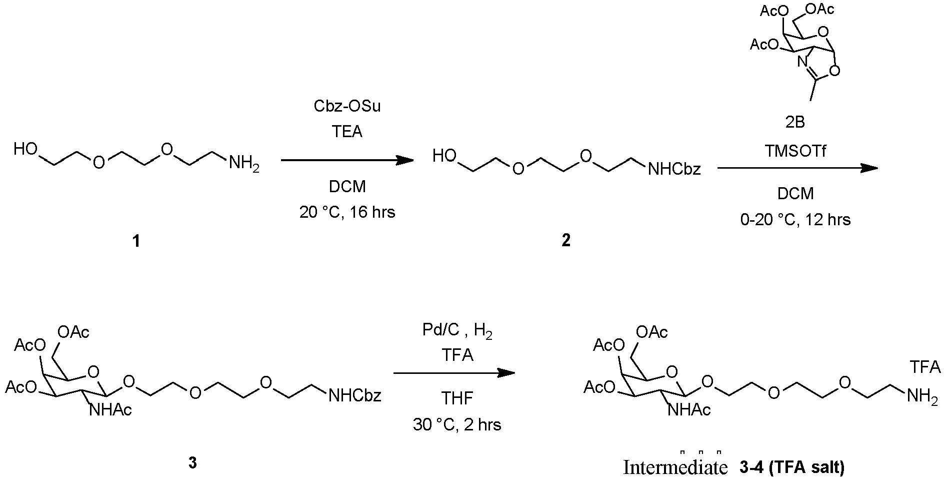

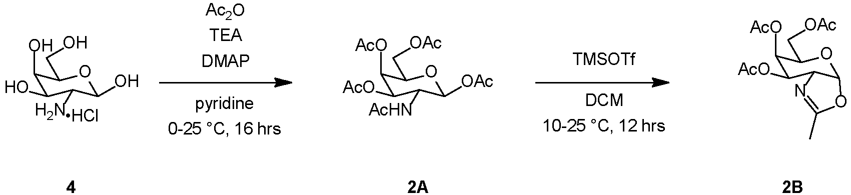

### 1. Preparation of a compound 2

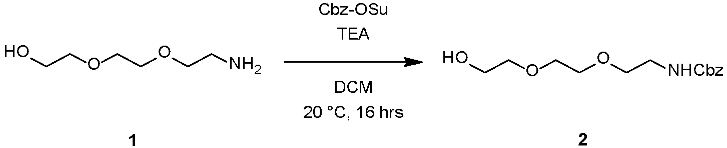

Benzyl(2,5-dioxopyrrolidine-1-yl)carbonate was slowly added to DCM (1.80 L) of a compound 1 (300 g, 2.01 mol) at 15°C, and TEA (203 g, 2.01 mol, 280 mL) was added dropwise. After the addition, a mixture was stirred at 25°C for 16 h. TLC (dichloromethane:methanol=10:1) showed that the reactant 1 (R_{f}=0.32) was retained and an important new point (R_{f}=0.52) was detected. The reaction mixture was washed with a saturated sodium bicarbonate solution (1.00 L×2), and an organic phase was washed with a salt solution (1.00 L), dried with anhydrous Na₂SO₄ and concentrated under vacuum. A yellow oily compound 2 (about 385 g) was obtained without purification.

### 2. Preparation of a compound 2A

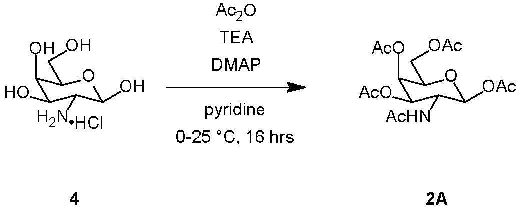

DMAP (19.8 g, 162 mmol) was added at one time to a pyridine solution (1.75 L) of a compound 4 (350 g, 1.62 mol, HCl) and AczO (994 g, 9.74 mol, 912 mL) at 0-15°C, and TEA (164 g, 1.62 mol, 226 mL) was added dropwise. A mixture was stirred at 25°C for 16 h. LCMS (product: RT=0.687 min) showed that initial reactants were completely consumed. EtOAc (1.40 L) was added to the mixture at 25°C and stirred for 30 min, then the mixture was filtered, and a filter cake was cleaned with EtOAc (300 mL). The filter cake was ground with water (1.45 L) at 25°C for 30 min. A mixture was filtered, and a filter cake was cleaned with water (175 mL×3) and then collected to obtain a white solid compound 2A (about 580 g).

### 3. Preparation of a compound 2B

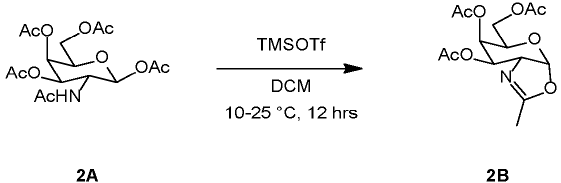

Three reactions were carried out in parallel.

TMSOTf (137 g, 616 mmol, 111 mL) was added dropwise to a DCM solution (800 mL) of the compound 2A (200 g, 514 mmol) at 10-15°C for 0.5 h. Then, a mixture was stirred at 25°C for 3 h. TLC (dichloromethane:methanol=20:1) showed that the compound 2A (R_{f}=0.54) was completely consumed and a new point (R_{f}=0.24) was formed. The three reactions were combined. The mixture was cooled to 0-15°C and slowly poured to a NaHCO₃ solution (300 g dissolved in 3.00 L of water) at 0-5°C. An organic phase was separated, and an aqueous phase was extracted with DCM (1.00 L×3). Organic layers were combined, dried with Na₂SO₄, filtered and concentrated under vacuum. A yellow oily compound 2B (about 507 g) was obtained and directly used in a next step without purification.

### 4. Preparation of a compound 3

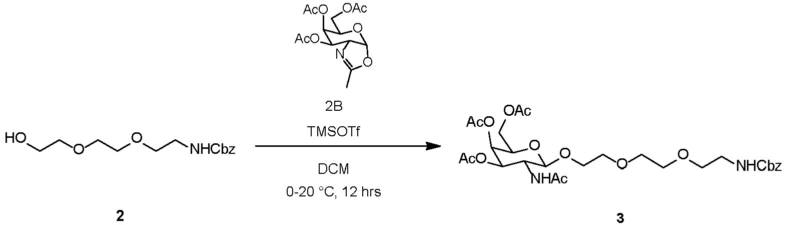

TMSOTf (84.4 g, 380 mmol, 69.0 mL) was added dropwise to a mixture of the compound 2B (250 g, 759 mmol) and the compound 2 (151 g, 531 mmol) in DCM (1.00 L) at 0-10°C, and the mixture was stirred at 20°C for 12 h. TLC (dichloromethane:methanol=20:1) showed that the compound 2 (R_{f}=0.33) was completely consumed and an new point (R_{f}=0.03) was formed. Combined reactants were cooled to 0-5°C, and then the reactants were poured to NaHCO₃ (aqueous solution, 100 g dissolved in 1 L of water) and stirred at 5-10°C for 10 min to separate phases. An aqueous phase was extracted with DCM (500 mL×2), combined organic phases were dried with a₂SO₄ and filtered, and a filtrate was concentrated under vacuum. A yellow oily compound 3 (about 360 g) was obtained without purification. ¹H NMR: (400 MHz, DMSO). δ 7.79-7.37 (m, 1H), 7.35-7.26 (m, 5H), 5.21-5.20 (m, 1H), 5.00-4.95 (m, 3H), 4.55-4.53 (m, 1H), 4.03-3.86 (m, 3H), 3.61-3.59 (m, 1H), 3.59-3.57 (m, 1H), 3.48-3.40 (m, 6H), 3.39-3.31 (m, 2H), 3.14-3.13 (m, 2H), 2.09 (s, 3H), 1.99 (s, 3H), 1.88 (s, 3H), 1.76-1.74 (m, 3H).

### 5. Preparation of an intermediate 3-4 (TFA salt)

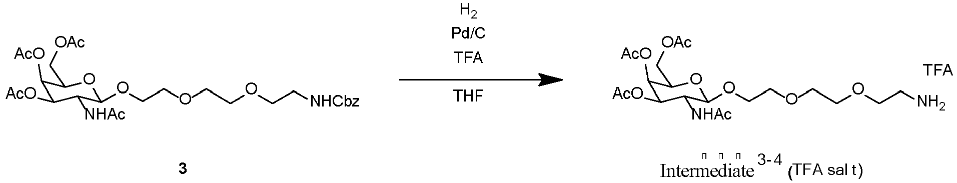

Three reactions were carried out in parallel.

The compound 3 (180 g, 293 mmol) and TFA (33.5 g, 293 mmol, 21.8 mL) were added to a mixture of Pd/C (18.0 g, 16.3 mmol, content: 10%) in THF (1.80 L) in an argon atmosphere. A suspension was subjected to degasification and hydrogen ventilation for three times. The mixture was stirred in H₂ (50 Psi) at 30°C for 2 h. LCMS (product: RT=0.697 min) showed that the compound 3 was completely consumed and a product peak was detected. The three reactions were combined. The mixture was filtered with celite, and a filtrate was concentrated under reduced pressure to remove a solvent. A yellow solid intermediate 3-4 (TFA salt) (393 g, 660 mmol, yield: 74.8%, purity: 99.6%, TFA) was obtained without purification.

### ¹H NMR: (400 MHz, DMSO-d6)

δ = 7.92 (d, *J* = 9.1 Hz, 4H), 5.27-5.17 (m, 1H), 5.03-4.91 (m, 1H), 4.60-4.50 (m, 1H), 4.09-3.97 (m, 4H), 3.85 (s, 2H), 3.65-3.46 (m, 10H), 3.04-2.92 (m, 2H), 2.10 (s, 3H), 2.00 (s, 3H), 1.94-1.86 (m, 3H), 1.82-1.71 (m, 4H).

### Example 24: Preparation of an intermediate 3-3 and an intermediate 5

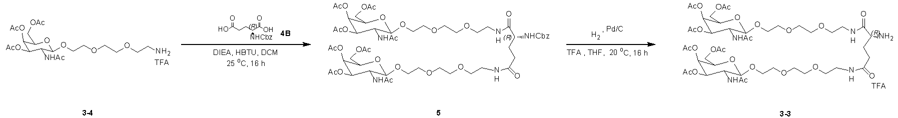

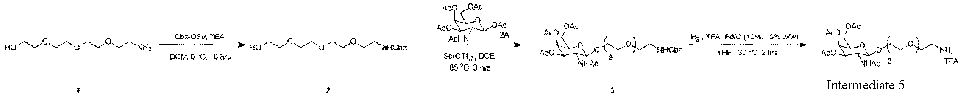

### 1. Preparation of a compound 5

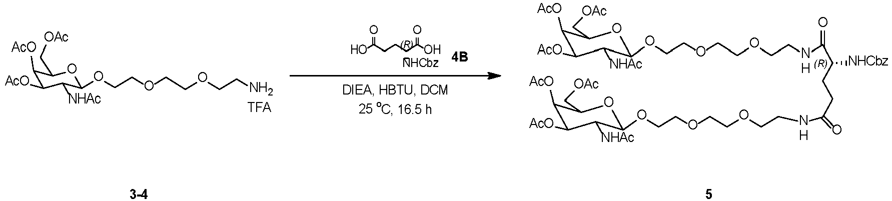

DIEA (30.3 g, 234 mmol, 40.8 mL, 6.60 eq) was added at one time to a DCM solution (1.00 L) of a compound 4B (10.0 g, 35.5 mmol, 1.00 *eq*) and a compound 3-4 (46.3 g, 78.2 mmol, 2.20 *eq,* TFA) at 25°C. Stirring was carried out at 25°C for half an hour. HBTU (30.3 g, 234 mmol, 40.8 mL, 6.60 *eq*) was added to a mixture. Stirring was carried out at 25°C for 16 h. LCMS (product: RT=0.681 mins) showed that a reaction was completed. The mixture was concentrated under vacuum. 0.50 N HCl (200 mL×2) was added to the mixture at 20°C, and the mixture was extracted with DCM (3×500 mL). Organic phases were combined, cleaned with saturated NaHCOs (3×800 mL) until a pH value was 8, cleaned with a salt solution (3×500 mL), dried with Na₂SO₄, concentrated under vacuum and purified. A residue was purified by column chromatography (SiO₂, DCM: MeOH=50:1 to 15:1). The residue was concentrated under vacuum at 40°C and purified by preparative-MPLC (column: 800 g Agela C18; mobile phase: [water-ACN]; 15-45%, 25 min; 45%, 10 min). Vacuum drying was carried out to obtain a yellow solid compound 5 (about 180 g+75.0 g+87.0 g+40.0 g+38.0 g).

417.0 g of the compound 3-4 was converted into the compound 5 in 9 batches.

### 2. Preparation of an intermediate 3-3

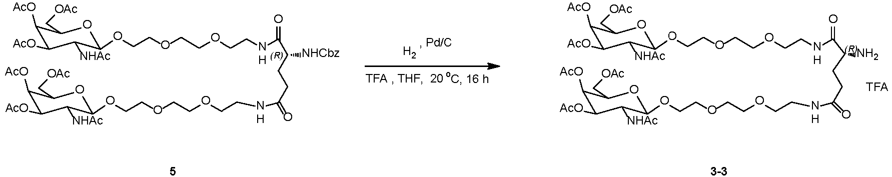

The compound 5 (73.0 g, 61.7 mmol, 1.00 *eq*) and TFA (7.04 g, 61.7 mmol, 4.57 mL, 1.00 *eq*) were added to THF (300 mL) of Pd/C (3.00 g, content: 10%) in an argon atmosphere. A suspension was subjected to degasification and hydrogen ventilation for three times. Stirring was carried out in H₂ (20 Psi) at 20°C for 16 h. TLC (dichloromethane:methanol=8: 1, R_{f}=0.0) showed that a reaction was completed. A mixture was filtered with celite, and a filtrate was concentrated under increased pressure to remove a solvent so as to obtain a white solid intermediate 3-3 (about 33.4 g+129 g+75.0 g).

¹H NMR: (400 MHz, DMSO)

*δ* 8.53 (t, *J =* 5.2 Hz, 1H), 8.18 (d, *J =* 2.4 Hz, 3H), 8.03 (t, *J =* 5.2 Hz, 1H), 7.84 (dd, *J =* 3.6 Hz, 2H), 5.22 (d, *J =* 3.2 Hz, 2H), 4.96 (dd, *J =* 3.2 Hz, 2H), 4.55 (d, *J* =8.4 Hz, 2H), 4.02 (t, *J* =8.8 Hz, 6H), 3.77-3.59 (m, 5H), 3.58-3.45 (m, 21H), 3.40-3.20 (m, 4H), 2.18 (t*, J* = 7.6 Hz, 2H), 2.17 (d, *J* =8.0 Hz, 6H), 2.10 (s, 6H), 1.99 (s, 6H), 1.90-1.80 (m, 8H), 1.77 (s, 6H).

### 3. Preparation of a compound 2

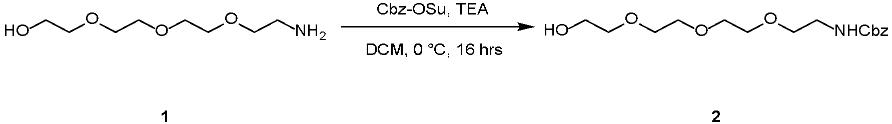

TEA (47.1 g, 465 mmol, 64.8 mL, 1.00 *eq)* and Cbz-OSu (110 g, 442 mmol, 0.95 *eq)* were added to a DCM solution (540 mL) of a compound 1 (90.0 g, 465 mmol, 1.00 *eq*) at 0°C. A mixture was stirred at 20°C for 16 h. LCMS (product: RT=0.625 mins) showed that a reaction was completed. The reaction mixture was cleaned with a saturated sodium bicarbonate solution (450 mL×2), and an organic phase was cleaned with a salt solution (500 mL), dried with anhydrous Na₂SO₄ and concentrated to obtain a yellow oily compound 2 (about 136 g).

### 4. Preparation of a compound 3

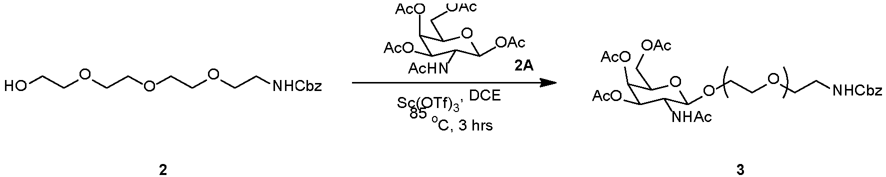

A compound 2A (84.9 g, 218 mmol, 1.40 *eq*) and Sc(OTf)₃ (12.0 g, 62.3 mmol, 0.40 *eq*) were added to a DCE solution (300 mL) of the compound 2 (51.0 g, 155 mmol, 1.00 *eq*) at 20°C. Stirring was carried out at 85°C for 3 h. LCMS (product: RT=0.783 mins) showed that a reaction was completed. A reaction mixture was cooled to 15°C, cooled by adding saturated NaHCO₃ (300 mL) at 15-20°C and extracted with DCM (300 mL). Combined organic phases were cleaned with 1 M HCl (50.0 mL) and a salt solution (300 mL), dried with Na₂SO₄, filtered and concentrated under reduced pressure at 45°C to obtain a brown oily compound 3 (84.0 g).

### 5. Preparation of an intermediate 5

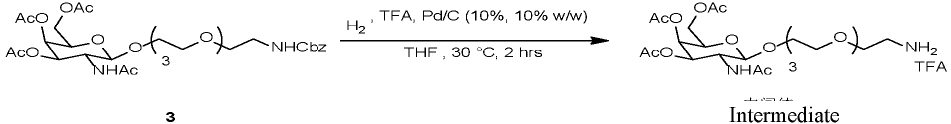

The compound 3 (70.0 g, 109 mmol, 1.00 *eq*) and TFA (12.5 g, 109 mmol, 8.14 mL, 1.00 *eq*) were added to THF (300 mL) of Pd/C (7.00 g, content: 10%) in an argon atmosphere. A suspension was subjected to degasification and hydrogen ventilation for three times. A mixture was stirred in H₂ (40 Psi) at 30°C for 2 h. TLC (dichloromethane:methanol=8: 1) showed that a reaction was completed. The mixture was filtered with celite, and a filtrate was concentrated under increased pressure to remove a solvent so as to obtain a yellow oily compound 5 (66.0 g, 103 mmol, yield: 94.29%, TFA).

¹H NMR: (400 MHz, DMSO)

*δ* 7.86-7.84 (m, 4H), 5.22 (d, *J =* 3.6 Hz, 1H), 4.97 (dd, *J =* 3.2 Hz, 1H), 4.54 (d, *J =* 8.4 Hz, 1H), 4.03 (s, 3H), 3.77-3.60 (m, 3H), 3.60-3.51 (m, 15H), 3.00-2.97 (m, 2H), 2.10 (s, 3H), 2.00 (s, 3H), 1.89 (s, 3H), 1.81 (s, 3H).

### Example 25: Preparation of an intermediate 1-3

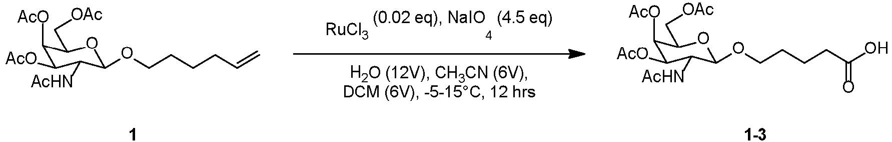

NaIO₄ (224 g, 1.05 mol) and RuCl₃ (966 mg, 4.66 mmol) were added at one time to a mixture of DCM (100 mL), ACN (100 mL) and H₂O (300 mL) at -5°C to 0°C. A compound 1 was dissolved in a mixture of DCM (100 mL) and ACN (100 mL), and then a resulting solution was added dropwise to the above reaction mixture at -5°C to 0°C. The reaction mixture was stirred at 15°C for 12 h. TLC (petroleum ether/ethyl acetate =1/1, product R_{f}=0.20) showed that a new point was formed. The pH of the reaction mixture was adjusted to 8 with NaHCOs (50.0 g), and the mixture was extracted with DCM (500 mL×4). The pH of an aqueous phase was adjusted to 3 with citric acid (100 g). Then, the mixture was extracted with DCM (500 mL×4). An organic phase was separated, dried with Na₂SO₄, filtered and concentrated under reduced pressure to form a residue. The residue was purified from EtOAc (1.00 L) by recrystallization at 15°C. A crude product was washed with EtOAc (1.00 L). A grayish white solid intermediate 1-3 (55.3 g, yield: 52.2%, purity: 98.4%) was obtained.

¹HNMR: (400 MHz DMSO-*d*₆)

11.9 (s, 1H), 7.80 (d, *J =* 9.2 Hz, 9H), 5.20 (d, *J =* 3.6 Hz 1H), 4.97-4.93 (m, 1H), 4.48 (d, *J =* 8.8 Hz, 1H), 4.02 (s, 3H), 3.90-3.83 (m, 1H), 3.73-3.69 (m, 1H), 3.44-3.39 (m, 1H), 2.19 (t, *J =* 6.8 Hz, 2 H), 2.10 (s, 3H), 1.99 (s, 3H), 1.88 (s, 3H), 1.76 (s, 3H), 1.49-1.48 (m, 4H).

### Example 26: Preparation of an intermediate 2-2

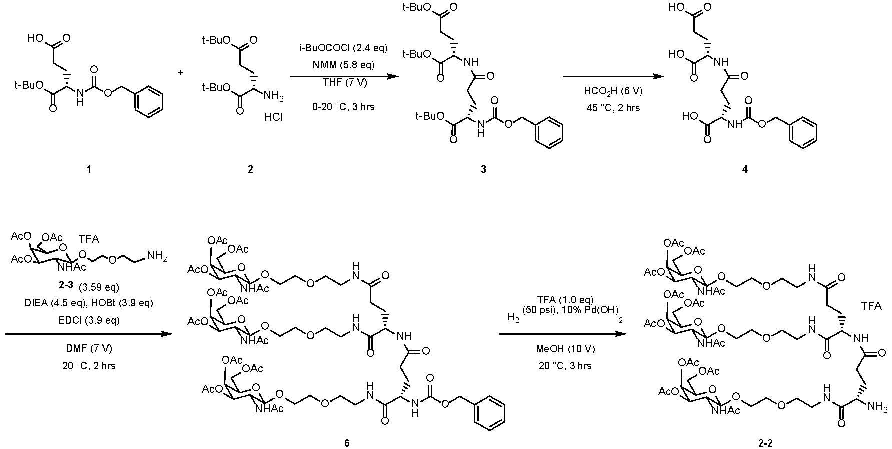

### 1. Preparation of a compound 3

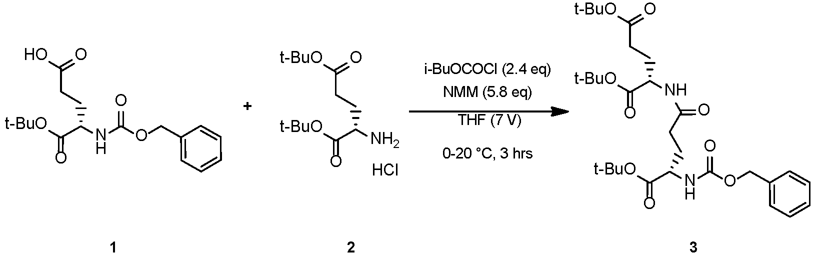

### 4 reactions were carried out in parallel.

4-methylmorpholine (434 g, 4.30 mol, 472 mL) was added to a THF solution (1.75 L) of a compound 1 (250 g, 741 mmol) and then cooled to 0°C. Isobutyl chloroformate (243 g, 1.78 mol, 233 mL) was added to a reaction mixture within 10 min to maintain a reaction temperature less than 4.0°C. After the addition, the mixture was stirred for 40 min or above, and a compound 2 (526 g, 1.78 mol) was added to the reaction mixture successively within 10 min to maintain a reaction temperature less than 4.0°C. After the addition, an ice bath was removed, and then reactants were allowed to be heated to room temperature continuously for 2 h. TLC (petroleum ether/ethyl acetate=1/1, compound 1 R_{f}=0.43) showed that the compound 1 was completely consumed and a new point was formed. The 4 reactions were combined. A reaction solution was poured to a stirred cold (0°C) 0.50 M HCl (aq.) (12.0 L) solution and stirred for about 10 min. Then, EtOAc (4.00 L×3) was added and stirred for a period of time, liquid separation was carried out, and an organic phase was washed with a salt solution (10.0 L), dried with Na₂SO₄ and concentrated under vacuum to produce a thick colorless oily compound. Hexane (1.20 L) was added to the stirred oily compound. White smoke appeared in a solution and then disappeared after further stirring. A seed crystal (1.20 g, 0.10 wt %) was added, and a white crystal was formed slowly at this time. Within 20 min, a suspension was thick enough to hinder stirring. At this time, additional hexane (6.00 L) was added and stirred for 12 h. The suspension was filtered, cleaned with hexane (1.20 L) and dried to obtain a white solid compound 3 (about 1.20 kg).

¹H NMR: (400 MHz DMSO)

*δ* 8.10-8.08 (m, 1H), 7.62-7.60 (m, 1H), 7.38-7.31 (m, 5H), 5.11-4.94 (m, 2H), 4.12-4.09 (m, 1H), 3.92-3.87 (m, 1H), 2.25-2.19 (m, 4H), 1.90-1.88 (m, 2H), 1.73-1.67 (m, 2H), 1.40-1.39 (m, 27H).

### 2. Preparation of a compound 4

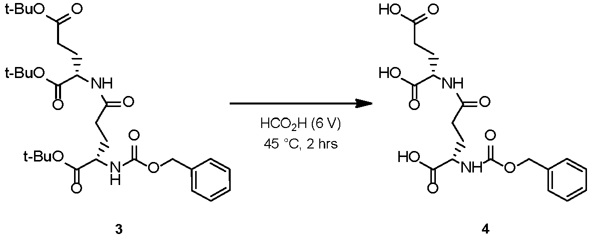

5 reactions were carried out in parallel.

An HCOOH solution (2.40 L) of the compound 3 (240 g, 415 mmol) was stirred at 45°C for 2 h. LCMS (compound 4=0.570 min) showed that the compound 3 was completely consumed and a main peak having a required m/z value appeared. The 5 reactions were combined. Toluene and ACN (each 1.50 L) were used for dilution and concentration. Azeotropic treatment was carried out with ACN and toluene (500 mL) at 1:1 and ACN for three times (500 mL each time) to remove formic acid. A compound 4 was subjected to high vacuum drying. Then, a residue was stirred with DCM (500 mL), and an organic layer was poured off. Then, azeotropic drying was carried out for two times with ACN (400 mL), vacuum drying was carried out, and then azeotropic treatment was carried out with toluene (400 mL) for 9 times to obtain a white solid compound 4 (about 800 g).

¹H NMR: (400 MHz DMSO)

*δ* 12.4-12.2 (m, 2H), 8.12-8.10 (m, 1H), 7.62-7.60 (m, 1H), 7.38-7.34 (m, 5H), 5.07-5.02 (m, 2H), 4.22-4.17 (m, 1H), 3.99-3.96 (m, 1H), 2.31-2.21 (m, 4H), 2.00-1.93 (m, 2H), 1.76-1.75 (m, 2H).

### 3. Preparation of a compound 6

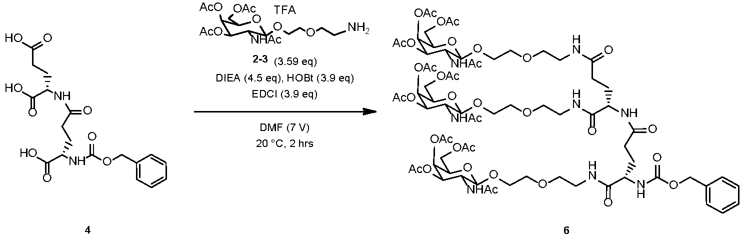

HOBT (103 g, 760 mmol), EDCI (146 g, 760 mmol) and DIEA (113 g, 877 mmol, 153 mL) were sequentially added to a stirred DMF solution (2.00 L) of the compound 4 (80.0 g, 194 mmol) and a compound 2-3 (384 g, 700 mmol, TFA). Reactants were stirred at 20°C for 2 h. TLC (dichloromethane/methanol=5/1, compound 6 R_{f}=0.43) showed that the compound 4 was completely consumed and a new point was formed. A reaction mixture was slowly poured to a stirred cold 0.5 M HCl aqueous solution (230 mL), stirred for 10 min to form a white solid and then filtered, and an aqueous phase was extracted with DCM (1.50 L) for two times. Combined organic phases were cleaned with 5% NaHCOs (aq.) (200 mL), then dried (Na₂SO₄) and evaporated and concentrated under increased pressure. A residue was purified by column chromatography (SiO₂, dichloromethane/methanol=5/1, compound 6 R_{f}=0.43) to obtain a yellow solid compound 6 (about 180 g).

¹H NMR: (400 MHz DMSO)

*δ* 7.95-7.91 (m, 3H), 7.82-7.80 (m, 4H), 7.39-7.31 (m, 6H), 5.21-5.01 (m, 3H), 5.00-4.96 (m, 5H), 4.56-4.53 (m, 3H), 4.02 (s, 1H), 3.88 (s, 9H), 3.85 (s, 4H), 3.76 (s, 3H), 3.50-349 (m, 9H), 3.39-3.36 (m, 6H), 3.19-3.15 (m, 6H), 2.15-2.05 (m, 12H), 1.99 (s, 9H), 1.89-1.77 (m, 21H).

### 4. Preparation of an intermediate 2-2

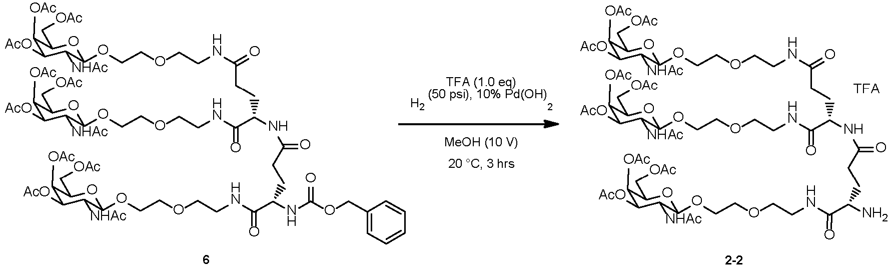

Pd(OH)₂/C (4.94 g, 3.52 mmol, content: 10%) was added to a dry hydrogenation flask in an argon atmosphere, MeOH (350 mL) was added, and the compound 6 (47.4 g, 28.5 mmol) and TFA (3.26 g, 28.5 mmol, 2.11 mL) were sequentially added and stirred in H₂ (50 psi) at 20°C for 3 h. TLC (dichloromethane/methanol=5/1, intermediate 2-2 R_{f}=0.25) monitored a reaction and showed that the compound 6 was consumed. A reaction mixture was filtered, and a filtrate was concentrated under vacuum to obtain a white solid intermediate 2-2 (TFA salt) (46.3 g, yield: 94.6%, purity: 95.7%, TFA).

¹H NMR: (400 MHz DMSO)

δ 8.56-8.50 (m, 1H), 8.14-8.05 (m, 4H), 7.99 (s, 1H), 7.88-7.82 (m, 4H), 5.22-5.21 (m, 3H), 4.99-4.96 (m, 3H), 4.55-4.53 (m, 3H), 4.09 (s, 1H), 4.03 (s, 9H), 3.87-3.79 (m, 6H), 3.59-3.52 (m, 1H), 3.51-347 (m, 9H), 3.39-3.37 (m, 5H), 3.17-3.16 (m, 6H), 2.22-2.10 (m, 12H), 1.99 (s, 9H), 1.93-1.73 (m, 21H).

### Example 27: Preparation of an intermediate 1-2

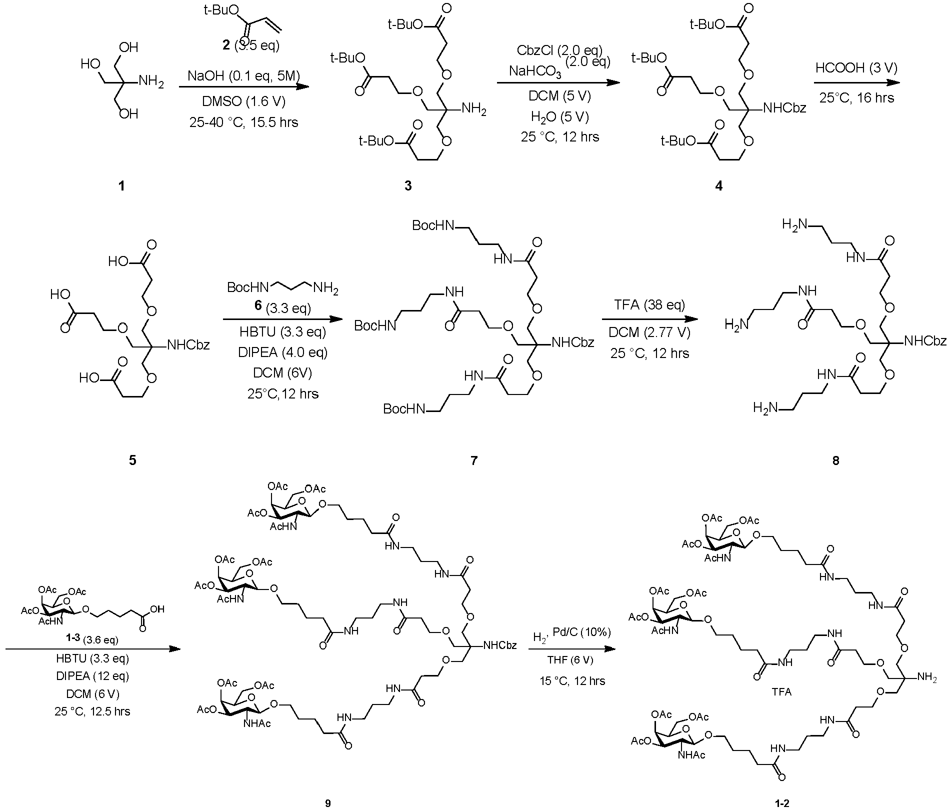

### 1. Preparation of a compound 3

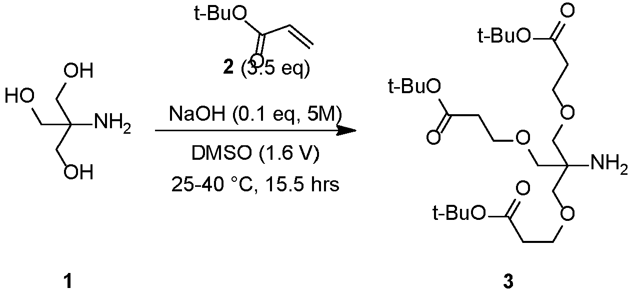

NaOH (5 M, 82.6 mL) was added to a DMSO solution (800 L) of a compound 1 (500 g, 4.13 mol), and then a compound 2 (2.12 kg, 16.5 mol) was added at 25°C. A mixture was stirred at 40°C for 12 h. The mixture was subjected to reflux at 80°C for 3.5 h. TLC (petroleum ether/ethyl acetate=3/1, compound 3 R_{f}=0.49) showed that a reaction was completed. MTBE (1.00 L) was added to the mixture, and then the mixture was cleaned with water (1.00 L×2) and a salt solution (300 mL). An organic phase was dried with Na₂SO₄, filtered and concentrated under reduced pressure to obtain a crude product. A residue was purified by column chromatography (SiO₂, petroleum ether/ethyl acetate=1/0 to 5/1, compound 3 R_{f}=0.49) to obtain a yellow oily compound 3 (about 690 g).

¹H NMR: (400 MHz, DMSO-*d₆*)

*δ* 3.53-3.56 (m, 6H), 3.71 (s, 6H), 2.39 (t, *J* = 6.0 Hz, 6H), 1.40 (s, 27H), 1.29 (s, 2H).

### 2. Preparation of a compound 4

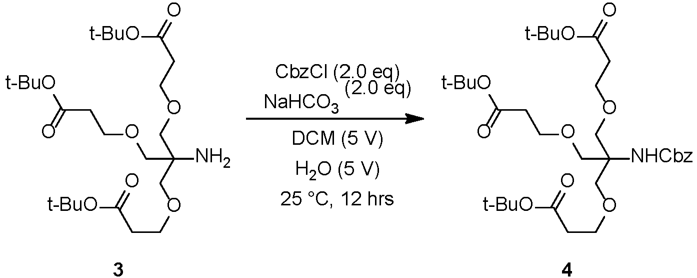

2 reactions were carried out in parallel.

An aqueous solution (1.25 L) of NaHCOs (121 g, 1.44 mol, 56.2 mL) was added to a DCM solution (1.25 L) of the compound 3 (365 g, 722 mmol) at 25°C, and then CbzCl (185 g, 1.08 mol, 154 mL) was added dropwise and stirred for 12 h. TLC (petroleum ether/ethyl acetate=5/1, compound 4 R_{f}=0.46) showed that the compound 3 was completely consumed. The 2 reactions were combined. A reaction mixture was extracted with DCM (1.00 L×2), and combined organic layers were washed with a salt solution (200 mL), dried with Na₂SO₄ and concentrated under vacuum to obtain a residue. The residue was purified by column chromatography (SiO₂, petroleum ether/ethyl acetate=1/0 to 5/1, compound 4 R_{f}=0.46) to obtain a yellow oily compound 4 (about 735 g).

¹H NMR: (400 MHz, DMSO-d6)

δ 7.31-7.35 (m, 5H), 4.97 (s, 2H), 3.49-.3.45 (m, 12H), 2.37-2.40 (m, 6H), 1.39 (s, 27H).

### 3. Preparation of a compound 5

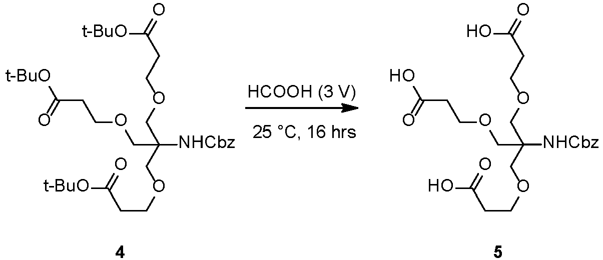

HCOOH (2.20 L) was added to the compound 4 (735 g, 1.15 mol) at 25°C. Then, a solution was stirred at 25°C for 16 h. TLC (petroleum ether/ethyl acetate=5/1, compound 5 R_{f}=0.1) showed that a reaction was completed. Reactants were concentrated under vacuum to form a residue. The residue was diluted with toluene (200 ml) and acetonitrile (200 mL) and then concentrated under vacuum to obtain a yellow oily compound 5 (about 545 g, crude product).

¹H NMR: (400 MHz, DMSO-d6)

δ 12.2 (s, 3H), 7.30-7.39 (m, 5H), 4.98 (s, 2H), 3.54-3.57 (m, 6H), 3.49 (s, 6H), 2.40-2.50 (m, 6H).

### 4. Preparation of a compound 7

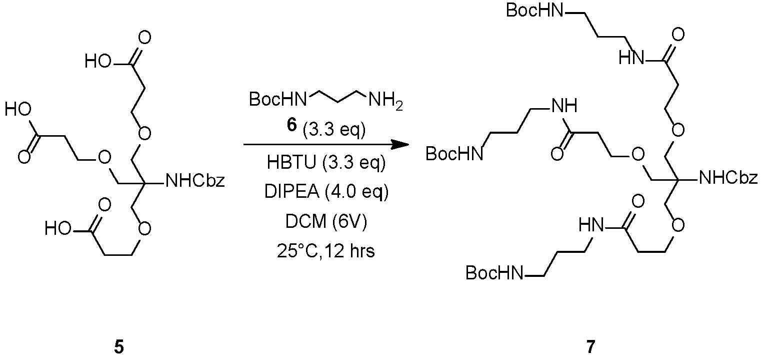

DIPEA (570 g, 4.41 mol), HBTU (1.38 kg, 3.64 mol) and a compound 6 (634 g, 3.64 mol) were added to a DCM solution (600 L) of the compound 5 (520 g, 1.10 mol). A mixture was stirred at 25°C for 12 h. TLC (dichloromethane/methanol=10/1, compound 7 R_{f}=0.22) showed that a reaction was completed. Reactants were cleaned with a saturated NaHCO₃ solution ((500 mL×3) and a salt solution (200 mL), dried with Na₂SO₄, filtered and concentrated under vacuum to obtain a residue. The residue was purified by column chromatography (SiOz, dichloromethane/methanol=1/0 to 5/1, dichloromethane/methanol=10/1, compound 7 R_{f}=0.22) to obtain a yellow oily compound 7 (about 361 g).

¹H NMR: (400 MHz, DMSO-d6)

δ 7.80-7.83(m, 3H), 7.29-7.35 (m, 5H), 6.76-6.77 (m, 3H), 4.97 (s, 2H), 3.52-3.55 (m, 6H), 3.47 (s, 6H), 2.99-3.04 (m, 6H), 2.87-2.92 (m, 6H), 2.25-2.28 (m, 6H), 1.46-1.50 (m, 6H), 1.36 (s, 27H).

### 5. Preparation of a compound 8

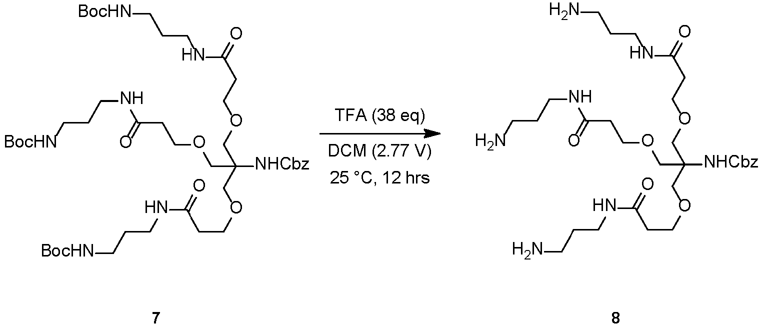

TFA (1.66 kg, 14.6 mol, 1.08 L) was added to a DCM solution (1.00 L) of the compound 7 (360 g, 382.9 mmol) at 25°C. Reactants were stirred at 25°C for 12 h. TLC (dichloromethane:methanol=10: 1, compound 8 R_{f}=0.0) showed that a reaction was completed. A solution was concentrated under vacuum to obtain a residue. The residue was diluted with toluene (500 mL) and then concentrated under vacuum to obtain a yellow oily compound 8 (about 245 g).

### 6. Preparation of a compound 9

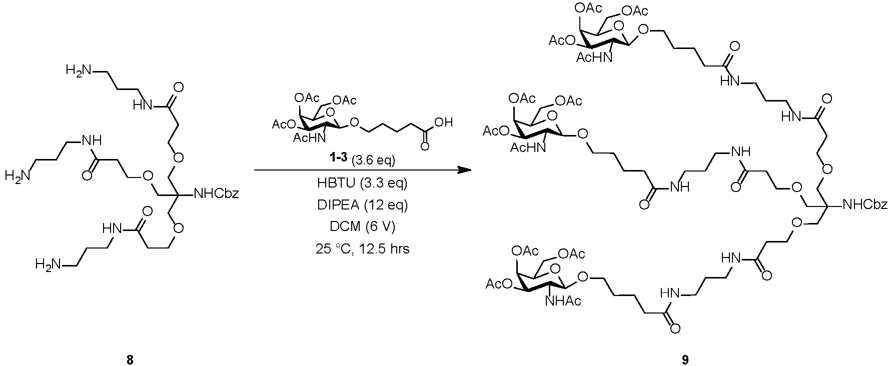

DIPEA (339 g, 2.63 mol) and HBTU (256 g, 675 mmol) were added to a DCM solution (720 mL) of a compound 1-3 (302 g, 675 mmol), and a mixture was stirred at 25°C for 30 min. Then, the compound 8 (120 g, 188 mmol) was added to the mixture, and the mixture was stirred at 25°C for 12 h. LCMS (compound 9 RT=1.737 min) showed that a reaction was completed. A solution was cleaned with a saturated NaHCO₃ aqueous solution ((400 mL×3) and a salt solution (200 mL), dried with Na₂SO₄, filtered and concentrated under reduced pressure to obtain a residue. The residue was purified by column chromatography (SiO₂, dichloromethane/methanol=1/0 to 5/1, compound 9 R_{f}=0.35) to obtain a crude product. The crude product was purified by reverse HPLC (MeCN/H₂O) to obtain a yellow oily compound 9 (about 200 g).

¹H NMR: (400 MHz, DMSO-d6)

δ 7.81-7.84 (m, 6H), 7.72-7.75 (m, 3H), 7.33-7.35 (m, 5H), 5.20-5.21 (m, 3H), 4.94-4.97 (m, 5H), 4.46-4.49 (m, 3H), 4.02 (s, 9H), 3.83-.3.90 (m, 3H), 3.67-3.72 (m, 3H), 3.52-3.55 (m, 6H), 3.47 (s, 5H), 3.34-3.42 (m, 3H), 2.99-3.05 (m, 12H), 2.27 (t, *J =* 6.4 Hz, 6H), 2.10 (s, 9H), 2.03 (t, *J =* 7.2H), 1.99 (s, 9H), 1.88 (s, 9H), 1.77 (s, 9H), 1.72-1.53 (m, 14H).

### 7. Preparation of an intermediate 1-2

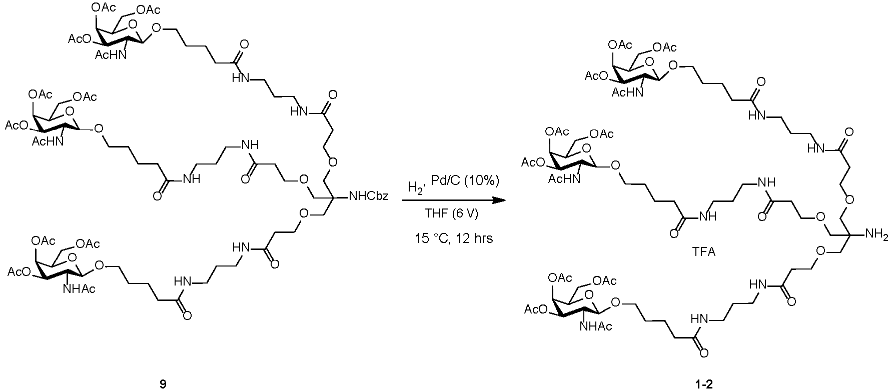

TFA (6.45 g, 56.5 mmol, 4.19 mL) and Pd/C (21.8 g, content: 10%) were added to a THF solution (654 mL) of the compound 9 (109 g, 56.5 mmol) at 15°C in an argon atmosphere. A mixture was stirred in H₂ (15 Psi) at 15°C for 12 h. LCMS (intermediate 1-2 RT=0.696 min) showed that a reaction was completed. The mixture was filtered, and a filtrate was concentrated under vacuum to obtain a white solid intermediate 1-2 (84.2 g, yield: 73.3%, purity: 94.1%, TFA).

¹H NMR: (400 MHz, DMSO-d6)

δ 7.76-7.91 (m, 9H), 5.21 (d, *J* = 3.6 Hz, 3H), 4.94-4.98 (m, 3H), 4.48 (d, *J* = 8.4 Hz, 3 H), 3.99-4.04 (m, 9H), 3.83-3.90 (m, 3H), 3.67-3.72 (m, 3H), 3.57-3.64 (m, 9H), 3.58-3.65 (m, 9H), 3.44 (s, 5H), 3.01-3.07 (m, 12H), 2.34 (t, *J* = 6.0 Hz, 6H), 2.10 (s, 9H), 2.02-2.06 (m, 6H), 1.99 (s, 9H), 1.88 (s, 9H), 1.77 (s, 9H), 1.46-1.53 (m, 17H).

### Example 28: Preparation of siRNA

The siRNA of the present invention is prepared by a solid-phase phosphoramidite method known in the field. The method can specifically refer to, for example, PCT publication No. WO2016081444 and No. WO2019105419, and is briefly described below.

### 1. Connection of a ligand and a CPG carrier

Connection of a ligand and a CPG carrier is illustrated below with the compound E7 as an example, and other GalNAc ligands of the present invention can be connected to the CPG carrier by a similar method.

After the compound E7 (53 mg, 0.018 mmol) and HBTU (13.3 mg, 0.035 mmol) were mixed, acetonitrile (5 mL) was added for dissolution by oscillation, and then DIEA (9.0 mg, 0.07 mmol) and DMAP (2.1 mg, 0.018 mmol) were added for dissolution by oscillation until a solution was clear. A blank carrier Resin (550 mg, CPG pore size 1,000 Å) was weighed and added to the reaction solution, the temperature was controlled at 20°C, and shaking was conducted to carry out a reaction overnight. A sample was taken and monitored, and TLC was carried out with DCM/methanol=4/1 as a developing agent and phosphomolybdic acid as a color developing agent. Results showed that a reaction was completed. Filtration was carried out by a sand core funnel, a filter cake was washed with anhydrous acetonitrile (20 mL*5), and the filter cake was taken and subjected to suction filtration under reduced pressure by an oil pump for 6 h to obtain 530 mg of a white-like solid.

530 mg of the condensed product was placed in a 50 mL round-bottomed flask, CapC (DMAP/acetonitrile), CapB (N-methylimidazole/pyridine/acetonitrile) and CapA (acetic anhydride/acetonitrile) were sequentially added, and shaking was carried out overnight at room temperature. Filtration was carried out, a filter cake was washed with acetonitrile (20 mL*4), and the filter cake was taken and subjected to suction filtration under reduced pressure by an oil pump for 8 h to obtain 200 mg of a white-like solid for solid-phase synthesis.

### 2. Synthesis of a sense strand (SS strand) and an antisense strand (AS strand)

Through the solid-phase phosphoramidite synthesis method, with a blank CPG solid support or a solid support connected with GalNAc ligands according to the above step 1 as an initial cycle, nucleoside monomers or GalNAc ligands were connected one by one in a direction from 3' to 5' according to a nucleotide arrangement sequence. Connection of each of the nucleoside monomers includes reactions in four steps including deprotection, coupling, capping, and oxidation or thiolation. Synthesis conditions of an oligonucleotide with a synthesis scale of 5 umol are as follows:
the nucleoside monomers were provided by a 0.05 mol/L acetonitrile solution, and the reaction in each step was carried out under a same condition, namely a temperature of 25°C; the deprotection was carried out with a 3% trichloroacetic acid-dichloromethane solution, and the deprotection was carried out for 3 times; the coupling reaction was carried out with a 0.25 mol/L ETT-acetonitrile solution as an activator, and the coupling was carried out for 2 times; the capping was carried out with 10% acetic anhydride-acetonitrile and pyridine/N-methylimidazole/acetonitrile (10:14:76, v/v/v), and the capping was carried out for 2 times; the oxidation was carried out with a 0.05 mol/L tetrahydrofuran/pyridine/water (70/20/10, v/v/v) solution of iodine, and the oxidation was carried out for 2 times; and the thiolation was carried out with 0.2 mol/L acetonitrile/3-methylpyridine (1/1, v/v) of PADS, and the thiolation was carried out for 2 times.

### 3. Purification and annealing of oligonucleotide

### 3.1. Ammonolysis

A synthesized solid support (sense strand or antisense strand) was added to a 5 mL centrifuge tube, 3% diethylamine/ammonia water (v/v) was added, and a reaction was carried out in a constant-temperature water bath at 35°C (or 55°C) for 16 h (or 8 h). Filtration was carried out, the solid support was washed with ethanol/water for three times with 1 mL each time, a filtrate was centrifuged and concentrated, and a crude product was purified.

### 3.2. Purification

Methods for purification and desalination are well known to persons in the field. For example, a strong anion filler column can be used, a sodium chloride-sodium hydroxide system was used for elution and purification, a product was collected in a tube, a gel filler purification column can be used for desalination, and an elution system was pure water.

### 3.3. Annealing

The sense strand (SS strand) and the antisense strand (AS strand) were mixed at a molar ratio (SS strand/AS strand=1/1.05), heated to 70-95°C in a water bath pot, maintained for 3-5 min, and then naturally cooled to room temperature, and the system was freeze-dried to obtain a product.

Example 29: Verification of long-term efficacy of a compound of the present invention in a C57BL/6 mouse model

C57BL/6 mice (male, 18-21 g, 6- to 8-week-old) were randomly grouped. An administration dose of each animal was calculated according to a body weight, and single administration was carried out by a subcutaneous injection method. An siRNA conjugate was administered in a 1 mg/mL solution (with a 0.9% sodium chloride aqueous solution as a solvent). Specifically, before an experiment, the siRNA conjugate was dissolved in the 0.9% sodium chloride aqueous solution and metered to a required concentration and a volume. An administration volume of normal saline (control group) and the siRNA conjugate was 5 mL/kg.

Before administration (recorded as day 0) and after administration on day 7, day 14, day 21, day 28, day 35, day 42, day 56 and day 70, 10 mg of liver was taken, placed in an RNAlater^{™} solution and cryopreserved at -80°C for detection of mTTR mRNA of the liver, respectively.

The mTTR mRNA of the liver was detected.

According to an operation protocol of a high-throughput tissue RNA extraction kit (FireGen, FG0412), a nucleic acid extraction apparatus (Hangzhou Allsheng, Auto-pure96) was used for cell RNA extraction. Reverse transcription was carried out with reference to a PrimeScript^{™} II 1st Strand cDNA Synthesis Kit (Takara, 6210B). 20 µL of a system was subjected to fluorescence quantitative PCR reaction (ABI, QuantStudio3) detection with reference to a TaqMan^{™} Fast Advanced Master Mix (ABI, 4444965). Primers are shown in Table 1.

**Table 1: Primer information**

| Primer name | Sequence information (5'-3') | Fluorescent group |
|---|---|---|
| mTTR-PF | GGGAAGACCGCGGAGTCT (SEQ ID NO: 44) | |
| mTTR-PR | CAGTTCTACTCTGTACACTCCTTCTACAAA (SEQ ID NO: 45) | |
| mTTR-P | CTGCACGGGCTCACCACAGATGA (SEQ ID NO: 46) | 5' 6-FAM, 3'BHQ1 |
| mGAPDH-PF | CGGCAAATTCAACGGCACAG (SEQ ID NO: 47) | |
| mGAPDH-PR | CCACGACATACTCAGCACCG (SEQ ID NO: 48) | |
| mGAPDH-P | ACCATCTTCCAGGAGCGAGACCCCACT (SEQ ID NO: 49) | 5'TET, 3'BHQ2 |

### Data statistics and analysis

A 2^{-△△Ct} value was calculated and converted into a percentage to obtain a residual inhibitory rate.
△△Ct=[(Ct target gene in an experimental group-Ct internal reference in the experimental group)-(Ct target gene in a control group-Ct internal reference in the control group)],
where the target gene is mTTR, and the internal reference is mGAPDH.

**Table 2: Compound information**

| | Compound information | Administration dose |
|---|---|---|
| 1 | Normal saline | / |
| 2 | DR002234 | 1mpk |
| 3 | DR002235 | 1mpk |
| 4 | DR002238 | 1mpk |
| 5 | DR002240 | 1mpk |
| 6 | DR002242 | 1mpk |
| 7 | DR002243 | 1mpk |
| 8 | DR002244 | 1mpk |
| 9 | DR002245 | 1mpk |
| 10 | DR002246 | 1mpk |
| 11 | DR002248 | 1mpk |
| 12 | DR002247 | 1mpk |
| 13 | DR002249 | 1mpk |
| 14 | DR002220 | 1mpk |

**Table 3: Experimental results of long-term efficacy of a compound in a C57BL/6 mouse model**

| | Test compound | Day7 | | Day14 | | Day21 | | Day28 | | Day42 | | Day56 | | Day70 | |
|---|---|---|---|---|---|---|---|---|---|---|---|---|---|---|---|
| | | Residual mRNA of a target gene | SD | Residual mRNA of a target gene | SD | Residual mRNA of a target gene | SD | Residual mRNA of a target gene | SD | Residual mRNA of a target gene | SD | Residual mRNA of a target gene | SD | Residual mRNA of a target gene | SD |
| 1 | Normal saline | 100.3% | 8.7 % | 100.5% | 11.0% | 100.9% | 15.9% | 100.1% | 4.9% | 100.0% | 3.6% | 100.1% | 4.8% | 100.6% | 13.3% |
| 2 | DR002234 | 7.4% | 3.0% | 5.7% | 3.1% | 11.4% | 9.1% | 14.5% | 3.3% | / | / | / | / | / | / |
| 3 | DR002235 | 7.4% | 1.3% | 7.1% | 0.6% | 10.7% | 0.5% | 12.9% | 4.2% | / | / | / | / | / | / |
| 4 | DR002238 | 11.6% | 3.0% | 14.9% | 2.9% | 10.1% | 6.8% | 32.9% | 5.9% | / | / | / | / | / | / |
| 5 | DR002240 | 6.8% | 0.9% | 7.5% | 2.3% | 15.9% | 7.4% | 16.2% | 5.7% | / | / | / | / | / | / |
| 6 | DR002242 | / | / | 1.6% | 0.7% | / | / | 1.7% | 0.5% | / | / | / | / | / | / |
| 7 | DR002243 | 8.3% | 1.7% | 6.6% | 1.5% | 7.8% | 4.6% | 8.4% | 3.3% | / | / | / | / | / | / |
| 8 | DR002244 | 5.1 % | 2.0% | 3.4% | 0.6% | 3.4% | 0.5% | 4.2% | 0.3% | / | / | / | / | / | / |
| 9 | DR002245 | 5.8 % | 1.7% | 5.8% | 1.2% | 3.1% | 0.7% | 7.2% | 2.8% | / | / | / | / | / | / |
| 10 | DR002246 | 5.2% | 1.3% | 5.1% | 1.3% | 3.0% | 1.3% | 8.2% | 6.0% | / | / | / | / | / | / |
| 11 | DR002248 | 3.1% | 0.6% | 3.6% | 0.5% | 2.4% | 1.7% | 6.6% | 2.8% | / | / | / | / | / | / |
| 12 | DR002247 | 9.7% | 2.7% | 9.6% | 1.7% | 60% | 2.2% | 8.6% | 2.0% | 17.1% | 1.4% | 36.1% | 6.0% | 53.1% | 10.2% |
| 13 | DR002249 | 6.4% | 1.2% | 4.0% | 0.6% | 3.3% | 0.5% | 5.5% | 1.3% | 16.6% | 6.6% | 24.3% | 7.9% | 34.3% | 2.0% |
| 14 | DR002220 | 4.2 % | 1.2% | 3.8% | 0.3% | 4.2% | 0.7% | 6.8% | 2.7% | 18.5% | 2.8% | 49.8% | 3.0% | 60.2% | 10.5% |

| | | | | | | | | | | | | | | | |
|---|---|---|---|---|---|---|---|---|---|---|---|---|---|---|---|
| where "P' represents no test. | | | | | | | | | | | | | | | |

### Example 30: Verification of long-term efficacy of a compound of the present invention in a C57BL/6 mouse model

With reference to experimental steps in Example 29, long-term efficacy of a test compound (shown in Table 4) in a C57BL/6 mouse model was verified. An administration dose of each animal was calculated according to a body weight, and single administration was carried out by a subcutaneous injection method. An siRNA conjugate was administered in a 1 mg/mL solution (with a 0.9% sodium chloride aqueous solution as a solvent) at an administration dose of 1 mpk. At various time points, 10 mg of liver was taken, placed in an RNAlater^{™} solution and cryopreserved at -80°C for detection of mTTR mRNA of the liver. Experimental results are shown in Table 5.

**Table 4: Compound information**

| | Compound information | Administration dose |
|---|---|---|
| 1 | Normal saline | / |
| 2 | DR002220 | 1mpk |
| 3 | DR005649 | 1mpk |
| 4 | DR002242 | 1mpk |
| 5 | DR005651 | 1mpk |
| 6 | DR005648 | 1mpk |

**Table 5: Experimental results of long-term efficacy of a compound in a C57BL/6 mouse model**

| | Day7 | | Day21 | | Day28 | | Day42 | | Day56 | | Day70 | |
|---|---|---|---|---|---|---|---|---|---|---|---|---|
| In vivo compound number | Residual mRNA of a target gene | SD | Residual mRNA of a target gene | SD | Residual mRNA of a target gene | SD | Residual mRNA of a target gene | SD | Residual mRNA of a target gene | SD | Residual mRNA of a target gene | STD |
| Normal saline | 102.5% | 25.8% | 102.5% | 28.8% | 100.8% | 14.2% | 100.4% | 9.9% | 100.4% | 9.7% | 100.3% | 8.3% |
| DR002220 | 7.5% | 3.1% | 9.4% | 6.5% | 26.7% | 4.0% | 44.0% | 5.6% | 60.3% | 13.9% | 85.7% | 13.3% |
| DR005649 | 2.0% | 0.8% | 1.7% | 0.4% | 8.3% | 5.6% | 14.7% | 6.1% | 23.4% | 8.6% | 46.7% | 12.6% |
| DR002242 | 3.5% | 1.7% | 3.9% | 0.7% | 9.2% | 6.8% | 15.3% | 3.8% | 34.3% | 3.7% | 56.8% | 17.9% |
| DR005651 | 5.0% | 4.8% | 3.4% | 0.8% | 7.0% | 1.5% | 14.1% | 1.8% | 25.0% | 4.1% | 45.5% | 5.7% |
| DR005648 | 2.2% | 0.6% | 3.1% | 0.7% | 8.6% | 3.0% | 14.8% | 1.9% | 19.5% | 4.3% | 28.0% | 3.0% |

### Example 31: Verification of long-term efficacy of a compound of the present invention in a C57BL/6 mouse model

With reference to experimental steps in Example 29, long-term efficacy of a test compound (shown in Table 6) in a C57BL/6 mouse model was verified.

An administration dose of each animal was calculated according to a body weight, and single administration was carried out by a subcutaneous injection method. An siRNA conjugate was administered in a 1 mg/mL solution (with a 0.9% sodium chloride aqueous solution as a solvent) at an administration dose of 1 mpk. At various time points, orbital blood was taken and detected by an ELISA kit (Abeam, ab282297) to obtain an mTTR protein in serum. Experimental results are shown in Table 7.

**Table 6: Compound information**

| | Compound information | Administration dose |
|---|---|---|
| 1 | Saline | / |
| 2 | DR002220 | 1mpk |
| 3 | DR002242 | 1mpk |
| 4 | DR005728 | 1mpk |
| 5 | DR005729 | 1mpk |
| 6 | DR005730 | 1mpk |

**Table 7: Experimental results of long-term efficacy of a compound in a C57BL/6 mouse model**

| | | Day14 | | Day28 | | Day42 | | Day56 | |
|---|---|---|---|---|---|---|---|---|---|
| | Compound information | Residual mTTR protein | SD | Residual mTTR protein | SD | Residual mTTR protein | SD | Residual mTTR protein | SD |
| 1 | Saline | 100.00% | 1000% | 100.00% | 8.10% | 100.00% | 6.30% | 100.00% | 4.90% |
| 2 | DR002220 | 2.30% | 0.80% | 6.80% | 2.40% | 16.30% | 460% | 33.40% | 7.60% |
| 3 | DR002242 | 2.80% | 1.40% | 5.80% | 2.90% | 12.00% | 3.50% | 17.70% | 4.90% |
| 4 | DR005728 | 2.00% | 0.40% | 3.90% | 1.20% | 8.30% | 3.10% | 16.10% | 6.20% |
| 5 | DR005729 | 2.50% | 1.30% | 3.20% | 0.50% | 9.60% | 3.90% | 11.50% | 0.90% |
| 6 | DR005730 | 1.90% | 0.60% | 5.70% | 3.20% | 11.70% | 400% | 18.70% | 7.40% |

### Example 32: Activity detection in an HDI mouse model (APOC3)

### 1. HDI animal modeling

6- to 8-week-old female Balb/c mice were subjected to in vivo transfection modeling using a double gene stable transfection system by a high-pressure tail vein injection method. Via the tail vein, a TransIT^{®}-QR Delivery Solution (total volume: 10% of an animal body weight, Mirusbio-MIR 5240) containing a Piggy-Bac transposon plasmid of a target gene cDNA sequence and a helper plasmid at different mass ratios was injected into the mice by a 27-gauge needle within 5-7 seconds. The mice were placed back into a cage and observed for 30 min after the injection. With a molding day as day 0, an SEAP expression level was detected at various time points (Day7-Day35) after the molding.

### 2. Detection of SEAP expression

A standard product of a kit (Phospha-Light^{™} SEAP reporter gene test system, Invitrogen, T1016) was diluted at 15 mU/mL by 2 times in a total of 7 concentration points.

A CSPD substrate was mixed with a reaction buffer diluent at a ratio of 1:20 to form a reaction solution.

A 5× buffer diluent was diluted to a 1× buffer diluent with RNase-free DNase distilled water.

Serum was mixed with the 1× buffer diluent to obtain a sample diluent in a centrifuge tube, and the sample diluent was incubated at 65°C for 30 min and then cooled to room temperature.

50 µL of the sample diluent was added to a 96-well plate, and then 50 µL of an analytical buffer was added to each well and incubated at room temperature for 5 min.

50 µL of the reaction solution was added to each well and incubated at room temperature for 20 min, and an SEAP chemiluminescence value was read on a microplate reader (Tecan, Infinite 200).

### 3. Activity detection

A total of 50 ug of plasmids including a Piggy-Bac helper plasmid and a Piggy-Bac transposon recombinant plasmid at a mass ratio of 1:1 were selected for modeling. On day 15 after the modeling, according to Table 10, the mice were administered subcutaneously in one time with 200 µl of normal saline containing a 3 mg/kg (mpk) Apoc3 RNAi reagent or 200 µl of normal saline without an Apoc3 RNAi reagent as a control. Experimental screening results of an HDI model are shown in Table 11.

**Table 10 Single subcutaneous administration dose of an RNAi reagent in mice**

| | Compound information | Administration dose |
|---|---|---|
| 1 | Normal saline | / |
| 2 | DR005636 | 3mpk |
| 3 | DR005679 | 3mpk |
| 4 | DR005681 | 3mpk |

**Table 11 Experimental results of an RNAi reagent in an HDI mouse model with stable transfection of plasmids**

| | | Day7 | | Day14 | | Day21 | | Day28 | | Day35 | | Day42 | |
|---|---|---|---|---|---|---|---|---|---|---|---|---|---|
| | Compoun d | Averag e value | SD | Averag e value | SD | Averag e value | SD | Averag e value | SD | Averag e value | SD | Averag e value | SD |
| 1 | Normal saline | 137.50 % | 32.70 % | 129.50 % | 37.20 % | 129.60 % | 20.90 % | 140.20 % | 14.60 % | 110.60 % | 6.90% | 97.40% | 9.30% |
| 2 | DR00563 6 | 9.00% | 2.60% | 11.70% | 3.80% | 17.70% | 5.90% | 29.00% | 7.50% | 53.00% | 17.50 % | 65.10% | 11.30 % |
| 3 | DR00567 9 | 9.40% | 3.70% | 11.60% | 4.30% | 21.40% | 10.40 % | 38.40% | 21.20 % | 75.40% | 39.10 % | 92.00% | 27.10 % |
| 4 | DR00568 1 | 11.20% | 2.80% | 13.40% | 3.60% | 23.80% | 8.20% | 41.60% | 11.20 % | 59.60% | 8.50% | 77.60% | 14.90 % |

| | | | | | | | | | | | | | |
|---|---|---|---|---|---|---|---|---|---|---|---|---|---|
| Note: Serum SEAP was standardized to 100% before administration. | | | | | | | | | | | | | |

### Example 33: Activity detection in an HDI mouse model (ANGPTL3)

With reference to experimental steps in Example 32, 6- to 8-week-old female Balb/c mice were selected to construct an HDI model and carry out an administration test in this experiment. A total of 100 ug of plasmids including a Piggy-Bac helper plasmid and a Piggy-Bac transposon recombinant plasmid at a mass ratio of 1:1 were selected for modeling. On day 14 after the modeling, according to Table 14, the mice were administered subcutaneously in one time with 200 µl of normal saline containing a 1 mg/kg (mpk) ANGPTL3 RNAi reagent or 200 µl of normal saline without an ANGPTL3 RNAi reagent as a control. Experimental screening results of an HDI model are shown in Table 15.

**Table 14 Single subcutaneous administration dose of an RNAi reagent in mice**

| Number | Group | Administration dose |
|---|---|---|
| 1 | Normal saline | / |
| 2 | DR005685 | 1 mpk |
| 3 | DR005686 | 1 mpk |
| 4 | DR005687 | 1 mpk |

**Table 15 Experimental results of an RNAi reagent in an HDI mouse model with stable transfection of plasmids**

| | Normal saline | DR005685 | DR005686 | DR005687 |
|---|---|---|---|---|
| Day7 | 97.2% | 19.4% | 17.0% | 16.8% |
| Day14 | 95.7% | 16.3% | 15.3% | 11.1% |
| Day21 | 92.1% | 22.5% | 24.0% | 18.5% |
| Day28 | 83.2% | 34.5% | 35.0% | 29.7% |
| Day35 | 90.4% | 48.0% | 52.9% | 47.8% |

| | | | | |
|---|---|---|---|---|
| Note: Serum SEAP was standardized to 100% before administration. | | | | |

### Example 34: In vivo activity detection (PCSK9)

Experimental animals: 40 humanized PCSK9 Tg mice that were male and 11- to 12-week-old were selected.

A baseline level of a PCSK9 protein was determined by ELISA on D-12 (namely., day 12 before administration, same below) and D-5 before administration of the animals, and the baseline level was defined as 100%.

On D0, a test compound was administered subcutaneously at 3 mg/kg by single administration, as shown in Table 19 for detail.

**Table 19 Administration details for in vivo activity screening**

| Group information | siRNA | Animal number | Administration dose | Administration volume | Administration route and frequency |
|---|---|---|---|---|---|
| Vehicle | PBS | 5 | 3 mg/kg | 5 mL/kg | Subcutaneous, single time |
| TA01 | DR002221 | 5 | 3 mg/kg | 5 mL/kg | Subcutaneous, single time |
| TA02 | DR005642 | 5 | 3 mg/kg | 5 mL/kg | Subcutaneous, single time |
| TA03 | DR005656 | 5 | 3 mg/kg | 5 mL/kg | Subcutaneous, single time |
| TA04 | DR002359 | 5 | 3 mg/kg | 5 mL/kg | Subcutaneous, single time |
| TA05 | DR005672 | 5 | 3 mg/kg | 5 mL/kg | Subcutaneous, single time |
| TA06 | DR005675 | 5 | 3 mg/kg | 5 mL/kg | Subcutaneous, single time |
| TA07 | DR005674 | 5 | 3 mg/kg | 5 mL/kg | Subcutaneous, single time |

On D7, D14, D21 and D28 after the administration, whole blood was taken, respectively. After standing was carried out at room temperature, serum was obtained by centrifugation, and a PCSK9 protein level was determined by ELISA. Results are shown in Table 20.

**Table 20 In vivo activity screening results**

| Compound | D7 | | D14 | | D21 | | D28 | |
|---|---|---|---|---|---|---|---|---|
| | Average value | SD | Average value | SD | Average value | SD | Average value | SD |
| PBS | 122.0% | 39.4% | 110.5% | 36.8% | 126.5% | 31.5% | 120.4% | 36.1% |
| DR002221 | 13.3% | 2.9% | 12.9% | 2.1% | 21.7% | 3.0% | 29.6% | 4.0% |
| DR005642 | 13.8% | 2.2% | 13.5% | 3.5% | 19.9% | 5.8% | 22.4% | 5.5% |
| DR005656 | 15.8% | 4.4% | 15.0% | 5.8% | 26.3% | 12.3% | 27.6% | 11.9% |
| DR002359 | 11.6% | 3.3% | 17.7% | 7.0% | 24.4% | 20.7% | 39.7% | 5.1% |
| DR005672 | 11.3% | 2.3% | 9.2% | 2.0% | 16.1% | 3.7% | 25.9% | 8.3% |
| DR005675 | 12.4% | 1.4% | 16.2% | 4.0% | 25.4% | 4.8% | 49.0% | 13.5% |
| DR005674 | 7.2% | 1.9% | 8.4% | 1.8% | 8.5% | 3.3% | 11.1% | 3.2% |

The above contents are further detailed descriptions of the present invention in combination with specific preferred embodiments, which cannot be assumed that the specific embodiments of the present invention are limited to these descriptions. For persons of ordinary skill in the technical field to which the present invention belongs, various simple inferences or substitutions can also be made without departing from the concept of the present invention and should be regarded as falling within the scope of protection of the present invention.

## Claims

1. A ligand containing N-acetylgalactosamine, wherein the ligand comprises a conjugated group shown in Formula (X'): 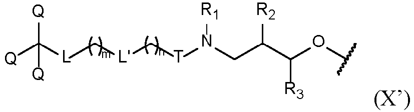 wherein, 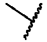
represents a position connected with a biomolecule;
Q is independently H, 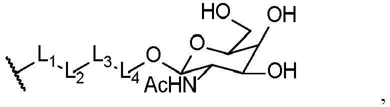 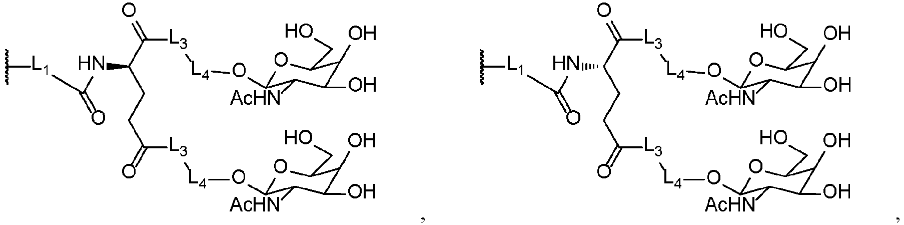 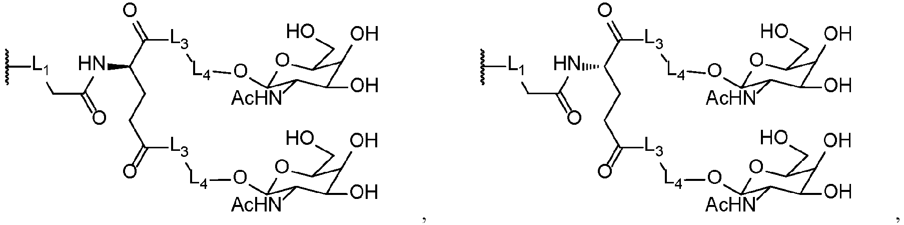 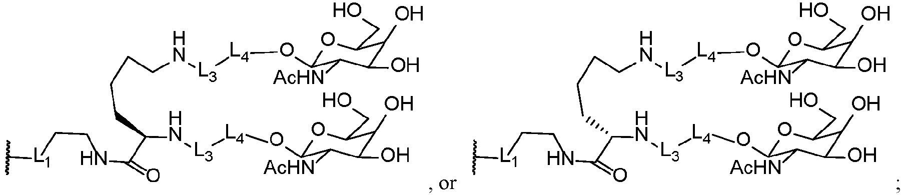
wherein L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
wherein a is 0, 1, 2, or 3;
b is 1, 2, 3, 4, or 5;
c is 1, 2, 3, 4, or 5;
d is 1,2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
wherein e is 1, 2, 3, 4, or 5;
T is bond, -CH₂-, -C(O)-, -M-, -CH₂-M-, or -C(O)-M-;
wherein M is 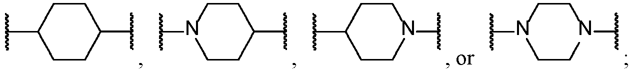
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

2. The ligand according to claim 1, wherein the conjugated group is shown in Formula (I'): 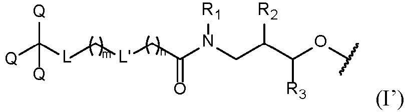 wherein, 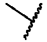
represents a position connected with a biomolecule;
Q is independently H, 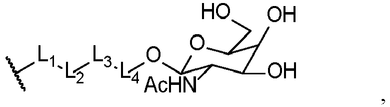 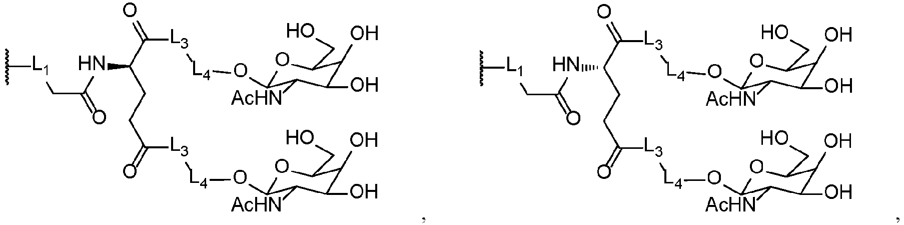 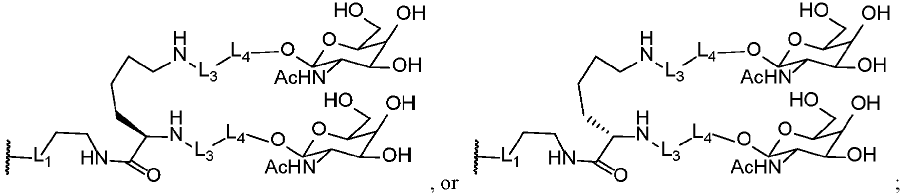
wherein L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
wherein a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond, -C(O)NH-, or -NHC(O)-;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

3. The ligand according to claim 2, wherein,
Q is independently H or 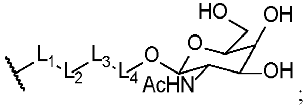
wherein L₁ is -CH₂O- or -NHC(O)-(CH₂NHC(O))ₐ-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein a is 0, 1, 2, or 3;
b is 1, 2, 3, 4, or 5;
c is 1, 2, 3, 4, or 5;
d is 1,2, 3, 4, 5, 6, 7, or 8;
L is -CH₂O-;
L' is bond;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

4. The ligand according to claim 2, wherein the conjugated group is shown in Formula (I'-1), Formula (I'-2), or Formula (I'-3): 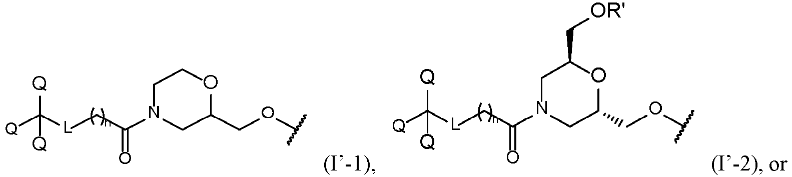 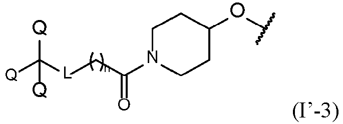 wherein, 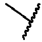
represents a position connected with a biomolecule;
Q is 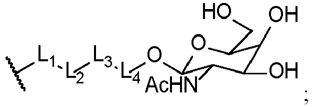
wherein L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1,2, 3, 4, 5, 6, 7, or 8;
L is -CH₂O-;
R' is H, a hydroxyl protective group, or a solid support, and the hydroxyl protective group is preferably - C(O)CH₂CH₂C(O)OH or 4,4'-dimethoxytriphenylmethyl; and
nis0, 1, 2, 3, 4, 5, 6, 7, 8, 9, or 10.

5. The ligand according to claim 2, wherein,
Q is independently H, 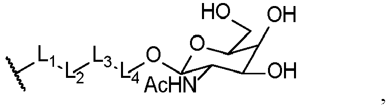 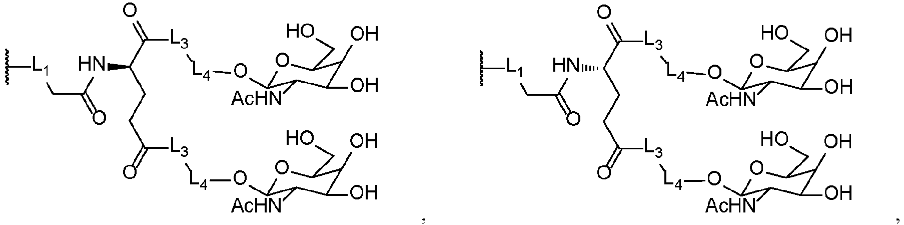 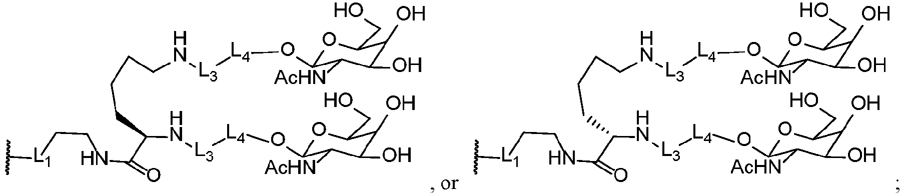
wherein L₁ is -CH₂O-, -CH₂O-CH₂CH₂O-, or -NHC(O)-(CH₂NHC(O))ₐ-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

6. The ligand according to claim 5, wherein the conjugated group is shown in Formula (II'-1) or Formula (II'-2): 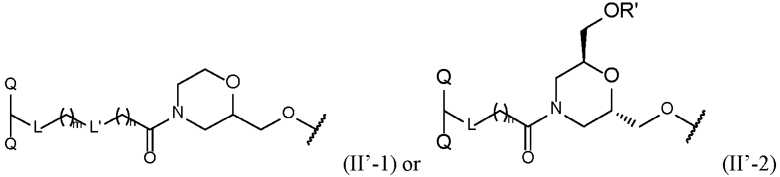 wherein, 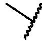
represents a position connected with a biomolecule;
Q is independently 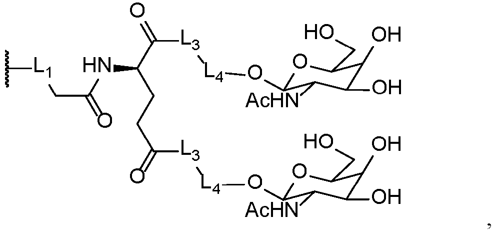 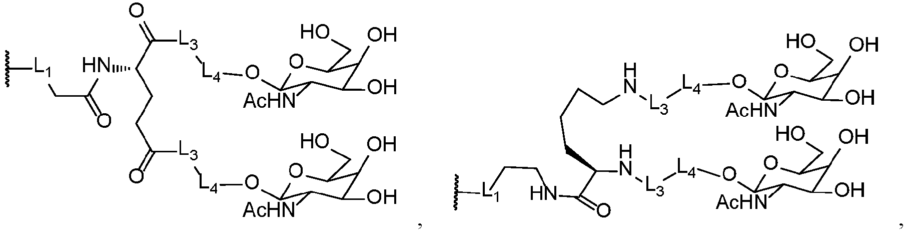 or 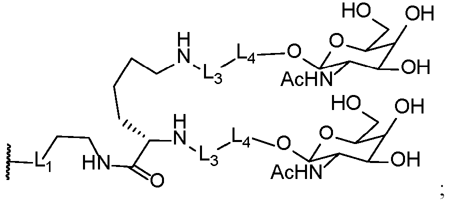
wherein L₁ is -CH₂O- or -CH₂O-CH₂CH₂O-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1,2, 3, 4, 5, 6, 7, or 8;
L is -NHC(O)-;
L' is bond or -C(O)NH-;
R' is H, a hydroxyl protective group, or a solid support, and the hydroxyl protective group is preferably - C(O)CH₂CH₂C(O)OH or 4,4'-dimethoxytriphenylmethyl;
m is 0, 1, 2, 3, 4, 5, 6, 7, 8, 9, or 10; and
nis 0, 1,2, 3, 4, 5, 6, 7, 8,9, or 10.

7. The ligand according to claim 2, wherein,
Q is independently H, 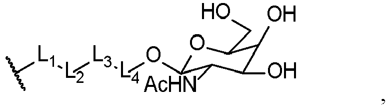 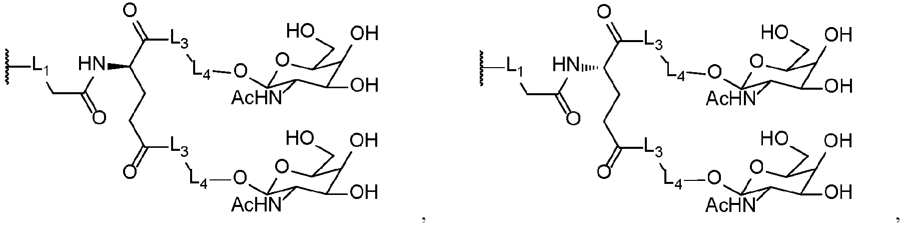 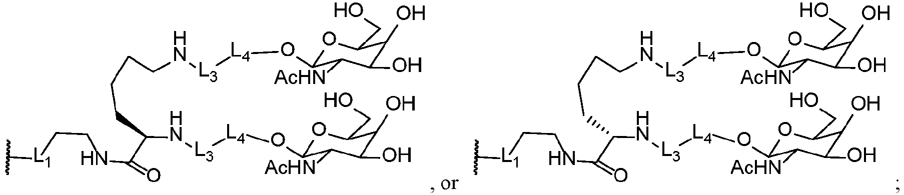
wherein L₁ is -CH₂-, -C(O)-, -CH₂O-, -CH₂O-CH₂CH₂O-, or -NHC(O)-(CH₂NHC(O))ₐ-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein a is 0, 1, 2, or 3;
b is 1, 2, 3, 4, or 5;
c is 1, 2, 3, 4, or 5;
d is 1,2, 3, 4, 5, 6, 7, or 8;
L is -CH₂O- or -NHC(O)-;
L' is bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

8. The ligand according to claim 7, wherein the conjugated group is shown in Formula (II'-2): 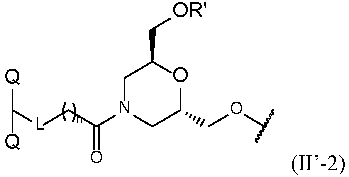 wherein, 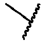
represents a position connected with a biomolecule;
Q is 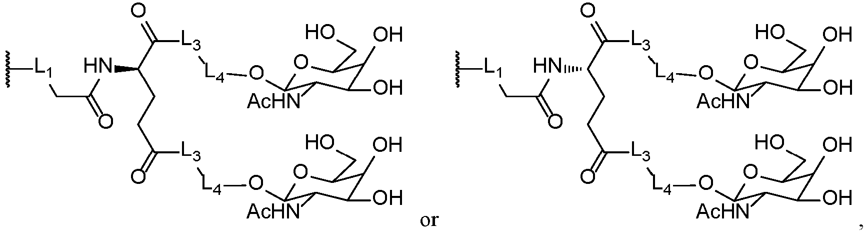 preferably 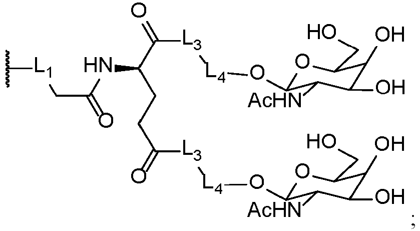
L₁ is -CH₂O-;
L₃ is -NHCH₂CH₂-;
L₄ is -(OCH₂CH₂)_{c}-;
c is 1, 2, or 3;
L is -NHC(O)-;
R' is H; and
n is 6, 7, 8, 9, or 10, preferably 7, 8, or 9, more preferably 8.

9. The ligand according to claim 7, wherein the conjugated group is shown in Formula (II'-2): 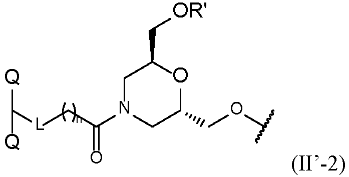 wherein, 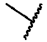
represents a position connected with a biomolecule;
Q is independently 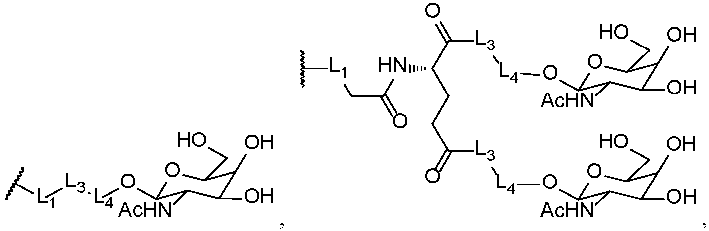
or 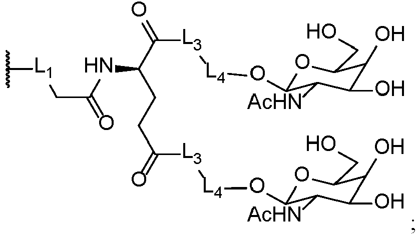
wherein L₁ is -CH₂- or -C(O)-;
L₃ is -(NHCH₂CH₂)_{b}-,
L₄ is -(OCH₂CH₂)_{c}-;
wherein b is 1, 2, 3, 4, or 5;
c is 1, 2, 3, 4, or 5;
L is -CH₂O- or -NHC(O)-;
R' is H, a hydroxyl protective group, or a solid support, and the hydroxyl protective group is preferably - C(O)CH₂CH₂C(O)OH or 4,4'-dimethoxytriphenylmethyl; and
n is 0, 1, 2, 3, 4, 5, 6, 7, 8, 9, or 10.

10. The ligand according to claim 9, wherein the conjugated group is shown in Formula (II'-3): 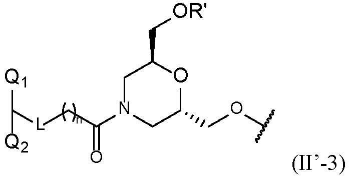 wherein, 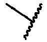
represents a position connected with a biomolecule;
Q₁ is 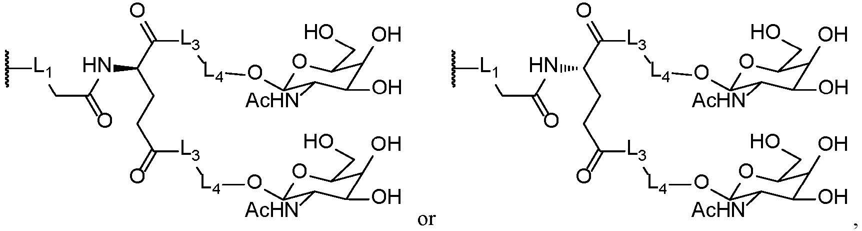 preferably 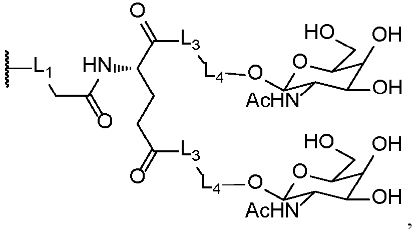 wherein L₁ is -CH₂-;
Q₂ is 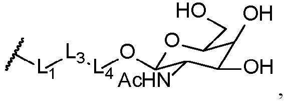 wherein L₁ is -C(O)-;
L₃ is -NHCH₂CH₂-;
L₄ is -(OCH₂CH₂)_{c}-;
c is 1, 2, or 3;
L is -NHC(O)-;
R' is H; and
n is 6, 7, 8, 9, or 10, preferably 7, 8, or 9, more preferably 8.

11. The ligand according to claim 1, wherein,
Q is independently H, 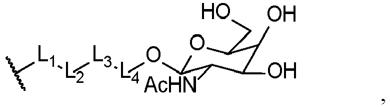 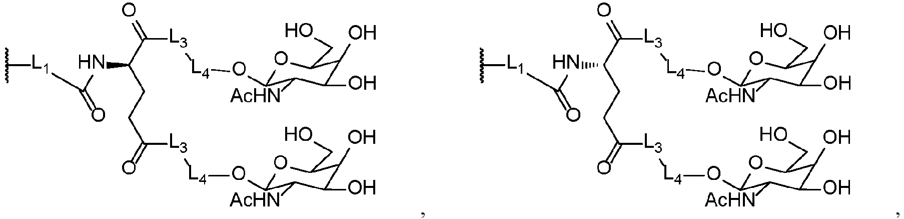 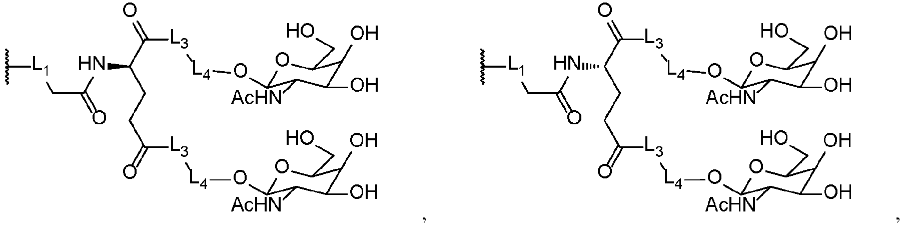 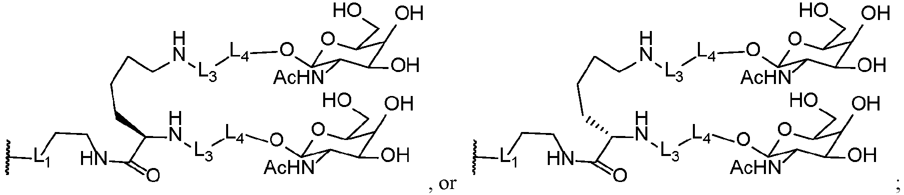
wherein L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
wherein a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is bond, -C(O)NH-, -NHC(O)-, or -O(CH₂CH₂O)ₑ-;
wherein the e is 1, 2, 3, 4, or 5;
T is bond, -CH₂-, -M-, -CH₂-M-, or -C(O)-M-;
wherein the M is 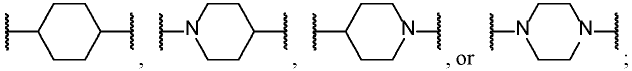
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1,2, 3, 4, 5, 6, 7, 8, 9, or 10.

12. The ligand according to claim 11, wherein,
T is -M-, -CH₂-M-, or -C(O)-M-, wherein the M is

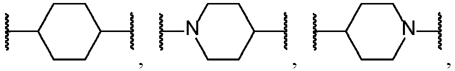

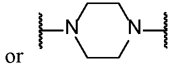

13. The ligand according to claim 11 or 12, wherein,
Q is independently H or 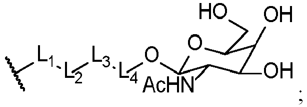
wherein L₁ is -CH₂O- or -NHC(O)-(CH₂NHC(O))ₐ-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein a is 0, 1, 2, or 3;
b is 1, 2, 3, 4, or 5;
c is 1, 2, 3, 4, or 5;
d is 1,2, 3, 4, 5, 6, 7, or 8;
L is bond or -CH₂O-;
L' is bond or -O(CH₂CH₂O)ₑ-;
wherein the e is 1, 2, 3, 4, or 5;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to claim 11 or 12.

14. The ligand according to claim 13, wherein the conjugated group is shown in Formula (III'-1), Formula (III'-2), or Formula (III'-3): 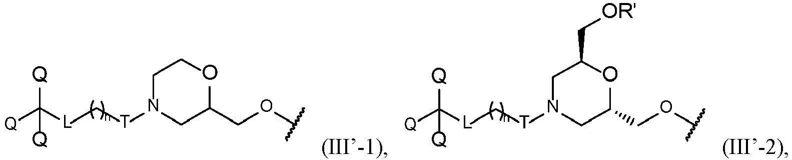 or wherein, 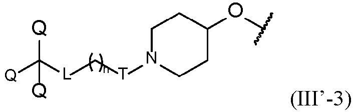 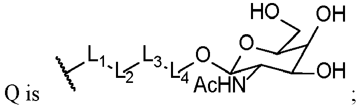
wherein L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1, 2, 3, 4, 5, 6, 7, or 8;
L is bond or -CH₂O-;
wherein R' is H, a hydroxyl protective group, or a solid support, and the hydroxyl protective group is preferably -C(O)CH₂CH₂C(O)OH or 4,4'-dimethoxytriphenylmethyl;
n is 0, 1, 2, 3, 4, 5, 6, 7, 8, 9, or 10; and
T is as defined according to claim 11 or 12.

15. The ligand according to claim 11 or 12, wherein,
Q is independently H, 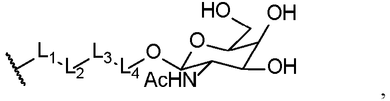 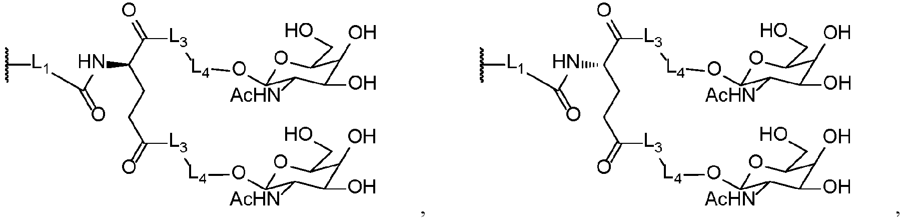 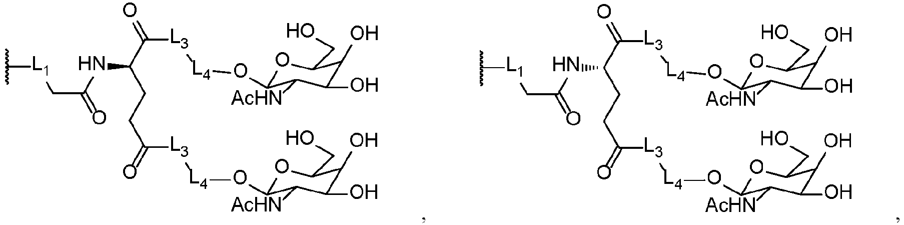 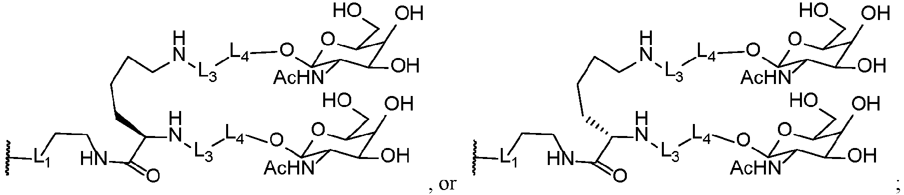
wherein L₁ is -CH₂-, -CH₂O-, or -C(O)-;
L₂ is bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1,2, 3, 4, 5, 6, 7, or 8;
L is bond or -NHC(O)-;
L' is bond;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to claim 11 or 12.

16. The ligand according to claim 11 or 12, wherein the conjugated group is shown in Formula (IV'-1) or Formula (IV'-2): 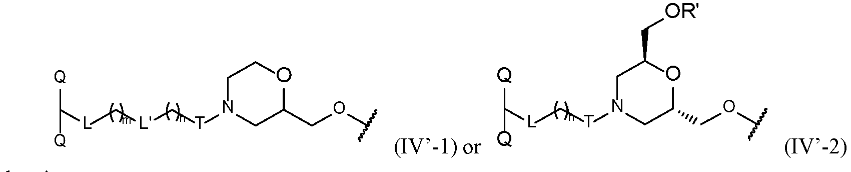 wherein,
Q is independently 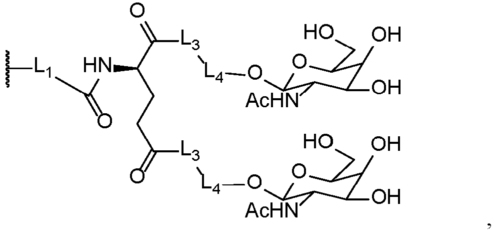 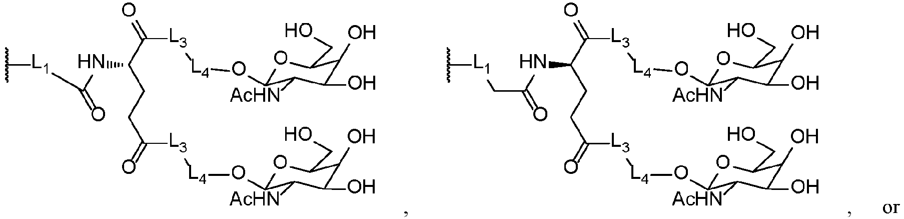 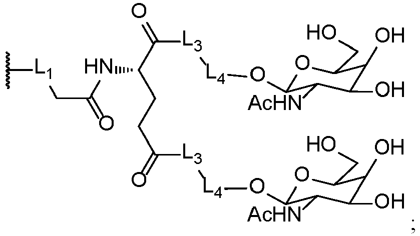
wherein L₁ is -CH₂-, -CH₂O-, or -C(O)-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1,2, 3, 4, 5, 6, 7, or 8;
L is bond or -NHC(O)-;
L' is bond;
wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to claim 11 or 12.

17. The ligand according to claim 1, wherein the conjugated group is shown in Formula (II'-3): 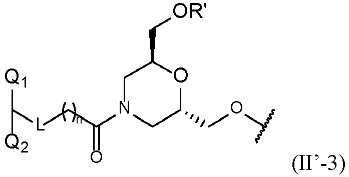 wherein, 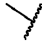
represents a position connected with a biomolecule;
Q₁ is 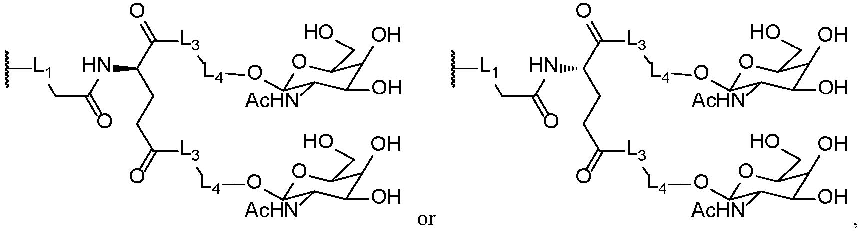 preferably 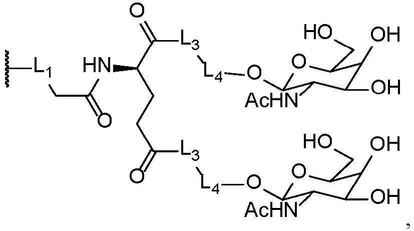 wherein L₁ is -CH₂-;
Q₂ is 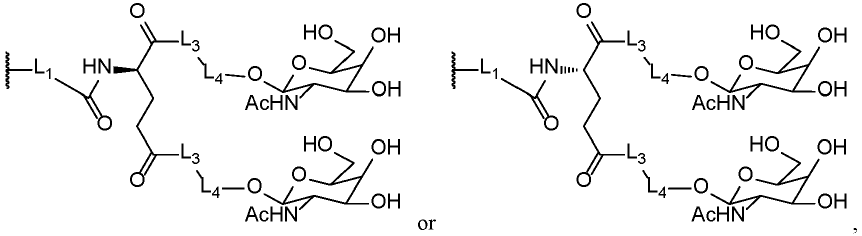 preferably 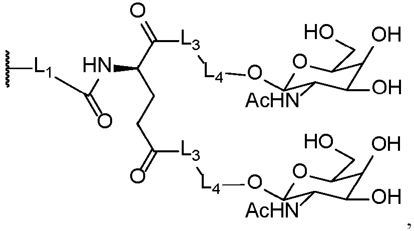 wherein L₁ is bond;
L₃ is -NHCH₂CH₂-;
L₄ is -(OCH₂CH₂)_{c}-;
c is 1, 2, or 3;
L is -NHC(O)-;
R' is H; and
n is 6, 7, 8, 9, or 10, preferably 7, 8, or 9, more preferably 8.

18. The ligand according to claim 1, wherein,
Q is independently H, 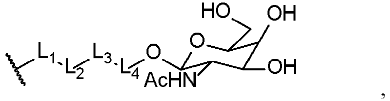 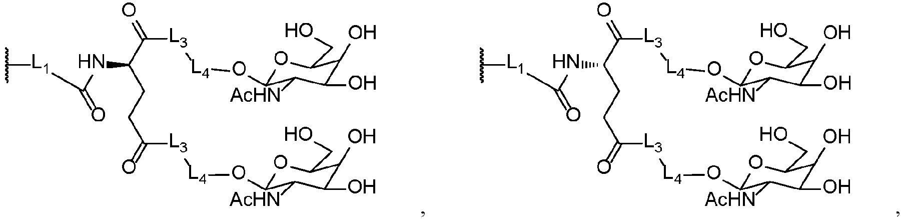 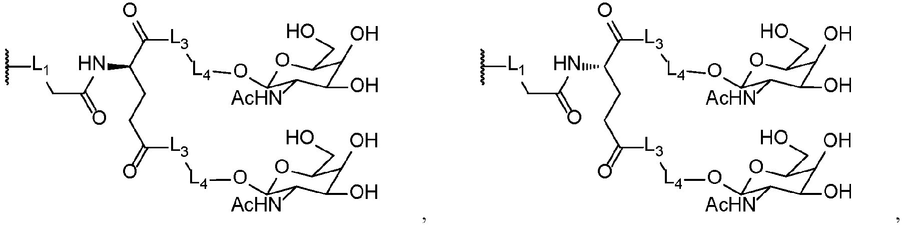 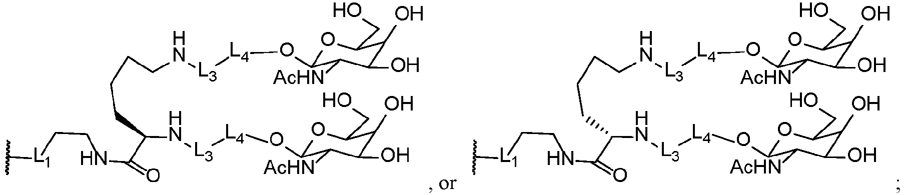
wherein L₁ is bond, -CH₂-, -CH₂CH₂-, -C(O)-, -CH₂O-, -CH₂O-CH₂CH₂O-, or -NHC(O)-(CH₂NHC(O))ₐ-;
L₂ is bond or -CH₂CH₂C(O)-;
L₃ is bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, -(OCH₂CH₂CH₂CH₂CH₂)_{c}-, or - NHC(O)-(CH₂)_{d}-;
wherein a is 0, 1, 2, or 3;
b is 1, 2, 3, 4, or 5;
c is 1, 2, 3, 4, or 5;
d is 1, 2, 3, 4, 5, 6, 7, or 8;
L is bond, -CH₂O-, or -NHC(O)-;
L' is -O(CH₂CH₂O)ₑ-;
wherein the e is 1, 2, 3, 4, or 5;
T is bond, -CH₂-, -C(O)-, -M-, -CH₂-M-, or -C(O)-M-;
wherein the M is 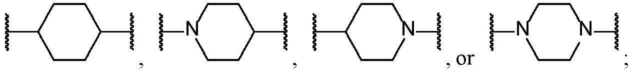
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

19. The ligand according to claim 1, wherein the conjugated group is selected from the following:
| Numb er | Structure |
|---|---|
| GL1 | 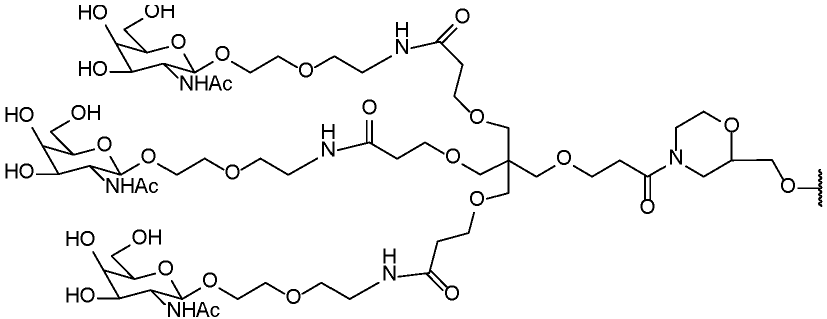 |
| GL2 | 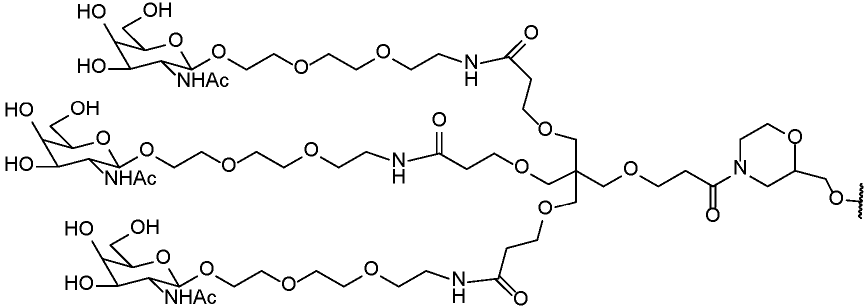 |
| GL3 | 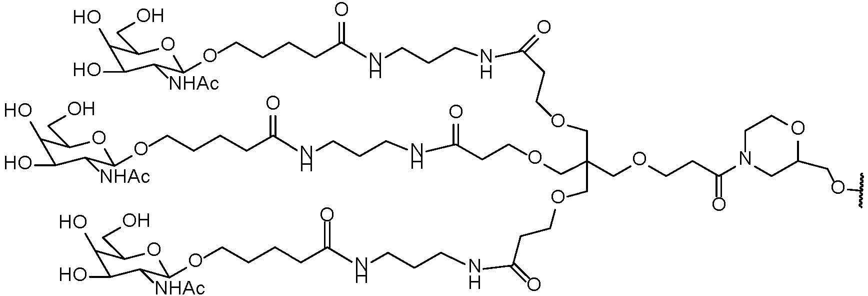 |
| GL4 | 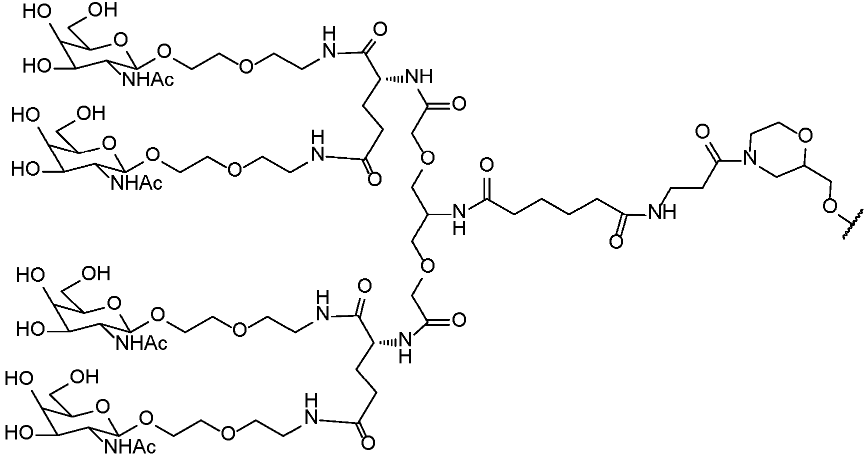 |
| GL5 | 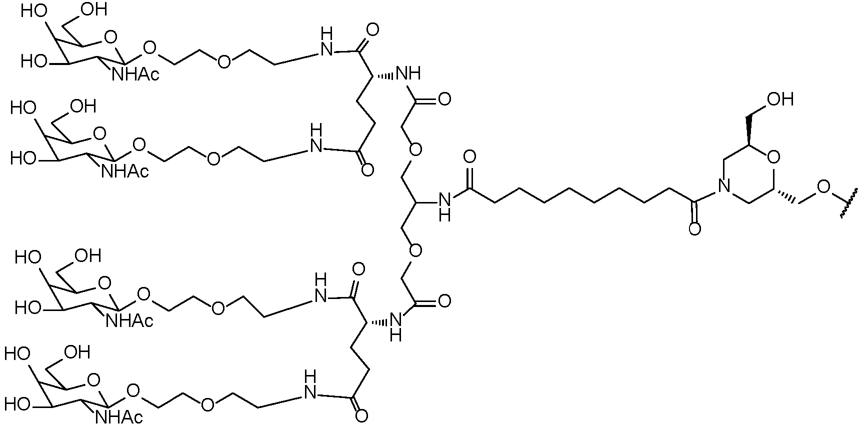 |
| GL6 | 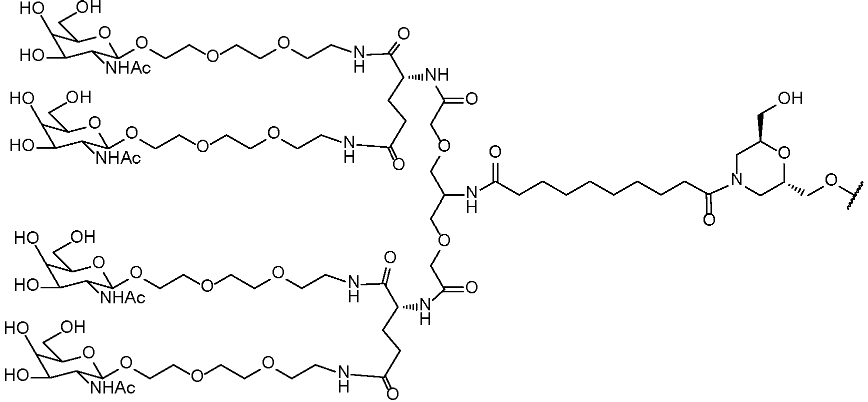 |
| GL7 | 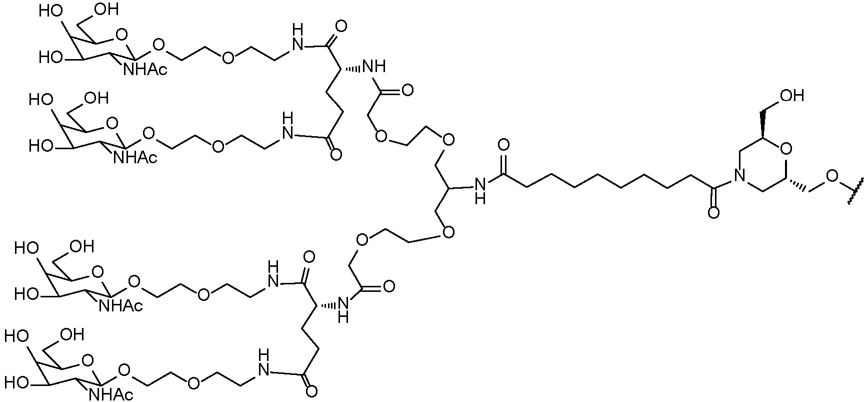 |
| GL8 | 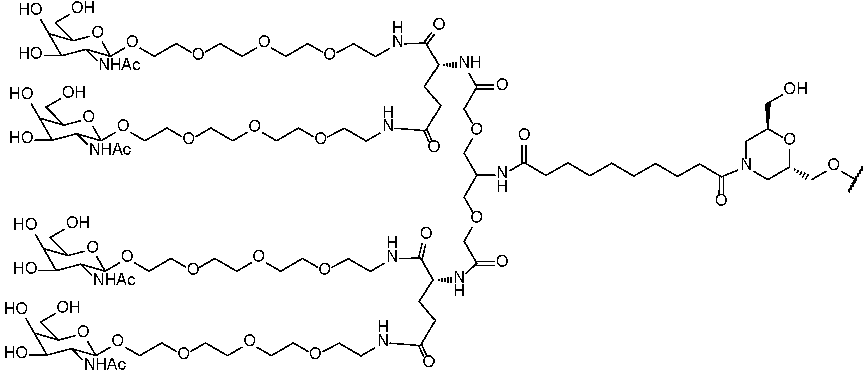 |
| GL9 | 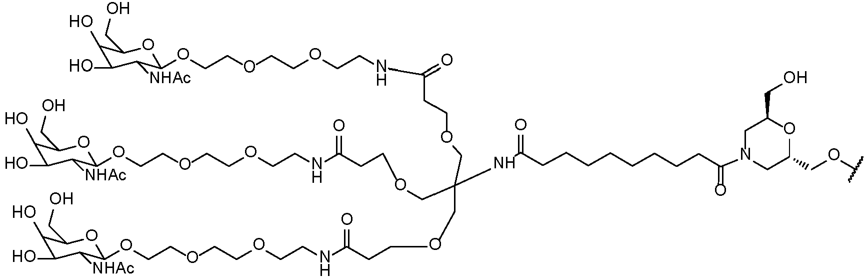 |
| GL10 | 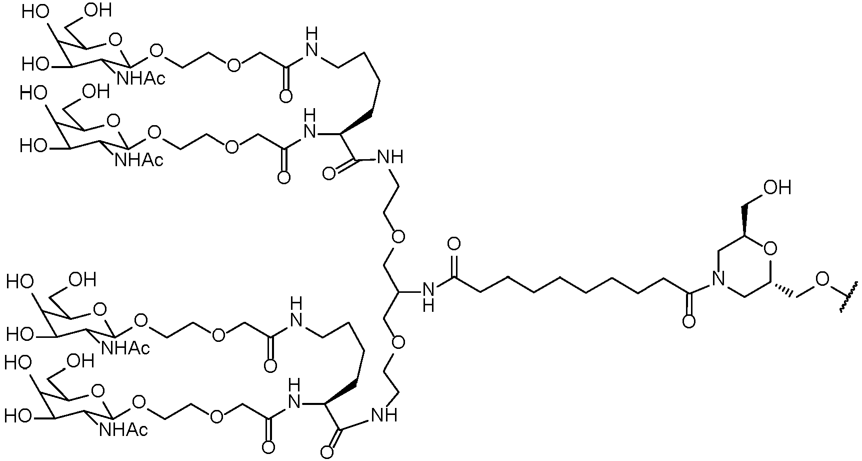 |
| GL11 | 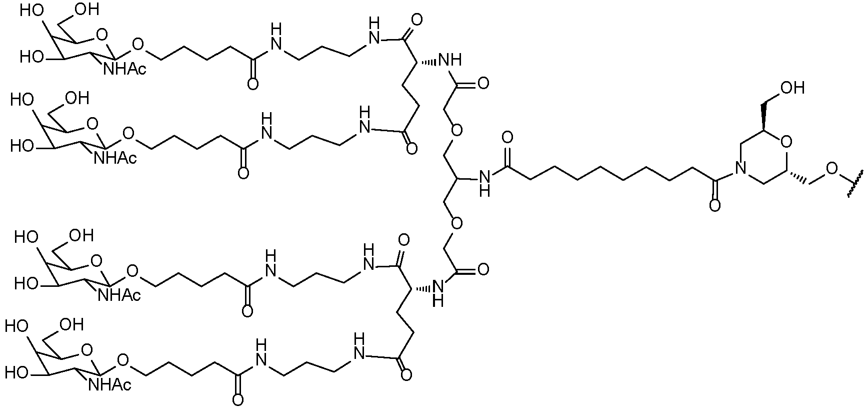 |
| GL12 | 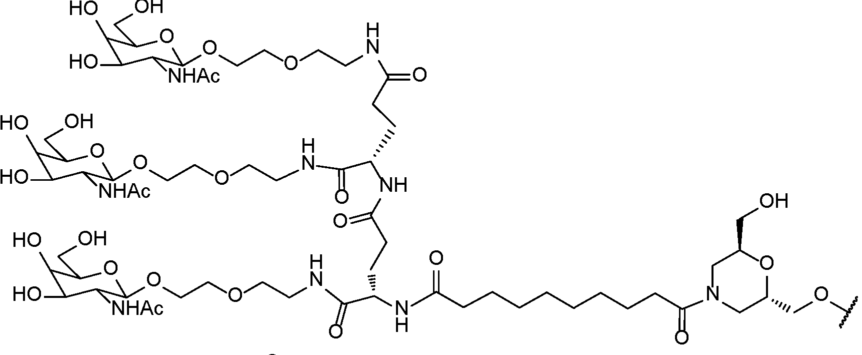 |
| GL13 | 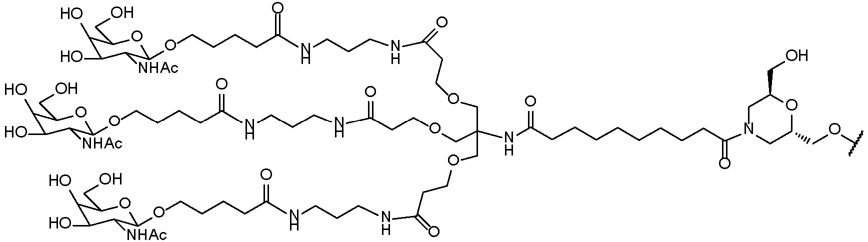 |
| GL14 | 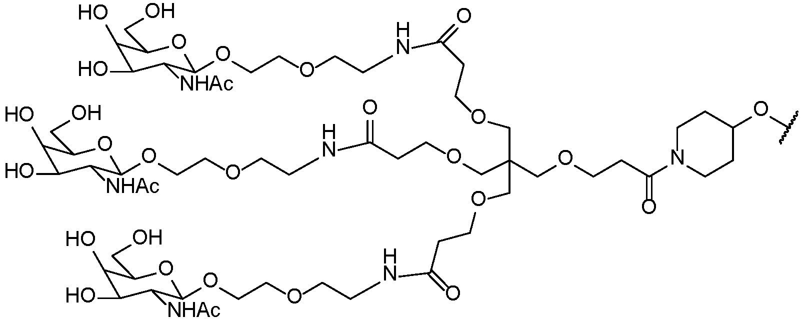 |
| GL15 | 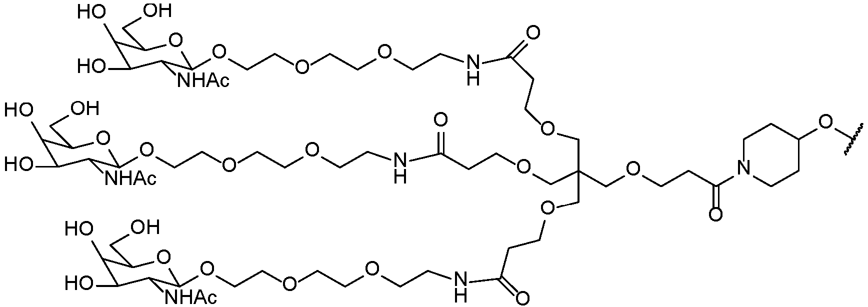 |
| GL16 | 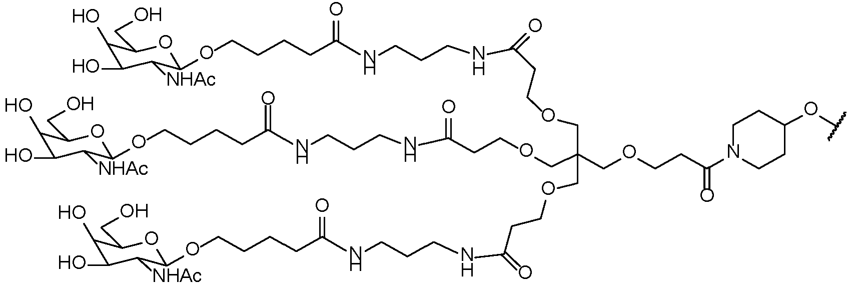 |

20. The ligand according to claim 1, wherein the conjugated group is selected from the following:
| Numb er | Structure |
|---|---|
| GL17 | 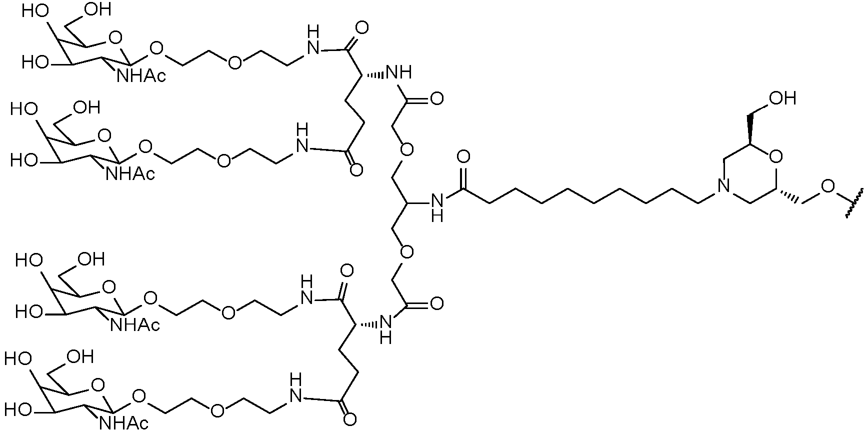 |
| GL18 | 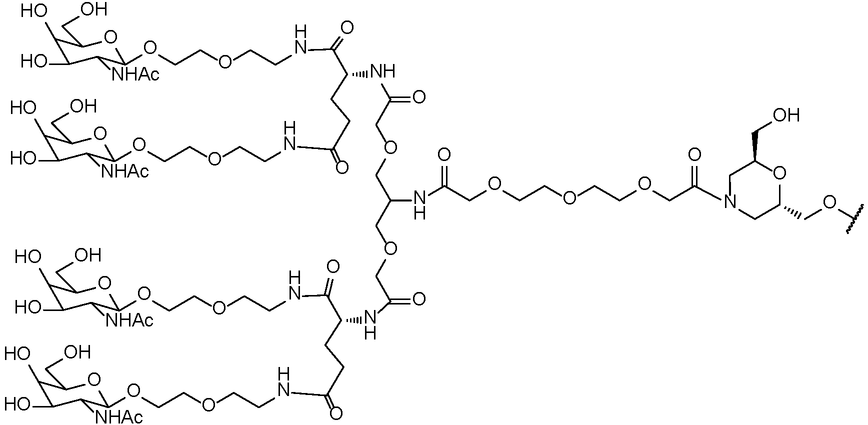 |
| GL19 | 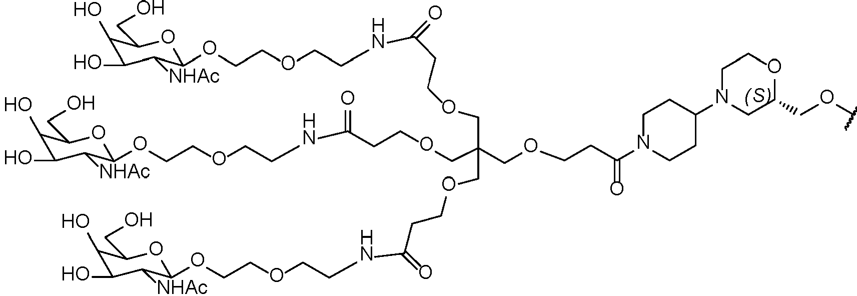 |
| GL20 | 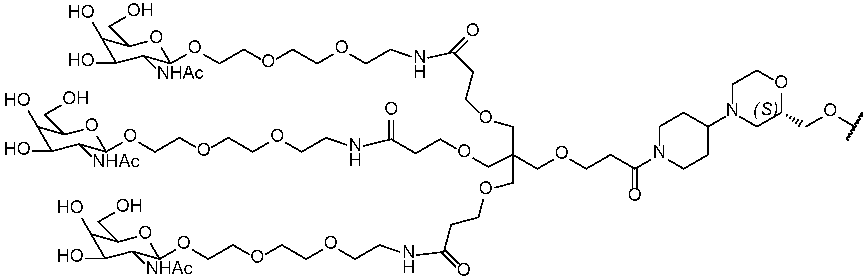 |
| GL21 | 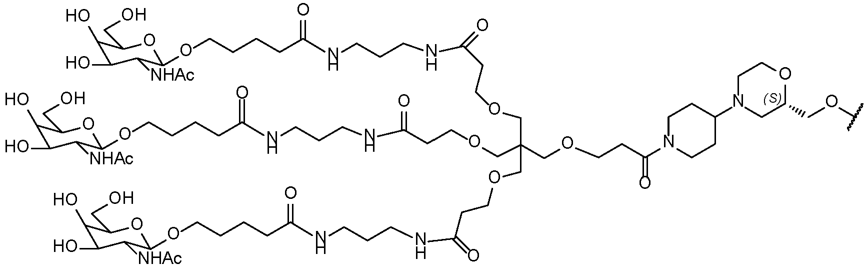 |
| GL22 | 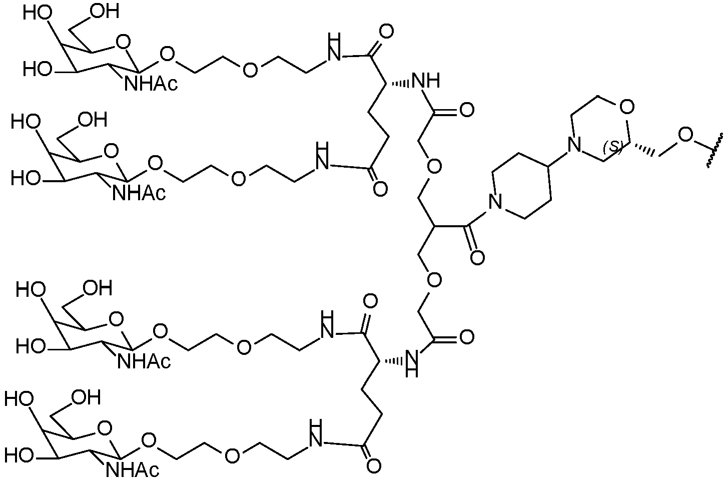 |
| GL23-1 | 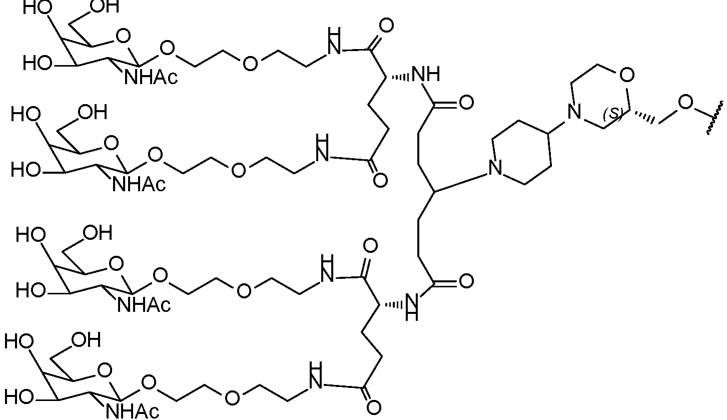 |
| GL23-2 | 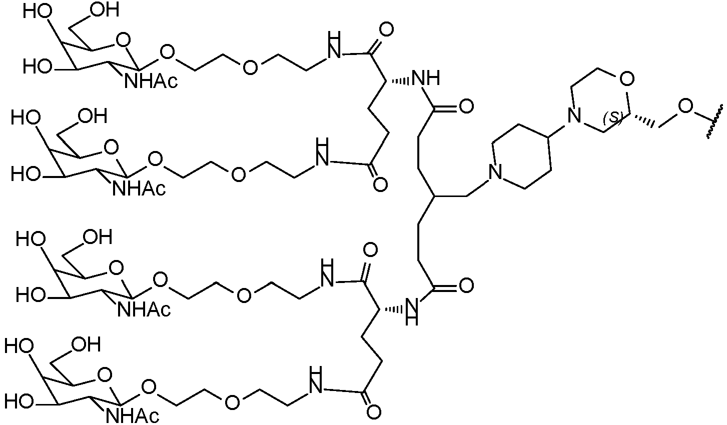 |
| GL24 | 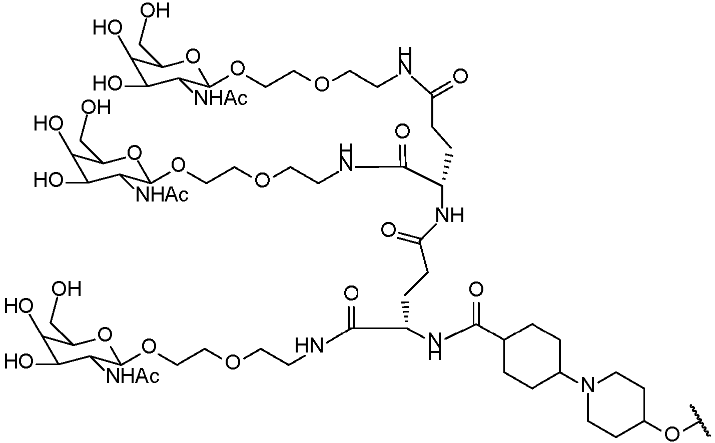 |
| GL25 | 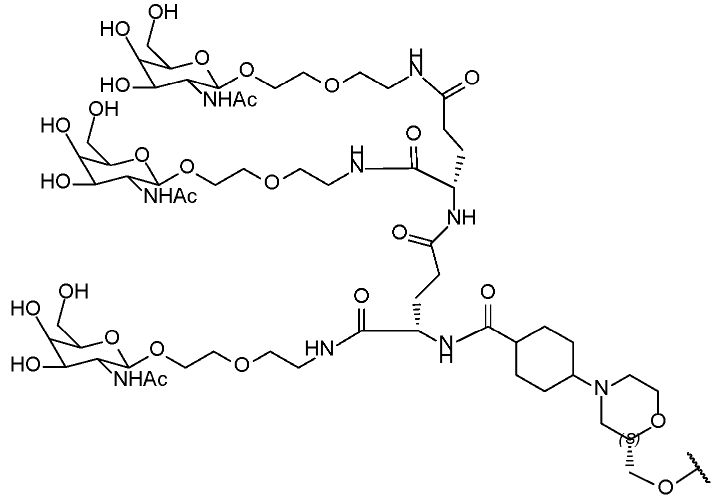 |
| GL26 | 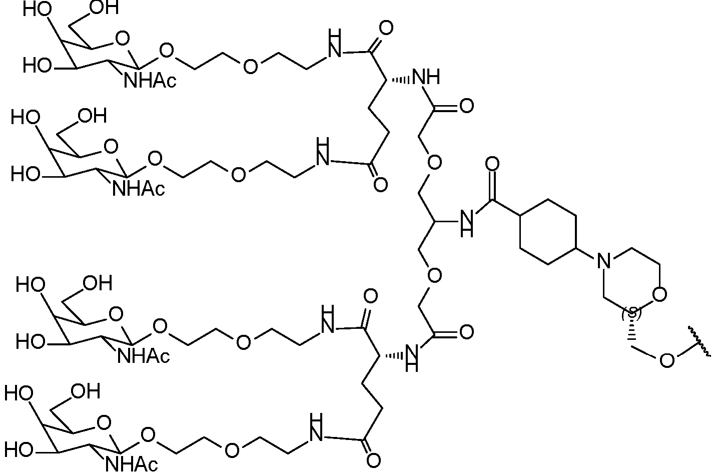 |
| GL27 | 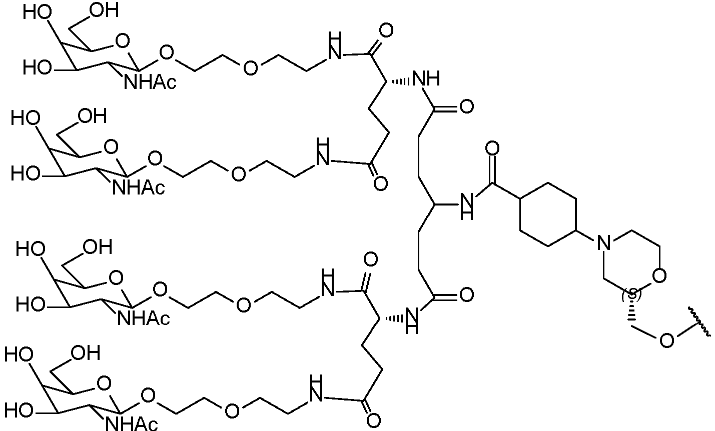 |
| GL28 | 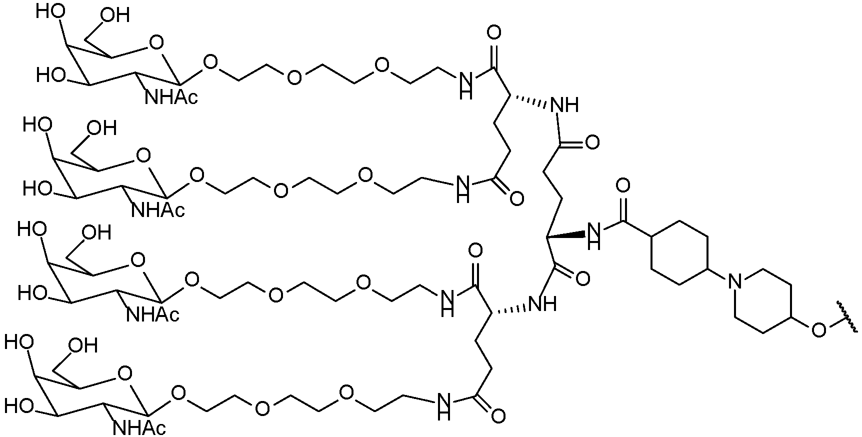 |
| GL29 | 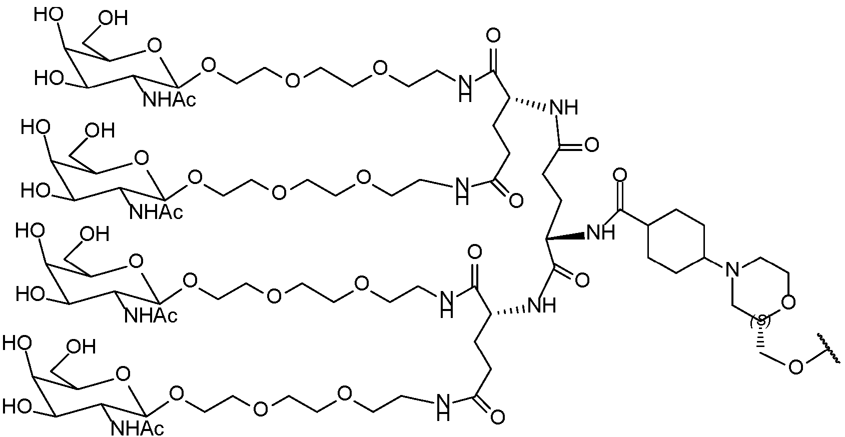 |
| GL30 | 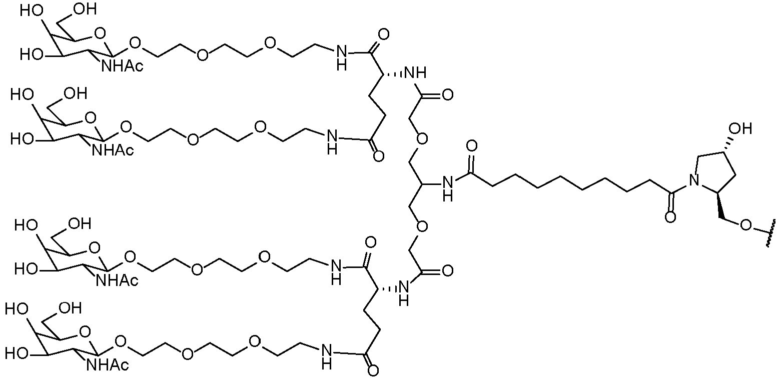 |
| GL31 | 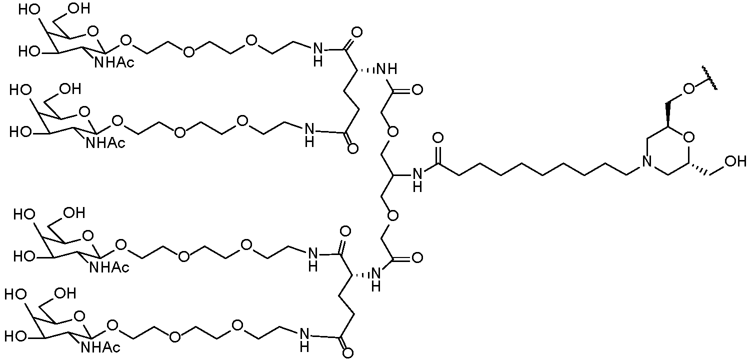 |
| GL32 | 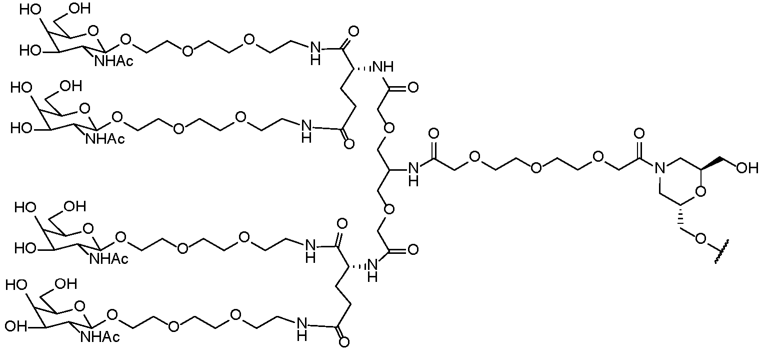 |
| GL33 | 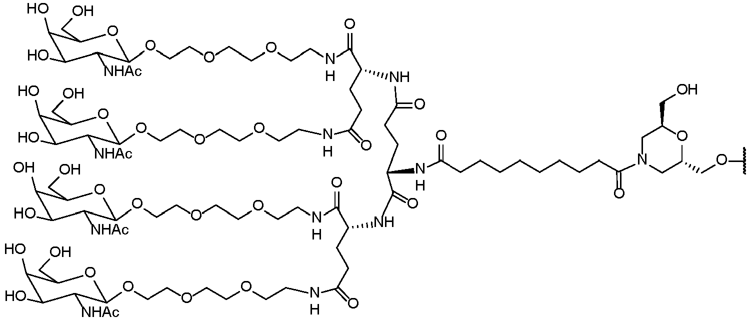 |

21. The ligand according to any one of claims 1-20, targeting ASGPR.

22. A nucleic acid molecule, comprising a nucleic acid and the ligand connected thereto according to any one of claims 1-21.

23. The nucleic acid molecule according to claim 22, wherein the nucleic acid is selected from a DNA, an RNA, and a DNA/RNA hybrid.

24. The nucleic acid molecule according to claim 23, wherein the nucleic acid molecule is single-stranded or double-stranded.

25. The nucleic acid molecule according to claim 23, wherein the nucleic acid molecule is selected from a small interfering RNA (siRNA) and a short hairpin RNA (shRNA).

26. The nucleic acid molecule according to claim 23, wherein the nucleic acid molecule is a double-stranded RNA molecule comprising a sense strand and an antisense strand, and a conjugated group is connected to a 3' end or 5' end of the sense strand of the double-stranded RNA molecule, preferably the 3' end.

27. The nucleic acid molecule according to claim 26, wherein the sense strand and the antisense strand of the double-stranded RNA molecule each has 10 to 30 nucleotides, preferably 15 to 25 nucleotides, more preferably 20 to 25 nucleotides.

28. The nucleic acid molecule according to claim 26 or 27, wherein one or more nucleotides in the sense strand and/or the antisense strand of the double-stranded RNA molecule comprise chemical modification.

29. The nucleic acid molecule according to claim 26 or 27, wherein one or more nucleotides in the sense strand and/or the antisense strand of the double-stranded RNA molecule are substituted with nucleotide analogs.

30. The nucleic acid molecule according to any one of claims 22-29, specifically binding to an asialoglycoprotein receptor (ASGPR) on surfaces of hepatocytes.

31. A pharmaceutical composition, comprising the nucleic acid molecule according to any one of claims 22-30 and a pharmaceutically acceptable carrier or excipient.

32. A kit, comprising the nucleic acid molecule according to any one of claims 22-30.

33. An intermediate compound, being a compound of Formula (V): 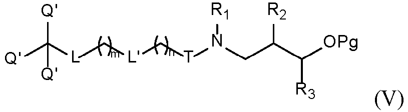 wherein,
Pg is a hydroxyl protective group, for example, selected from the following: 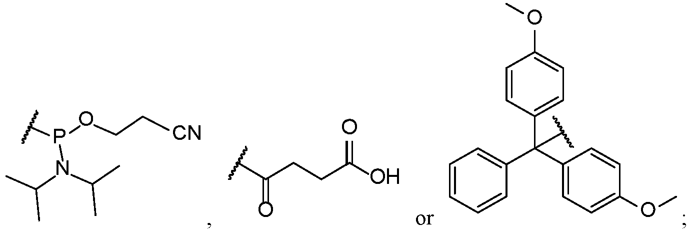
Q' is independently H, 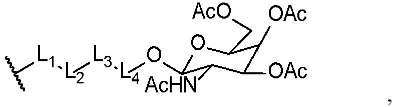 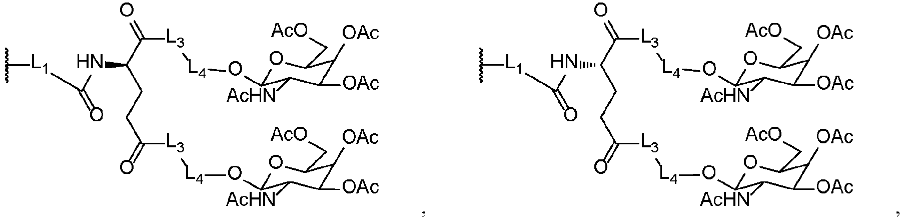 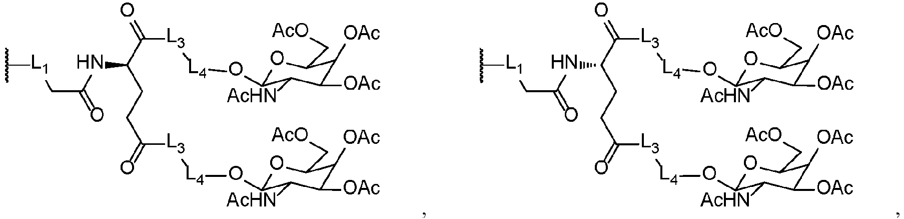 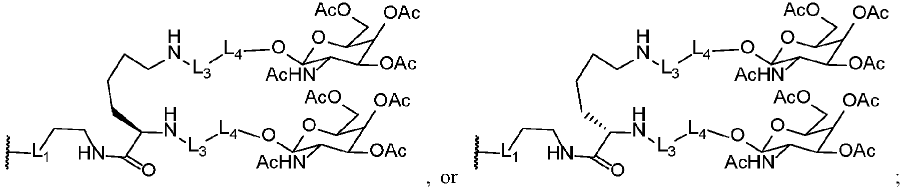 and
other groups are as defined according to any one of claims 1-18.

34. An intermediate compound, selected from:
| Num ber | Structure |
|---|---|
| 1a | 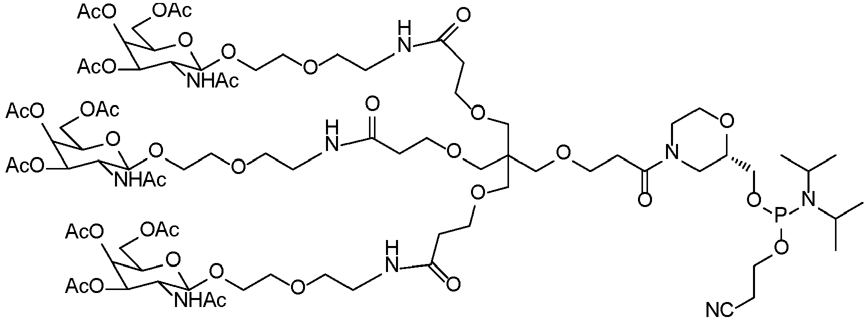 |
| 2a | 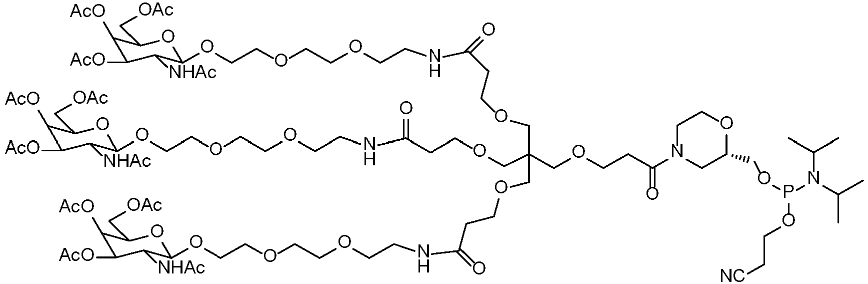 |
| 3a | 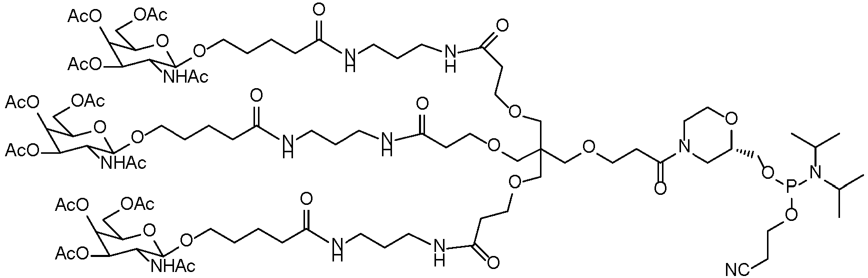 |
| 4a | 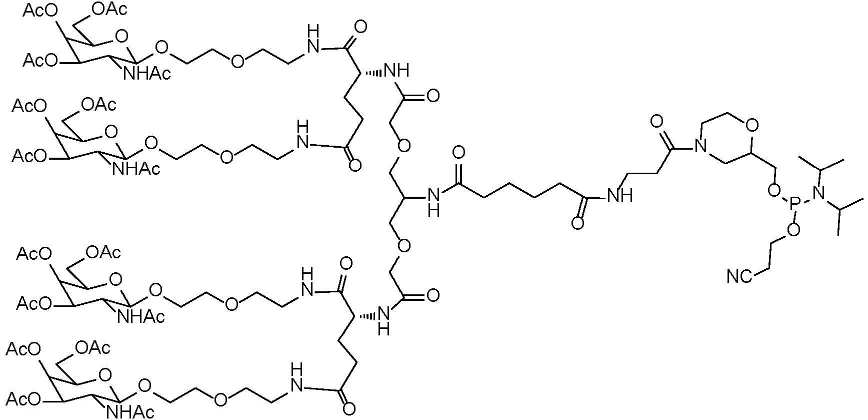 |
| 5a | 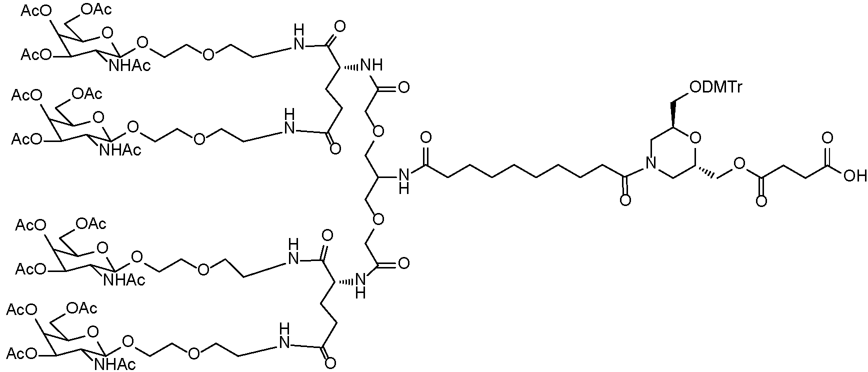 |
| 6a | 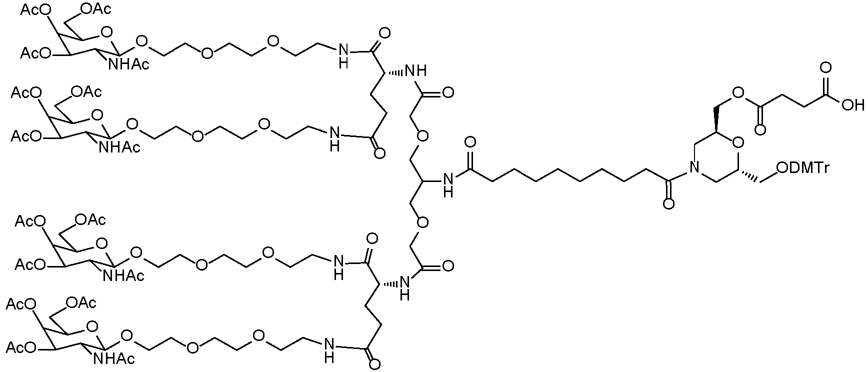 |
| 7a | 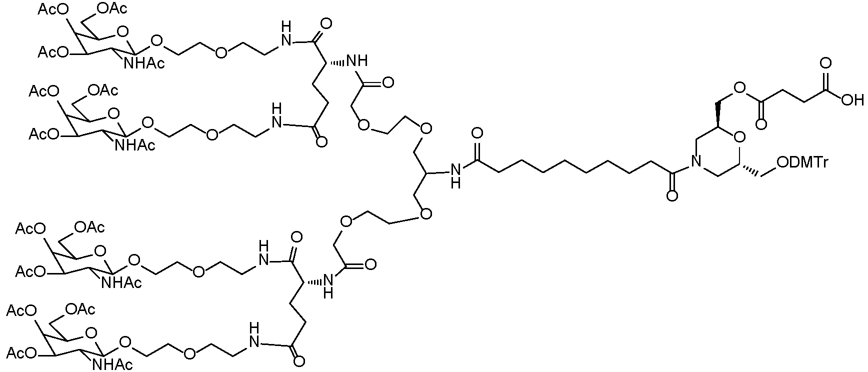 |
| 8a | 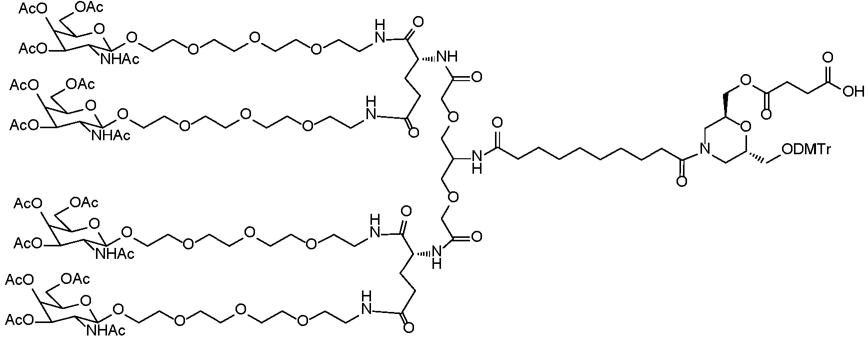 |
| 9a | 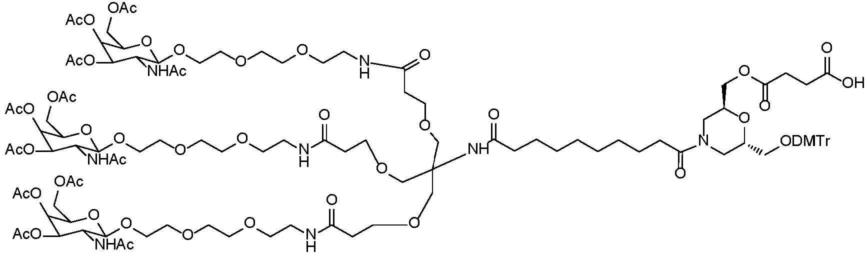 |
| 10a | 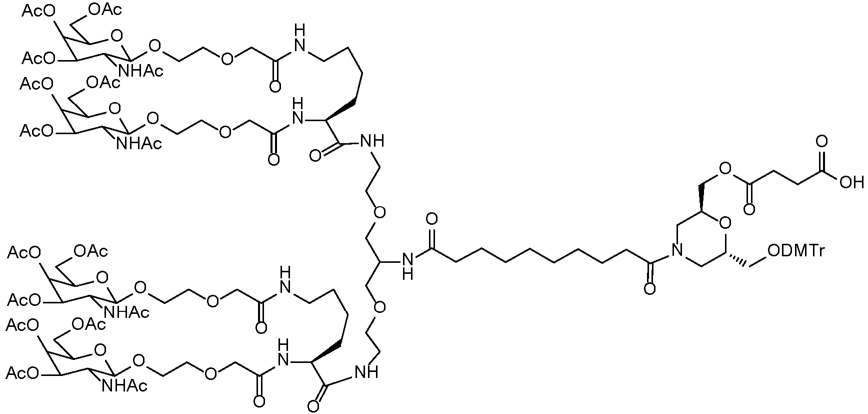 |
| 11a | 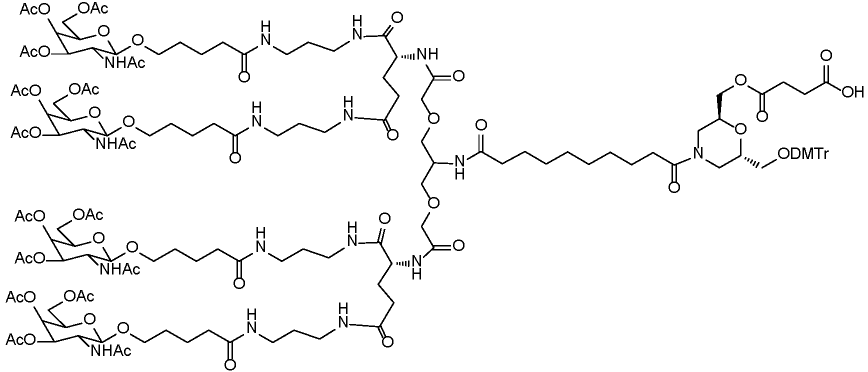 |
| 12a | 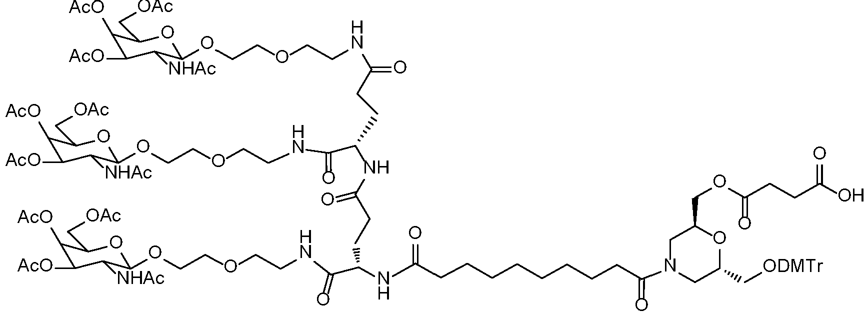 |
| 13a | 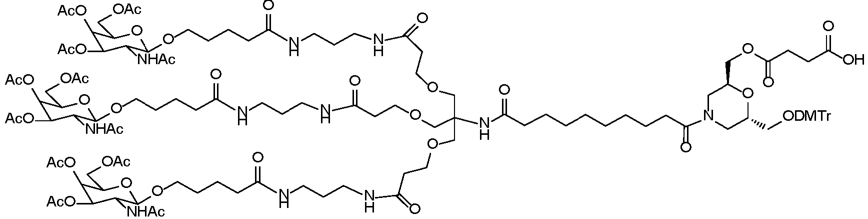 |
| 14a | 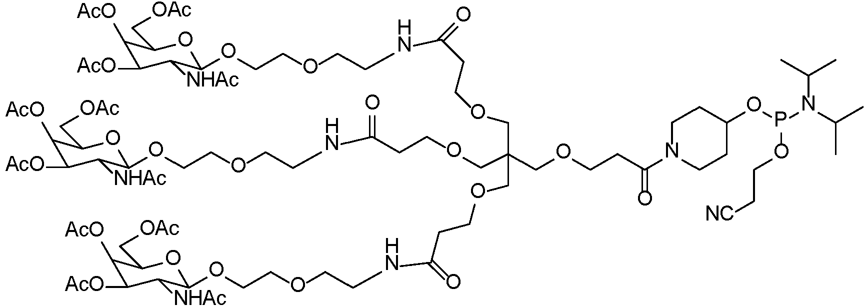 |
| 15a | 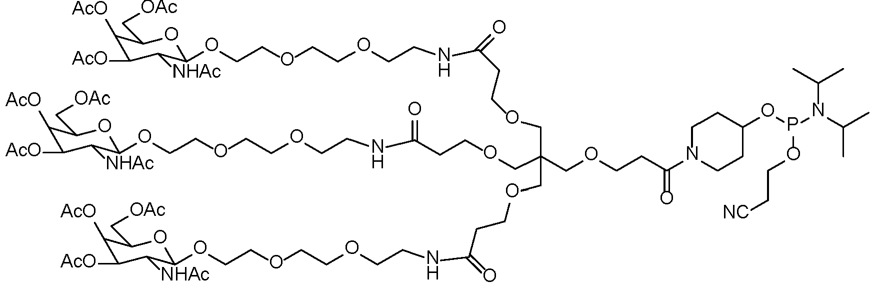 |
| 16a | 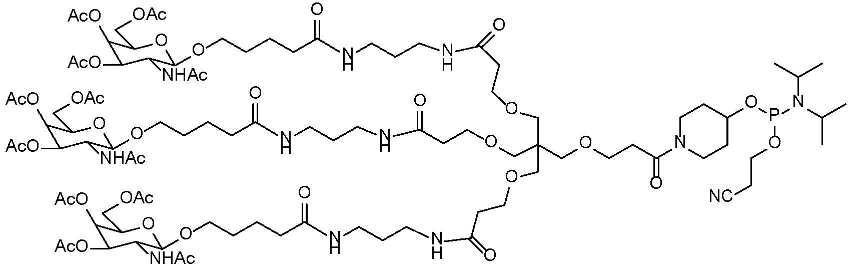 |

35. An intermediate compound, selected from:
| Num ber | Structure |
|---|---|
| 17a | 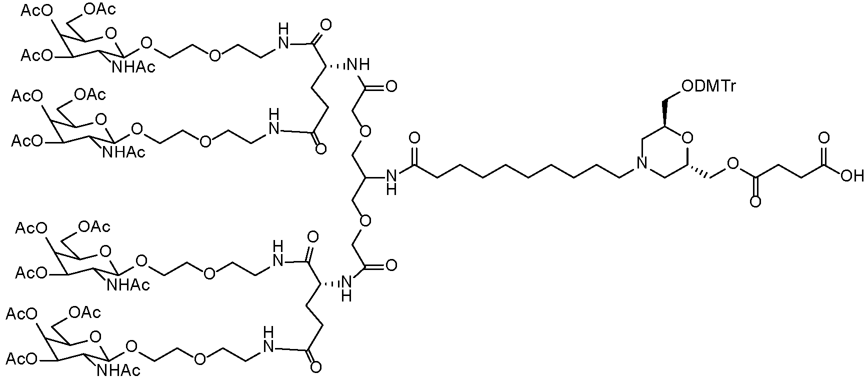 |
| 18a | 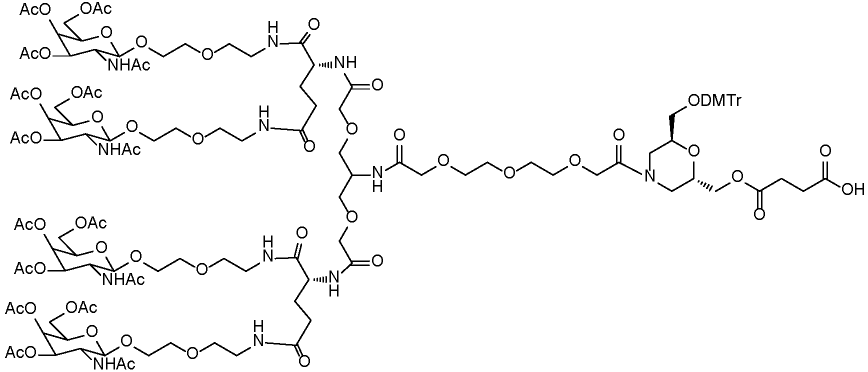 |
| 19a | 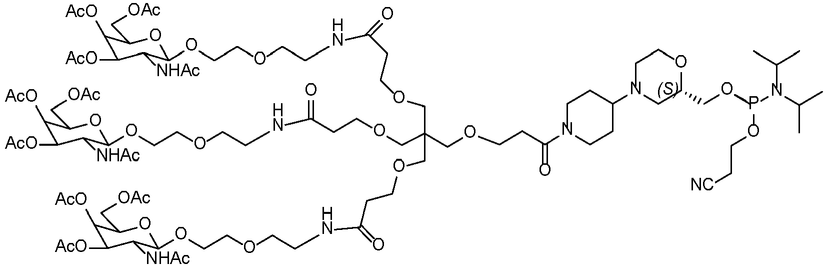 |
| 20a | 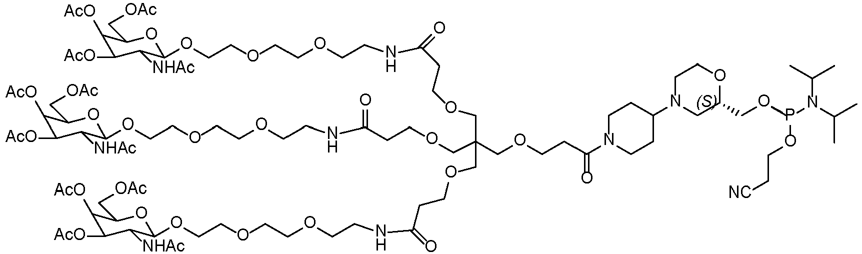 |
| 21a | 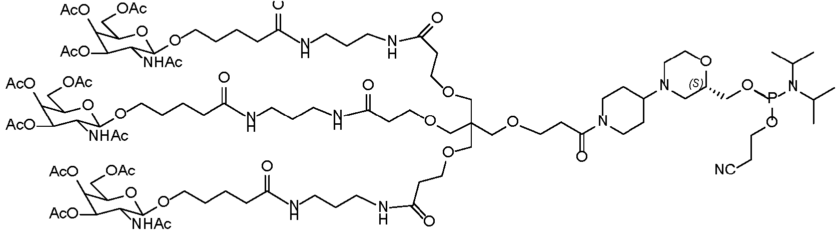 |
| 22a | 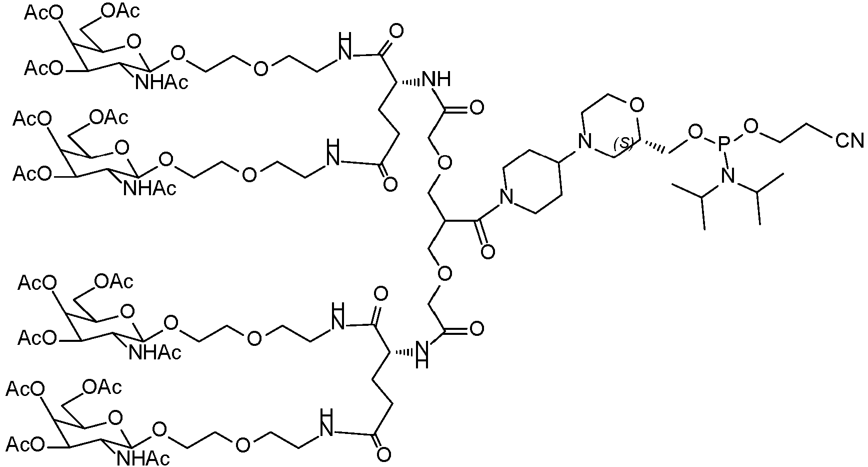 |
| 23 a-1 | 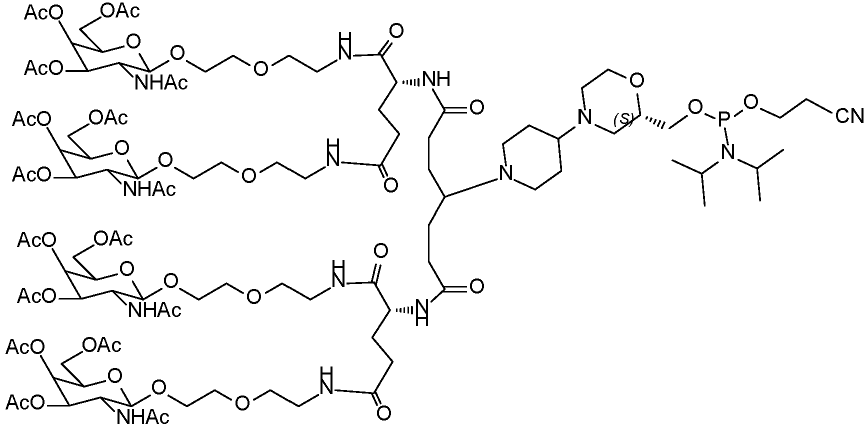 |
| 23 a-2 | 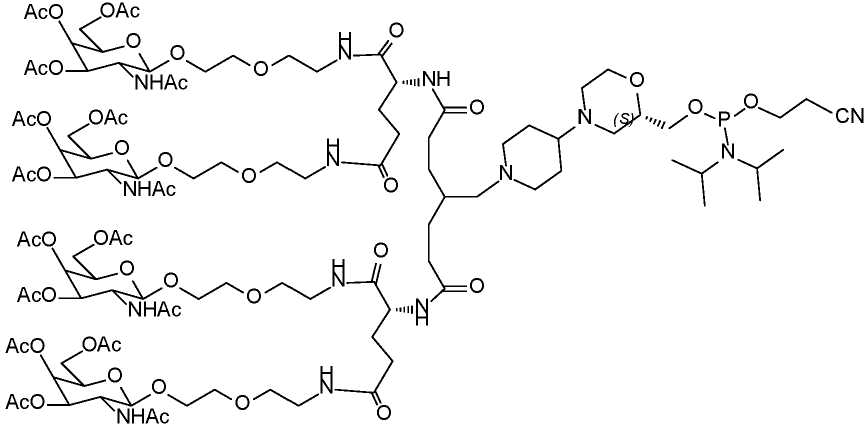 |
| 24a | 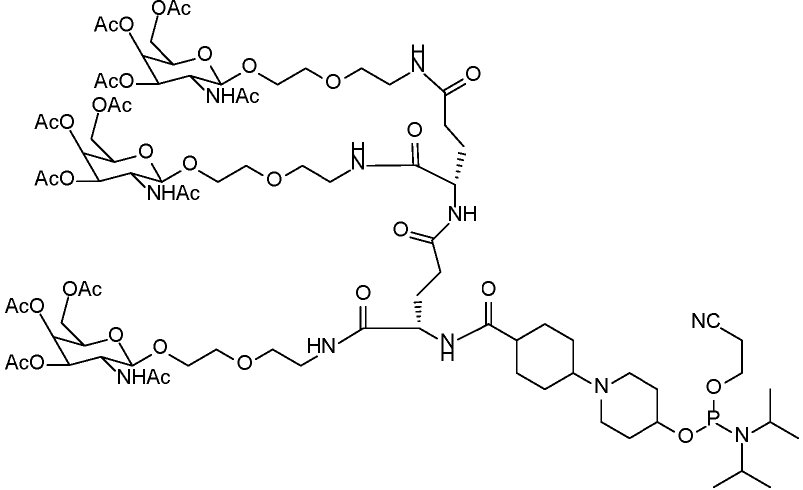 |
| 25a | 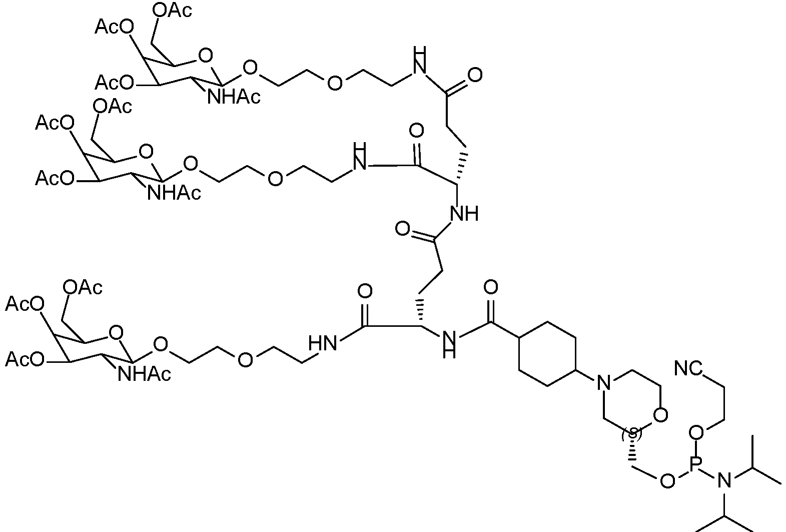 |
| 26a | 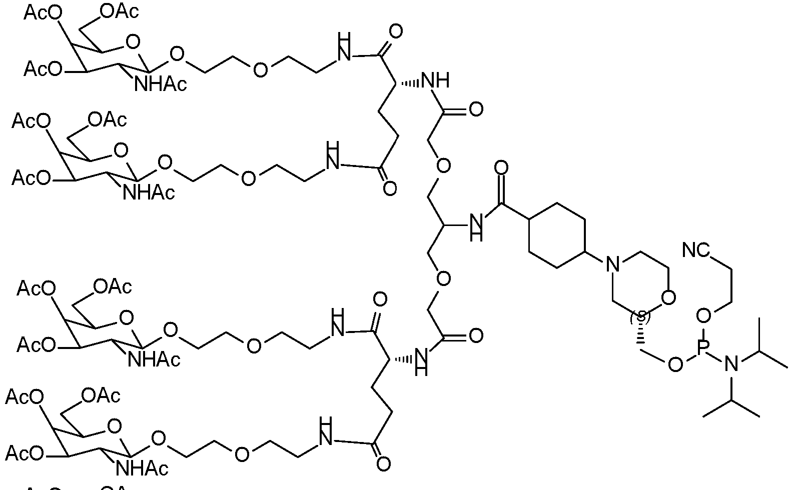 |
| 27a | 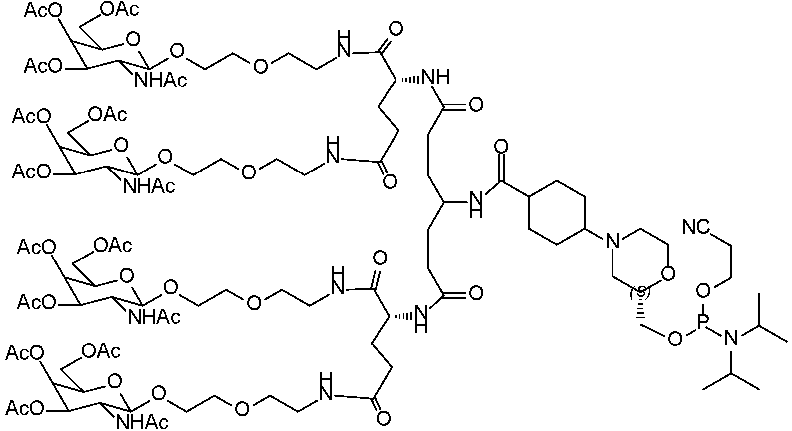 |
| 28a | 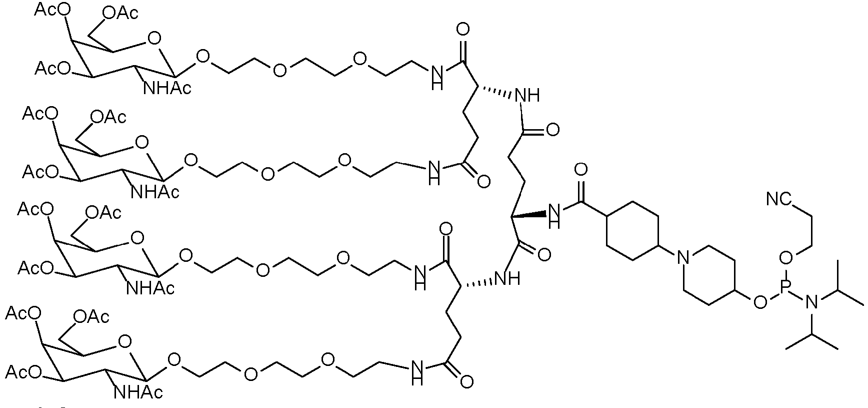 |
| 29a | 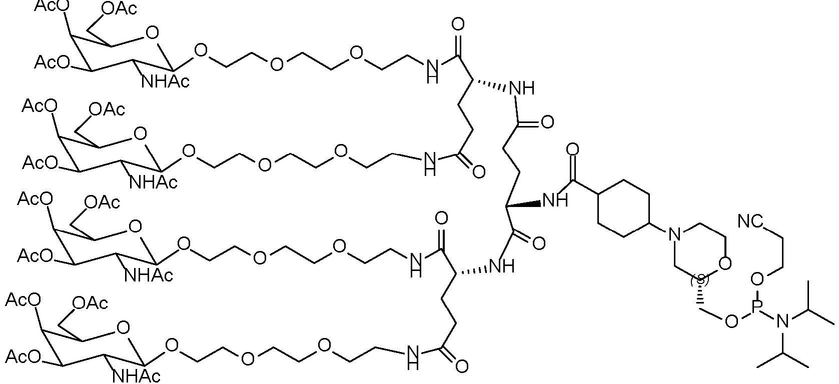 |
| 30a | 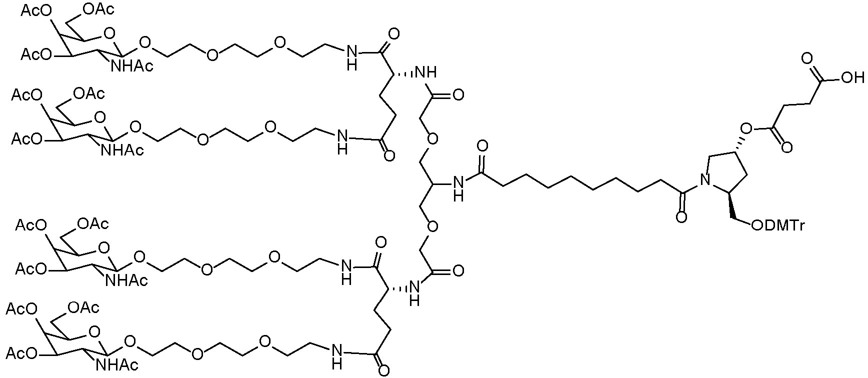 |
| 31a | 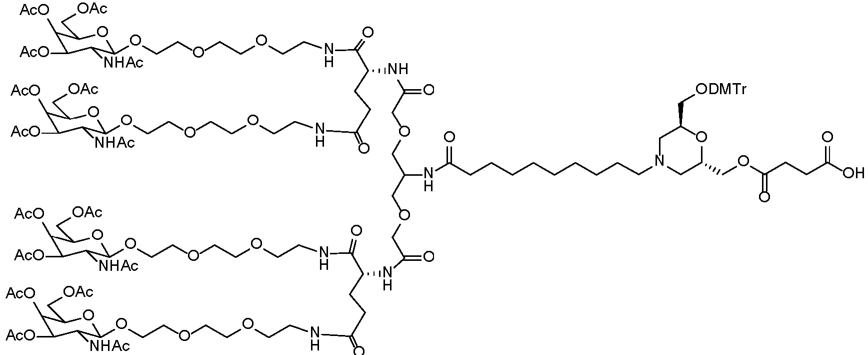 |
| 32a | 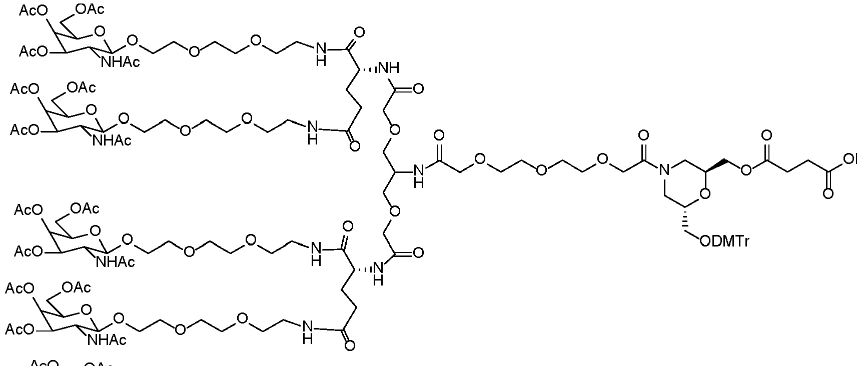 |
| 33a | 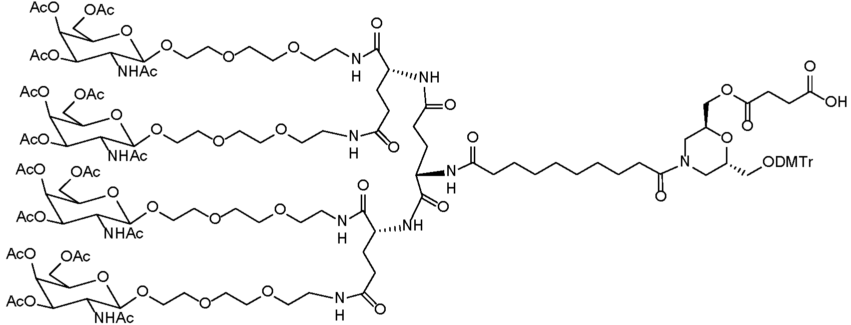 |

36. A method for preparing a compound of Formula (I), comprising treating a compound of Formula (V) under the condition of removing a protective hydroxyl group: 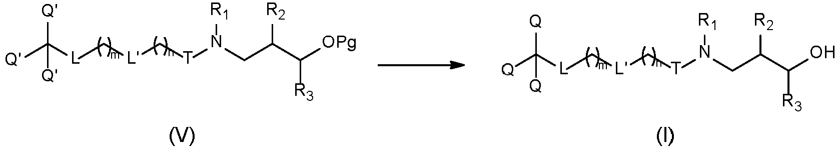 wherein various groups are as defined according to any one of claims 1-18.
